(19) Europäisches Patentamt
European Patent Office
Office européen des brevets

(11) EP 1 141 293 B1

(12) EUROPEAN PATENT SPECIFICATION

(45) Date of publication and mention of the grant of the patent:
**01.08.2007 Bulletin 2007/31**

(21) Application number: **99968929.2**

(22) Date of filing: **21.12.1999**

(51) Int Cl.:
*C12N 15/12* (2006.01) *C12N 5/10* (2006.01)
*C12N 1/21* (2006.01) *C07K 14/52* (2006.01)
*C07K 16/22* (2006.01) *G01N 33/50* (2006.01)
*G01N 33/566* (2006.01) *A01K 67/027* (2006.01)
*A61K 31/70* (2006.01) *A61K 38/18* (2006.01)
*A61K 39/395* (2006.01) *A61P 9/00* (2006.01)

(86) International application number:
**PCT/US1999/030503**

(87) International publication number:
**WO 2000/037641 (29.06.2000 Gazette 2000/26)**

(54) **VASCULAR ENDOTHELIAL GROWTH FACTOR-X**

VASKULÄRER ENDOTHELIALER WACHSTUMSFAKTOR X

FACTEUR DE CROISSANCE ENDOTHELIALE VASCULAIRE- X

(84) Designated Contracting States:
**AT BE CH CY DE DK ES FI FR GB GR IE IT LI LU MC NL PT SE**
Designated Extension States:
**AL LT LV MK RO SI**

(30) Priority: **22.12.1998 GB 9828377**
**18.03.1999 US 124967 P**
**08.11.1999 US 164131 P**

(43) Date of publication of application:
**10.10.2001 Bulletin 2001/41**

(73) Proprietor: **JANSSEN PHARMACEUTICA N.V.**
**2340 Beerse (BE)**

(72) Inventors:
- **GORDON, Robert Douglas,**
**Janssen Pharmaceutica N.V.**
**2340 Beerse (BE)**
- **SPRENGEL, Jorg Jurgen,**
**Janssen Pharmaceutica N.V.**
**2340 Beerse (BE)**
- **YON, Jeffrey Roland,**
**Janssen Pharmaceutica N.V.**
**2340 Beerse (BE)**
- **DIJKMANS, Josiena Johanna Huberdina**
**2340 Beerse (BE)**
- **GOSIEWSKA, Anna,**
**Johnson & Johnson Cons. Products**
**199 Grandview Road,**
**Skillman, NJ 08588 (US)**
- **DHANARAJ, Sridevi N.,**
**Johnson & Johnson Con. Prod.**
**199 Grandview Road,**
**Skillman, NJ08588 (US)**
- **XU, Jean,**
**Johnson & Johnson Consumer Products**
**199 Grandview Road,**
**Skillman, NJ 08588 (US)**

(74) Representative: **Daelemans, Frank F.R. et al**
**Janssen Pharmaceutica N.V.,**
**Patent Department,**
**Turnhoutseweg 30**
**2340 Beerse (BE)**

(56) References cited:
**EP-A- 0 984 063** **WO-A-98/07832**
**WO-A-98/24811** **WO-A-99/37671**
**WO-A-99/47677**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 141 293 B1

## Description

The present invention is concerned with a novel vascular endothelial growth factor (VEGF) herein designated "VEGF-X", and characterisation of the nucleic acid and amino acid sequences of VEGF-X.

### Introduction

Angiogenesis involves formation and proliferation of new blood vessels, and is an essential physiological process for normal growth and development of tissues in, for example, embryonic development, tissue regeneration and organ and tissue repair.
Angiogenesis also features in the growth of human cancers which require continuous stimulation of blood vessel growth. Abnormal angiogenesis is associated with other diseases such as rheumatoid arthritis psoriasis and diabetic retinopathy.
Capillary vessels consist of endothelial cells which carry the genetic information necessary to proliferate to form capillary networks. Angiogenic molecules which can initiate this process have previously been characterised. A highly selective mitogen for vascular enothelial cells is vascular endothelial growth factor (VEGF) (Ferrara et al., "Vascular Endothelial Growth Factor: Basic Biology and Clinical Implications". Regulation of angiogenesis, by I.D. Goldberg and E.M. Rosen 1997 Birkhauser Verlag Basle/Switzerland). VEGF is a potent vasoactive protein which is comprised of a glycosylated cationic 46-49 kd dimer having two 24 kd subunits. It is inactivated by sulfhydryl reducing agents and is resistant to acidic pH and to heating and binds to immobilised heparin.
VEGF-A has four different forms of 121, .165, 189 and 206 amino acids respectively due to alternative splicing. VEGF121 and VEGF165 are soluble and are capable of promoting angiogenesis, whereas VEGF189 and VEGF206 are bound to heparin containing proteoglycans in the cell surface. The temporal and spatial expression of VEGF has been correlated with physiological proliferation of the blood vessels (Gajdusek, C.M., and Carbon, S.J., Cell Physiol., 139:570-579. (1989)); McNeil, P.L., Muthukrishnan, L., Warder, E., D'Amore, P.A., J. Cell. Biol., 109:811-822, (1989)). Its high affinity binding sites are localized only on endothelial cells in tissue sections (Jakeman, L.B., et al., Clin. Invest. 89:244-253 (1989)). The growth factor can be isolated from pituitary cells and several tumor cell lines, and has been implicated in some human gliomas (Plate, K.H. Nature 359:845-348, (1992))- The inhibition of VEGF function by anti-VEGF monoclonal antibodies was shown to inhibit tumor growth in immune-deficient mice (Kim, K.J., Nature 362:841-844, (1993)).
VEGF proteins have been described in the following patents and applications all of which are hereby incorporated by reference EF-0,506,477, WO-95/24473, WO-98/28621, WO-90/13649, EP-0,476,983, EP-0,550,296, WO-90/13649, WO-96/26736, WO-96/27007, WO-98/49300, WO-98/36075, WO-98/840124, WO-90/11084, WO-98/24811, WO-98/10071, WO-98/07832, WO-98/02543, WO-97/05250, WO-91/02058, WO-96/39421, WO-96/39515, WO-98/16551.
A news member of the VEGF family, annotated as as VEGF-R in EP-0,984, 063 as VEGF-D in WO- 98/07832, and VEGF-X in the present application, was found to have potentially significant benefits for the treatment of tumours and other conditions mediated by inappropriate angiogenic activity.

### Summary of the Invention

In the present application, there is provided the sequence of a CUB domain present in the sequence of VEGF-X which domain itself prevents angiogenesis and which is used to treat diseases associated with inappropriate vascularisation or angiogenesis.

### Detailed Description of the Invention

The present inventors have identified in the sequence encoding the VEGF-X protein a CUB domain, which has heretofore not previously been identified in VEGF-type growth factors. The VEGF-X protein may therefore exert dual regulatory effects via interaction with the VEGF tyrosine kinase receptors or with neuropilin receptors mediated by the CUB domain. Thus, the sequence encoding said CUB domain may be included in an expression vector for subsequent transformation of a host cell, tissue or organism.
VEGF-X or fragments thereof may be able to modulate the effects of pro-angiogenic growth factors such as VEGF as indicated in the findings presented in the examples below that the N-terminal part of the VEGF-X protein, a CUB-like domain, is able to inhibit VEGF-stimulated proliferation of HUVECs. VEGF-X or fragments thereof may therefore be useful in therapy of conditions involving inappropriate angiogenesis. Inhibition of the angiogenic activity of VEGF has been linked with inhibition of tumour growth in several models eg Kim K. J. et al, Nature 362:841-844, (1993). Additionally, agents able to inhibit angiogenesis would be expected to be useful in treating other angiogenesis-dependent diseases such a retinopathy, osteoarthritis and psoriasis (Folkman, J., Nature Medicine 1:27-31, (1995).
As identified in more detail in the Examples described herein the present inventors have surprisingly identified that the CUB domain of VEGF-X is able to inhibit stimulation of proliferation of HUVECs induced by either VEGF or bFGF. The CUB domain may, therefore, be utilised as a therapuetic agent for inhibition of angiogenesis and for treatment of condition associated with inappropriate vascularisation or angiogenesis.

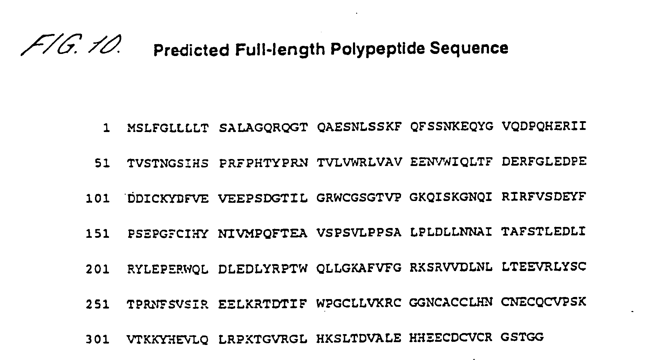

Therefore according to a further aspect of the invention there is provided a method of inhibiting angiogenic activity and inappropriate vascularisation including formation and proliferation of new blood vessels, growth and development of tissues, tissue regeneration and organ and tissue repair in a subject said method comprising administering to said subject an amount of a polypeptide having an amino acid sequence from position 40 to 150 of the sequence illustrated in Figure 10 or a nucleic acid molecule encoding the CUB domain according to the invention in sufficient concentration to reduce or prevent said angiogenic activity.

Furthermore there is also provided a method of treating or preventing any of cancer, rheumatoid arthritis, psoriasis and diabetic retinopathy, said method comprising administering to said subject an amount of a polypeptide having an amino acid sequence from position 40 to 150 of the sequence illustrated in Figure 10 or a nucleic acid molecule encoding the CUB domain according to the invention in sufficient concentration to treat or prevent said disorders.

The CUB domain may also be used to identify compounds that inhibit or enhance angiogenic activity such as inappropriate vascularisation, in a method comprising contacting a cell expressing a VEGF receptor and/or a neuropilin 1 or 2 type receptor with said compound in the presence of a VEGF-X protein according to the invention and monitoring for the effect of said compound or said cell when compared to a cell which has not been contacted with said compound. Such compounds may then be used as appropriate to prevent or inhibit angiogenic activity to treat the disorders or conditions described herein, or in a pharmaceutical composition. An antibody to said CUB domain may also be useful in identifying other proteins having said sequences.

Also provided by this aspect of the present invention is a nucleic acid molecule such as an antisense molecule capable of hybridising to the nucleic acid molecules according to the invention under high stringency conditions, which conditions would be well known to those skilled in the art.

Stringency of hybridisation as used herein refers to conditions under which polynucleic acids are stable. The stability of hybrids is reflected in the melting temperature (Tm) of the hybrids. Tm can be approximated by the formula:

$$81.5°C+16.6(\log_{10}[Na^+]+0.41\ (\%G\&C)-600/1$$

wherein I is the length of the hybrids in nucleotides. Tm decreases approximately by 1-1-5°C with every 1% decrease in sequence homology.

The term "stringency" refers to the hybridisation conditions wherein a single-stranded nucleic acid joins with a complementary strand when the purine or pyrimidine bases therein pair with their corresponding base by hydrogen bonding. High stringency conditions favour homologous base pairing whereas low stringency conditions favour non-homologous base pairing.

"Low stringency" conditions comprise, for example, a temperature of about 37°C or less, a formamide concentration of less than about 50%, and a moderate to low salt (SSC) concentration; or, alternatively, a temperature of about 50°C or less, and a moderate to high salt (SSPE) concentration, for example 1M NaCl.

"High stringency" conditions comprise, for example, a temperature of about 42°C or less, a formamide concentration of less than about 20%, and a low salt (SSC) concentration; or, alternatively, a temperature of about 65°C, or less, and a low salt (SSPE) concentration. For example, high stringency conditions comprise hybridization in 0.5 M $NaHPO_4$, 7% sodium dodecyl sulfate (SDS), 1 mM EDTA at 65°C (Ausubel, F.M. et al. Current Protocols in Molecular Biology, Vol. I, 1989; Green Inc. New York, at 2.10.3).

"SSC" comprises a hybridization and wash solution. A stock 20X SSC solution contains 3M sodium chloride, 0.3M sodium citrate, pH 7.0.

"SSPE" comprises a hybridization and wash solution. A 1X SSPE solution contains 180 mM NaCl, 9mM $Na_2HPO_4$ and 1 mM EDTA, pH 7.4.

The nucleic acid capable of hybridising to nucleic acid molecules according to the invention will generally be at least 70%, preferably at least 80 or 90% and more preferably at least 95% homologous to the nucleotide sequences according to the invention.

The antisense molecule capable of hybridising to the nucleic acid according to the invention may be used as a probe or as a medicament or may be included in a pharmaceutical composition with a pharmaceutically acceptable carrier, diluent or excipient therefor.

The term "homologous" describes the relationship between different nucleic acid molecules or amino acid sequences wherein said sequences or molecules are related by partial identity or similarity at one or more blocks or regions within said molecules or sequences.

The present invention also comprises within its scope proteins or polypeptides encoded by the nucleic acid molecules according to the invention or a functional equivalent, derivative or bioprecursor thereof.

The DNA molecules according to the invention may, advantageously, be included in a suitable expression vector to express a CUB domain encoded therefrom in a suitable host. Incorporation of cloned DNA into a suitable expression vector for subsequent transformation of said cell and subsequent selection of the transformed cells is well known to those skilled in the art as provided in Sambrook et al. (1989), molecular cloning, a laboratory manual, Cold Spring Harbour Laboratory Press.

An expression vector according to the invention includes a vector having a nucleic acid according to the invention operably linked to regulatory sequences, such as promoter regions, that are capable of effecting expression of said DNA fragments. The term "operably linked" refers to a juxta position wherein the components described are in a relationship permitting them to function in their intended manner. Such vectors may be transformed into a suitable host cell to provide for expression of a polypeptide according to the invention. Thus, in a further aspect, the invention provides a process for preparing polypeptides according to the invention which comprises cultivating a host cell, transformed or transfected with an expression vector as described above under conditions to provide for expression by the vector of a coding sequence encoding the polypeptides, and recovering the expressed polypeptides.

The vectors may be, for example, plasmid, virus or phage vectors provided with an origin of replication, and optionally a promoter for the expression of said nucleotide and optionally a regulator of the promoter.

The vectors may contain one or more selectable markers, such as, for example, ampicillin resistance.

Regulatory elements required for expression include promoter sequences to bind RNA polymerase and transcription initiation sequences for ribosome binding. For example, a bacterial expression vector may include a promoter such as the lac promoter and for translation initiation the Shine-Dalgarno sequence and the start codon AUG. Similarly, a eukaryotic expression vector may include a heterologous or homologous promoter for RNA polymerase II, a downstream polyadenylation signal, the start codon AUG, and a termination codon for detachment of the ribosome. Such vectors may be obtained commercially or assembled from the sequences described by methods well known in the art.

Nucleic acid molecules according to the invention may be inserted into the vectors described in an antisense orientation in order to provide for the production of antisense RNA. Antisense RNA or other antisense nucleic acids may be produced by synthetic means.

In accordance with the present invention, a defined nucleic acid includes not only the identical nucleic acid but also any minor base variations including in particular, substitutions in cases which result in a synonymous codon (a different codon specifying the same amino acid residue) due to the degenerate code in conservative amino acid substitutions. The term "nucleic acid sequence" also includes the complementary sequence to any single stranded sequence given regarding base variations.

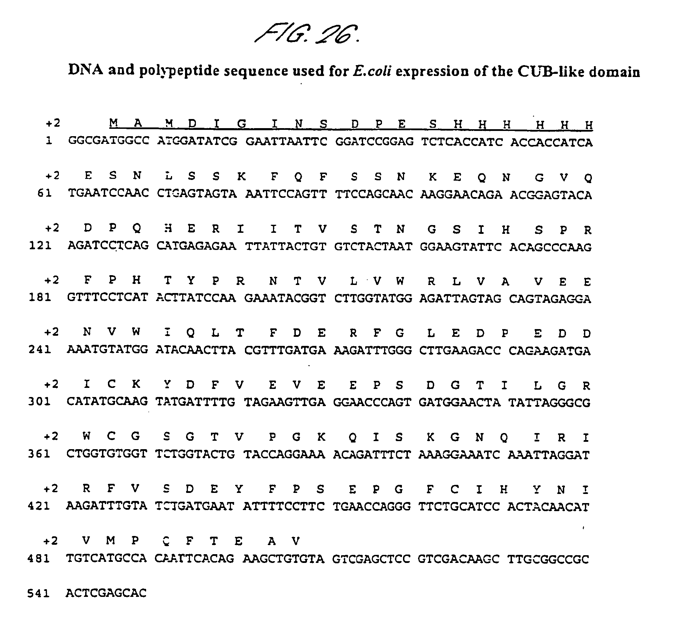

The present invention also advantageously provides nucleic acid sequences of at least approximately 10 contiguous nucleotides of a nucleic acid according to the invention and preferably from 10 to 50 nucleotides even more preferably, the nucleic acid sequence comprise the sequences illustrated in Figure 26. These sequences may, advantageously be used as probes or primers to initiate replication, or the like. Such nucleic acid sequences may be produced according to techniques well known in the art, such as by recombinant or synthetic means. They may also be used in diagnostic kits or the like for detecting the presence,of a nucleic acid according to the invention. These tests generally comprise contacting the probe with the sample under hybridising conditions and detecting for the presence of any duplex or triplex formation between the probe and any nucleic acid in the sample.

According to the present invention these probes may be anchored to a solid support. Preferably, they are present on an array so that multiple probes can simultaneously hybridize to a single biological sample. The probes can be spotted onto the array or synthesised *in situ* on the array. (See Lockhart et al., Nature Biotechnology, vol. 14, December 1996 "Expression monitoring by hybridisation to high density oligonucleotide arrays". A single array can contain more than 100, 500 or even 1,000 different probes in discrete locations.

The nucleic acid sequences, according to the invention may be produced using such recombinant or synthetic means, such as for example using PCR cloning mechanisms which generally involve making a pair of primers, which may be from approximately 10 to 50 nucleotides to a region of the gene which is desired to be cloned, bringing the primers into contact with mRNA, cDNA, or genomic DNA from a human cell, performing a polymerase chain reaction under conditions which brings about amplification of the desired region, isolating the amplified region or fragment and recovering the amplified DNA. Generally, such techniques are well known in the art, such as described in Sambrook et al. (Molecular Cloning: a Laboratory Manual, 1989).

The nucleic acids or oligonucleotides according to the invention may carry a revealing label. Suitable labels include radioisotopes such as $^{32}P$ or $^{35}S$, enzyme labels or other protein labels such as biotin or fluorescent markers. Such labels may be added to the nucleic acids or oligonucleotides of the invention and may be detected using known techniques per se.

Advantageously, human allelic variants or polymorphisms of the DNA molecule according to the invention may be identified by, for example, probing cDNA or genomic libraries from a range of individuals, for example, from different populations. Furthermore, nucleic acids and probes according to the invention may be used to sequence genomic DNA from patients using techniques well known in the art, such as the Sanger Dideoxy chain termination method, which may, advantageously, ascertain any predisposition of a patient to certain disorders associated with a growth factor according to the invention.

The protein according to the invention includes all possible amino acid variants encoded by the nucleic acid molecule according to the invention including a polypeptide encoded by said molecule and having conservative amino acid changes. Conservative amino acid substitution refers to a replacement of one or more amino acids in a protein as identified in Table 1. Proteins or polypeptides according to the invention further include variants of such sequences, including naturally occurring allelic variants which are substantially homologous to said proteins or polypeptides. In this context, substantial homology is regarded as a sequence which has at least 70%, preferably 80 or 90% and preferably 95% amino acid homology with the proteins or polypeptides encoded by the nucleic acid molecules according to the invention. The protein according to the invention may be recombinant, synthetic or naturally occurring, but is preferably recombinant.

The nucleic acid or protein according to the invention may be used as a medicament or in the preparation of a medicament for treating cancer or other diseases or conditions associated with expression of VEGF-X protein.

Advantageously, the nucleic acid molecule or the protein according to the invention may be provided in a pharmaceutical composition together with a pharmacologically acceptable carrier, diluent or excipient therefor.

A further aspect of the invention provides a host cell or organism, transformed or transfected with an expression vector according to the invention.

According to a further aspect of the invention there is also provided a transgenic cell, tissue or organism comprising a transgene capable of expressing a CUB domain protein according to the invention. The term "transgene capable of expression" as used herein means a suitable nucleic acid sequence which leads to expression of proteins having the same function and/or activity. The transgene, may include, for example, genomic nucleic acid isolated from human cells or synthetic nucleic acid, including DNA integrated into the genome or in an extrachromosomal state. Preferably, the transgene comprises the nucleic acid sequence encoding the proteins according to the invention as described herein.

Antibodies to the protein or polypeptide of the present invention may, advantageously, be prepared by techniques which are known in the art. For example, polyclonal antibodies may be prepared by inoculating a host animal, such as a mouse or rabbit, with the polypeptide according to the invention or an epitope thereof and recovering immune serum. Monoclonal antibodies may be prepared according to known techniques such as described by Kohler R. and Milstein C., Nature (1975) 256, 495-497. Advantageously, such antibodies may be included in a kit for identifying a CUB domain polypeptide in a sample, together with means for contacting the antibody with the sample.

The invention also further provides a pharmaceutical composition comprising said antibody together with a pharmaceutically acceptable carrier diluent or excipient therefor.

Proteins which interact with the polypeptide of the invention may be identified by investigating protein-interactions using the two-hybrid vector system first proposed by Chien et al., (1991) Proc. Natl. Acad. Sci. USA 88 : 9578-9582.

This technique is based on functional reconstitution in vivo of a transcription factor which activates a reporter gene. More particularly the technique comprises providing an appropriate host cell with a DNA construct comprising a reporter gene under the control of a promoter regulated by a transcription factor having a DNA binding domain and an activating domain, expressing in the host cell a first hybrid DNA sequence encoding a first fusion of a fragment or all of a nucleic acid sequence according to the invention and either said DNA binding domain or said activating domain of the transcription factor, expressing in the host at least one second hybrid DNA sequence, such as a library or the like, encoding putative binding proteins to be investigated together with the DNA binding or activating domain of the transcription - factor which is not incorporated in the first fusion; detecting any binding of the proteins to be investigated with a protein according to the invention by detecting for the presence of any reporter gene product in the host cell; optionally isolating second hybrid DNA sequences encoding the binding protein.

An example of such a technique utilises the GAL4 protein in yeast. GAL4 is a transcriptional activator of galactose metabolism in yeast and has a separate domain for binding to activators upstream of the galactose metabolising genes as well as a protein binding domain. Nucleotide vectors may be constructed, one of which comprises the nucleotide residues encoding the DNA binding domain of GAL4. These binding domain residues may be fused to a known protein encoding sequence, such as for example, the nucleic acids according to the invention. The other vector comprises the residues encoding the protein binding domain of GAL4. These residues are fused to residues encoding a test protein. Any interaction between polypeptides encoded by the nucleic acid according to the invention and the protein to be tested leads to transcriptional activation of a reporter molecule in a GAL-4 transcription deficient yeast cell into which the vectors have been transformed. Preferably, a reporter molecule such as β-galactosidase is activated upon restoration of transcription of the yeast galactose metabolism genes.

**Deposited Plasmids**

| | Date of Deposit | Accession No. |
|---|---|---|
| Plasmid VEGFX/pCR2.1 1TOPO FL | 1 March 1999 | LMBP 3925 |
| Plasmid VEGFX/pRSETB BD amino acids G230-G345 | 1 March 1999 | LMBP 3926 |
| Plasmid VEGFX/pcR.2.1 FL Clone 9 | 20 October 1999 | LMBP 3977 |
| Plasmid VEGF-X CUB PET22b | 20 December 1999 | ------ |

The above plasmids were deposited at the Belgian Coordinated Collections of Microorganisms (BCCM) at Laboratorium Voor Moleculaire Biologie-Plasmidencollectie (LMBP) B-9000, Ghent, Belgium, in accordance with the provisions of the Budapest Treaty of 28 April 1977.

The invention may be more clearly understood with reference to the accompanying example, which is purely exemplary, with reference to the accompanying drawings, wherein:

Figure 1: is a DNA sequence identified in the Incyte LifeSeq™ database coding for a novel VEGF-X protein.

Figure 2: is an illustration of amino acid sequence of the nucleic acid sequence of Figure 1.

Figure 3: is an illustration of PCR primer sequences utilised to identify the VEGF-X protein according to the invention.

Figure 4: is a diagrammatic illustration of the spatial relationships in the VEGF-X sequence of the clones identified using the PCR primer sequences of Figure 3.

Figure 5: is an illustration of the nucleotide sequences of the 5' RACE primers used to identify the 5' end of the VEGF-X open reading frame.

Figure 6: is an illustration of the sequence obtained from the RACE experiment.

Figure 7: is an illustration of the nucleotide sequences obtained from the search of LifeSeq™ database using the sequence in Figure 6.

Figure 8: is an illustration of the primers used to clone the entire coding sequence of VEGF-X.

Figure 9: is an illustration of the entire coding sequence of VEGF-X.

Figure 10: is an illustration of the predicted amino acid sequence of the nucleotide sequence of Figure 9.

Figure 11: is an alignment of the sequence of Figure 10 with the sequences of VEGF-A to D.

Figure 12: is an illustration of variant sequences of the VEGF-X protein according to the invention.

Figure 13: is an illustration of the oligonucleotide primers used for E.coli expression of VEGF-X domains and for expression of the full length sequence of VEGF-X in a baculovirus/insect cell expression system.

Figure 14: depicts nucleic acid sequences of 18 human EST clones obtained from a BLAST search of the LifeSeq™ database used to identify the full sequence encoding VEGF-X.

Figure 15: depicts the nucleotide sequences of 50 human EST clones obtained from the LifeSeq™ database.

Figure 16: is an illustration of nucleotide sequences utilised as primers to identify the nucleotide sequence encoding VEGF-X.

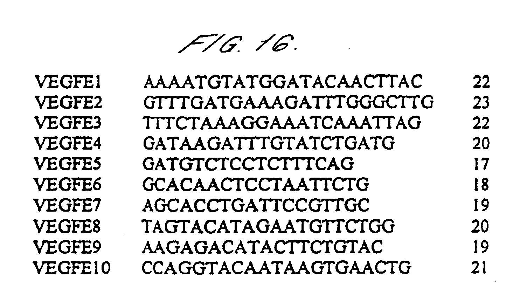

Figure 17: is a nucleotide sequence coding for a partial VEGF-X protein according to the invention.

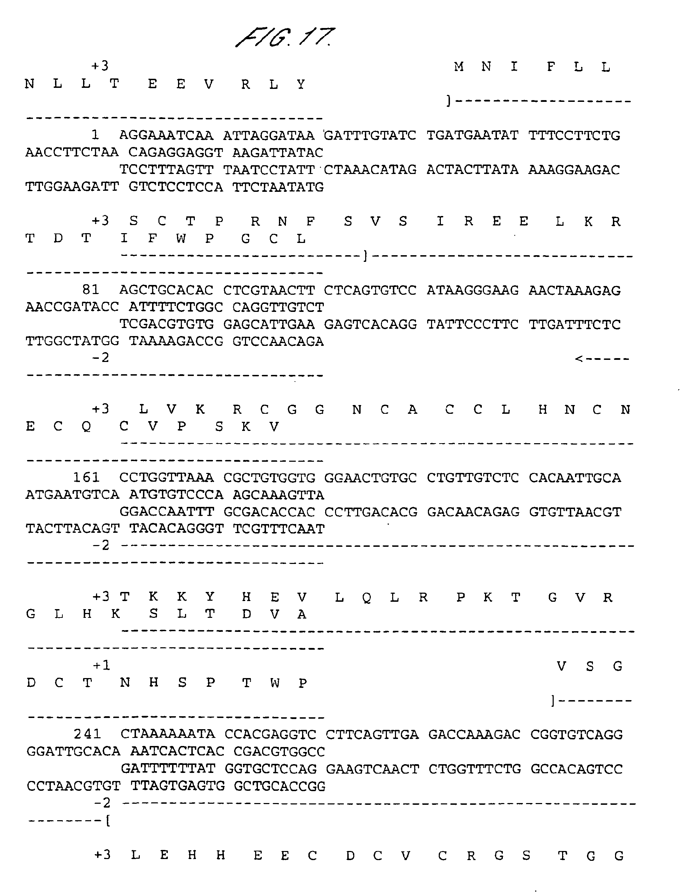

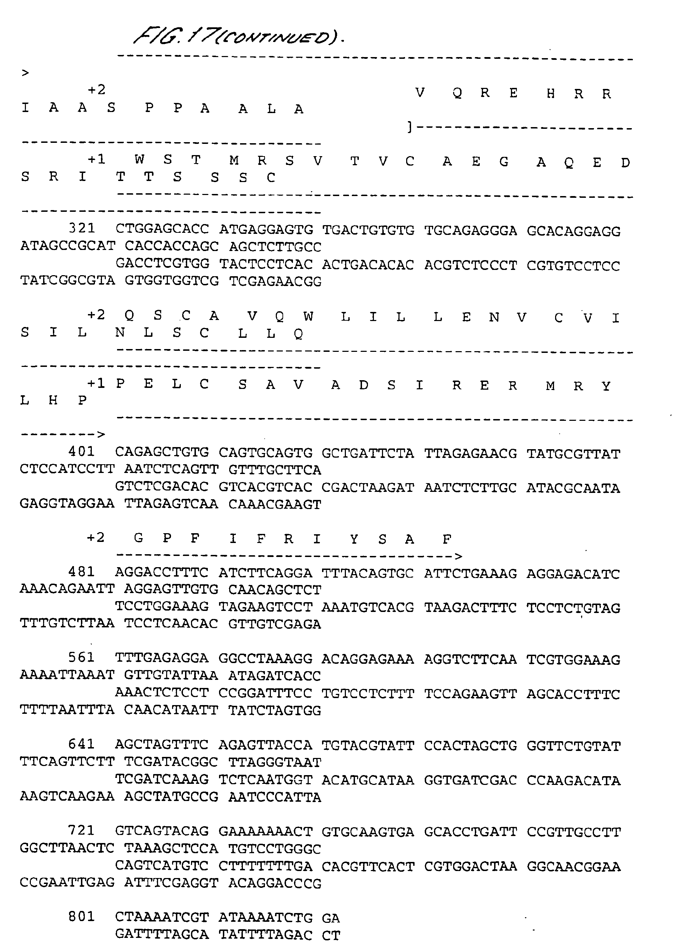

Figure 18: is an illustration of a partial nucleotide sequence encoding VEGF-X protein according to the invention.

Figure 19: is an illustration of a DNA and polypeptide sequence used for mammalian cell expression of VEGF-X. The predicted VEGF-X signal sequence is in lower case letters. The C-terminal V5 epitope and His6 sequences are underlined.

Figure 20: is an illustration of a DNA and polypeptide sequence used for baculovirus/insect cell expression of VEGF-X. In the polypeptide sequence the signal sequence is shown in lower case. The N-terminal peptide tag added to the predicted mature VEGF-X sequence is underlined.

Figure 21: is an illustration of a DNA and polypeptide sequence used for *E. coli* expression of VEGF-X. The polypeptide sequences at the N- and C- termini derived from the MBP fusion and His6 tag respectively are underlined.

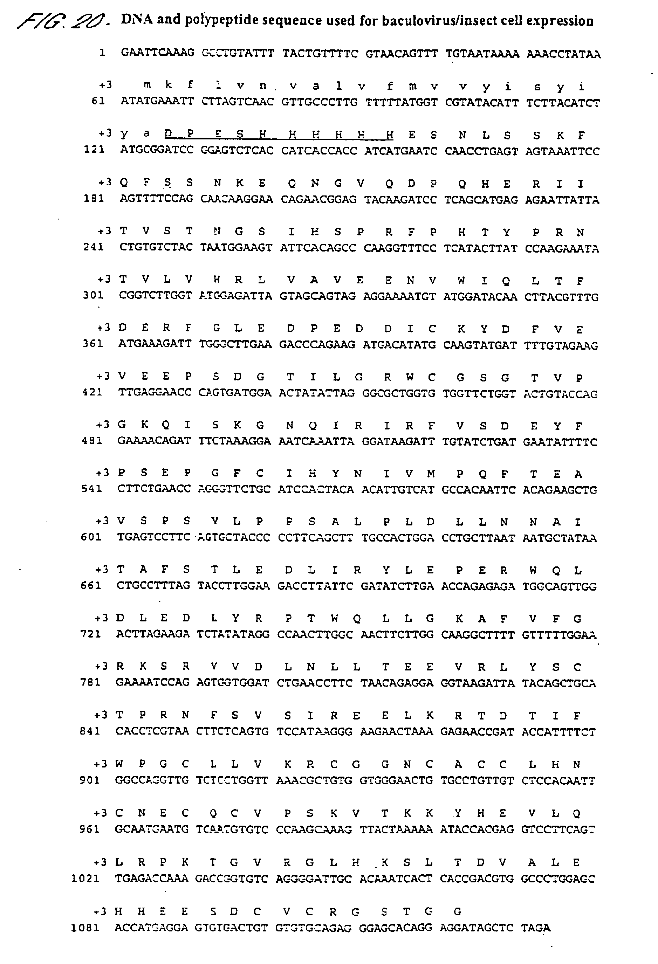

Figure 22: illustrates the disulphide-linked dimerisation of VEGF-X. Protein samples were analysed by SDS-PAGE. Prior to loading the gel, samples were heated to 95°C for 5 minutes in sample buffer in the presence (+) or absence (-) of reducing agent. (A) samples from COS cell expression of a C-terminally V5/His6 peptide-tagged construct. The left hand panel is total conditioned medium, the right hand panel is material purified on Nickel agarose resin. Reduced monomer and putative disulphide-linked, non-reduced dimer are indicated by arrows. There appears to be proteolysis of the protein during purification. Gels were blotted onto nylon membranes and protein detected with an anti V5 monoclonal antibody. (B) Samples from E.coli expression of a maltose-binding protein/His6 dual fusion construct. M indicates the molecular weight markers (Benchmark, LifeTechnologies). The gel was stained with Coomassie Blue by standard procedures. The fusion protein has an apparent molecular weight of 80kDa.

Figure 23: illustrates the glycosylation of VEGF-X. VEGF-X was purified from the culture supernatant of COS cells transfected with the pcDNA6/V5-His construct. Supernatants were harvested 72h post-transfection and purified on nickel resin. Samples were then treated with EndoH (+) or untreated (-) before SDS-PAGE and blotting, as described in the legend to Figure 22.

Figure 24: is an illustration of the DNA and polypeptide sequence used for *E. coli* expression of the VEGF-like domain of VEGF-X. Polypeptide sequences at the N-terminus of the protein derived from the vector are underlined.

Figure 25: shows expression of the VEGF-X VEGF domain in E. coli. Lane 1-10μl broad range marker (New England Biolabs), lane 2-10μl unreduced sample, lane 3-10μl reduced sample. The reduced PDGF domain protein (lane 3) has an apparent molecular weight of approximately 19kDa on SDS-PAGE.

Figure 26: illustrates a DNA and polypeptide sequence used for E. coli expression of the CUB-like domain of VEGF-X. The polypeptide sequence at the N-terminus derived from the vector-encoded signal and the introduced His6 tag are underlined.

Figure 27: shows expression of the VEGF-X CUB domain in *E. coli.* The CUB domain protein was purified on Nickel chelate resin. The protein migrates at approximately 23kDa on SDS-PAGE.

Figure 28: illustrates the effect of truncated VEGF-X (CUB domain) on HUVEC proliferation. (A) Human Umbilical Vein Endothelial Cells (one-day-treatment). (B) Human Umbilical Vein Endothelial Cells (24-hour starving followed by one-day-treatment). (C) Effect of VEGF-$A_{165}$ and VEGF-X CUB domain on the proliferation of HUVEC (two-day-treatment).

Figure 29: depicts the tissue distribution of VEGF-X mRNA analysed by Northern blotting and RT-PCR in (A) normal tissues and (B) tumour tissue and cell lines.

Figure 30: depicts the partial intron/exon structure of the VEGF-X gene. (A) Genomic DNA sequences of 2 exons determined by sequencing; exon sequence is in upper case, intron sequence is in lower case. (B) Shows the location of splice sites within the VEGF-X cDNA sequence. The location of mRNA splicing events is indicated by vertical lines. The cryptic splice donor/acceptor site at nt. 998/999 (diagonal lines) gives rise to the splice variant forms of VEGF-X. No splice site information is given for the region shown in italics.

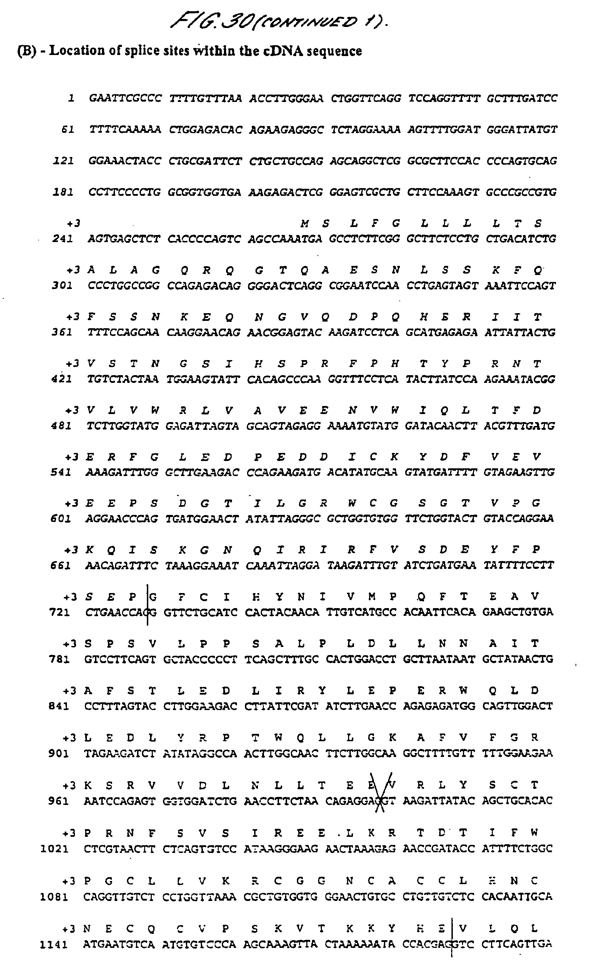

Figure 31: is a graphic representation of the effect of FL-VEGF-X on HuVEC proliferation: (24 hour serum starvation followed by one day treatment).

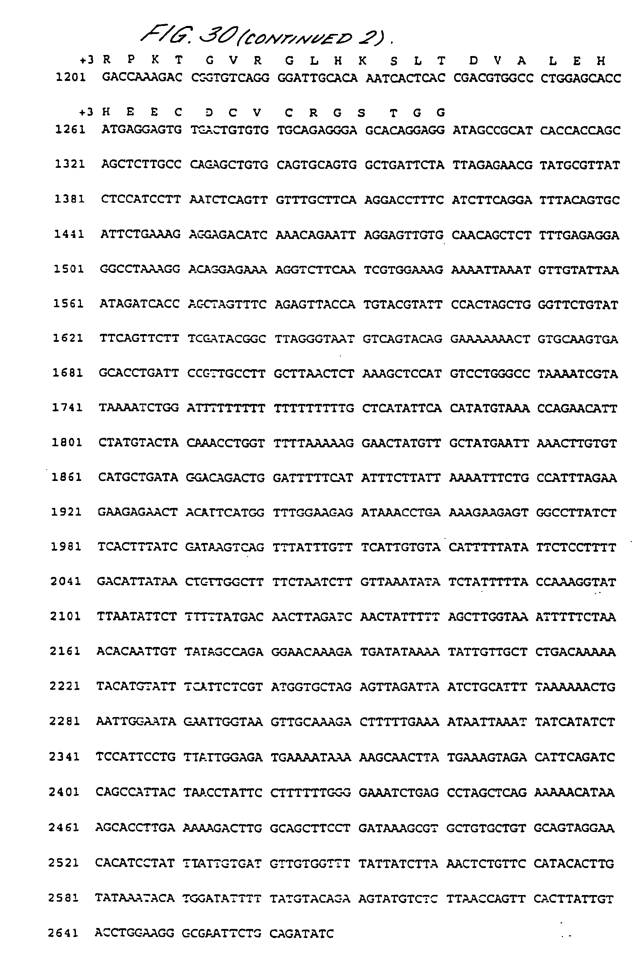

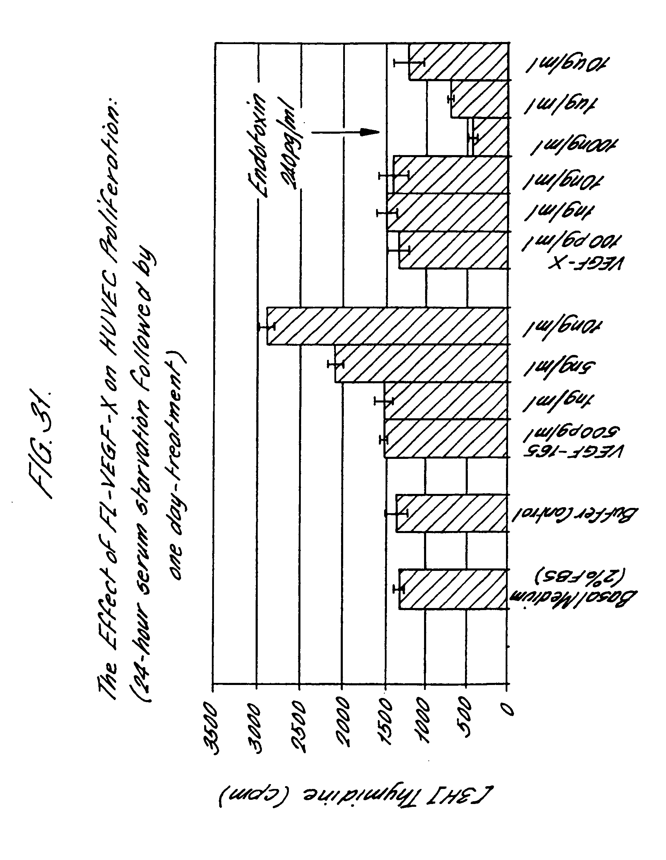

Figure 32: is a graphic representation of the combined effect of truncated VEGF-X (CUB domain) and human recom-

binant $VEGF_{165}$ on HuVEC proliferation: (24 hour serum starvation followed by two day treatment).

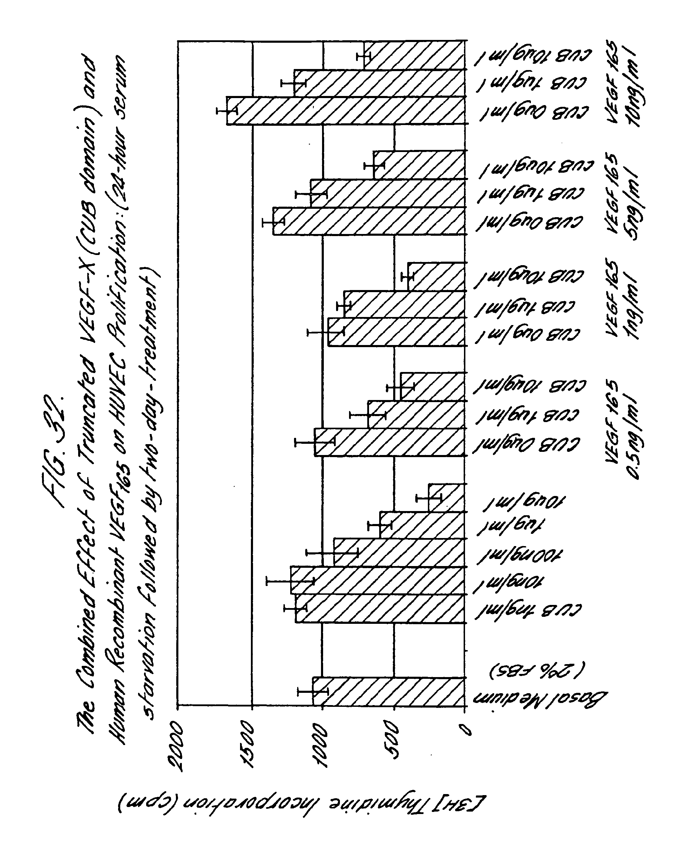

Figure 33: is a graphic representation of the combined effect of the CUB domain and human recombinant bFGF on HuVEC proliferation: (24 hour serum starvation followed by two day treatment).

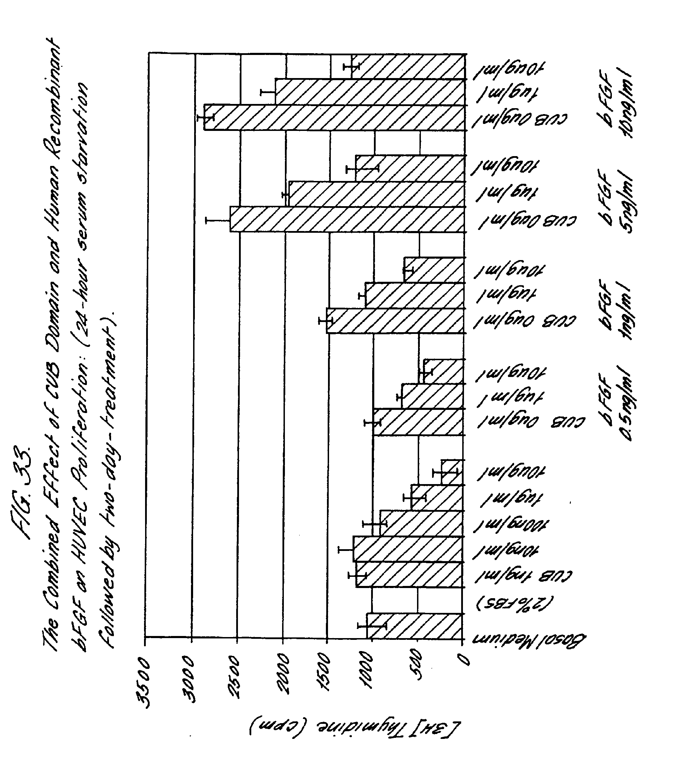

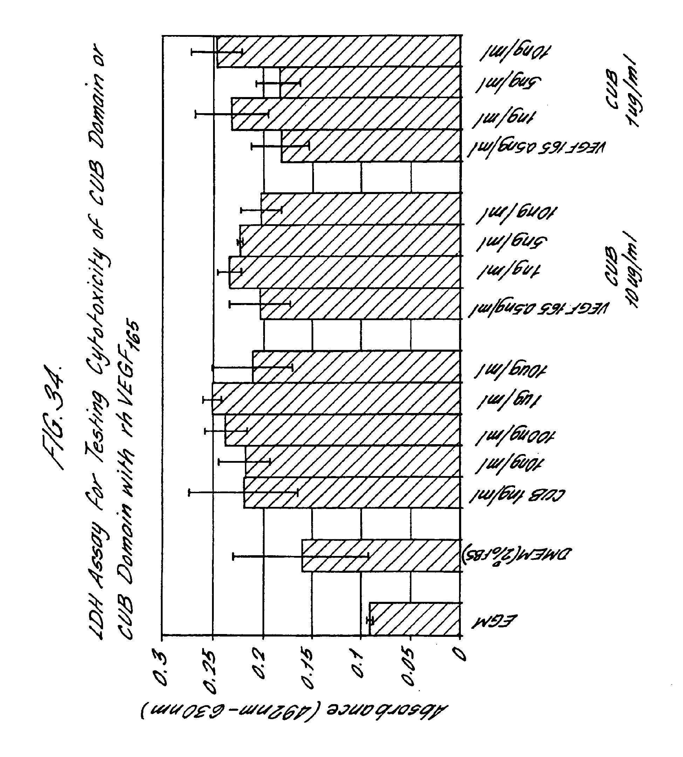

Figure 34: is a graphic representation of the results of a LDH assay for testing cytotoxicity of the CUB domain or the CUB domain with $rhVEGF_{165}$.

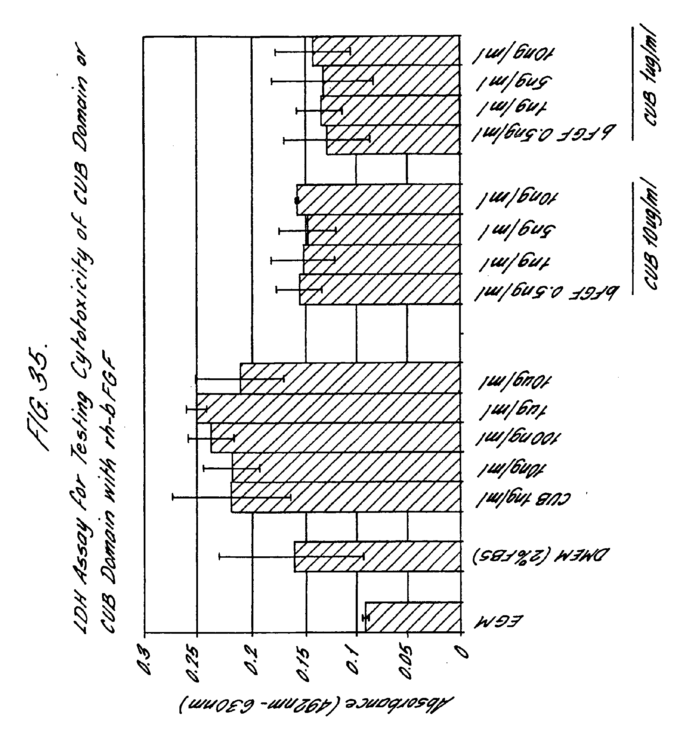

Figure 35: is a graphic representation of the results obtained from a LDH assay for testing cytotoxicity of the CUB domain or CUB domain with rh-bFGF.

A BLAST (Basic Local Alignment Search Tool; Altschul et al., 1990 J. Mol. Biol. 215, 403-410) search was performed in the proprietary LifeSeq™ human EST database (Incyte Pharmaceuticals, Inc., Palo Alto, CA, USA). BLAST produces alignments of both nucleotide and amino acid sequences to determine sequence similarity. Because of the local nature of the alignments, BLAST is especially useful in determining exact matches or in identifying homologues. While it is useful for matches which do not contain gaps, it is inappropriate for performing motif-style searching. The fundamental unit of BLAST algorithm output is the High-scoring Segment Pair (HSP).

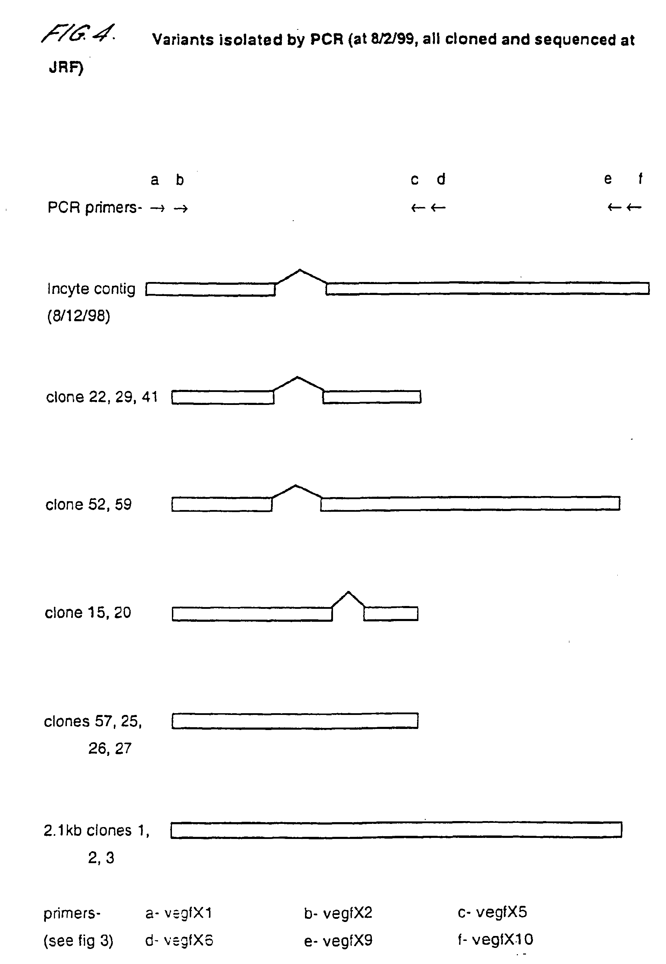

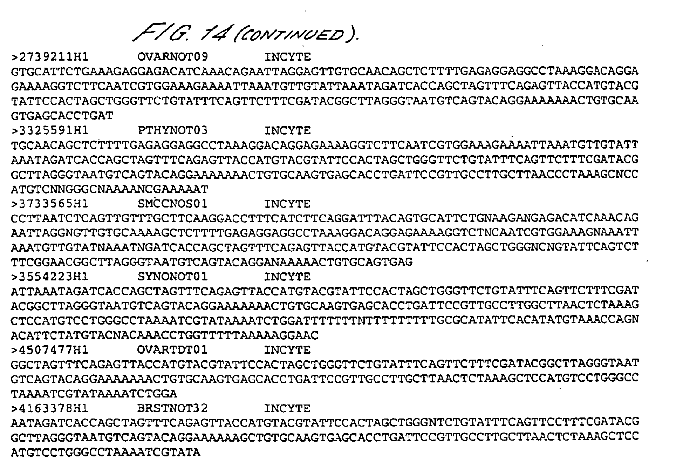

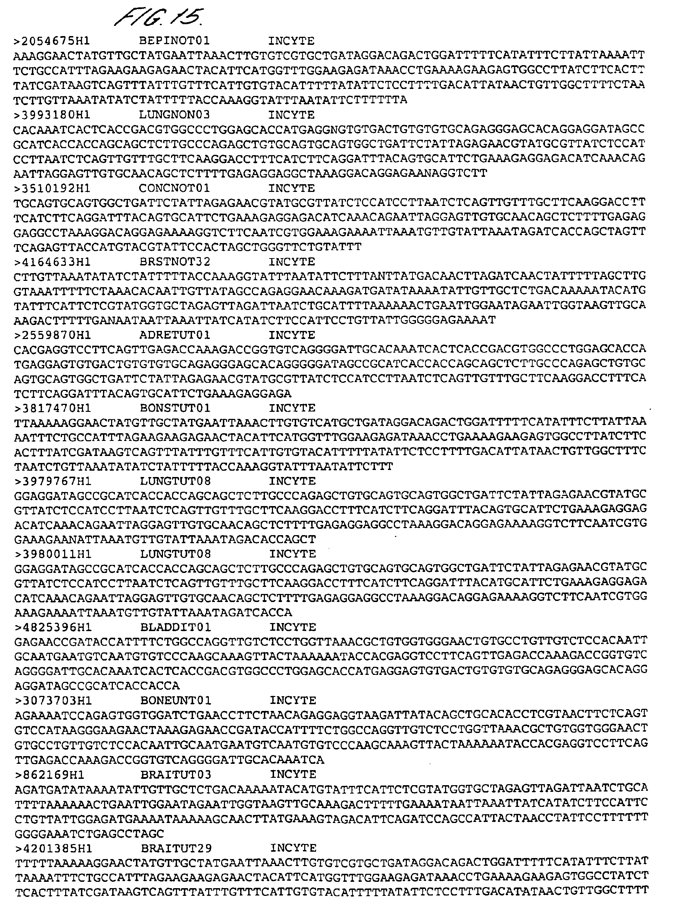

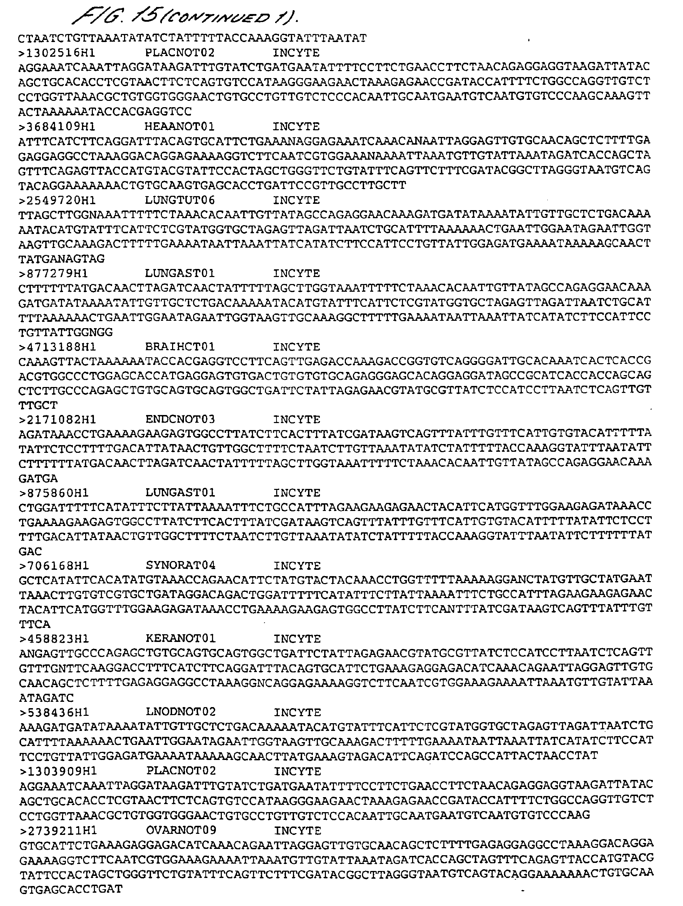

Eighteen human EST clones (Figure 14) with high similarity to the previously identified VEGF proteins were identified and a further fifty EST clones (Figure 15) were identified using these sequences as query sequences, allowing us to deduce the putative sequence for the new VEGF-X protein. The sequences obtained were compared to known sequences to determine regions of homology and to identify the sequence as a novel VEGF-type protein. Using the DNA sequence information in the databases we were able to prepare suitable primers having the sequences of VEGF-X 1-10 illustrated in Figure 3 for use in subsequent RACE experiments to obtain the complete DNA sequence for the VEGF-X gene.

Cloning

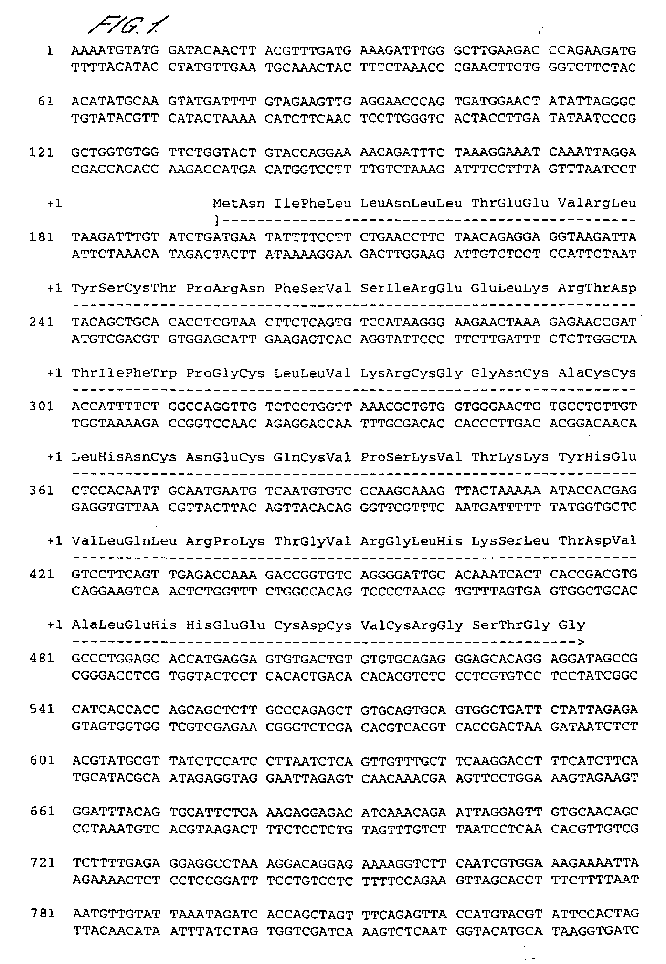

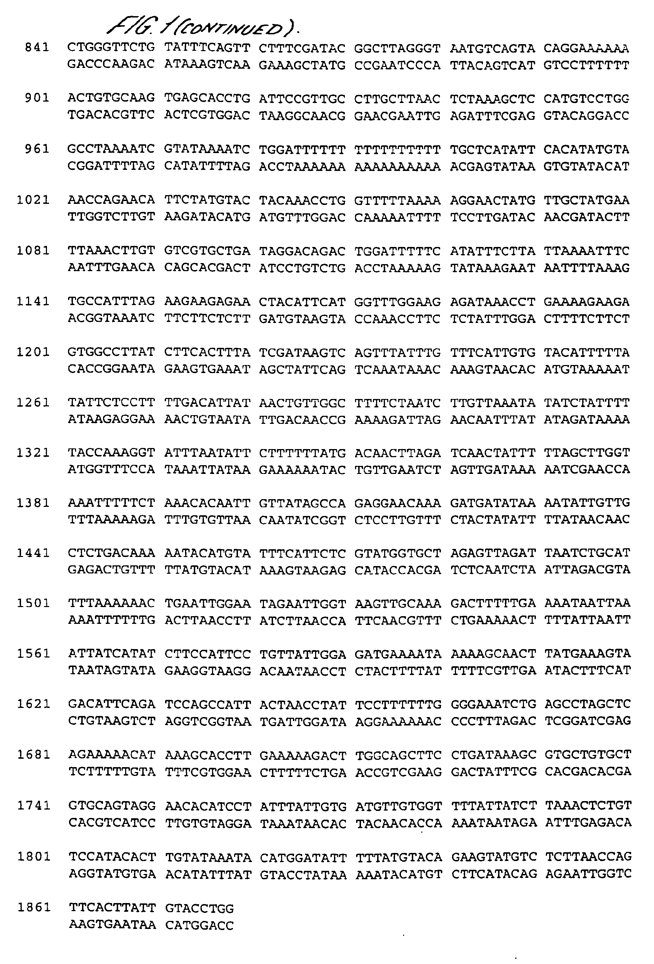

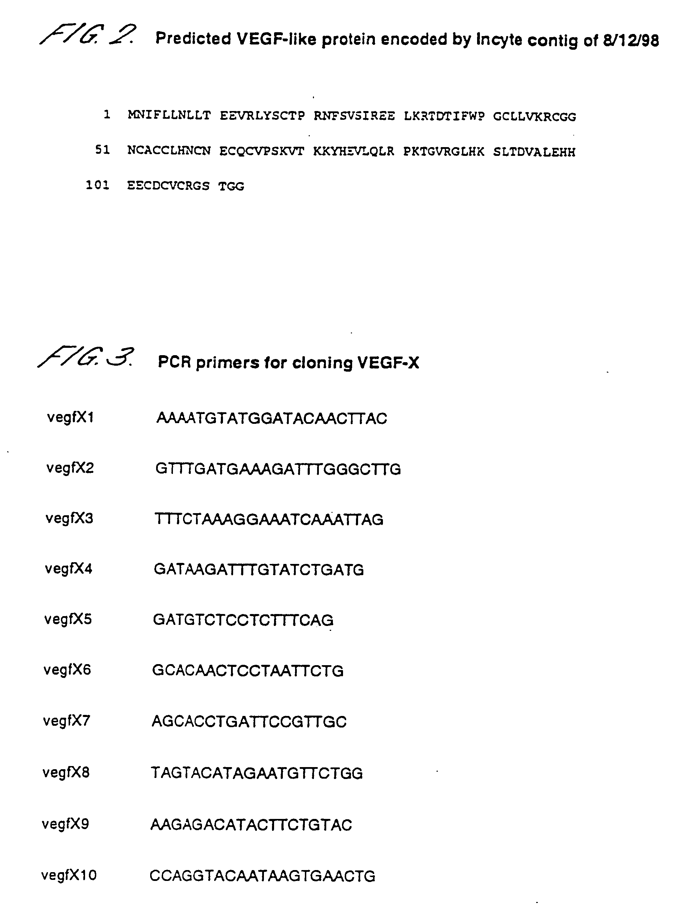

A profile was developed based on the VEGF-like domain in existing VEGF sequences (VEGF-A, B, C and D). This was used to search the public databases and the Incyte LifeSeq™ database. No significant novel matching sequences were found in the public databases. All of the matching sequences found in the LifeSeq™ database (-1000) were assembled to give a smaller number of sequences (-30), which included the known VEGFs and a potential novel VEGF (figures 1 and 2). This sequence was named VEGF-X.

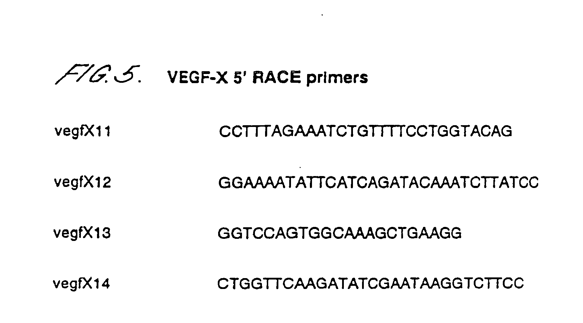

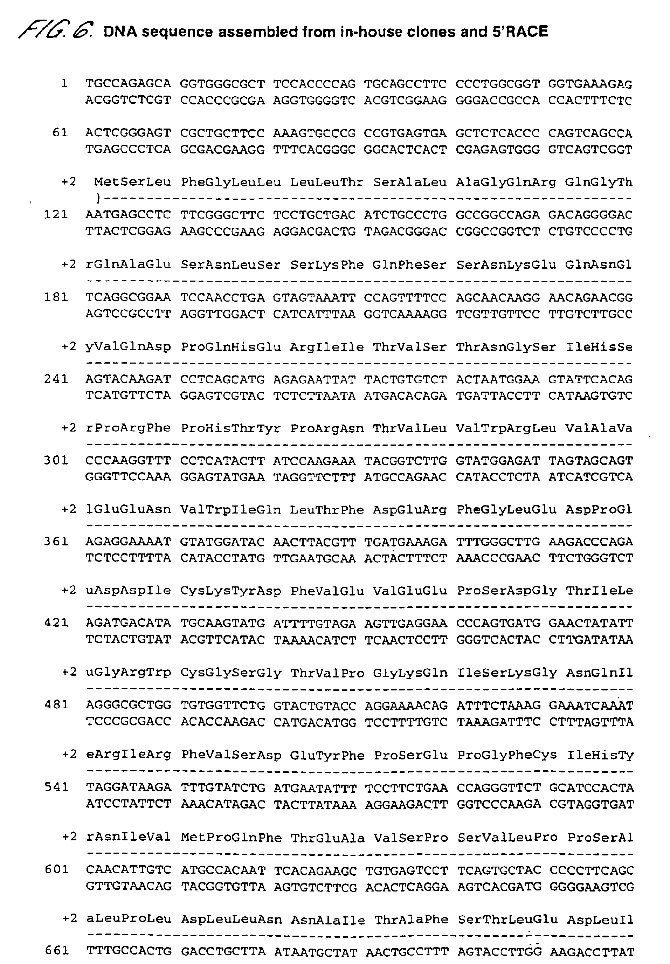

Oligonucleotides were designed to amplify the VEGF-X sequence from cDNA (figure 3). The ESTs found in LifeSeq™ were from a range of tissues, with a slight predominance of sequences from ovary, testis, placenta and lung (Figure 14 and 15). Accordingly the oligonucleotides were used to amplify cDNA derived from lung and placenta. First-round PCR products were found at -200bp larger than the expected sizes, while 3 major species appeared after a second round of PCR amplification, the smallest of which was of the expected size. These fragments were cloned and sequenced. The smallest fragment did indeed have the sequence originally identified from the LifeSeq database, while the others contained insertions (figure 4).

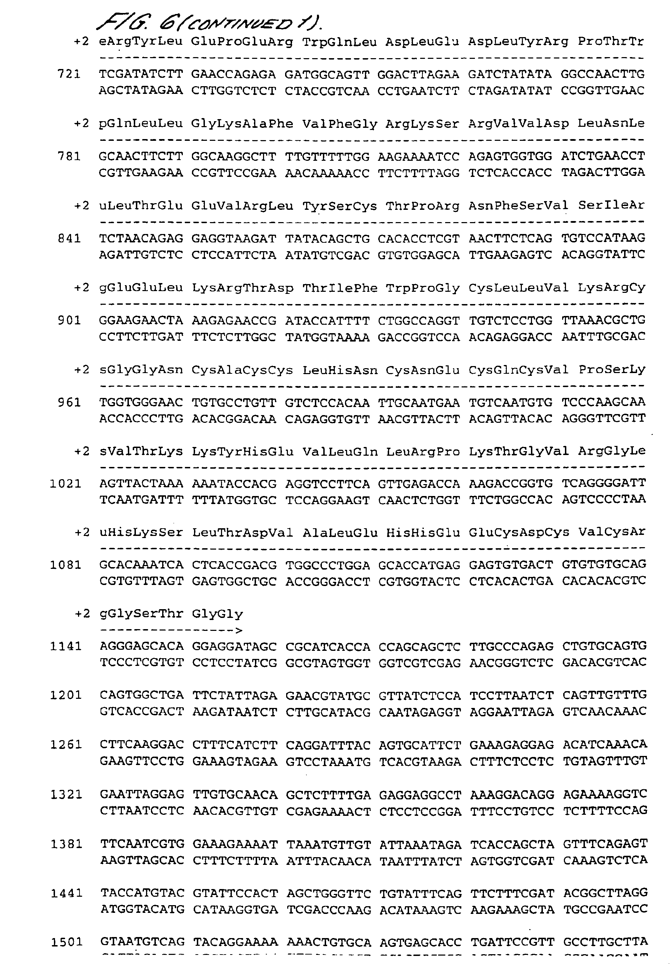

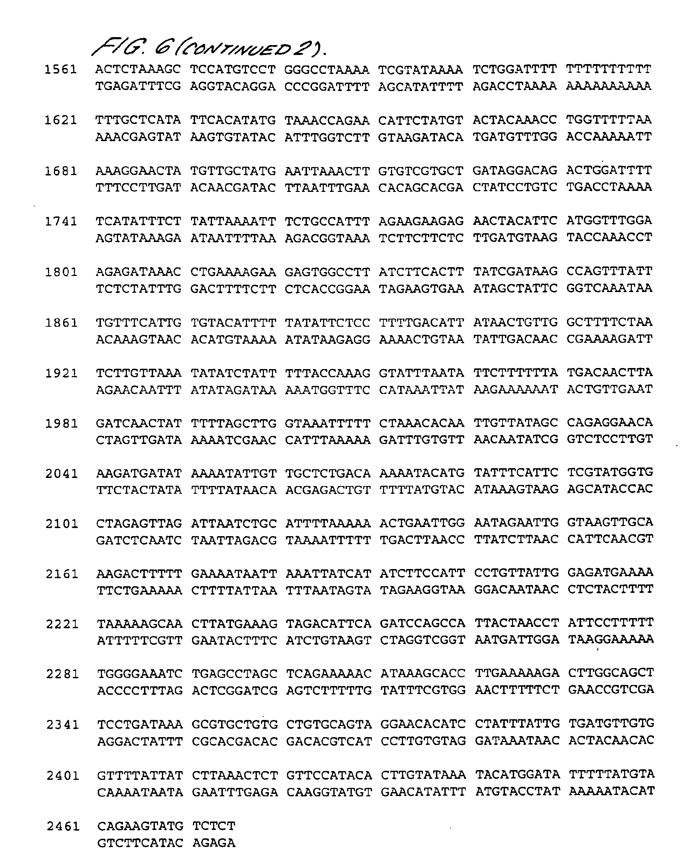

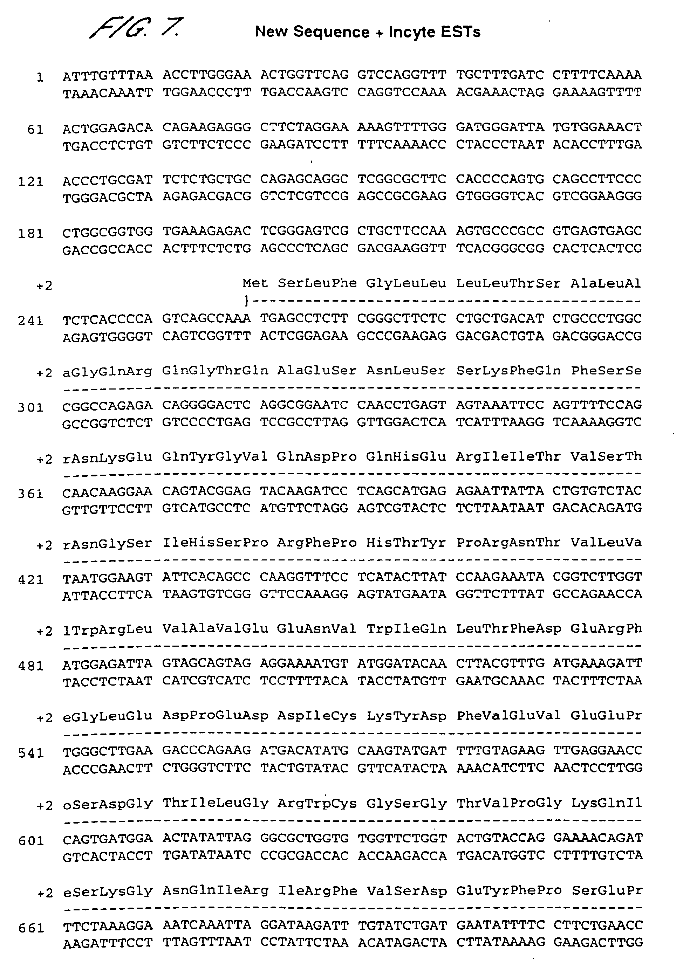

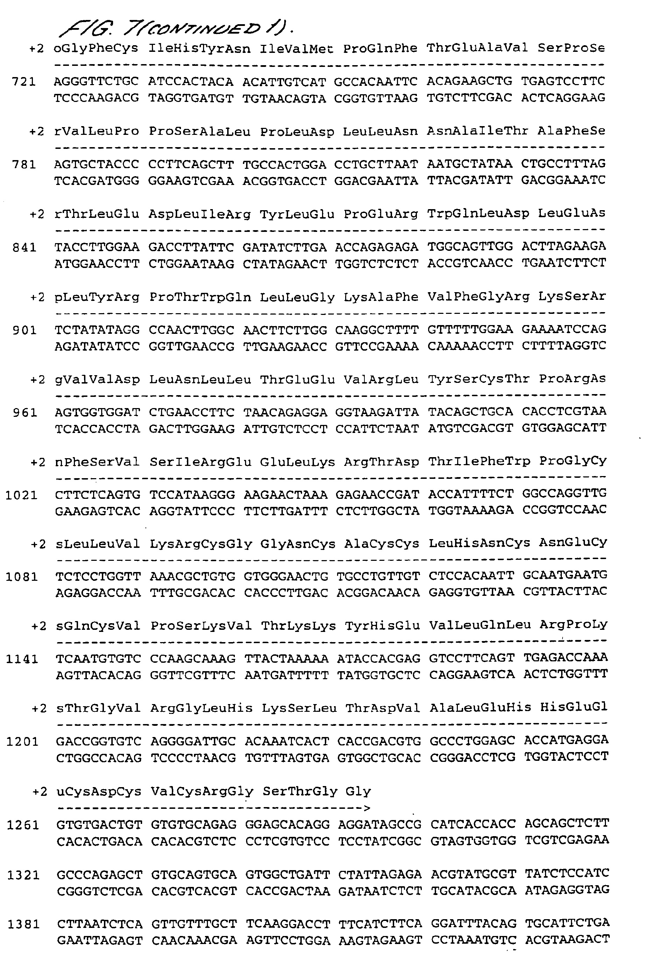

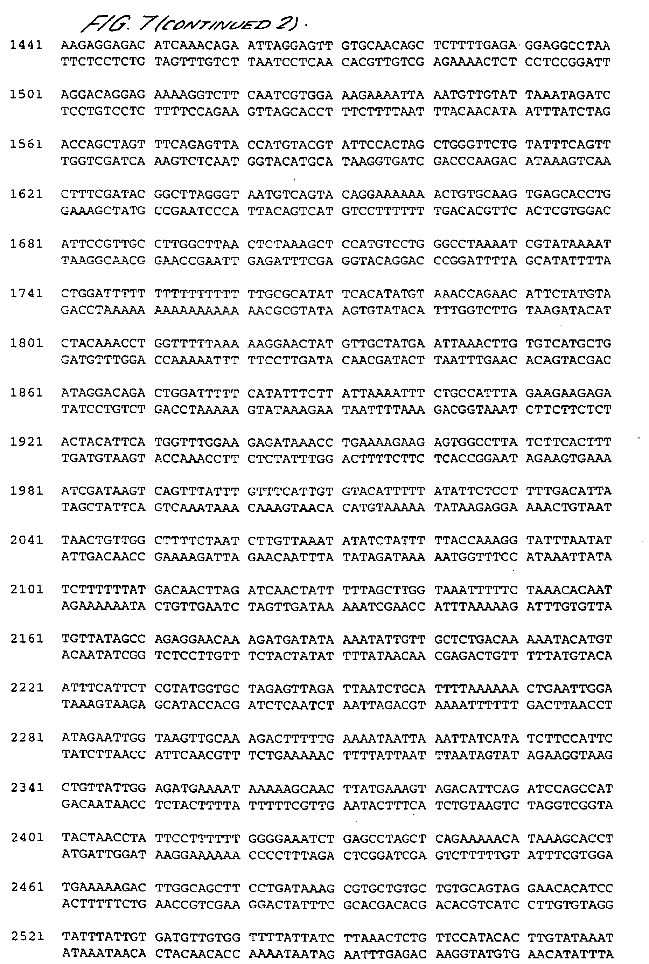

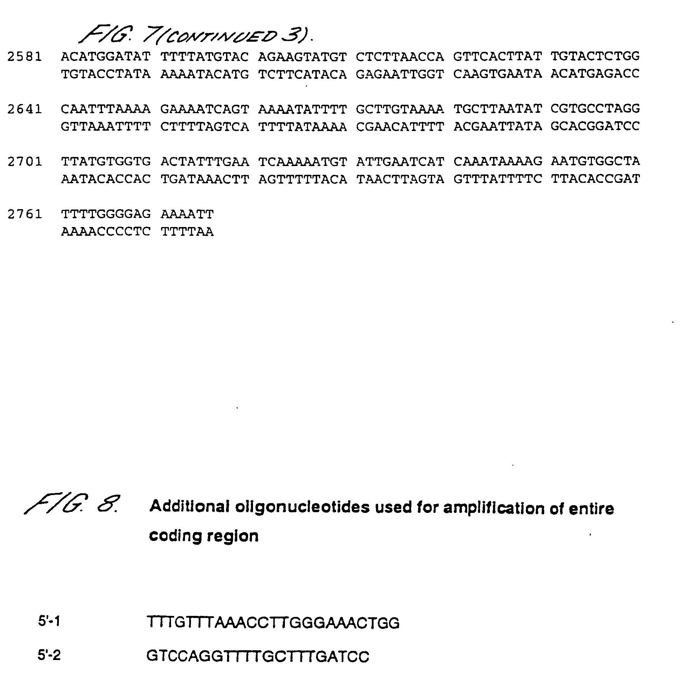

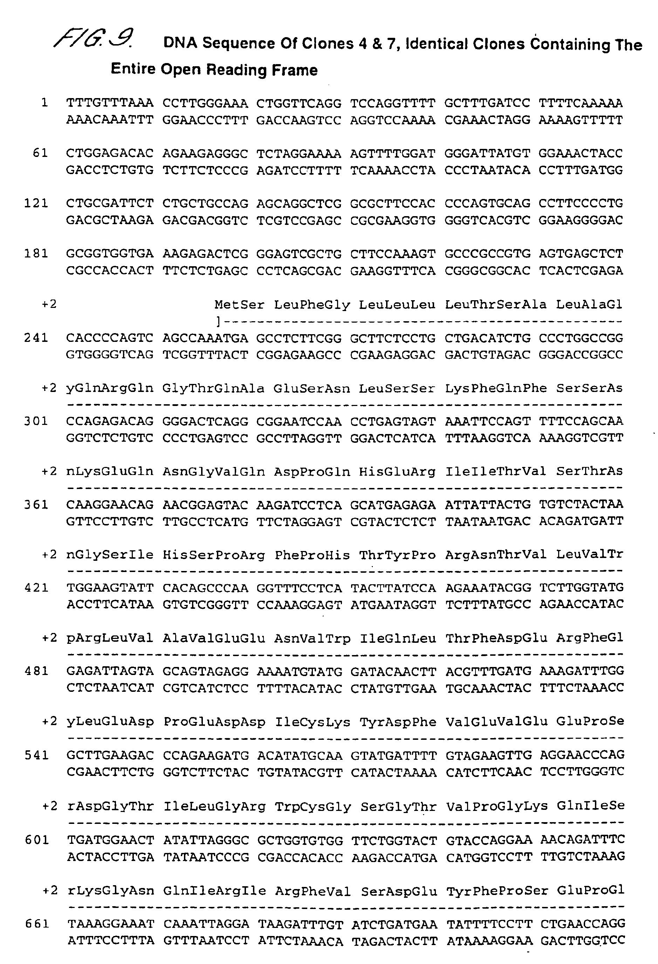

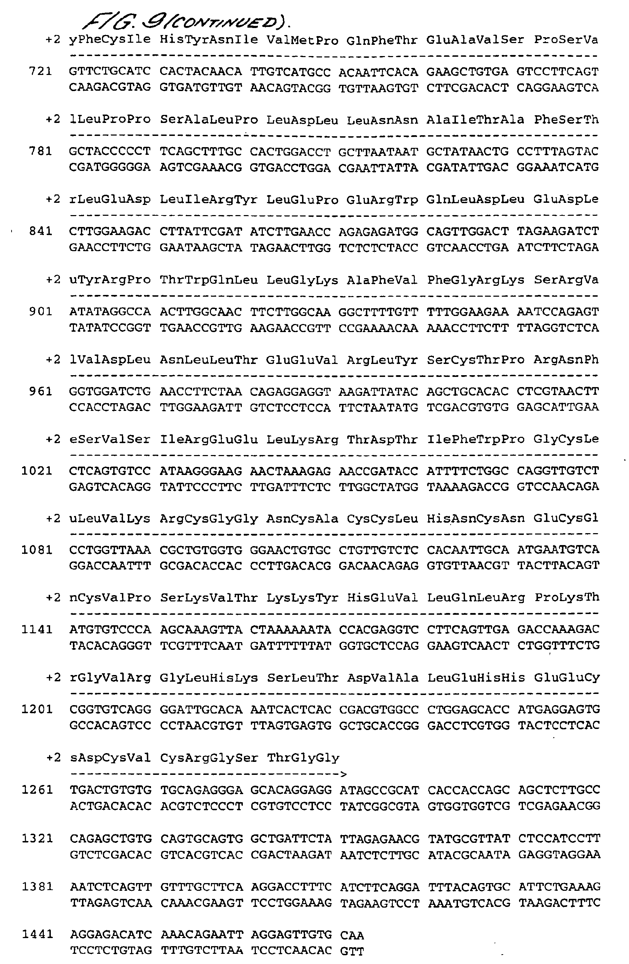

As the first round of amplification suggested that the major species found in cDNA from ovary and placenta was not that originally identified in the LifeSeq™database, the focus of effort was switched to the presumed major species (it seemed likely that clones 57, 25-27 and 2.1kb clones 1-3 in fig 4 represented the major mRNA species). Conceptual translation of the DNA sequences of these cloned PCR fragments indicated that the complete open reading frame was not present in the clones or in the sequence from LifeSeq™. While all clones contained the same sequence in the region of the translation termination codon, indicating that the end of the open reading frame had been identified, the 5' end of the open reading frame had not been cloned. 5' RACE experiments were therefore carried out in order to find the start of the reading frame. PCR primers designed for RACE experiments are shown in figure 5. RACE PCR products were sequenced directly. Sequence could be obtained from the 3' end of these RACE products but not from the 5' end; probably because the products were not cloned and were therefore heterogeneous at the 5' end. This new sequence was assembled with the existing cloned sequence to give the sequence shown in figure 6. Searching the LifeSeq™ database with this sequence identifies ESTs which extend the sequence a further 140bp in the 5' direction and a further 160bp in the 3' direction (figure 7). This longer contig was used to design oligonucleotide primers to amplify the entire coding sequence (these primer sequences are shown in figure 8). PCR was carried out using primers 5'-1 and vegfX10 (in order to clone a "full-length" cDNA), and with primers 5'-1 and vegfX6 (in order to clone the full coding region, see figure 3 for sequences of vegfX10 and vegfX6). A number of clones were obtained for the shorter fragment, of which clones 4 and 7 contain no PCR errors (sequence of clones 4 & 7 in figure 9). A single clone was obtained for the longer fragment (clone 9), but this sequence appears to contain 2 PCR errors.

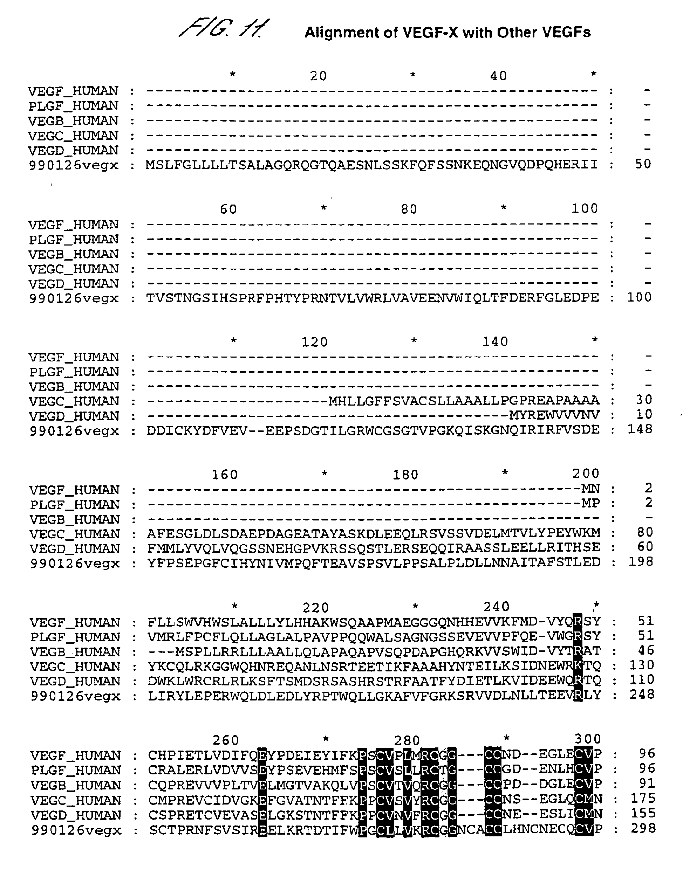

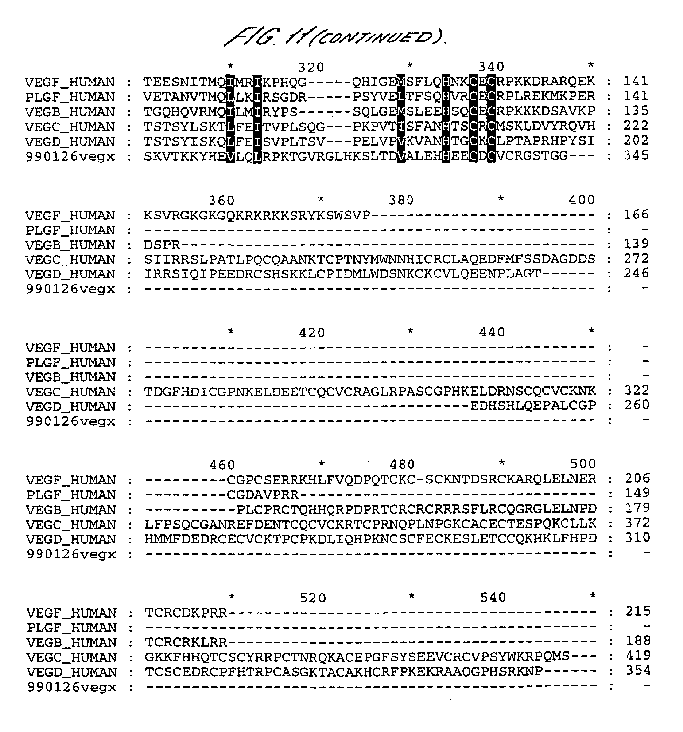

The predicted polypeptide from these longer contigs is shown in figure 10. Amino acids 1-22 are predicted to encode a signal sequence (von Heijne, 1986, Nucleic Acids Res. 14, 4683-4690). **Figure 11** shows an alignment of the protein sequence with VEGFs A-D. The region homologous to the other VEGFs is located towards the C-terminus of the protein. As the VEGF homology domain is expected to belong to the TGF-beta superfamily of growth factors and to consist of a dimer containing both intra- and intermolecular disulphide bonds, initial alignments focussed on the cysteines. However, mapping of the sequence onto the known x-ray structure of the VEGF-A receptor-binding domain (Muller et al (1997) Proc. Natl. Acad. Sci USA 94, 7192-7197) suggests that the alignment in figure 11 is plausible, as the extra 4 cysteine residues within the VEGF-homology region of VEGF-X (compared to this region of VEGF-A) correspond to residues which are spatially close in VEGF-A, and may therefore be able to form disulphide bonds.

A search of the PFAM database of protein domains with the full-length polypeptide sequence from figure 10 identifies two domain consensus sequences within the polypeptide. The more C-terminal domain is a "VEGF" domain: (the known VEGFs all contain this domain and the structure of this region of VEGF-A is similar to that of PDGF). Additionally towards the N-terminus of the polypeptide there is a CUB domain (amino acids -40-150). The CUB domain is a 100-110 amino acid extracellular domain found in a number of developmentally-regulated proteins. When the full-length protein is used to search the protein databases using the BLAST 2 algorithm, the scores for matches to CUB domain-containing proteins are more significant than those to the other VEGFs. Interestingly, the most significant matches are to the CUB domains of Neuropilins, and Neuropilin-1 was recently identified as a receptor of one of the VEGF-A isoforms VEGF-$A_{165}$ (Soker et al. (1998) Cell 92, 735-745).

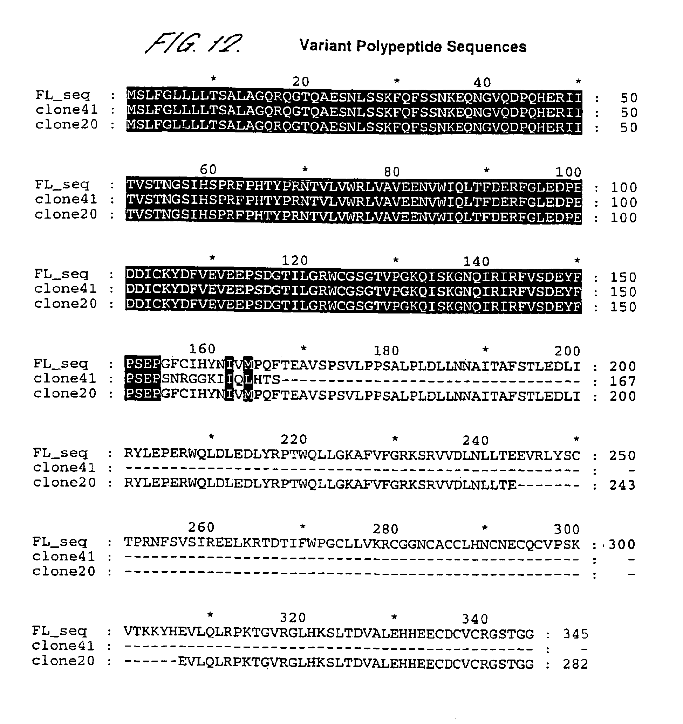

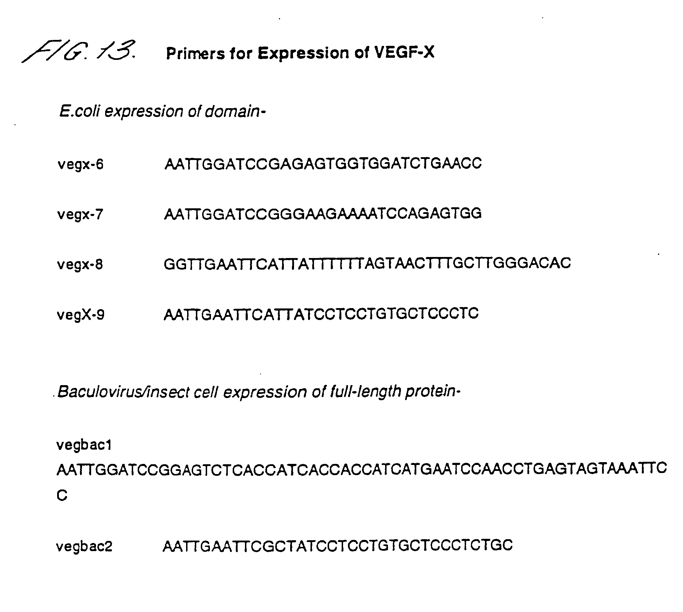

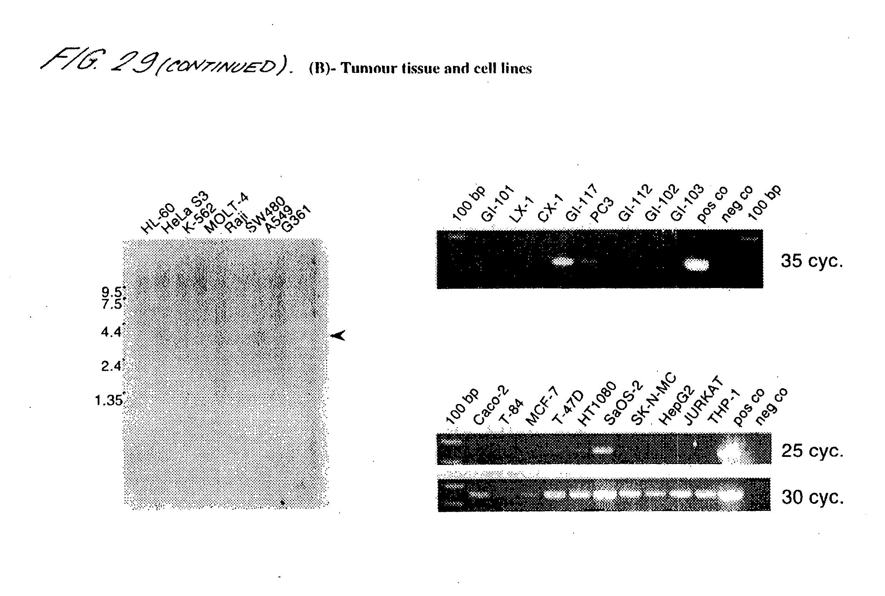

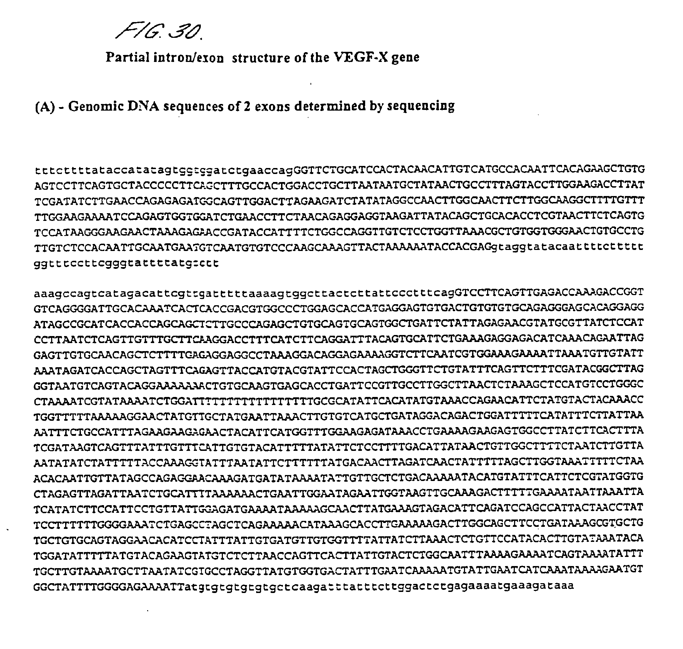

Assuming that the variant sequences isolated by PCR (i.e. the smaller PCR fragments) use the same translation initiation site as the full-length sequence, they would result in production of the variant proteins shown in figure 12. It may be significant that both of these variant proteins retain the CUB domain and delete all or part of the VEGF-like domain. The production of these variant sequences can be explained by the use of a cryptic splice donor/acceptor site within the VEGF-X sequence (figure 30B, between nt. 998/999): one variant arises by splicing out of the region between nt. 729-998, the other by splicing out of the region between nt. 999-1187.

## Expression

### Full-length expression constructs

Mammalian cells

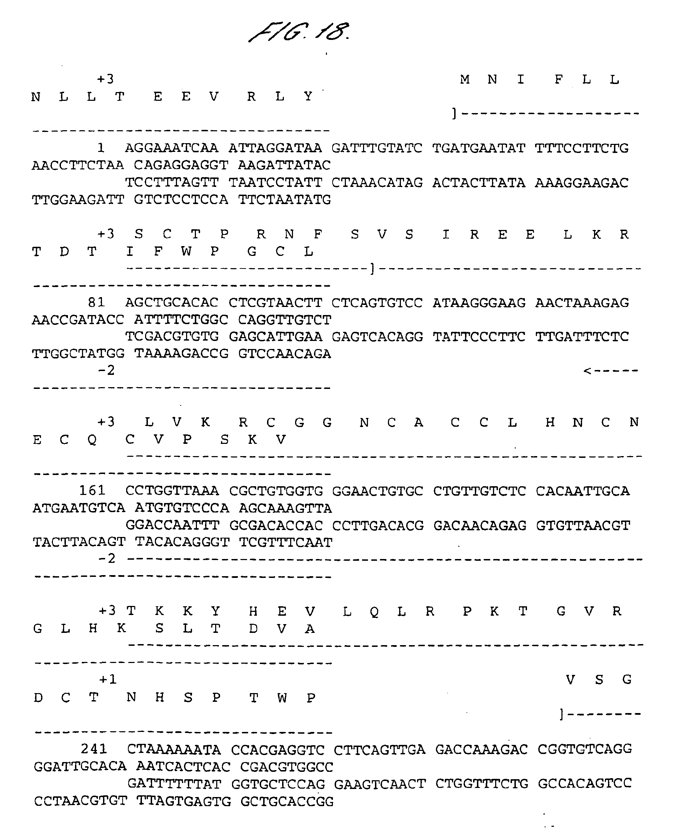

Clone 4 containing the full CDS of VEGF-X (see figure 9), was used to generate constructs for expression of full-length protein. The sequence was amplified by PCR and cloned into the vector pCDNA6/V5-His so as to add a C-terminal V5 epitope tag and $His_6$ tag. The DNA and polypeptide sequence in this vector is shown in figure 19. Transient expression in COS cells followed by western blotting and detection via an anti-V5 mAb demonstrates the secretion of a protein of ~50K into the medium in transfected cells only (figure 22A). This construct can also be used to generate VEGF-X expressing stable CHO cell lines.

Baculovirus/Insect-cell expression system

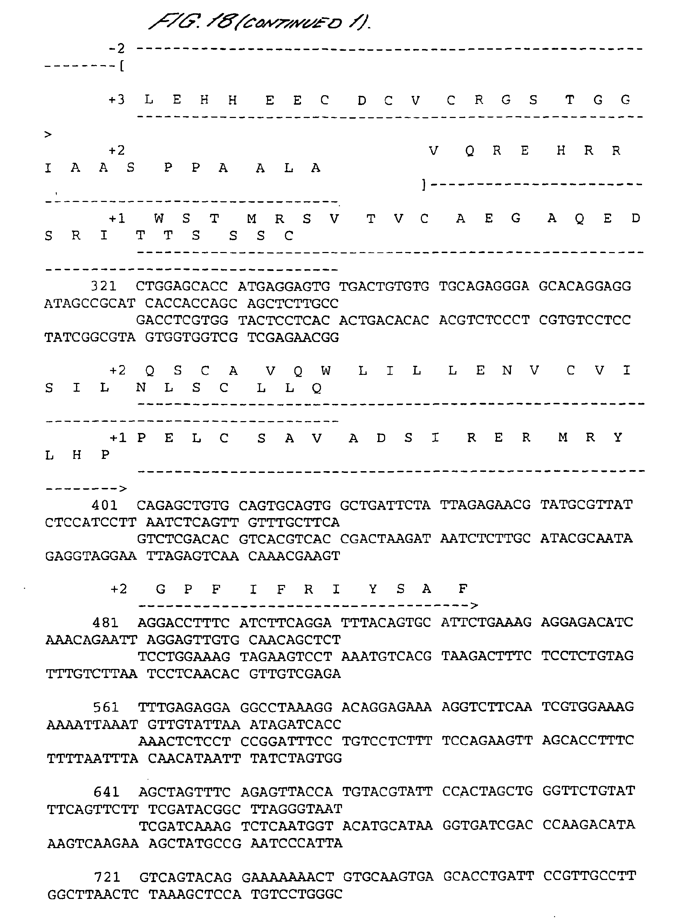

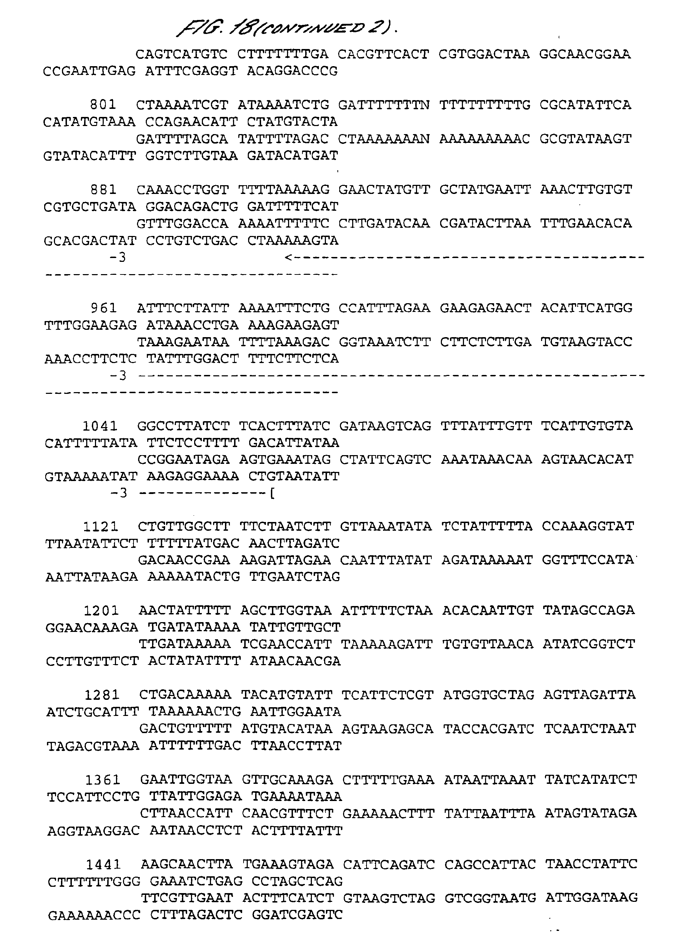

For expression in the baculovirus/insect cell system the DNA encoding the predicted mature VEGF-X polypeptide sequence was fused to a sequence encoding a signal derived from melittin, a secreted insect protein. An N-terminal 6His tag was also added to facilitate purification. The insert was then cloned into the baculovirus expression vector pFASTBAC. The DNA and polypeptide sequence of this construct is shown in figure 20. Infection of *Trichoplusia ni* Hi5 cells with this recombinant baculovirus results in the secretion of a protein of approximately 45K into the medium (data not shown).

*E. coli*

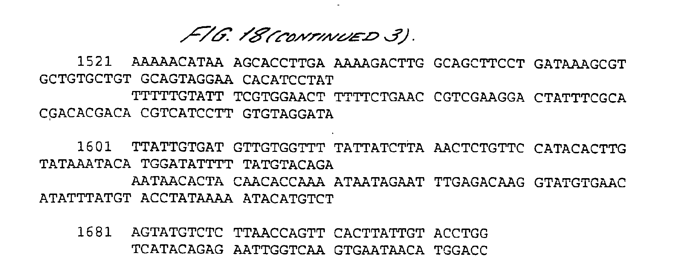

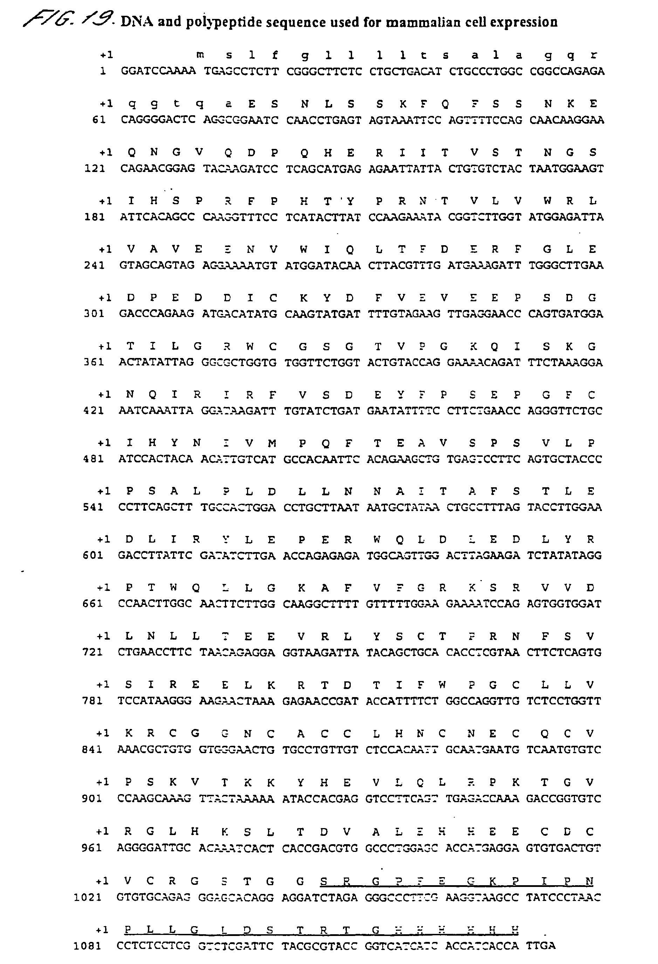

The coding region of VEGF-X has been cloned in a variety of ways for expression as a secreted protein in E.coli. A particularly useful expression clone carries an N-terminal fusion to the E.coli maltose-binding protein (MBP-derived from the expression vector pMAL-p2, New England Biolabs) and a C-terminal fusion to a 6His tag. The DNA and polypeptide sequence of this vector is shown in figure 21. Sequential purification of cell fractions on Ni-NTA resin and amylose resin allows the isolation of the expressed protein (see figure 22B).

**Expression of fragments**

VEGF

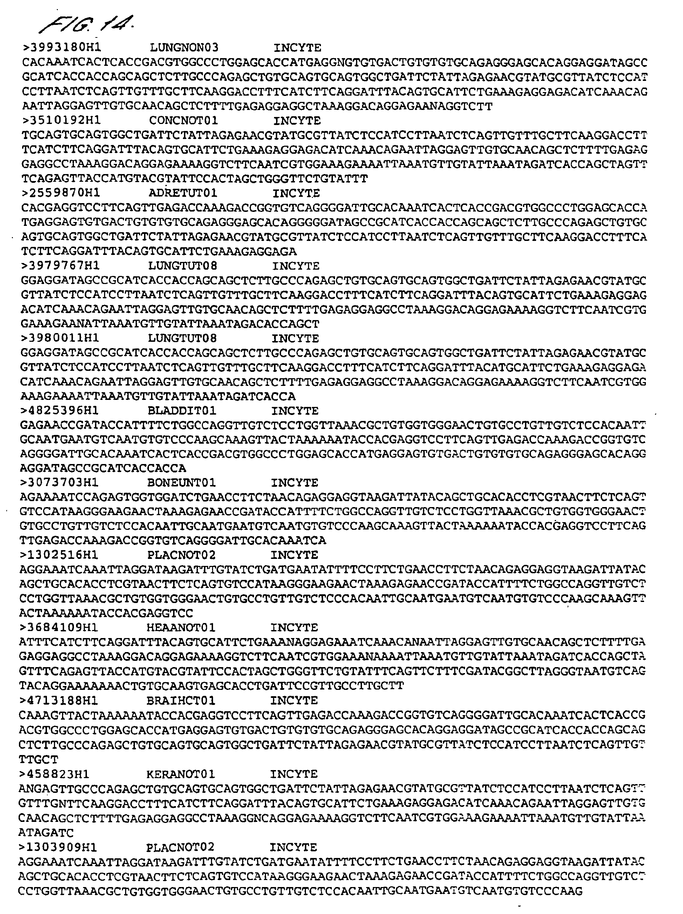

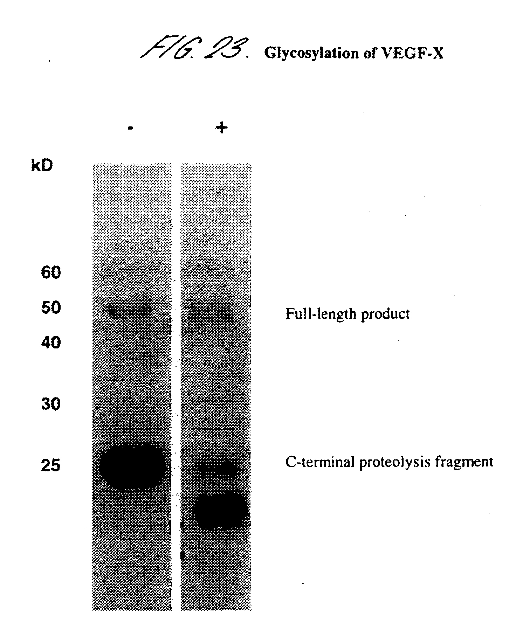

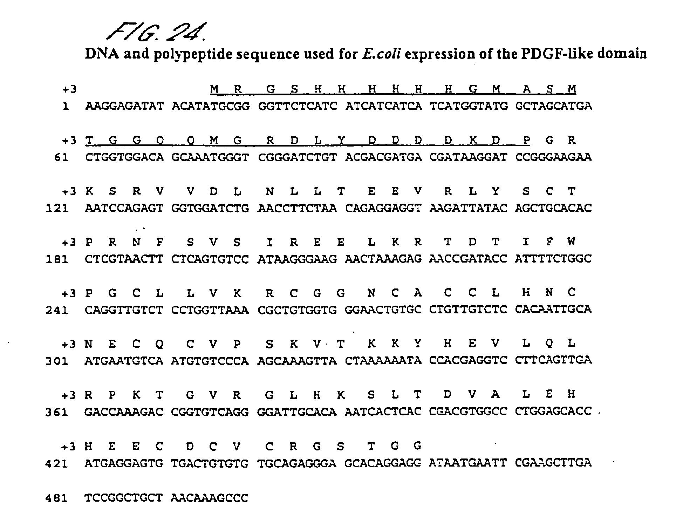

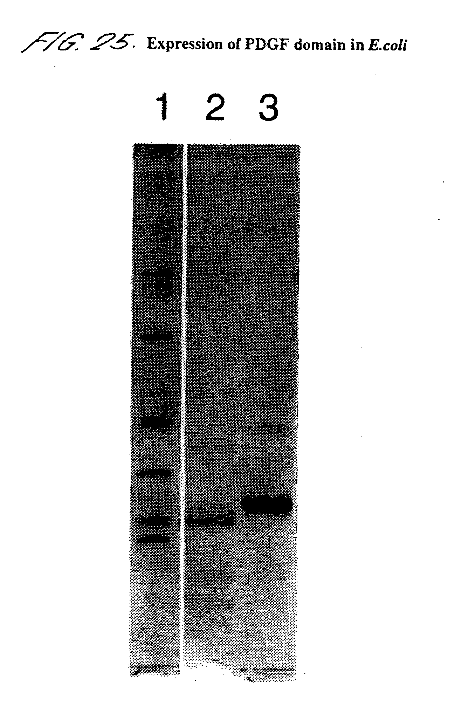

The VEGF domain of VEGF-X has been expressed in *E.coli.* Similar domains from VEGF-A (Christinger et al. (1996) PROTEINS: Structure, Function and Genetics 26, 353-357), and VEGF-D (Achen et al (1998) Proc. Natl. Acad. Sci USA 95, 548-553) have been shown to be capable of binding to the respective receptors. Expression of these domains was carried out using the bacterium E.coli. Additionally, the full-length protein was expressed using the baculovirus/insect cell expression system. The oligonucleotide primers which have been obtained for these experiments are shown in figure 13. The construct directed expression in the bacterial cytoplasm, and as expected the protein was produced in insoluble form in inclusion bodies (the DNA and polypeptide sequence used for PDGF domain expression is shown in figure 24). Inclusion bodies were washed, solubilized with urea and the protein purified under denaturing conditions, before refolding by dialysis to remove the urea. Soluble protein was obtained, but shows little evidence of the disulphide bond linked dimers seen with material derived from animal cells (figure 25, compare with figure 22A & B). It is not clear therefore whether this protein is correctly folded.

CUB

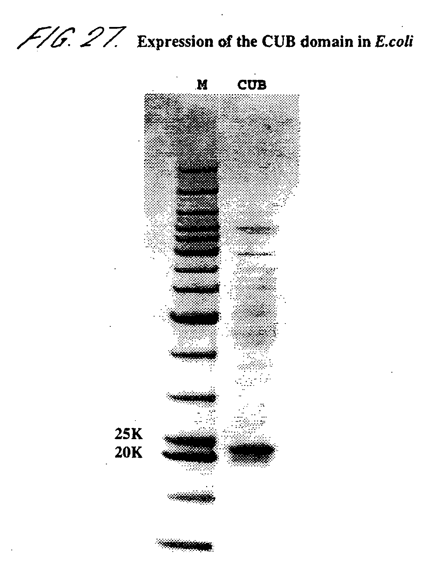

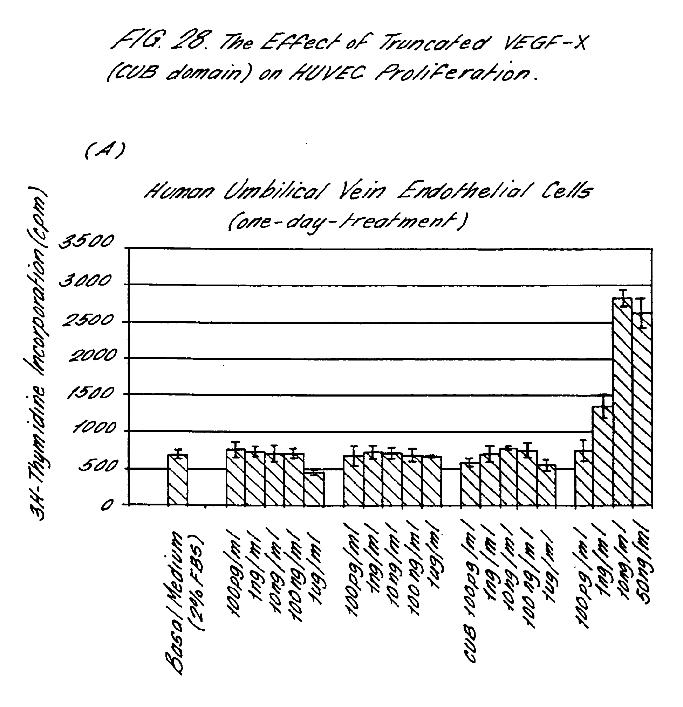

The CUB domain has been expressed as a soluble secreted protein in *E.coli* (figure 26). The protein was purified by binding to Ni-NTA resin (figure 27) and assayed for activity on HUVECs in an in-vitro proliferation assay.

**Properties of the VEGF-X protein**

The transient mammalian cell expression system described above has been used to generate full-length VEGF-X protein, as shown by antibody detection following Western blotting (see figure 22A).

Disulphide bond linked dimers

The other members of the PDGF family of growth factors, the PDGFs and VEGFs, all exist as dimers in which two monomers constituting the dimer are linked by interchain disulphide bonds. The x-ray structures of PDGF-BB (Oefner et al, 1992), and VEGF-A (Muller et al, 1997) are known and indicate that at least these two members of the family contain two interchain disulphide bonds. Practically this means that in SDS-PAGE analysis of these growth factors the presence of interchain disulphide bonds is shown by a large decrease in mobility in the absence of reducing agent (ie. the nonreduced dimer migrates more slowly through the gel than the reduced monomer). This effect was also expected for VEGF-X, and has been demonstrated for the material obtained from transient mammalian cell expression (figure 22A). In the case of the full length material produced in *E.coli* only some 10% of the total VEGF-X protein appears to be present as disulphide bond-linked dimers (figure 22B). However, these results provide evidence that the mammalian cell-derived protein is correctly folded, and that a portion of the E.coli-derived protein is too.

Glycosylation

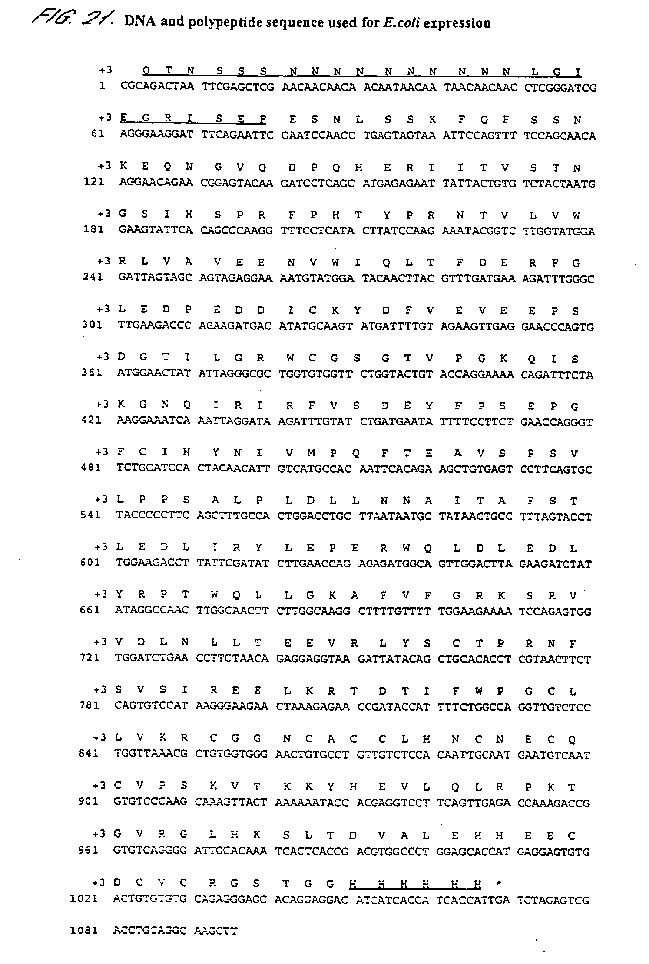

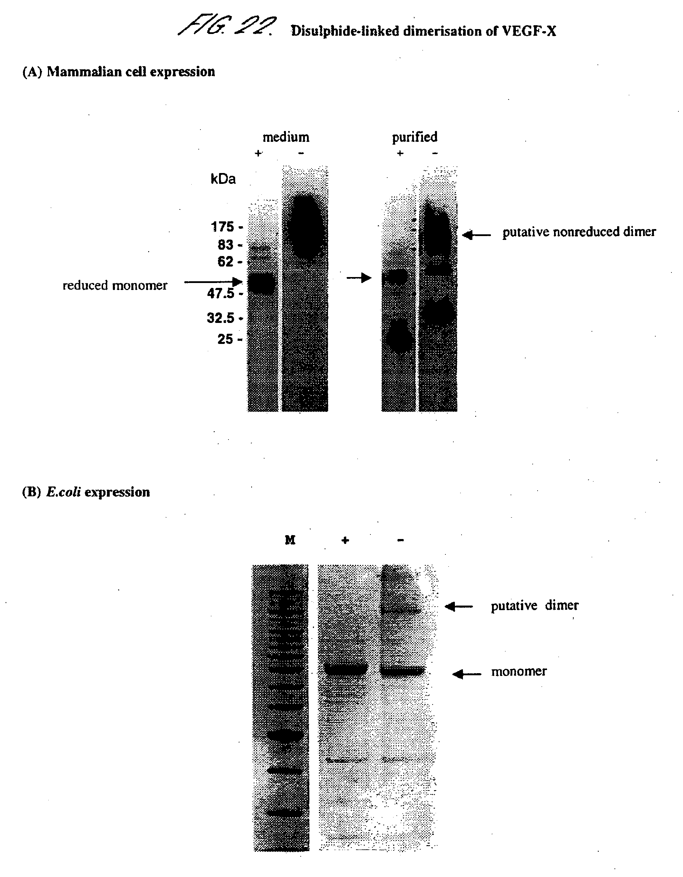

There are 3 predicted potential N-linked glycosylation sites within the VEGF-X protein: at residues 25, 55 and 254 of the polypeptide sequence. The predicted molecular mass of the mature VEGF-X protein is 40kDa, but SDS-PAGE and western blotting (detection via an introduced C-terminal epitope tag-see figure 19) of the full-length protein expressed in COS cells gives a band slightly larger than the expected size (45-50kDa) as well as one at 25kDa (figure 22A). This smaller band is presumed to be a C-terminal proteolysis fragment derived from the full-length molecule (controls from uninfected cells do not show this band), probably corresponding to a cleavage between the CUB and VEGF domains. EndoH treatment of the preparation gives a slight mobility change for the full-length protein (figure 23), but for the smaller VEGF domain fragment there is a clear change, indicating that the predicted glycosylation site within the VEGF domain at residue 254 is indeed glycosylated.

**Activity of proteins in cell-based assays**

Protein samples were tested for activity in cell proliferation, cell migration and in-vitro angiogenesis assays. Active samples can also be tested in the in vivo matrigel mouse model of angiogenesis.

Full-length VEGF-X protein

Conditioned medium derived from COS cells transiently expressing VEGF-X (see figure 22A) displayed no detectable activity in any of the assays. However, as VEGF-X protein could only be detected in this preparation by Western blotting, and not by Coomassie-staining of gels, it is clearly present at very low levels and this may be the reason for the observed lack of activity in the cell proliferation, migration or *in vitro* angiogenesis tests.

VEGF domain

The VEGF domain protein described above has been tested in cell proliferation (on a range of cell types), cell migration and in vitro angiogenesis assays and has failed to show activity in any of these tests. As suggested above, this may be due to incorrect folding of this protein.

CUB domain

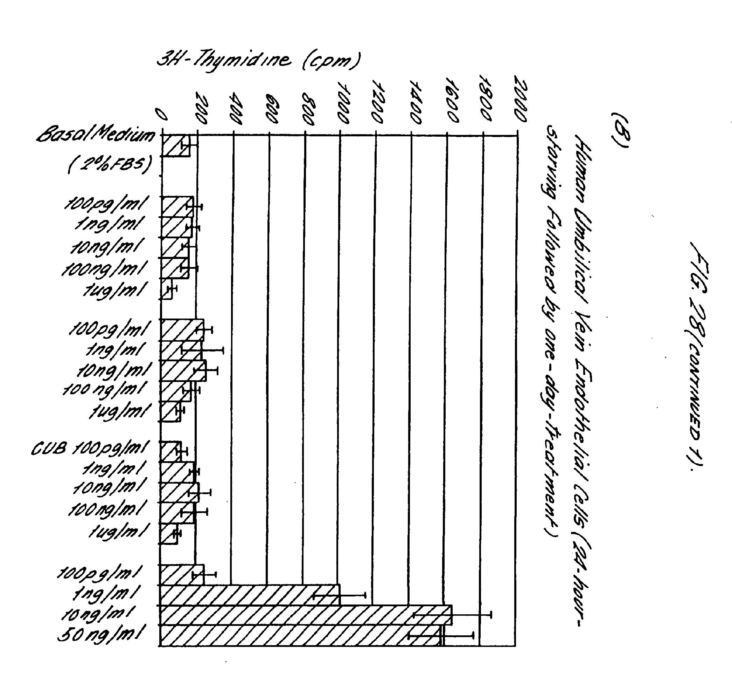

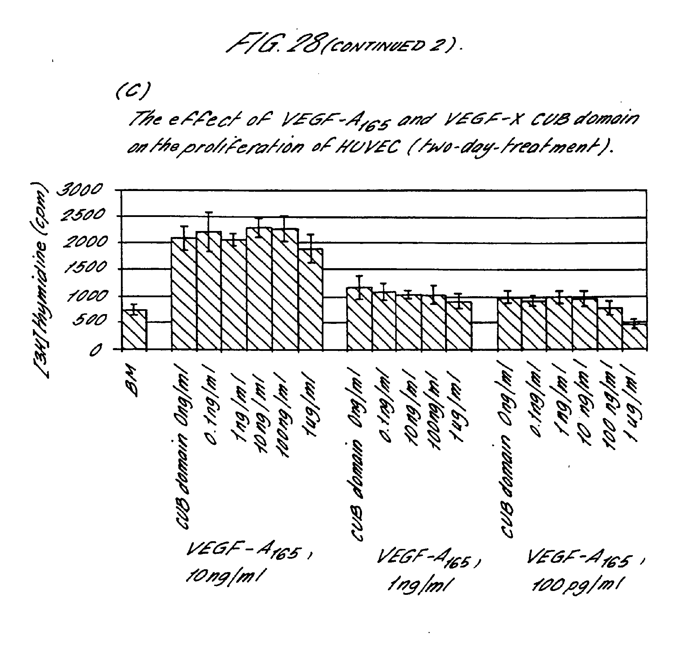

The CUB domain protein at the highest dose tested (1μg/ml) appears to inhibit proliferation of HUVECs in the absence of other stimulation (figure 28A & B). This effect is also seen following stimulation with the lowest VEGF-$A_{165}$ dose tested (1ng/ml- figure 28C). The CUB domain of VEGF-X therefore appears to show antiproliferative activity on HUVECs, even in the presence of low VEGF-$A_{165}$ doses.

**Tissue distribution of mRNA**

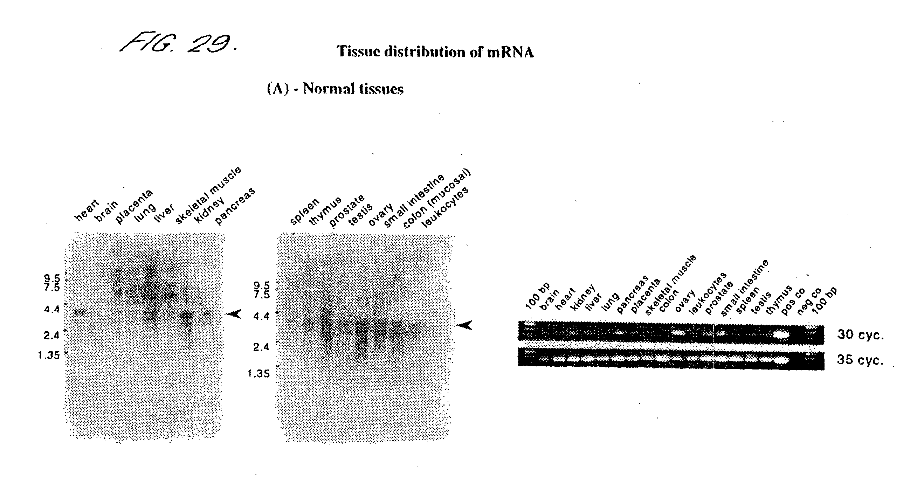

VEGF-A mRNA expression has been shown to be upregulated in a wide variety of human tumors (lung, breast, ovarian, colon, stomach, liver, pancreas, kidney, bladder and prostate- Takahashi et al, 1995). Tumor VEGF-A expression has been shown to correlate with tumor growth rate, microvascular density and tumor metastasis (Takahashi et al, 1995). It was thus of interest to examine the mRNA expression patterns of VEGF-X. Accordingly, Northern blot analysis of mRNA derived from different tissues has been carried out. The results indicate that although the VEGF-X mRNA is expressed at low levels, it is present in a wide range of tissues. PCR amplification of cDNA from a range of tissue sources supports this idea (figure 29A). The major mRNA species is approximately 3.1kb in size. There is no significant upregulation seen in tumour cell lines or in tumour tissues tested (figure 29B), with the possible exception of the cell lines GI-117 (lung carcinoma) and SaOS-2 (osteosarccma). The results of these initial tissue distribution studies do not, therefore, provide evidence for upregulation of VEGF-X in tumour growth, as is seen with VEGF-A.

**Genomic structure of the VEGF-X gene**

A genomic BAC clone covering the 3' part of the VEGF-X locus was isolated by hybridisation screening of nylon filters containing a human BAC library. Direct sequencing of this clone using oligonucleotide primers based on the VEGF-X cDNA sequence allowed the determination of several intron/exon boundaries (figure 30). Interestingly, the position of the mRNA splice site within the PDGF domain (nt 1187/1188 in figure 30B) is conserved with respect to those in the VEGF-A and VEGF-D genes (Tischer et al, 1991; Rocchigiani et al, 1998).

**Materials & Methods**

**PCR, Cloning, DNA sequence determination and BAC screening.**

All primers were purchased from Eurogentec, Seraing, Belgium. Insert-specific sequencing primers (15- and 16-mers) were designed by visual inspection of the DNA sequences. DNA was prepared on Qiagen-tip-20 columns or on Qiaquick spin columns (Qiagen GmbH, Düsseldorf, Germany) and recovered from the spin columns in 30μl Tris/EDTA-buffer (10mM TrisHCl pH 7.5, 1 mM EDTA (sodium salt)). Sequencing reactions were performed using BigDye™ Terminator Cycle Sequencing Ready Reaction kits (Perkin Elmer, ABI Division, Foster City, CA, USA) and were run on an Applied Biosystems 377 DNA sequencer (Perkin Elmer, ABI Division, Foster City, CA, USA). Polymerase chain reactions were carried out according to standard procedures (Ausubel et al, 1997). The PCR fragments were cloned into vectors pCR2.1 (Invitrogen, Carlsbad, CA. USA) or pCR-TOPO (Invitrogen,NL) according to the manufacturer's instructions. One of those vectors, plasmid VEGFX/pCR2.1 1TOPO FL was deposited on 1 March 1999 under Accession No. LMBP 3925. After sequence determination, the inserts were cloned into the desired expression vectors (see figures 19, 20, 21, 24 & 26).

A human genomic BAC library (Genome Systems, Inc., St Louis, MI, USA) was screened by hybridisation to oligonucleotides derived from the VEGF-X cDNA sequence, according to the manufacturer's instructions. BAC DNA was prepared using a Qiagen plasmid midi kit (Qiagen GmbH, Düsseldorf, Germany ) according to the manufacturer's instructions with some modifications (after clearing of the lysate from chromosomal DNA, supernatants from individual preparations were pooled on a single column (tip 100), and after the 70 % EtOH wash, the pellet was resuspended overnight at 4°C in 100 μl TE). 20-mer sequencing primers were designed based on the known cDNA sequence, and sequencing carried out as above.

**5' RACE**

In order to extend the cDNA clone in a 5' direction RACE reactions were carried out. Since it was known that the mRNA is present in placenta and skeletal muscle, Marathon-Ready™ placenta and skeletal muscle cDNAs were purchased from Clontech (Palo Alto CA. USA) and used according to the manufacturer's instructions. DNA fragments were excised from agarose gels, purified using QiaQuick PCR purification columns (Qiagen GmbH, Düsseldorf, Germany) and sequenced directly.

VEGF-X protein expression and purification DNA fragments encoding the desired protein sequences were amplified by PCR and cloned into appropriate expression vector systems.

For mammalian cell expression, the full coding sequence was cloned into the vector pcDNA6/V5-his (Invitrogen Leek, NL, see figure 19 for construct sequence), so as to add a C-terminal peptide tag to assist in detection and purification.

For insect cell expression the sequence of the predicted mature polypeptide was initially amplified to add an N-terminal 6His peptide and then cloned into the pMelBacB vector (Invitrogen, Leek, NL) to add an insect cell signal sequence. The entire insert was then PCR-cloned into the vector pFASTBAC-1 (LifeTechnologies, Gaithersburg, MA, USA) for construction of a baculovirus according to the manufacturer's instructions.

For E.coli expression, the coding region was PCR amplified to add a C-terminal 6His tag and then cloned into the vector pMAL-p2 (New England Biolabs, Beverly, MA, USA). The coding sequence of this construct is shown in figure 21). The protein was purified first on Ni-NTA resin (Qiagen GmbH, Düsseldorf, Germany) and then on amylose resin (New England Biolabs, Beverly, MA, USA), according to the manufacturers' instructions.

DNA sequences encoding the CUB and VEGF domain fragments of VEGF-X were PCR amplified and cloned into pET22b and pET21a (Novagen, Madison, WI, USA) respectively. The CUB domain protein was prepared either from the periplasm or medium of induced cultures by standard methods (Ausubel et al, 1997). The protein was initially purified by precipitation with 20% ammonium sulphate. After overnight dialysis vs 20mM Tris Hcl pH7.5, 100mM NaCl to remove ammonium sulphate, the protein was further purified on Ni-NTA resin as described above. The VEGF domain protein was expressed in insoluble form, and preparation of inclusion bodies was carried out using standard procedures (Ausubel et al 1997). Inclusion bodies were dissolved in 6M guanidine hydrochloride, 20mM Tris Hcl pH8.0, 200mM NaCl, 1mM 2-mercaptoethanol, and purified on Ni-NTA resin (Qiagen GmbH, Düsseldorf, Germany) according to the manufacturer's instructions. The protein was refolded by dialysis against several changes of buffer containing decreasing concentrations of denaturant.

Analysis of protein glycosylation was carried out using EndoH (Roche Molecular Biochemicals, Brussels, BE) according to the manufacturer's instructions.

**Cell Proliferation Assay**

Human umbilical vein endothelial cells (HUVECs) (Clonetics, San Diego, CA.) were trypsinized with 0.05% trypsin/0.53mM EDTA (Gibco, Gaithersburg, MD.), resuspended in the EGM-2(Clonetics, San Diego, CA.), counted, and distributed in a 96-well tissue culture plate at 5,000 cells/well. Following cell attachment and monolayer formation (16 hours), cells were stimulated with various concentrations of truncated VEGF-X (CUB domain or VEGF domain) or dilutions of culture supernatants of the full-length VEGF-X (COS 7 or HEK293) in DMEM (Gibco, Gaithersburg, MD.) containing 0.5% to 2% FBS (HyClone, Logan, UT) as indicated. For human fetal dermal fibroblasts (American Type Culture Collection, Rockville, MD.), the growth medium was replaced by DMEM containing 0.1% BSA (Sigma, St. Louise, MO.) with or without various concentrations of truncated VEGF-X proteins. For HCASMC (Clonetics, San Diego, CA.), the medium was replaced by DMEM containing 0.5% FBS. The cells were treated for a further 24 hr-72 hr. For the measurement of proliferation, the culture media were replaced with 100 μl of DMEM containing 5% FBS and 3μ Ci/ml of [3H]-thymidine (Amersham, Arlington Heights, IL.). Following pulse labeling, cells were fixed with methanol/acetic acid (3:1, vol/vol) for 1 hour at room temperature. The cells were washed twice with 250 μl/well of 80% methanol. The cells were solubilized in 0.05% trypsin (100μl/well) for 30 minutes then in 0.5% SDS (100 μl/well) for another 30 minutes. Aliquots of cell lysates (180 μl) were combined with 2 ml of scintillation cocktail (Fisher, Springfiled, NJ) and the radioactivity of cell lysates was measured using a liquid scintillation counter (Wallac 1409). In each case, samples were performed in quadruplicate.

**Chemotaxis Assay**

The chemotactic response of HUVECs was assayed using a 48-well modified Boyden chamber (NeuroProbe, Cabin John, MD.) and collagen-coated (0.1mg/ml type I collagen, Collaboratic Biomedical, Bedford, MA.) polycarbonate membrane filters with a pore diameter of 8 μm (NeuroProbe, Cabin John, MD.). Cell suspensions (15,000/well) were loaded to the upper part of the chemotaxis chamber and stimulated for 4 hours with rhVEGF$_{165}$ (0.1-10 ng/ml) (Calbiochem, San Diego, CA.) or various concentrations of truncated VEGF-X (PDGF domain). Cells remaining on the top of the membrane were removed. Migration was assessed by counting the number of cells that migrated to the lower side of the filter membrane. The membrane was fixed with 10% formaldehyde for 15 min, followed by staining with Gill's hemotoxylin III (Poly Scientific, Bay Shore, NY.). The assay was performed in triplicates and six independent high power fields per well were counted using a light microscope at 250 magnification. The results were expressed as the fold of unstimulated cells (EGM containing 0.1% BSA).

***In Vitro* Angiogenesis Assay**

*In vitro* angiogenesis in fibrin gels was quantitated using spheroids of human umbilical vein endothelial cells (Korff et al., 1998). To generate endothelial cell spheroids of defined size and cell number, a specific number of cells (-800 cells per spheroid) was suspended in EGM-2 culture medium containing 20% methylcellulose (Sigma, St. Louis, MO.), seeded into nonadherent round-bottom 96-well plates. All suspended cells in one well contributed to the formation of a single endothelial cell spheroid within 24 hours. A fibrin gel stock solution was prepared freshly prior to use by mixing 3mg/ml fibrinogen (Calbiochem, San Diego, CA.) in Medium 199 (Gibco, Gaithersburg, MD.). Assays were performed in 24-well culture plates. The 1ml fibrinogen stock was mixed with 50 HUVEC spheroids and the corresponding test substance including rh-VEGF$_{165}$ or various concentration of VEGF-X. The spheriod-containing fibrinogen was rapidly transferred into 24-well plates. Fifteen microliters of thrombin (100 NIH U/ml stock, Sigma, St. Louis, MO.) was added to the gel for the fibrin gel formation. The gel formation usually occurred within 30 seconds. After gel formation, Iml/well of Medium 199 supplemented with 20% FBS, 1mg/ml ε-aminocaproic acid (Calbiochem, San Diego, CA.) and antibiotics were added. The gel was incubated at 37°C (5%$CO_2$, 95% air, 100% humidity). After 3 days, *in vitro* angiogenesis was quantitated by measuring the length of the three longest capillary sprouts that had grown out of each spheroid (100X magnification), analyzing at least 10 spheroids per experimental group and experiment.

**Matrigel Mouse Assay**

The matrigel mouse assay is carried out as described by Passanti et al (1992).

**Analysis of VEGF-X gene expression by RT-PCR analysis.**

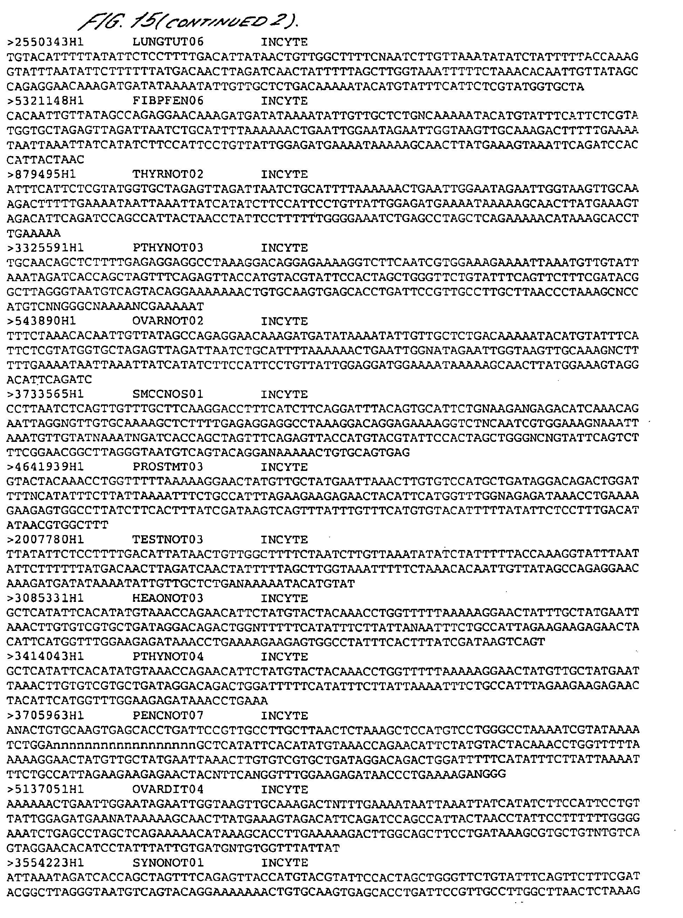

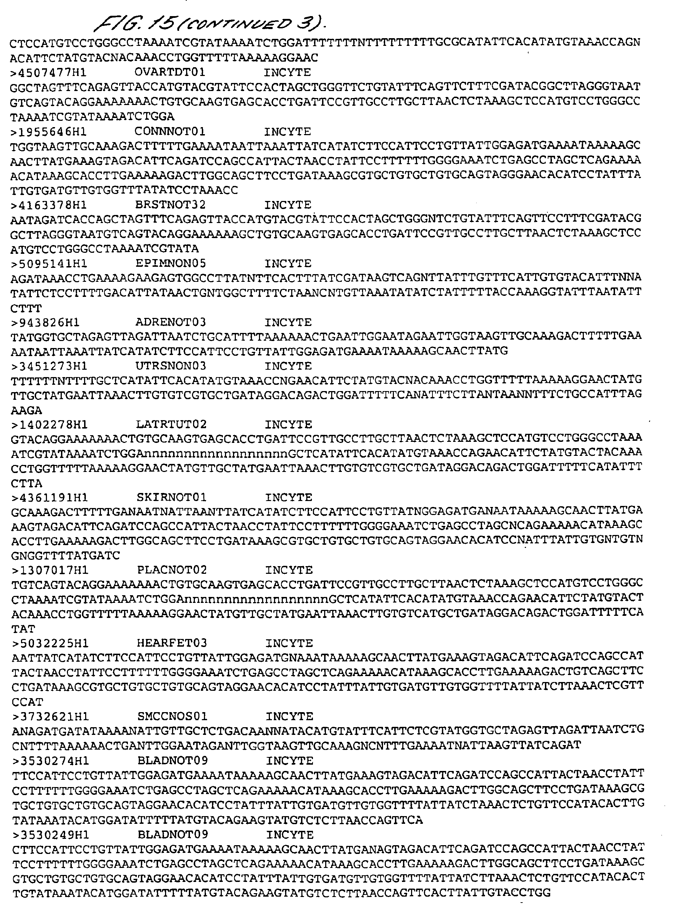

Oligonucleotide primers VEGF-E2 and VEGF-X14 (figure 16; figure 5) were used for the specific PCR amplification of a 350 bp fragment from VEGF-X. PCR amplifications were performed on human multiple tissue cDNA (MTC™) panels (Clontech human MTC panels I and II and human Tumor MTC panel) normalised to the mRNA expression levels of six different housekeeping genes. In addition, cDNA was made from different tumor cell cultures (Caco-2 colorectal adenocarcinoma; T-84 colorectal carcinoma; MCF-7 breast adenocarcinoma; T-47D breast ductal gland carcinoma; HT1080 bone fibrosarcoma; SaOS-2 osteosarcoma; SK-N-MC neuroblastoma; HepG2 hepatoblastoma; JURKAT T-cell leukemia and THP-1 myelomonocytic leukemia). For the preparation of tumor cell cDNA, cells were homogenised and total RNA prepared using the RNeasy Mini kit (Qiagen GmbH, Hilden, Germany) according to manufacturer's instructions. 1 μg of total RNA was reverse transcribed using oligo(dT)15 as a primer and 50 U of Expand™ Reverse Transcriptase (Boehringer Mannheim, Mannheim, Germany) according to the manufacturer's instructions. PCR reactions with VEGF-X-specific or glyceraldehyde-3-phosphate dehydrogenase (G3PDH)-specific primers were then performed on 1 μl of this cDNA. For all cDNAs, PCR reactions with VEGF-X specific primers were performed in a total volume of 50 μl, containing 5 μl (± 1 ng) of cDNA, 1x Advantage KlenTaq PCR reaction buffer, 0.2 mM dNTP, 250 nM of primers VEGF-E2 and VEGF-X14 and 1 μl of Advantage KlenTaq polymerase mix. Samples were heated to 95°C for 30 s and cycling was done for 30 s at 95°C and 30 s at 68°C for 25, 30 or 35 cycles. Control reactions using specific primers that amplify a 1 kb fragment of the housekeeping gene G3PDH were also performed according to the manufacturer's instructions.

**Northern blot analysis of VEGF-X.**

Northern blots containing 2 μg of poly(A)-rich RNA derived from different human tissues (Clontech Laboratories; MTN™ blot, MTN™ blot II and Cancer Cell Line MTN™ blot) were hybridized according to the manufacturers instructions

with a α-[$^{32}P$]-dCTP random-priming labelled (Multiprime labelling kit, Roche Diagnostics) 293 bp specific VEGF-X fragment (PinAI-StuI fragment including 92 bp of the 3' end coding region and 201 bp of the 3' untranslated region of VEGF-X). The blots were hybridized overnight at 68°C and final washes at high stringency were at 68°C in 0.1x SSC/ 0.1 % SDS. The membranes were autoradiographed for 1 to 3 days with intensifying screens.

**Full length VEGF-X**

The effect of full length VEGF-X on proliferation of HuVEC cells was determined by the $^{3}H$-Thymidine incorporation assay. HuVEC cells were serum starved for 24 hours prior to treatment with the full length VEGF-X at the concentration range from 100 pg/ml-10 μg/ml. There was no effect of VEGF-X at 100 pg/ml-10 ng/ml on endothelial cell proliferation. At the higher concentrations of FL-VEGF-X (100 ng/ml and 1 μg/ml) there was a marked inhibition of endothelial cell proliferation. This is probably due to the very high endotoxin level in the samples. The VEGF-X sample was purified in order to decrease the endotoxin level and is currently tested in the cell proliferation assay.

**The Summary from Testing the CUB Domain**

The effect of CUB domain on inhibition of HuVEC prolieration either serum- (2%), rh-VEGF or bFGF-stimulated, was assessed by the $^{3}H$-Thymidine incorporation assay. Cells were serum starved followed by the treatment with the CUB domain and various growth factors. Results showed that the CUB domain inhibited endothelial cell proliferation, either serum- (2%), rh-VEGF or bFGF-stimulated in a dose dependent manner with maximal inhibition at 10 μg/ml. There was approximately a 2-fold inhibition of proliferation (at 10 μg/ml) of cells stimulated with VEGF and bFGF and nearly a 5-fold inhibition of cells stimulated with serum (2%). Results with the LDH assay showed that there was no cytotoxicity associated with the inhibition of cell proliferation by the CUB domain.

Therefore, the N-terminus of the polypeptide from Figure 10 has been shown to possess a CUB domain. When database searches are carried out using the full-length coding sequence the best matches (i.e. for a BLAST search, those with the lowest probability score) are found with the CUB domain rather than with the VEGF-like domain. The best match from searching release 37 of the SWISSPROT database (Feb 1999) is to the CUB domain of a neuropilin from Xenopus laevis, and the matches to the CUB domains of human neuropilins 1 and 2 are also more significant than matches to the VEGFs.

This similarity is provocative, given the identification of neuropilin-1 and -2 as cellular receptors for the VEGF-A 165 (Stoker *et al.* 1998, reviewed in Neufeld et al. 1999). It is plausible therefore that VEGF-X could exert dual regulatory effects: via interaction with the tyrosine kinase VEGF-receptors mediated by the VEGF-like domain, as well as via interaction with VEGF isoforms or with the neurophilin receptors, mediated by the CUB domain.

To the best of our understanding the latter would be entirely novel, and searches on the most recent release of the Incyte database do not reveal any other proteins containing both CUB and VEGF-like domains. This arrangement of domains suggests possible positive or negative models of regulation:

*Positive*- the VEGF-like domain is able to interact productively with the tyrosine kinase VEGF receptors giving activation, and the CUB domain is able to interact productively with the neuropilin receptor giving activation.

*Negative*- the VEGF-like domain does not interact productively with the tyrosine kinase VEGF receptors, either preventing receptor dimerisation or blocking the VEGF binding sites. Further, the CUB domain does not interact productively with the neuropilin receptors, either preventing receptor activation or blocking the VEGF binding sites, or indeed by binding to VEGF isoforms and preventing their interaction with receptors.

TABLE 1

| ORIGINAL RESIDUE | EXEMPLARY SUBSTITUTIONS |
|---|---|
| ALA | SER, THR |
| ARG | LYS |
| ASN | HIS, SER |
| ASP | GLU, ASN |
| CYS | SER |
| GLN | ASN, HIS |
| GLU | ASP, GLU |
| GLY | ALA, SER |

(continued)

| ORIGINAL RESIDUE | EXEMPLARY SUBSTITUTIONS |
|---|---|
| HIS | ASN, GLN |
| ILE | LEU, VAL, THR |
| LEU | ILE, VAL |
| LYS | ARG, GLN, GLU, THR |
| MET | LEU, ILE, VAL |
| PHE | LEU, TYR |
| SER | THR, ALA, ASN |
| THR | SER, ALA |
| TRP | ARG, SER |
| TYR | PHE |
| VAL | ILE, LEU ALA |
| PRO | ALA |

**References**

1. Ausubel, FM, R Brent, RE Kingston, DD Moore, JG Seidman, JA Smith, K Struhl (Eds). (1997) Current Protocols in Molecular Biology, John Wiley and Sons.

2. von Heijne, G. (1986) Nucleic Acids Res. 14, 4683-4690.

3. Muller, YA, B Li, HW Christinger, JA Wells, BC Cunningham and AM de Vos. (1997) Vascular endothelial growth factor: crystal structure and functional mapping of the kinase domain receptor binding site. Proc. Natl. Acad. Sci USA 94, 7192-7197.

4. Korff, T and Augustic, H.G. (1998) Integration of endothelial cells in multicellular spheroids prevents apoptosis and induced differentiation. The Journal of Cell Biology. 143, 1341-1352

5. Christinger, HW, YA Muller, LT Berleau, BA Keyt, BC Cunningham, N Ferrara and AM de Vos. (1996) PROTEINS: Structure, Function and Genetics 26, 353-357.

6. Achen, MG, M Jeltsch, E Kukk, T Makinen, A Vitali, AF Wilks, K Alitalo and SA Stacker. (1998) Proc. Natl. Acad. Sci USA 95, 548-553.

7. Siemeister, G, B Schnurr, K Mohrs, C Schachtele, C Marme and G Martiny-Baron. (1996) Biochem. Biophys. Res. Commun. 222, 249-255.

8. Soker, S, S Takashima, HQ Miao, G Neufeld and M Klagsbrun (1998). Neuropilin-1 is expressed by endothelial and tumor cells as an isoform-specific receptor for vascular endothelial growth factor, Cell 92: 735-745.

9. Neufeld, G, T Cohen, S Gengrinovitch and Z Poltorak (1999). Vascular endothelial growth factor and its receptors, FASEB J. 13:9-22.

10. Oefner, C., D'Arcy, A., Winkler, F.K., Eggimann, B. and Hosang, M. (1992). Crystal structure of human platelet-derived growth factor BB. EMBO J. 11, 3921-3926.

11. Passanti, A.., Taylor, R.M., Pili, R., Guo, Y., Long, P.V., Haney, J.A., Pauly, R., Grant, D.S. and Martin, G.R. (1992) A simple, quantitative method for assessing angiogenesis and antiangiogenic agents using reconstituted basement membrane, heparin and fibroblast growth factor. Laboratory Investigation, 67, 519-528.

12. Rocchigiani, M., Lestingi, M., Luddi, A., Orlandini, M., Franco, B., Rossi, E., Ballabio, A., Zuffardi, 0. and Oliviero, S. (1990). Human FIGF: cloning, gene structure, and mapping to chromosome Xp22.1 between the PIGA and the GRPR genes. Genomics, 47, 207-216.

13. Takahashi, Y., Kitadai, Y., Bucana, C.D., Cleary, K.R. and Ellis, L.M. (1995). Expression of vascular endothelial growth factor and its receptor, KDR, correlates with vascularity, metastasis and proliferation of human colon cancer. Cancer Research, 55: 3964-3968.

14. Tischer, E., Mitchell, R., Hartman, T., Silva, M., Gospodarowicz, D., Fiddes, J.C. and Abraham, J.A. (1991). The human gene for vascular endothelial growth factor: Multiple protein forms are encoded through alternative exon splicing. J. Biol. Chem. 266, 11947-11954.

**SEQUENCE LISTING**

| | |
|---|---|
| Sequence ID No 1 | corresponds to the amino acid sequence from position 23 to 345 of the amino acid sequence illustrated in Figure 10. |
| Sequence ID No.2 | is the amino acid sequence illustrated in Figure 10. |
| Sequence ID No 3 | corresponds to the sequence from position 257 to 1291 of the nucleotide sequence illustrated in Figure 9. |
| Sequence ID No 4 | corresponds to the polynucleotide sequence of VEGFX1 illustrated in Figure 3. |
| Sequence ID No 5 | corresponds to the polynucleotide sequence of VEGFX2 illustrated in Figure 3. |
| Sequence ID No 6 | corresponds to the polynucleotide sequence of VEGFX3 illustrated in Figure 3. |
| Sequence ID No 7 | corresponds to the polynucleotide sequence of VEGFX4 illustrated in Figure 3. |
| Sequence ID No 8 | corresponds to the polynucleotide sequence of VEGFX5 illustrated in Figure 3. |
| Sequence ID No 9 | corresponds to the polynucleotide sequence of VEGFX6 illustrated in Figure 3. |
| Sequence ID No 10 | corresponds to the polynucleotide sequence of VEGFX7 illustrated in Figure 3. |
| Sequence ID No 11 | corresponds to the polynucleotide sequence of VEGFX8 illustrated in Figure 3. |
| Sequence ID No 12 | corresponds to the polynucleotide sequence of VEGFX9 illustrated in Figure 3. |
| Sequence ID No 13 | corresponds to the polynucleotide sequence of VEGFX10 illustrated in Figure 3. |
| Sequence ID No 14 | corresponds to the polynucleotide sequence of VEGFX11 illustrated in Figure 4. |
| Sequence ID No 15 | corresponds to the polynucleotide sequence of VEGFX12 illustrated in Figure 4. |
| Sequence ID No 16 | corresponds to the polynucleotide sequence of VEGFX13 illustrated in Figure 4. |
| Sequence ID No 17 | corresponds to the polynucleotide sequence of VEGFX14 illustrated in Figure 4. |
| Sequence ID No 18 | corresponds to the polynucleotide sequence 5'-1 in Figure 8. |
| Sequence ID No 19 | corresponds to the polynucleotide sequence 5'-2 in Figure 8. |
| Sequence ID No 20 | corresponds to the polynucleotide sequence of VEGFX6 illustrated in Figure 13. |
| Sequence ID No 21 | corresponds to the polynucleotide sequence of VEGFX7 illustrated in Figure 13. |

Sequence ID No 22 corresponds to the polynucleotide sequence of VEGFX8 illustrated in Figure 13.

Sequence ID No 23 corresponds to the polynucleotide sequence of VEGFX9 illustrated in Figure 13.

Sequence ID No 24 corresponds to the polynucleotide sequence of VEGBAC1 illustrated in Figure 13.

Sequence ID No 25 corresponds to the polynucleotide sequence of VEGBAC2 illustrated in Figure 13.

Sequence ID No 26 corresponds to a polypeptide having the amino acid sequence from amino acid position 40 to 150 of the sequence of Figure 10.

Sequence ID No 27 corresponds to a polypeptide having the amino acid sequence illustrated in Figure 26.

Sequence ID No 28 corresponds to the sequence from position 5 to 508 of the nucleotide sequence illustrated in Figure 26.

Sequence ID No 29 corresponds to the nucleotide sequence from position 5 to 508 of the nucleotide sequence illustrated in Figure 26.

Sequence ID No 30 corresponds to the sequence from position 214 to 345 of the nucleotide sequence illustrated in Figure 10.

**Claims**

1. A nucleic acid molecule encoding a CUB domain polypeptide, consisting of the N-terminal part of the VEGF-X protein of SEQ. ID. No. 2 and wherein said polypeptide is able to inhibit stimulation of proliferation of HUVECs induced by either VEGF or bFGF.

2. A nucleic acid molecule according to claim 1, said nucleic acid molecule encoding a polypeptide comprising the amino acid sequence from position 40 to 150 of the sequence of SEQ ID No. 2.

3. A nucleic acid molecule according to claims 1 or 2, said nucleic acid molecule encoding a polypeptide comprising the amino acid sequence of SEQ ID No. 27.

4. A nucleic acid molecule according to any of claims 1 to 3, comprising the nucleotide sequence of SEQ ID No. 28.

5. A nucleic acid molecule according to any of claims 1 to 4 comprising the nucleotide sequence of SEQ ID No. 29.

6. An expression vector comprising a nucleic acid molecule according to any of claims 1 to 5.

7. A host cell transformed or transfected with an expression vector according to claim 6.

8. A protein expressed by the cell according to claim 7

9. A nucleic acid molecule according to any of claims 1 to 5 for use as a medicament.

10. Use of a nucleic acid molecule according to any of claims 1 to 5 in the manufacture of a medicament for treatment of disease conditions associated with inappropriate angiogenesis such as tumour or cancer growth, retinopathy, osteoarthritis or psoriasis.

11. A polypeptide encoded by a nucleic acid molecule as claimed in any of claims 1 to 5 for use as a medicament.

12. A polypeptide consisting of the amino acid sequence from position 40 to 150 of the sequence of SEQ ID No.2 for use as a medicament.

13. Use or a polypeptide consisting of the amino acid sequence from position 40 to 150 of the sequence of SEQ ID No. 2 in the manufacture of a medicament for the treatment of disease conditions associated with inappropriate angiogenesis such as tumour growth, retinopathy, osteoarthritis or psoriasis.

14. use of polypeptide encoded by a nucleic acid molecule as claimed in any of claims 1 to 5, in the manufacturing of a medicament for the treatment of disease conditions associated with inappropriate angiogenesis such as tumour growth, retinopathy, osteoarthritis or psoriasis.

15. An antisense molecule capable of hybridising to a molecule according to any of claims 1 to 5 under high stringency conditions.

16. An antibody capable of binding to a protein according to claim 8 or an epitope thereof.

17. A pharmaceutical composition comprising an antibody according to claim 16 together with a pharmaceutically acceptable carrier diluent or excipient therefor.

**Patentansprüche**

1. Nukleinsäuremolekül, codierend für ein CUB-Domäne-Polypeptid, bestehend aus dem N-terminalen Teil des VEGF-X-Proteins der SEQ ID Nr. 2, wobei das Polypeptid in der Lage ist, die durch VEGF oder bFGF induzierte Stimulierung der Proliferation von HUVECs zu hemmen.

2. Nukleinsäuremolekül nach Anspruch 1, wobei das Nukleinsäuremolekül für ein Polypeptid mit der Aminosäuresequenz von Position 40 bis 150 der Sequenz der SEQ ID Nr. 2 codiert.

3. Nukleinsäuremolekül nach Anspruch 1 oder 2, wobei das Nukleinsäuremolekül für ein Polypeptid mit der Aminosäuresequenz der SEQ ID Nr. 27 codiert.

4. Nukleinsäuremolekül nach einem der Ansprüche 1 bis 3, umfassend die Nukleotidsequenz der SEQ ID Nr. 28.

5. Nukleinsäuremolekül nach einem der Ansprüche 1 bis 4, umfassend die Nukleotidsequenz der SEQ ID Nr. 29.

6. Expressionsvektor, umfassend ein Nukleinsäuremolekül nach einem der Ansprüche 1 bis 5.

7. Wirtszelle, transformiert oder transfiziert mit einem Expressionsvektor nach Anspruch 6.

8. Protein, exprimiert von der Zelle nach Anspruch 7.

9. Nukleinsäuremolekül nach einem der Ansprüche 1 bis 5 zur Verwendung als Arzneimittel.

10. Verwendung eines Nukleinsäuremoleküls nach einem der Ansprüche 1 bis 5 bei der Herstellung eines Arzneimittels zur Behandlung von Krankheitszuständen im Zusammenhang mit unangemessener Angiogenese, wie beispielsweise Tumor- oder Krebswachstum, Retinopathie, Osteoarthritis oder Psoriasis.

11. Polypeptid, codiert von einem Nukleinsäuremolekül gemäß einem der Ansprüche 1 bis 5 zur Verwendung als Arzneimittel.

12. Polypeptid; bestehend aus der Aminosäuresequenz von Position 40 bis 150 der Sequenz der SEQ ID Nr. 2, zur Verwendung als Arzneimittel.

13. Verwendung eines Polypeptids, bestehend aus der Aminosäuresequenz von Position 40 bis 150 der Sequenz der SEQ ID Nr. 2, bei der Herstellung eines Arzneimittels zur Behandlung von Krankheitszuständen im Zusammenhang mit unangemessener Angiogenese, wie beispielsweise Tumorwachstum, Retinopathie, Osteoarthritis oder Psoriasis.

14. Verwendung des von einem Nukleinsäuremolekül gemäß einem der Ansprüche 1 bis 5 codierten Polypeptids bei der Herstellung eines Arzneimittels zur Behandlung von Krankheitszuständen im Zusammenhang mit unangemessener Angiogenese, wie beispielsweise Tumorwachstum, Retinopathie; Osteoarthritis oder Psoriasis.

**15.** Antisense-Molekül, fähig zur Hybridisierung an ein Molekül nach einem der Ansprüche 1 bis 5 unter Bedingungen mit hoher Stringenz.

**16.** Antikörper, fähig zur Bindung an ein Protein nach Anspruch 8 oder ein Epitop davon.

**17.** Pharmazeutische Zusammensetzung, umfassend einen Antikörper nach Anspruch 16 zusammen mit einem pharmazeutisch unbedenklichen Trägerverdünnungsmittel oder Hilfsstoff dafür.

**Revendications**

**1.** Molécule d'acide nucléique codant pour un polypeptide à domaine CUB, constitué de la partie N-terminale de la protéine VEGF-X de SEQ ID NO : 2, ledit polypeptide étant capable d'inhiber la stimulation de la prolifération des cellules HUVEC induite soit par VEGF soit par bFGF.

**2.** Molécule d'acide nucléique selon la revendication 1, ladite molécule d'acide nucléique codant pour un polypeptide comprenant la séquence d'acides aminés de la position 40 à 150 de la séquence de SEQ ID NO : 2.

**3.** Molécule d'acide nucléique selon les revendications 1 ou 2, ladite molécule d'acide nucléique codant pour un polypeptide comprenant la séquence d'acides aminés de SEQ ID NO : 27.

**4.** Molécule d'acide nucléique selon l'une quelconque des revendications 1 à 3, comprenant la séquence nucléotidique de SEQ ID NO : 28.

**5.** Molécule d'acide nucléique selon l'une quelconque des revendications 1 à 4, comprenant la séquence nucléotidique de SEQ ID NO : 29.

**6.** Vecteur d'expression comprenant une molécule d'acide nucléique selon l'une quelconque des revendications 1 à 5.

**7.** Cellule hôte transformée par ou transfectée avec un vecteur d'expression selon la revendication 6.

**8.** Protéine exprimée par la cellule selon la revendication 7.

**9.** Molécule d'acide nucléique selon l'une quelconque des revendications 1 à 5 destinée à être utilisée comme médicament.

**10.** Utilisation d'une molécule d'acide nucléique selon l'une quelconque des revendications 1 à 5, dans la fabrication d'un médicament destiné au traitement d'états pathologiques associés à une angiogenèse inappropriée tels que la croissance tumorale ou cancéreuse, la rétinopathie, l'ostéoarthrite ou le psoriasis.

**11.** Polypeptide codé par une molécule d'acide nucléique telle que revendiquée dans l'une quelconque des revendications 1 à 5, destiné à être utilisé comme médicament.

**12.** Polypeptide constitué de la séquence d'acides aminés de la position 40 à 150 de la séquence de SEQ ID NO : 2, destiné à être utilisé comme médicament.

**13.** Utilisation d'un polypeptide constitué de la séquence d'acides aminés de la position 40 à 150 de la séquence de SEQ ID NO : 2, dans la fabrication d'un médicament destiné au traitement d'états pathologiques associés à une angiogenèse inappropriée tels que la croissance tumorale, la rétinopathie, l'ostéoarthrite ou le psoriasis.

**14.** Utilisation d'un polypeptide codé par une molécule d'acide nucléique telle que revendiquée dans l'une quelconque des revendications 1 à 5, dans la fabrication d'un médicament destiné au traitement d'états pathologiques associés à une angiogenèse inappropriée tels que la croissance tumorale, la rétinopathie, l'ostéoarthrite ou le psoriasis.

**15.** Molécule antisens susceptible de s'hybrider à une molécule selon l'une quelconque des revendications 1 à 5, dans des conditions de forte stringence.

16. Anticorps susceptible de se lier à une protéine selon la revendication 8 ou à un épitope de celle-ci.

17. Composition pharmaceutique comprenant un anticorps selon la revendication 16 conjointement avec un support, diluant ou excipient pharmaceutiquement acceptable pour celle-ci.

FIG. 1

```
  1  AAAATGTATG GATACAACTT ACGTTTGATG AAAGATTTGG GCTTGAAGAC CCAGAAGATG
     TTTTACATAC CTATGTTGAA TGCAAACTAC TTTCTAAACC CGAACTTCTG GGTCTTCTAC

 61  ACATATGCAA GTATGATTTT GTAGAAGTTG AGGAACCCAG TGATGGAACT ATATTAGGGC
     TGTATACGTT CATACTAAAA CATCTTCAAC TCCTTGGGTC ACTACCTTGA TATAATCCCG

121  GCTGGTGTGG TTCTGGTACT GTACCAGGAA AACAGATTTC TAAAGGAAAT CAAATTAGGA
     CGACCACACC AAGACCATGA CATGGTCCTT TTGTCTAAAG ATTTCCTTTA GTTTAATCCT

 +1                      MetAsn IlePheLeu LeuAsnLeuLeu ThrGluGlu ValArgLeu
                         ]-----------------------------------------------
181  TAAGATTTGT ATCTGATGAA TATTTTCCTT CTGAACCTTC TAACAGAGGA GGTAAGATTA
     ATTCTAAACA TAGACTACTT ATAAAAGGAA GACTTGGAAG ATTGTCTCCT CCATTCTAAT

 +1  TyrSerCysThr ProArgAsn PheSerVal SerIleArgGlu GluLeuLys ArgThrAsp
     ----------------------------------------------------------------
241  TACAGCTGCA CACCTCGTAA CTTCTCAGTG TCCATAAGGG AAGAACTAAA GAGAACCGAT
     ATGTCGACGT GTGGAGCATT GAAGAGTCAC AGGTATTCCC TTCTTGATTT CTCTTGGCTA

 +1  ThrIlePheTrp ProGlyCys LeuLeuVal LysArgCysGly GlyAsnCys AlaCysCys
     ----------------------------------------------------------------
301  ACCATTTTCT GGCCAGGTTG TCTCCTGGTT AAACGCTGTG GTGGGAACTG TGCCTGTTGT
     TGGTAAAAGA CCGGTCCAAC AGAGGACCAA TTTGCGACAC CACCCTTGAC ACGGACAACA

 +1  LeuHisAsnCys AsnGluCys GlnCysVal ProSerLysVal ThrLysLys TyrHisGlu
     ----------------------------------------------------------------
361  CTCCACAATT GCAATGAATG TCAATGTGTC CCAAGCAAAG TTACTAAAAA ATACCACGAG
     GAGGTGTTAA CGTTACTTAC AGTTACACAG GGTTCGTTTC AATGATTTTT TATGGTGCTC

 +1  ValLeuGlnLeu ArgProLys ThrGlyVal ArgGlyLeuHis LysSerLeu ThrAspVal
     ----------------------------------------------------------------
421  GTCCTTCAGT TGAGACCAAA GACCGGTGTC AGGGGATTGC ACAAATCACT CACCGACGTG
     CAGGAAGTCA ACTCTGGTTT CTGGCCACAG TCCCCTAACG TGTTTAGTGA GTGGCTGCAC

 +1  AlaLeuGluHis HisGluGlu CysAspCys ValCysArgGly SerThrGly Gly
     ----------------------------------------------------------->
481  GCCCTGGAGC ACCATGAGGA GTGTGACTGT GTGTGCAGAG GGAGCACAGG AGGATAGCCG
     CGGGACCTCG TGGTACTCCT CACACTGACA CACACGTCTC CCTCGTGTCC TCCTATCGGC

541  CATCACCACC AGCAGCTCTT GCCCAGAGCT GTGCAGTGCA GTGGCTGATT CTATTAGAGA
     GTAGTGGTGG TCGTCGAGAA CGGGTCTCGA CACGTCACGT CACCGACTAA GATAATCTCT

601  ACGTATGCGT TATCTCCATC CTTAATCTCA GTTGTTTGCT TCAAGGACCT TTCATCTTCA
     TGCATACGCA ATAGAGGTAG GAATTAGAGT CAACAAACGA AGTTCCTGGA AAGTAGAAGT

661  GGATTTACAG TGCATTCTGA AAGAGGAGAC ATCAAACAGA ATTAGGAGTT GTGCAACAGC
     CCTAAATGTC ACGTAAGACT TTCTCCTCTG TAGTTTGTCT TAATCCTCAA CACGTTGTCG

721  TCTTTTGAGA GGAGGCCTAA AGGACAGGAG AAAAGGTCTT CAATCGTGGA AAGAAAATTA
     AGAAAACTCT CCTCCGGATT TCCTGTCCTC TTTTCCAGAA GTTAGCACCT TTCTTTTAAT

781  AATGTTGTAT TAAATAGATC ACCAGCTAGT TTCAGAGTTA CCATGTACGT ATTCCACTAG
     TTACAACATA ATTTATCTAG TGGTCGATCA AAGTCTCAAT GGTACATGCA TAAGGTGATC
```

FIG. 1 (CONTINUED).

841 CTGGGTTCTG TATTTCAGTT CTTTCGATAC GGCTTAGGGT AATGTCAGTA CAGGAAAAAA
GACCCAAGAC ATAAAGTCAA GAAAGCTATG CCGAATCCCA TTACAGTCAT GTCCTTTTTT

901 ACTGTGCAAG TGAGCACCTG ATTCCGTTGC CTTGCTTAAC TCTAAAGCTC CATGTCCTGG
TGACACGTTC ACTCGTGGAC TAAGGCAACG GAACGAATTG AGATTTCGAG GTACAGGACC

961 GCCTAAAATC GTATAAAATC TGGATTTTTT TTTTTTTTTT TGCTCATATT CACATATGTA
CGGATTTTAG CATATTTTAG ACCTAAAAAA AAAAAAAAAA ACGAGTATAA GTGTATACAT

1021 AACCAGAACA TTCTATGTAC TACAAACCTG GTTTTTAAAA AGGAACTATG TTGCTATGAA
TTGGTCTTGT AAGATACATG ATGTTTGGAC CAAAAATTTT TCCTTGATAC AACGATACTT

1081 TTAAACTTGT GTCGTGCTGA TAGGACAGAC TGGATTTTTC ATATTTCTTA TTAAAATTTC
AATTTGAACA CAGCACGACT ATCCTGTCTG ACCTAAAAAG TATAAAGAAT AATTTTAAAG

1141 TGCCATTTAG AAGAAGAGAA CTACATTCAT GGTTTGGAAG AGATAAACCT GAAAAGAAGA
ACGGTAAATC TTCTTCTCTT GATGTAAGTA CCAAACCTTC TCTATTTGGA CTTTTCTTCT

1201 GTGGCCTTAT CTTCACTTTA TCGATAAGTC AGTTTATTTG TTTCATTGTG TACATTTTTA
CACCGGAATA GAAGTGAAAT AGCTATTCAG TCAAATAAAC AAAGTAACAC ATGTAAAAAT

1261 TATTCTCCTT TTGACATTAT AACTGTTGGC TTTTCTAATC TTGTTAAATA TATCTATTTT
ATAAGAGGAA AACTGTAATA TTGACAACCG AAAAGATTAG AACAATTTAT ATAGATAAAA

1321 TACCAAAGGT ATTTAATATT CTTTTTTATG ACAACTTAGA TCAACTATTT TTAGCTTGGT
ATGGTTTCCA TAAATTATAA GAAAAAATAC TGTTGAATCT AGTTGATAAA AATCGAACCA

1381 AAATTTTTCT AAACACAATT GTTATAGCCA GAGGAACAAA GATGATATAA AATATTGTTG
TTTAAAAAGA TTTGTGTTAA CAATATCGGT CTCCTTGTTT CTACTATATT TTATAACAAC

1441 CTCTGACAAA AATACATGTA TTTCATTCTC GTATGGTGCT AGAGTTAGAT TAATCTGCAT
GAGACTGTTT TTATGTACAT AAAGTAAGAG CATACCACGA TCTCAATCTA ATTAGACGTA

1501 TTTAAAAAAC TGAATTGGAA TAGAATTGGT AAGTTGCAAA GACTTTTTGA AAATAATTAA
AAATTTTTTG ACTTAACCTT ATCTTAACCA TTCAACGTTT CTGAAAAACT TTTATTAATT

1561 ATTATCATAT CTTCCATTCC TGTTATTGGA GATGAAAATA AAAAGCAACT TATGAAAGTA
TAATAGTATA GAAGGTAAGG ACAATAACCT CTACTTTTAT TTTTCGTTGA ATACTTTCAT

1621 GACATTCAGA TCCAGCCATT ACTAACCTAT TCCTTTTTTG GGGAAATCTG AGCCTAGCTC
CTGTAAGTCT AGGTCGGTAA TGATTGGATA AGGAAAAAAC CCCTTTAGAC TCGGATCGAG

1681 AGAAAAACAT AAAGCACCTT GAAAAAGACT TGGCAGCTTC CTGATAAAGC GTGCTGTGCT
TCTTTTTGTA TTTCGTGGAA CTTTTTCTGA ACCGTCGAAG GACTATTTCG CACGACACGA

1741 GTGCAGTAGG AACACATCCT ATTTATTGTG ATGTTGTGGT TTTATTATCT TAAACTCTGT
CACGTCATCC TTGTGTAGGA TAAATAACAC TACAACACCA AAATAATAGA ATTTGAGACA

1801 TCCATACACT TGTATAAATA CATGGATATT TTTATGTACA GAAGTATGTC TCTTAACCAG
AGGTATGTGA ACATATTTAT GTACCTATAA AAATACATGT CTTCATACAG AGAATTGGTC

1861 TTCACTTATT GTACCTGG
AAGTGAATAA CATGGACC

**FIG. 2. Predicted VEGF-like protein encoded by Incyte contig of 8/12/98**

```
  1  MNIFLLNLLT EEVRLYSCTP RNFSVSIREE LKRTDTIFWP GCLLVKRCGG
 51  NCACCLHNCN ECQCVPSKVT KKYHEVLQLR PKTGVRGLHK SLTDVALEHH
101  EECDCVCRGS TGG
```

**FIG. 3. PCR primers for cloning VEGF-X**

| | |
|---|---|
| vegfX1 | AAAATGTATGGATACAACTTAC |
| vegfX2 | GTTTGATGAAAGATTTGGGCTTG |
| vegfX3 | TTTCTAAAGGAAATCAAATTAG |
| vegfX4 | GATAAGATTTGTATCTGATG |
| vegfX5 | GATGTCTCCTCTTTCAG |
| vegfX6 | GCACAACTCCTAATTCTG |
| vegfX7 | AGCACCTGATTCCGTTGC |
| vegfX8 | TAGTACATAGAATGTTCTGG |
| vegfX9 | AAGAGACATACTTCTGTAC |
| vegfX10 | CCAGGTACAATAAGTGAACTG |

FIG. 4. **Variants isolated by PCR (at 8/2/99, all cloned and sequenced at JRF)**

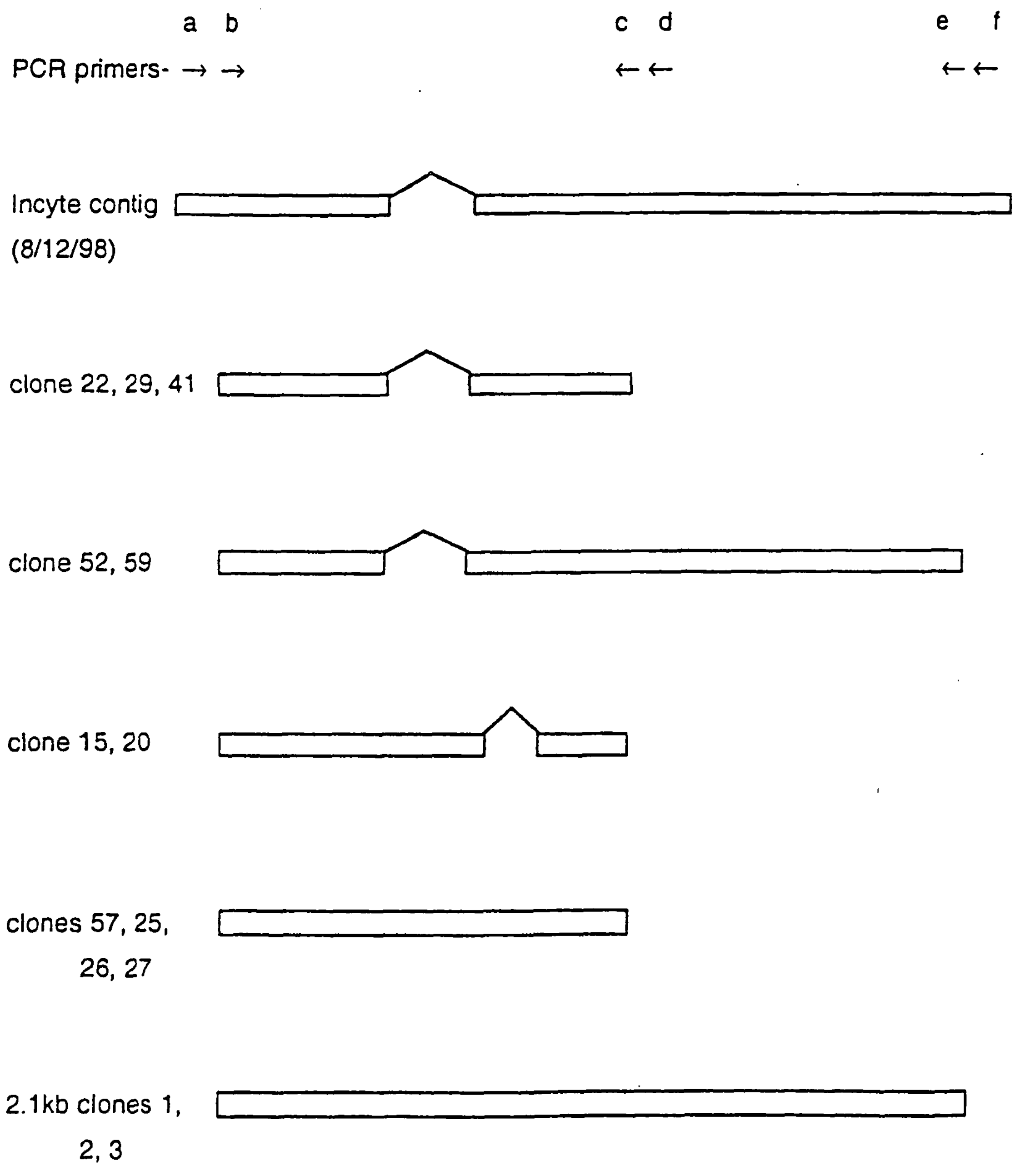


primers- (see fig 3)

| | | |
|---|---|---|
| a- vegfX1 | b- vegfX2 | c- vegfX5 |
| d- vegfX6 | e- vegfX9 | f- vegfX10 |

FIG. 5. **VEGF-X 5' RACE primers**

| | |
|---|---|
| vegfX11 | CCTTTAGAAATCTGTTTTCCTGGTACAG |
| vegfX12 | GGAAAATATTCATCAGATACAAATCTTATCC |
| vegfX13 | GGTCCAGTGGCAAAGCTGAAGG |
| vegfX14 | CTGGTTCAAGATATCGAATAAGGTCTTCC |

FIG. 6: DNA sequence assembled from in-house clones and 5'RACE

```
  1  TGCCAGAGCA GGTGGGCGCT TCCACCCCAG TGCAGCCTTC CCCTGGCGGT GGTGAAAGAG
     ACGGTCTCGT CCACCCGCGA AGGTGGGGTC ACGTCGGAAG GGGACCGCCA CCACTTTCTC

 61  ACTCGGGAGT CGCTGCTTCC AAAGTGCCCG CCGTGAGTGA GCTCTCACCC CAGTCAGCCA
     TGAGCCCTCA GCGACGAAGG TTTCACGGGC GGCACTCACT CGAGAGTGGG GTCAGTCGGT

 +2   MetSerLeu PheGlyLeuLeu LeuLeuThr SerAlaLeu AlaGlyGlnArg GlnGlyTh
      ]------------------------------------------------------------
121  AATGAGCCTC TTCGGGCTTC TCCTGCTGAC ATCTGCCCTG GCCGGCCAGA GACAGGGGAC
     TTACTCGGAG AAGCCCGAAG AGGACGACTG TAGACGGGAC CGGCCGGTCT CTGTCCCCTG

 +2  rGlnAlaGlu SerAsnLeuSer SerLysPhe GlnPheSer SerAsnLysGlu GlnAsnGl
     -------------------------------------------------------------
181  TCAGGCGGAA TCCAACCTGA GTAGTAAATT CCAGTTTTCC AGCAACAAGG AACAGAACGG
     AGTCCGCCTT AGGTTGGACT CATCATTTAA GGTCAAAAGG TCGTTGTTCC TTGTCTTGCC

 +2  yValGlnAsp ProGlnHisGlu ArgIleIle ThrValSer ThrAsnGlySer IleHisSe
     -------------------------------------------------------------
241  AGTACAAGAT CCTCAGCATG AGAGAATTAT TACTGTGTCT ACTAATGGAA GTATTCACAG
     TCATGTTCTA GGAGTCGTAC TCTCTTAATA ATGACACAGA TGATTACCTT CATAAGTGTC

 +2  rProArgPhe ProHisThrTyr ProArgAsn ThrValLeu ValTrpArgLeu ValAlaVa
     -------------------------------------------------------------
301  CCCAAGGTTT CCTCATACTT ATCCAAGAAA TACGGTCTTG GTATGGAGAT TAGTAGCAGT
     GGGTTCCAAA GGAGTATGAA TAGGTTCTTT ATGCCAGAAC CATACCTCTA ATCATCGTCA

 +2  lGluGluAsn ValTrpIleGln LeuThrPhe AspGluArg PheGlyLeuGlu AspProGl
     -------------------------------------------------------------
361  AGAGGAAAAT GTATGGATAC AACTTACGTT TGATGAAAGA TTTGGGCTTG AAGACCCAGA
     TCTCCTTTTA CATACCTATG TTGAATGCAA ACTACTTTCT AAACCCGAAC TTCTGGGTCT

 +2  uAspAspIle CysLysTyrAsp PheValGlu ValGluGlu ProSerAspGly ThrIleLe
     -------------------------------------------------------------
421  AGATGACATA TGCAAGTATG ATTTTGTAGA AGTTGAGGAA CCCAGTGATG GAACTATATT
     TCTACTGTAT ACGTTCATAC TAAAACATCT TCAACTCCTT GGGTCACTAC CTTGATATAA

 +2  uGlyArgTrp CysGlySerGly ThrValPro GlyLysGln IleSerLysGly AsnGlnIl
     -------------------------------------------------------------
481  AGGGCGCTGG TGTGGTTCTG GTACTGTACC AGGAAAAACAG ATTTCTAAAG GAAATCAAAT
     TCCCGCGACC ACACCAAGAC CATGACATGG TCCTTTTGTC TAAAGATTTC CTTTAGTTTA

 +2  eArgIleArg PheValSerAsp GluTyrPhe ProSerGlu ProGlyPheCys IleHisTy
     -------------------------------------------------------------
541  TAGGATAAGA TTTGTATCTG ATGAATATTT TCCTTCTGAA CCAGGGTTCT GCATCCACTA
     ATCCTATTCT AAACATAGAC TACTTATAAA AGGAAGACTT GGTCCCAAGA CGTAGGTGAT

 +2  rAsnIleVal MetProGlnPhe ThrGluAla ValSerPro SerValLeuPro ProSerAl
     -------------------------------------------------------------
601  CAACATTGTC ATGCCACAAT TCACAGAAGC TGTGAGTCCT TCAGTGCTAC CCCCTTCAGC
     GTTGTAACAG TACGGTGTTA AGTGTCTTCG ACACTCAGGA AGTCACGATG GGGGAAGTCG

 +2  aLeuProLeu AspLeuLeuAsn AsnAlaIle ThrAlaPhe SerThrLeuGlu AspLeuIl
     -------------------------------------------------------------
661  TTTGCCACTG GACCTGCTTA ATAATGCTAT AACTGCCTTT AGTACCTTGG AAGACCTTAT
```

FIG. 6 (CONTINUED 1).

```
  +2 eArgTyrLeu GluProGluArg TrpGlnLeu AspLeuGlu AspLeuTyrArg ProThrTr
     -----------------------------------------------------------------
 721 TCGATATCTT GAACCAGAGA GATGGCAGTT GGACTTAGAA GATCTATATA GGCCAACTTG
     AGCTATAGAA CTTGGTCTCT CTACCGTCAA CCTGAATCTT CTAGATATAT CCGGTTGAAC

  +2 pGlnLeuLeu GlyLysAlaPhe ValPheGly ArgLysSer ArgValValAsp LeuAsnLe
     -----------------------------------------------------------------
 781 GCAACTTCTT GGCAAGGCTT TTGTTTTTGG AAGAAAATCC AGAGTGGTGG ATCTGAACCT
     CGTTGAAGAA CCGTTCCGAA AACAAAAACC TTCTTTTAGG TCTCACCACC TAGACTTGGA

  +2 uLeuThrGlu GluValArgLeu TyrSerCys ThrProArg AsnPheSerVal SerIleAr
     -----------------------------------------------------------------
 841 TCTAACAGAG GAGGTAAGAT TATACAGCTG CACACCTCGT AACTTCTCAG TGTCCATAAG
     AGATTGTCTC CTCCATTCTA ATATGTCGAC GTGTGGAGCA TTGAAGAGTC ACAGGTATTC

  +2 gGluGluLeu LysArgThrAsp ThrIlePhe TrpProGly CysLeuLeuVal LysArgCy
     -----------------------------------------------------------------
 901 GGAAGAACTA AAGAGAACCG ATACCATTTT CTGGCCAGGT TGTCTCCTGG TTAAACGCTG
     CCTTCTTGAT TTCTCTTGGC TATGGTAAAA GACCGGTCCA ACAGAGGACC AATTTGCGAC

  +2 sGlyGlyAsn CysAlaCysCys LeuHisAsn CysAsnGlu CysGlnCysVal ProSerLy
     -----------------------------------------------------------------
 961 TGGTGGGAAC TGTGCCTGTT GTCTCCACAA TTGCAATGAA TGTCAATGTG TCCCAAGCAA
     ACCACCCTTG ACACGGACAA CAGAGGTGTT AACGTTACTT ACAGTTACAC AGGGTTCGTT

  +2 sValThrLys LysTyrHisGlu ValLeuGln LeuArgPro LysThrGlyVal ArgGlyLe
     -----------------------------------------------------------------
1021 AGTTACTAAA AAATACCACG AGGTCCTTCA GTTGAGACCA AAGACCGGTG TCAGGGGATT
     TCAATGATTT TTTATGGTGC TCCAGGAAGT CAACTCTGGT TTCTGGCCAC AGTCCCCTAA

  +2 uHisLysSer LeuThrAspVal AlaLeuGlu HisHisGlu GluCysAspCys ValCysAr
     -----------------------------------------------------------------
1081 GCACAAATCA CTCACCGACG TGGCCCTGGA GCACCATGAG GAGTGTGACT GTGTGTGCAG
     CGTGTTTAGT GAGTGGCTGC ACCGGGACCT CGTGGTACTC CTCACACTGA CACACACGTC

  +2 gGlySerThr GlyGly
     ---------------->
1141 AGGGAGCACA GGAGGATAGC CGCATCACCA CCAGCAGCTC TTGCCCAGAG CTGTGCAGTG
     TCCCTCGTGT CCTCCTATCG GCGTAGTGGT GGTCGTCGAG AACGGGTCTC GACACGTCAC

1201 CAGTGGCTGA TTCTATTAGA GAACGTATGC GTTATCTCCA TCCTTAATCT CAGTTGTTTG
     GTCACCGACT AAGATAATCT CTTGCATACG CAATAGAGGT AGGAATTAGA GTCAACAAAC

1261 CTTCAAGGAC CTTTCATCTT CAGGATTTAC AGTGCATTCT GAAAGAGGAG ACATCAAACA
     GAAGTTCCTG GAAAGTAGAA GTCCTAAATG TCACGTAAGA CTTTCTCCTC TGTAGTTTGT

1321 GAATTAGGAG TTGTGCAACA GCTCTTTTGA GAGGAGGCCT AAAGGACAGG AGAAAAGGTC
     CTTAATCCTC AACACGTTGT CGAGAAAACT CTCCTCCGGA TTTCCTGTCC TCTTTTCCAG

1381 TTCAATCGTG GAAAGAAAAT TAAATGTTGT ATTAAATAGA TCACCAGCTA GTTTCAGAGT
     AAGTTAGCAC CTTTCTTTTA ATTTACAACA TAATTTATCT AGTGGTCGAT CAAAGTCTCA

1441 TACCATGTAC GTATTCCACT AGCTGGGTTC TGTATTTCAG TTCTTTCGAT ACGGCTTAGG
     ATGGTACATG CATAAGGTGA TCGACCCAAG ACATAAAGTC AAGAAAGCTA TGCCGAATCC

1501 GTAATGTCAG TACAGGAAAA AAACTGTGCA AGTGAGCACC TGATTCCGTT GCCTTGCTTA
```

FIG. 6 (CONTINUED 2).

1561 ACTCTAAAGC TCCATGTCCT GGGCCTAAAA TCGTATAAAA TCTGGATTTT TTTTTTTTTT
TGAGATTTCG AGGTACAGGA CCCGGATTTT AGCATATTTT AGACCTAAAA AAAAAAAAAA

1621 TTTGCTCATA TTCACATATG TAAACCAGAA CATTCTATGT ACTACAAACC TGGTTTTTAA
AAACGAGTAT AAGTGTATAC ATTTGGTCTT GTAAGATACA TGATGTTTGG ACCAAAAATT

1681 AAAGGAACTA TGTTGCTATG AATTAAACTT GTGTCGTGCT GATAGGACAG ACTGGATTTT
TTTCCTTGAT ACAACGATAC TTAATTTGAA CACAGCACGA CTATCCTGTC TGACCTAAAA

1741 TCATATTTCT TATTAAAATT TCTGCCATTT AGAAGAAGAG AACTACATTC ATGGTTTGGA
AGTATAAAGA ATAATTTTAA AGACGGTAAA TCTTCTTCTC TTGATGTAAG TACCAAACCT

1801 AGAGATAAAC CTGAAAAGAA GAGTGGCCTT ATCTTCACTT TATCGATAAG CCAGTTTATT
TCTCTATTTG GACTTTTCTT CTCACCGGAA TAGAAGTGAA ATAGCTATTC GGTCAAATAA

1861 TGTTTCATTG TGTACATTTT TATATTCTCC TTTTGACATT ATAACTGTTG GCTTTTCTAA
ACAAAGTAAC ACATGTAAAA ATATAAGAGG AAAACTGTAA TATTGACAAC CGAAAAGATT

1921 TCTTGTTAAA TATATCTATT TTTACCAAAG GTATTTAATA TTCTTTTTTA TGACAACTTA
AGAACAATTT ATATAGATAA AAATGGTTTC CATAAATTAT AAGAAAAAAT ACTGTTGAAT

1981 GATCAACTAT TTTTAGCTTG GTAAATTTTT CTAAACACAA TTGTTATAGC CAGAGGAACA
CTAGTTGATA AAAATCGAAC CATTTAAAAA GATTTGTGTT AACAATATCG GTCTCCTTGT

2041 AAGATGATAT AAAATATTGT TGCTCTGACA AAAATACATG TATTTCATTC TCGTATGGTG
TTCTACTATA TTTTATAACA ACGAGACTGT TTTTATGTAC ATAAAGTAAG AGCATACCAC

2101 CTAGAGTTAG ATTAATCTGC ATTTTAAAAA ACTGAATTGG AATAGAATTG GTAAGTTGCA
GATCTCAATC TAATTAGACG TAAAATTTTT TGACTTAACC TTATCTTAAC CATTCAACGT

2161 AAGACTTTTT GAAAATAATT AAATTATCAT ATCTTCCATT CCTGTTATTG GAGATGAAAA
TTCTGAAAAA CTTTTATTAA TTTAATAGTA TAGAAGGTAA GGACAATAAC CTCTACTTTT

2221 TAAAAAGCAA CTTATGAAAG TAGACATTCA GATCCAGCCA TTACTAACCT ATTCCTTTTT
ATTTTTCGTT GAATACTTTC ATCTGTAAGT CTAGGTCGGT AATGATTGGA TAAGGAAAAA

2281 TGGGGAAATC TGAGCCTAGC TCAGAAAAAC ATAAAGCACC TTGAAAAAGA CTTGGCAGCT
ACCCCTTTAG ACTCGGATCG AGTCTTTTTG TATTTCGTGG AACTTTTTCT GAACCGTCGA

2341 TCCTGATAAA GCGTGCTGTG CTGTGCAGTA GGAACACATC CTATTTATTG TGATGTTGTG
AGGACTATTT CGCACGACAC GACACGTCAT CCTTGTGTAG GATAAATAAC ACTACAACAC

2401 GTTTTATTAT CTTAAACTCT GTTCCATACA CTTGTATAAA TACATGGATA TTTTTATGTA
CAAAATAATA GAATTTGAGA CAAGGTATGT GAACATATTT ATGTACCTAT AAAAATACAT

2461 CAGAAGTATG TCTCT
GTCTTCATAC AGAGA

FIG. 7. **New Sequence + Incyte ESTs**

```
  1  ATTTGTTTAA ACCTTGGGAA ACTGGTTCAG GTCCAGGTTT TGCTTTGATC CTTTTCAAAA
     TAAACAAATT TGGAACCCTT TGACCAAGTC CAGGTCCAAA ACGAAACTAG GAAAAGTTTT

 61  ACTGGAGACA CAGAAGAGGG CTTCTAGGAA AAAGTTTTGG GATGGGATTA TGTGGAAACT
     TGACCTCTGT GTCTTCTCCC GAAGATCCTT TTTCAAAACC CTACCCTAAT ACACCTTTGA

121  ACCCTGCGAT TCTCTGCTGC CAGAGCAGGC TCGGCGCTTC CACCCCAGTG CAGCCTTCCC
     TGGGACGCTA AGAGACGACG GTCTCGTCCG AGCCGCGAAG GTGGGGTCAC GTCGGAAGGG

181  CTGGCGGTGG TGAAAGAGAC TCGGGAGTCG CTGCTTCCAA AGTGCCCGCC GTGAGTGAGC
     GACCGCCACC ACTTTCTCTG AGCCCTCAGC GACGAAGGTT TCACGGGCGG CACTCACTCG

 +2                       Met SerLeuPhe GlyLeuLeu LeuLeuThrSer AlaLeuAl
                          ]-------------------------------------------
241  TCTCACCCCA GTCAGCCAAA TGAGCCTCTT CGGGCTTCTC CTGCTGACAT CTGCCCTGGC
     AGAGTGGGGT CAGTCGGTTT ACTCGGAGAA GCCCGAAGAG GACGACTGTA GACGGGACCG

 +2  aGlyGlnArg GlnGlyThrGln AlaGluSer AsnLeuSer SerLysPheGln PheSerSe
     -----------------------------------------------------------------
301  CGGCCAGAGA CAGGGGACTC AGGCGGAATC CAACCTGAGT AGTAAATTCC AGTTTTCCAG
     GCCGGTCTCT GTCCCCTGAG TCCGCCTTAG GTTGGACTCA TCATTTAAGG TCAAAAGGTC

 +2  rAsnLysGlu GlnTyrGlyVal GlnAspPro GlnHisGlu ArgIleIleThr ValSerTh
     -----------------------------------------------------------------
361  CAACAAGGAA CAGTACGGAG TACAAGATCC TCAGCATGAG AGAATTATTA CTGTGTCTAC
     GTTGTTCCTT GTCATGCCTC ATGTTCTAGG AGTCGTACTC TCTTAATAAT GACACAGATG

 +2  rAsnGlySer IleHisSerPro ArgPhePro HisThrTyr ProArgAsnThr ValLeuVa
     -----------------------------------------------------------------
421  TAATGGAAGT ATTCACAGCC CAAGGTTTCC TCATACTTAT CCAAGAAATA CGGTCTTGGT
     ATTACCTTCA TAAGTGTCGG GTTCCAAAGG AGTATGAATA GGTTCTTTAT GCCAGAACCA

 +2  lTrpArgLeu ValAlaValGlu GluAsnVal TrpIleGln LeuThrPheAsp GluArgPh
     -----------------------------------------------------------------
481  ATGGAGATTA GTAGCAGTAG AGGAAAATGT ATGGATACAA CTTACGTTTG ATGAAAGATT
     TACCTCTAAT CATCGTCATC TCCTTTTACA TACCTATGTT GAATGCAAAC TACTTTCTAA

 +2  eGlyLeuGlu AspProGluAsp AspIleCys LysTyrAsp PheValGluVal GluGluPr
     -----------------------------------------------------------------
541  TGGGCTTGAA GACCCAGAAG ATGACATATG CAAGTATGAT TTTGTAGAAG TTGAGGAACC
     ACCCGAACTT CTGGGTCTTC TACTGTATAC GTTCATACTA AAACATCTTC AACTCCTTGG

 +2  oSerAspGly ThrIleLeuGly ArgTrpCys GlySerGly ThrValProGly LysGlnIl
     -----------------------------------------------------------------
601  CAGTGATGGA ACTATATTAG GGCGCTGGTG TGGTTCTGGT ACTGTACCAG GAAAACAGAT
     GTCACTACCT TGATATAATC CCGCGACCAC ACCAAGACCA TGACATGGTC CTTTTGTCTA

 +2  eSerLysGly AsnGlnIleArg IleArgPhe ValSerAsp GluTyrPhePro SerGluPr
     -----------------------------------------------------------------
661  TTCTAAAGGA AATCAAATTA GGATAAGATT TGTATCTGAT GAATATTTTC CTTCTGAACC
     AAGATTTCCT TTAGTTTAAT CCTATTCTAA ACATAGACTA CTTATAAAAG GAAGACTTGG
```

FIG. 7(CONTINUED 1).

```
  +2 oGlyPheCys IleHisTyrAsn IleValMet ProGlnPhe ThrGluAlaVal SerProSe
     ------------------------------------------------------------------
 721 AGGGTTCTGC ATCCACTACA ACATTGTCAT GCCACAATTC ACAGAAGCTG TGAGTCCTTC
     TCCCAAGACG TAGGTGATGT TGTAACAGTA CGGTGTTAAG TGTCTTCGAC ACTCAGGAAG

  +2 rValLeuPro ProSerAlaLeu ProLeuAsp LeuLeuAsn AsnAlaIleThr AlaPheSe
     ------------------------------------------------------------------
 781 AGTGCTACCC CCTTCAGCTT TGCCACTGGA CCTGCTTAAT AATGCTATAA CTGCCTTTAG
     TCACGATGGG GGAAGTCGAA ACGGTGACCT GGACGAATTA TTACGATATT GACGGAAATC

  +2 rThrLeuGlu AspLeuIleArg TyrLeuGlu ProGluArg TrpGlnLeuAsp LeuGluAs
     ------------------------------------------------------------------
 841 TACCTTGGAA GACCTTATTC GATATCTTGA ACCAGAGAGA TGGCAGTTGG ACTTAGAAGA
     ATGGAACCTT CTGGAATAAG CTATAGAACT TGGTCTCTCT ACCGTCAACC TGAATCTTCT

  +2 pLeuTyrArg ProThrTrpGln LeuLeuGly LysAlaPhe ValPheGlyArg LysSerAr
     ------------------------------------------------------------------
 901 TCTATATAGG CCAACTTGGC AACTTCTTGG CAAGGCTTTT GTTTTTGGAA GAAAATCCAG
     AGATATATCC GGTTGAACCG TTGAAGAACC GTTCCGAAAA CAAAAACCTT CTTTTAGGTC

  +2 gValValAsp LeuAsnLeuLeu ThrGluGlu ValArgLeu TyrSerCysThr ProArgAs
     ------------------------------------------------------------------
 961 AGTGGTGGAT CTGAACCTTC TAACAGAGGA GGTAAGATTA TACAGCTGCA CACCTCGTAA
     TCACCACCTA GACTTGGAAG ATTGTCTCCT CCATTCTAAT ATGTCGACGT GTGGAGCATT

  +2 nPheSerVal SerIleArgGlu GluLeuLys ArgThrAsp ThrIlePheTrp ProGlyCy
     ------------------------------------------------------------------
1021 CTTCTCAGTG TCCATAAGGG AAGAACTAAA GAGAACCGAT ACCATTTTCT GGCCAGGTTG
     GAAGAGTCAC AGGTATTCCC TTCTTGATTT CTCTTGGCTA TGGTAAAAGA CCGGTCCAAC

  +2 sLeuLeuVal LysArgCysGly GlyAsnCys AlaCysCys LeuHisAsnCys AsnGluCy
     ------------------------------------------------------------------
1081 TCTCCTGGTT AAACGCTGTG GTGGGAACTG TGCCTGTTGT CTCCACAATT GCAATGAATG
     AGAGGACCAA TTTGCGACAC CACCCTTGAC ACGGACAACA GAGGTGTTAA CGTTACTTAC

  +2 sGlnCysVal ProSerLysVal ThrLysLys TyrHisGlu ValLeuGlnLeu ArgProLy
     ------------------------------------------------------------------
1141 TCAATGTGTC CCAAGCAAAG TTACTAAAAA ATACCACGAG GTCCTTCAGT TGAGACCAAA
     AGTTACACAG GGTTCGTTTC AATGATTTTT TATGGTGCTC CAGGAAGTCA ACTCTGGTTT

  +2 sThrGlyVal ArgGlyLeuHis LysSerLeu ThrAspVal AlaLeuGluHis HisGluGl
     ------------------------------------------------------------------
1201 GACCGGTGTC AGGGGATTGC ACAAATCACT CACCGACGTG GCCCTGGAGC ACCATGAGGA
     CTGGCCACAG TCCCCTAACG TGTTTAGTGA GTGGCTGCAC CGGGACCTCG TGGTACTCCT

  +2 uCysAspCys ValCysArgGly SerThrGly Gly
     ------------------------------------>
1261 GTGTGACTGT GTGTGCAGAG GGAGCACAGG AGGATAGCCG CATCACCACC AGCAGCTCTT
     CACACTGACA CACACGTCTC CCTCGTGTCC TCCTATCGGC GTAGTGGTGG TCGTCGAGAA

1321 GCCCAGAGCT GTGCAGTGCA GTGGCTGATT CTATTAGAGA ACGTATGCGT TATCTCCATC
     CGGGTCTCGA CACGTCACGT CACCGACTAA GATAATCTCT TGCATACGCA ATAGAGGTAG

1381 CTTAATCTCA GTTGTTTGCT TCAAGGACCT TTCATCTTCA GGATTTACAG TGCATTCTGA
     GAATTAGAGT CAACAAACGA AGTTCCTGGA AAGTAGAAGT CCTAAATGTC ACGTAAGACT
```

FIG. 7(CONTINUED 2).

```
1441  AAGAGGAGAC ATCAAACAGA ATTAGGAGTT GTGCAACAGC TCTTTTGAGA GGAGGCCTAA
      TTCTCCTCTG TAGTTTGTCT TAATCCTCAA CACGTTGTCG AGAAAACTCT CCTCCGGATT

1501  AGGACAGGAG AAAAGGTCTT CAATCGTGGA AAGAAAATTA AATGTTGTAT TAAATAGATC
      TCCTGTCCTC TTTTCCAGAA GTTAGCACCT TTCTTTTAAT TTACAACATA ATTTATCTAG

1561  ACCAGCTAGT TTCAGAGTTA CCATGTACGT ATTCCACTAG CTGGGTTCTG TATTTCAGTT
      TGGTCGATCA AAGTCTCAAT GGTACATGCA TAAGGTGATC GACCCAAGAC ATAAAGTCAA

1621  CTTTCGATAC GGCTTAGGGT AATGTCAGTA CAGGAAAAAA ACTGTGCAAG TGAGCACCTG
      GAAAGCTATG CCGAATCCCA TTACAGTCAT GTCCTTTTTT TGACACGTTC ACTCGTGGAC

1681  ATTCCGTTGC CTTGGCTTAA CTCTAAAGCT CCATGTCCTG GGCCTAAAAT CGTATAAAAT
      TAAGGCAACG GAACCGAATT GAGATTTCGA GGTACAGGAC CCGGATTTTA GCATATTTTA

1741  CTGGATTTTT TTTTTTTTTT TTGCGCATAT TCACATATGT AAACCAGAAC ATTCTATGTA
      GACCTAAAAA AAAAAAAAAA AACGCGTATA AGTGTATACA TTTGGTCTTG TAAGATACAT

1801  CTACAAACCT GGTTTTTAAA AAGGAACTAT GTTGCTATGA ATTAAACTTG TGTCATGCTG
      GATGTTTGGA CCAAAAATTT TTCCTTGATA CAACGATACT TAATTTGAAC ACAGTACGAC

1861  ATAGGACAGA CTGGATTTTT CATATTTCTT ATTAAAATTT CTGCCATTTA GAAGAAGAGA
      TATCCTGTCT GACCTAAAAA GTATAAAGAA TAATTTTAAA GACGGTAAAT CTTCTTCTCT

1921  ACTACATTCA TGGTTTGGAA GAGATAAACC TGAAAAGAAG AGTGGCCTTA TCTTCACTTT
      TGATGTAAGT ACCAAACCTT CTCTATTTGG ACTTTTCTTC TCACCGGAAT AGAAGTGAAA

1981  ATCGATAAGT CAGTTTATTT GTTTCATTGT GTACATTTTT ATATTCTCCT TTTGACATTA
      TAGCTATTCA GTCAAATAAA CAAAGTAACA CATGTAAAAA TATAAGAGGA AAACTGTAAT

2041  TAACTGTTGG CTTTTCTAAT CTTGTTAAAT ATATCTATTT TTACCAAAGG TATTTAATAT
      ATTGACAACC GAAAAGATTA GAACAATTTA TATAGATAAA AATGGTTTCC ATAAATTATA

2101  TCTTTTTTAT GACAACTTAG ATCAACTATT TTTAGCTTGG TAAATTTTTC TAAACACAAT
      AGAAAAAATA CTGTTGAATC TAGTTGATAA AAATCGAACC ATTTAAAAAG ATTTGTGTTA

2161  TGTTATAGCC AGAGGAACAA AGATGATATA AAATATTGTT GCTCTGACAA AAATACATGT
      ACAATATCGG TCTCCTTGTT TCTACTATAT TTTATAACAA CGAGACTGTT TTTATGTACA

2221  ATTTCATTCT CGTATGGTGC TAGAGTTAGA TTAATCTGCA TTTTAAAAAA CTGAATTGGA
      TAAAGTAAGA GCATACCACG ATCTCAATCT AATTAGACGT AAAATTTTTT GACTTAACCT

2281  ATAGAATTGG TAAGTTGCAA AGACTTTTTG AAAATAATTA AATTATCATA TCTTCCATTC
      TATCTTAACC ATTCAACGTT TCTGAAAAAC TTTTATTAAT TTAATAGTAT AGAAGGTAAG

2341  CTGTTATTGG AGATGAAAAT AAAAAGCAAC TTATGAAAGT AGACATTCAG ATCCAGCCAT
      GACAATAACC TCTACTTTTA TTTTTCGTTG AATACTTTCA TCTGTAAGTC TAGGTCGGTA

2401  TACTAACCTA TTCCTTTTTT GGGGAAATCT GAGCCTAGCT CAGAAAAACA TAAAGCACCT
      ATGATTGGAT AAGGAAAAAA CCCCTTTAGA CTCGGATCGA GTCTTTTTGT ATTTCGTGGA

2461  TGAAAAAGAC TTGGCAGCTT CCTGATAAAG CGTGCTGTGC TGTGCAGTAG GAACACATCC
      ACTTTTTCTG AACCGTCGAA GGACTATTTC GCACGACACG ACACGTCATC CTTGTGTAGG

2521  TATTTATTGT GATGTTGTGG TTTTATTATC TTAAACTCTG TTCCATACAC TTGTATAAAT
      ATAAATAACA CTACAACACC AAAATAATAG AATTTGAGAC AAGGTATGTG AACATATTTA
```

FIG. 7(CONTINUED 3).

```
2581 ACATGGATAT TTTTATGTAC AGAAGTATGT CTCTTAACCA GTTCACTTAT TGTACTCTGG
     TGTACCTATA AAAATACATG TCTTCATACA GAGAATTGGT CAAGTGAATA ACATGAGACC

2641 CAATTTAAAA GAAAATCAGT AAAATATTTT GCTTGTAAAA TGCTTAATAT CGTGCCTAGG
     GTTAAATTTT CTTTTAGTCA TTTTATAAAA CGAACATTTT ACGAATTATA GCACGGATCC

2701 TTATGTGGTG ACTATTTGAA TCAAAAATGT ATTGAATCAT CAAATAAAAG AATGTGGCTA
     AATACACCAC TGATAAACTT AGTTTTTACA TAACTTAGTA GTTTATTTTC TTACACCGAT

2761 TTTTGGGGAG AAAATT
     AAAACCCCTC TTTTAA
```

FIG. 8. **Additional oligonucleotides used for amplification of entire coding region**

| | |
|---|---|
| 5'-1 | TTTGTTTAAACCTTGGGAAACTGG |
| 5'-2 | GTCCAGGTTTTGCTTTGATCC |

**FIG. 9.** **DNA Sequence Of Clones 4 & 7, Identical Clones Containing The Entire Open Reading Frame**

```
  1  TTTGTTTAAA CCTTGGGAAA CTGGTTCAGG TCCAGGTTTT GCTTTGATCC TTTTCAAAAA
     AAACAAATTT GGAACCCTTT GACCAAGTCC AGGTCCAAAA CGAAACTAGG AAAAGTTTTT

 61  CTGGAGACAC AGAAGAGGGC TCTAGGAAAA AGTTTTGGAT GGGATTATGT GGAAACTACC
     GACCTCTGTG TCTTCTCCCG AGATCCTTTT TCAAAACCTA CCCTAATACA CCTTTGATGG

121  CTGCGATTCT CTGCTGCCAG AGCAGGCTCG GCGCTTCCAC CCCAGTGCAG CCTTCCCCTG
     GACGCTAAGA GACGACGGTC TCGTCCGAGC CGCGAAGGTG GGGTCACGTC GGAAGGGGAC

181  GCGGTGGTGA AAGAGACTCG GGAGTCGCTG CTTCCAAAGT GCCCGCCGTG AGTGAGCTCT
     CGCCACCACT TTCTCTGAGC CCTCAGCGAC GAAGGTTTCA CGGGCGGCAC TCACTCGAGA

 +2                       MetSer LeuPheGly LeuLeuLeu LeuThrSerAla LeuAlaGl
                          ]-----------------------------------------------
241  CACCCCAGTC AGCCAAATGA GCCTCTTCGG GCTTCTCCTG CTGACATCTG CCCTGGCCGG
     GTGGGGTCAG TCGGTTTACT CGGAGAAGCC CGAAGAGGAC GACTGTAGAC GGGACCGGCC

 +2  yGlnArgGln GlyThrGlnAla GluSerAsn LeuSerSer LysPheGlnPhe SerSerAs
     -----------------------------------------------------------------
301  CCAGAGACAG GGGACTCAGG CGGAATCCAA CCTGAGTAGT AAATTCCAGT TTTCCAGCAA
     GGTCTCTGTC CCCTGAGTCC GCCTTAGGTT GGACTCATCA TTTAAGGTCA AAAGGTCGTT

 +2  nLysGluGln AsnGlyValGln AspProGln HisGluArg IleIleThrVal SerThrAs
     -----------------------------------------------------------------
361  CAAGGAACAG AACGGAGTAC AAGATCCTCA GCATGAGAGA ATTATTACTG TGTCTACTAA
     GTTCCTTGTC TTGCCTCATG TTCTAGGAGT CGTACTCTCT TAATAATGAC ACAGATGATT

 +2  nGlySerIle HisSerProArg PheProHis ThrTyrPro ArgAsnThrVal LeuValTr
     -----------------------------------------------------------------
421  TGGAAGTATT CACAGCCCAA GGTTTCCTCA TACTTATCCA AGAAATACGG TCTTGGTATG
     ACCTTCATAA GTGTCGGGTT CCAAAGGAGT ATGAATAGGT TCTTTATGCC AGAACCATAC

 +2  pArgLeuVal AlaValGluGlu AsnValTrp IleGlnLeu ThrPheAspGlu ArgPheGl
     -----------------------------------------------------------------
481  GAGATTAGTA GCAGTAGAGG AAAATGTATG GATACAACTT ACGTTTGATG AAAGATTTGG
     CTCTAATCAT CGTCATCTCC TTTTACATAC CTATGTTGAA TGCAAACTAC TTTCTAAACC

 +2  yLeuGluAsp ProGluAspAsp IleCysLys TyrAspPhe ValGluValGlu GluProSe
     -----------------------------------------------------------------
541  GCTTGAAGAC CCAGAAGATG ACATATGCAA GTATGATTTT GTAGAAGTTG AGGAACCCAG
     CGAACTTCTG GGTCTTCTAC TGTATACGTT CATACTAAAA CATCTTCAAC TCCTTGGGTC

 +2  rAspGlyThr IleLeuGlyArg TrpCysGly SerGlyThr ValProGlyLys GlnIleSe
     -----------------------------------------------------------------
601  TGATGGAACT ATATTAGGGC GCTGGTGTGG TTCTGGTACT GTACCAGGAA AACAGATTTC
     ACTACCTTGA TATAATCCCG CGACCACACC AAGACCATGA CATGGTCCTT TTGTCTAAAG

 +2  rLysGlyAsn GlnIleArgIle ArgPheVal SerAspGlu TyrPheProSer GluProGl
     -----------------------------------------------------------------
661  TAAAGGAAAT CAAATTAGGA TAAGATTTGT ATCTGATGAA TATTTTCCTT CTGAACCAGG
     ATTTCCTTTA GTTTAATCCT ATTCTAAACA TAGACTACTT ATAAAAGGAA GACTTGGTCC
```

FIG. 9 (CONTINUED).

```
  +2 yPheCysIle HisTyrAsnIle ValMetPro GlnPheThr GluAlaValSer ProSerVa
     ------------------------------------------------------------------
 721 GTTCTGCATC CACTACAACA TTGTCATGCC ACAATTCACA GAAGCTGTGA GTCCTTCAGT
     CAAGACGTAG GTGATGTTGT AACAGTACGG TGTTAAGTGT CTTCGACACT CAGGAAGTCA

  +2 lLeuProPro SerAlaLeuPro LeuAspLeu LeuAsnAsn AlaIleThrAla PheSerTh
     ------------------------------------------------------------------
 781 GCTACCCCCT TCAGCTTTGC CACTGGACCT GCTTAATAAT GCTATAACTG CCTTTAGTAC
     CGATGGGGGA AGTCGAAACG GTGACCTGGA CGAATTATTA CGATATTGAC GGAAATCATG

  +2 rLeuGluAsp LeuIleArgTyr LeuGluPro GluArgTrp GlnLeuAspLeu GluAspLe
     ------------------------------------------------------------------
 841 CTTGGAAGAC CTTATTCGAT ATCTTGAACC AGAGAGATGG CAGTTGGACT TAGAAGATCT
     GAACCTTCTG GAATAAGCTA TAGAACTTGG TCTCTCTACC GTCAACCTGA ATCTTCTAGA

  +2 uTyrArgPro ThrTrpGlnLeu LeuGlyLys AlaPheVal PheGlyArgLys SerArgVa
     ------------------------------------------------------------------
 901 ATATAGGCCA ACTTGGCAAC TTCTTGGCAA GGCTTTTGTT TTTGGAAGAA AATCCAGAGT
     TATATCCGGT TGAACCGTTG AAGAACCGTT CCGAAAACAA AAACCTTCTT TTAGGTCTCA

  +2 lValAspLeu AsnLeuLeuThr GluGluVal ArgLeuTyr SerCysThrPro ArgAsnPh
     ------------------------------------------------------------------
 961 GGTGGATCTG AACCTTCTAA CAGAGGAGGT AAGATTATAC AGCTGCACAC CTCGTAACTT
     CCACCTAGAC TTGGAAGATT GTCTCCTCCA TTCTAATATG TCGACGTGTG GAGCATTGAA

  +2 eSerValSer IleArgGluGlu LeuLysArg ThrAspThr IlePheTrpPro GlyCysLe
     ------------------------------------------------------------------
1021 CTCAGTGTCC ATAAGGGAAG AACTAAAGAG AACCGATACC ATTTCTGGC CAGGTTGTCT
     GAGTCACAGG TATTCCCTTC TTGATTTCTC TTGGCTATGG TAAAAGACCG GTCCAACAGA

  +2 uLeuValLys ArgCysGlyGly AsnCysAla CysCysLeu HisAsnCysAsn GluCysGl
     ------------------------------------------------------------------
1081 CCTGGTTAAA CGCTGTGGTG GGAACTGTGC CTGTTGTCTC CACAATTGCA ATGAATGTCA
     GGACCAATTT GCGACACCAC CCTTGACACG GACAACAGAG GTGTTAACGT TACTTACAGT

  +2 nCysValPro SerLysValThr LysLysTyr HisGluVal LeuGlnLeuArg ProLysTh
     ------------------------------------------------------------------
1141 ATGTGTCCCA AGCAAAGTTA CTAAAAAATA CCACGAGGTC CTTCAGTTGA GACCAAAGAC
     TACACAGGGT TCGTTTCAAT GATTTTTTAT GGTGCTCCAG GAAGTCAACT CTGGTTTCTG

  +2 rGlyValArg GlyLeuHisLys SerLeuThr AspValAla LeuGluHisHis GluGluCy
     ------------------------------------------------------------------
1201 CGGTGTCAGG GGATTGCACA AATCACTCAC CGACGTGGCC CTGGAGCACC ATGAGGAGTG
     GCCACAGTCC CCTAACGTGT TTAGTGAGTG GCTGCACCGG GACCTCGTGG TACTCCTCAC

  +2 sAspCysVal CysArgGlySer ThrGlyGly
     -------------------------------->
1261 TGACTGTGTG TGCAGAGGGA GCACAGGAGG ATAGCCGCAT CACCACCAGC AGCTCTTGCC
     ACTGACACAC ACGTCTCCCT CGTGTCCTCC TATCGGCGTA GTGGTGGTCG TCGAGAACGG

1321 CAGAGCTGTG CAGTGCAGTG GCTGATTCTA TTAGAGAACG TATGCGTTAT CTCCATCCTT
     GTCTCGACAC GTCACGTCAC CGACTAAGAT AATCTCTTGC ATACGCAATA GAGGTAGGAA

1381 AATCTCAGTT GTTTGCTTCA AGGACCTTTC ATCTTCAGGA TTTACAGTGC ATTCTGAAAG
     TTAGAGTCAA CAAACGAAGT TCCTGGAAAG TAGAAGTCCT AAATGTCACG TAAGACTTTC

1441 AGGAGACATC AAACAGAATT AGGAGTTGTG CAA
     TCCTCTGTAG TTTGTCTTAA TCCTCAACAC GTT
```

FIG. 10. **Predicted Full-length Polypeptide Sequence**

```
  1  MSLFGLLLLT SALAGQRQGT QAESNLSSKF QFSSNKEQYG VQDPQHERII
 51  TVSTNGSIHS PRFPHTYPRN TVLVWRLVAV EENVWIQLTF DERFGLEDPE
101  DDICKYDFVE VEEPSDGTIL GRWCGSGTVP GKQISKGNQI RIRFVSDEYF
151  PSEPGFCIHY NIVMPQFTEA VSPSVLPPSA LPLDLLNNAI TAFSTLEDLI
201  RYLEPERWQL DLEDLYRPTW QLLGKAFVFG RKSRVVDLNL LTEEVRLYSC
251  TPRNFSVSIR EELKRTDTIF WPGCLLVKRC GGNCACCLHN CNECQCVPSK
301  VTKKYHEVLQ LRPKTGVRGL HKSLTDVALE HHEECDCVCR GSTGG
```

FIG. 11 **Alignment of VEGF-X with Other VEGFs**

```
                           *        20         *        40         *
VEGF_HUMAN  : -------------------------------------------------- :   -
PLGF_HUMAN  : -------------------------------------------------- :   -
VEGB_HUMAN  : -------------------------------------------------- :   -
VEGC_HUMAN  : -------------------------------------------------- :   -
VEGD_HUMAN  : -------------------------------------------------- :   -
990126vegx  : MSLFGLLLLTSALAGQRQGTQAESNLSSKFQFSSNKEQNGVQDPQHERII :  50

                     60         *        80         *       100
VEGF_HUMAN  : -------------------------------------------------- :   -
PLGF_HUMAN  : -------------------------------------------------- :   -
VEGB_HUMAN  : -------------------------------------------------- :   -
VEGC_HUMAN  : -------------------------------------------------- :   -
VEGD_HUMAN  : -------------------------------------------------- :   -
990126vegx  : TVSTNGSIHSPRFPHTYPRNTVLVWRLVAVEENVWIQLTFDERFGLEDPE : 100

                           *       120         *       140         *
VEGF_HUMAN  : -------------------------------------------------- :   -
PLGF_HUMAN  : -------------------------------------------------- :   -
VEGB_HUMAN  : -------------------------------------------------- :   -
VEGC_HUMAN  : --------------------MHLLGFFSVACSLLAAALLPGPREAPAAAA :  30
VEGD_HUMAN  : ----------------------------------------MYREWVVVNV :  10
990126vegx  : DDICKYDFVEV--EEPSDGTILGRWCGSGTVPGKQISKGNQIRIRFVSDE : 148

                    160         *       180         *       200
VEGF_HUMAN  : ------------------------------------------------MN :   2
PLGF_HUMAN  : ------------------------------------------------MP :   2
VEGB_HUMAN  : -------------------------------------------------- :   -
VEGC_HUMAN  : AFESGLDLSDAEPDAGEATAYASKDLEEQLRSVSSVDELMTVLYPEYWKM :  80
VEGD_HUMAN  : FMMLYVQLVQGSSNEHGPVKRSSQSTLERSEQQIRAASSLEELLRITHSE :  60
990126vegx  : YFPSEPGFCIHYNIVMPQFTEAVSPSVLPPSALPLDLLNNAITAFSTLED : 198

                           *       220         *       240         *
VEGF_HUMAN  : FLLSWVHWSLALLLYLHHAKWSQAAPMAEGGGQNHHEVVKFMD-VYQRSY :  51
PLGF_HUMAN  : VMRLFPCFLQLLAGLALPAVPPQQWALSAGNGSSEVEVVPFQE-VWGRSY :  51
VEGB_HUMAN  : ---MSPLLRRLLLAALLQLAPAQAPVSQPDAPGHQRKVVSWID-VYTRAT :  46
VEGC_HUMAN  : YKCQLRKGGWQHNREQANLNSRTEETIKFAAAHYNTEILKSIDNEWRKTQ : 130
VEGD_HUMAN  : DWKLWRCRLRLKSFTSMDSRSASHRSTRFAATFYDIETLKVIDEEWQRTQ : 110
990126vegx  : LIRYLEPERWQLDLEDLYRPTWQLLGKAFVFGRKSRVVDLNLLTEEVRLY : 248

                    260         *       280         *       300
VEGF_HUMAN  : CHPIETLVDIFQEYPDEIEYIFKPSCVPLMRCGG---CCND--EGLECVP :  96
PLGF_HUMAN  : CRALERLVDVVSEYPSEVEHMFSPSCVSLLRCTG---CCGD--ENLHCVP :  96
VEGB_HUMAN  : CQPREVVVPLTVELMGTVAKQLVPSCVTVQRCGG---CCPD--DGLECVP :  91
VEGC_HUMAN  : CMPREVCIDVGKEFGVATNTFFKPPCVSVYRCGG---CCNS--EGLQCMN : 175
VEGD_HUMAN  : CSPRETCVEVASELGKSTNTFFKPPCVNVFRCGG---CCNE--ESLICMN : 155
990126vegx  : SCTPRNFSVSIREELKRTDTIFWPGCLLVKRCGGNCACCLHNCNECQCVP : 298
```

FIG. 11 (CONTINUED).

```
                     *       320         *       340         *
VEGF_HUMAN : TEESNITMQIMRIKPHQG-----QHIGEMSFLQHNKCECRPKKDRARQEK : 141
PLGF_HUMAN : VETANVTMQLLKIRSGDR-----PSYVELTFSQHVRCECRPLREKMKPER : 141
VEGB_HUMAN : TGQHQVRMQILMIRYPS------SQLGEMSLEEHSQCECRPKKKDSAVKP : 135
VEGC_HUMAN : TSTSYLSKTLFEITVPLSQG---PKPVTISFANHTSCRCMSKLDVYRQVH : 222
VEGD_HUMAN : TSTSYISKQLFEISVPLTSV---PELVPVKVANHTGCKCLPTAPRHPYSI : 202
990126vegx : SKVTKKYHEVLQLRPKTGVRGLHKSLTDVALEHHEECDCVCRGSTGG--- : 345

                   360         *       380         *       400
VEGF_HUMAN : KSVRGKGKGQKRKRKKSRYKSWSVP------------------------- : 166
PLGF_HUMAN : -------------------------------------------------- :   -
VEGB_HUMAN : DSPR---------------------------------------------- : 139
VEGC_HUMAN : SIIRRSLPATLPQCQAANKTCPTNYMWNNHICRCLAQEDFMFSSDAGDDS : 272
VEGD_HUMAN : IRRSIQIPEEDRCSHSKKLCPIDMLWDSNKCKCVLQEENPLAGT------ : 246
990126vegx : -------------------------------------------------- :   -

                     *       420         *       440         *
VEGF_HUMAN : -------------------------------------------------- :   -
PLGF_HUMAN : -------------------------------------------------- :   -
VEGB_HUMAN : -------------------------------------------------- :   -
VEGC_HUMAN : TDGFHDICGPNKELDEETCQCVCRAGLRPASCGPHKELDRNSCQCVCKNK : 322
VEGD_HUMAN : ------------------------------------EDHSHLQEPALCGP : 260
990126vegx : -------------------------------------------------- :   -

                   460         *       480         *       500
VEGF_HUMAN : ---------CGPCSERRKHLFVQDPQTCKC-SCKNTDSRCKARQLELNER : 206
PLGF_HUMAN : ---------CGDAVPRR--------------------------------- : 149
VEGB_HUMAN : ----------PLCPRCTQHHQRPDPRTCRCRCRRRSFLRCQGRGLELNPD : 179
VEGC_HUMAN : LFPSQCGANREFDENTCQCVCKRTCPRNQPLNPGKCACECTESPQKCLLK : 372
VEGD_HUMAN : HMMFDEDRCECVCKTPCPKDLIQHPKNCSCFECKESLETCCQKHKLFHPD : 310
990126vegx : -------------------------------------------------- :   -

                     *       520         *       540         *
VEGF_HUMAN : TCRCDKPRR----------------------------------------- : 215
PLGF_HUMAN : -------------------------------------------------- :   -
VEGB_HUMAN : TCRCRKLRR----------------------------------------- : 188
VEGC_HUMAN : GKKFHHQTCSCYRRPCTNRQKACEPGFSYSEEVCRCVPSYWKRPQMS--- : 419
VEGD_HUMAN : TCSCEDRCPFHTRPCASGKTACAKHCRFPKEKRAAQGPHSRKNP------ : 354
990126vegx : -------------------------------------------------- :   -
```

FIG. 12. **Variant Polypeptide Sequences**

```
                 *        20         *        40         *
FL_seq   : MSLFGLLLLTSALAGQRQGTQAESNLSSKFQFSSNKEQNGVQDPQHERII :  50
clone41  : MSLFGLLLLTSALAGQRQGTQAESNLSSKFQFSSNKEQNGVQDPQHERII :  50
clone20  : MSLFGLLLLTSALAGQRQGTQAESNLSSKFQFSSNKEQNGVQDPQHERII :  50

                  60         *        80         *       100
FL_seq   : TVSTNGSIHSPRFPHTYPRNTVLVWRLVAVEENVWIQLTFDERFGLEDPE : 100
clone41  : TVSTNGSIHSPRFPHTYPRNTVLVWRLVAVEENVWIQLTFDERFGLEDPE : 100
clone20  : TVSTNGSIHSPRFPHTYPRNTVLVWRLVAVEENVWIQLTFDERFGLEDPE : 100

                 *       120         *       140         *
FL_seq   : DDICKYDFVEVEEPSDGTILGRWCGSGTVPGKQISKGNQIRIRFVSDEYF : 150
clone41  : DDICKYDFVEVEEPSDGTILGRWCGSGTVPGKQISKGNQIRIRFVSDEYF : 150
clone20  : DDICKYDFVEVEEPSDGTILGRWCGSGTVPGKQISKGNQIRIRFVSDEYF : 150

                 160         *       180         *       200
FL_seq   : PSEPGFCIHYNIVMPQFTEAVSPSVLPPSALPLDLLNNAITAFSTLEDLI : 200
clone41  : PSEPSNRGGKIIQLHTS--------------------------------- : 167
clone20  : PSEPGFCIHYNIVMPQFTEAVSPSVLPPSALPLDLLNNAITAFSTLEDLI : 200

                 *       220         *       240         *
FL_seq   : RYLEPERWQLDLEDLYRPTWQLLGKAFVFGRKSRVVDLNLLTEEVRLYSC : 250
clone41  : -------------------------------------------------- :   -
clone20  : RYLEPERWQLDLEDLYRPTWQLLGKAFVFGRKSRVVDLNLLTE------- : 243

                 260         *       280         *       300
FL_seq   : TPRNFSVSIREELKRTDTIFWPGCLLVKRCGGNCACCLHNCNECQCVPSK : 300
clone41  : -------------------------------------------------- :   -
clone20  : -------------------------------------------------- :   -

                 *       320         *       340
FL_seq   : VTKKYHEVLQLRPKTGVRGLHKSLTDVALEHHEECDCVCRGSTGG : 345
clone41  : --------------------------------------------- :   -
clone20  : ------EVLQLRPKTGVRGLHKSLTDVALEHHEECDCVCRGSTGG : 282
```

FIG. 13. **Primers for Expression of VEGF-X**

*E.coli expression of domain-*

vegx-6 AATTGGATCCGAGAGTGGTGGATCTGAACC

vegx-7 AATTGGATCCGGGAAGAAAATCCAGAGTGG

vegx-8 GGTTGAATTCATTATTTTTTAGTAACTTTGCTTGGGACAC

vegX-9 AATTGAATTCATTATCCTCCTGTGCTCCCTC

*Baculovirus/insect cell expression of full-length protein-*

vegbac1
AATTGGATCCGGAGTCTCACCATCACCACCATCATGAATCCAACCTGAGTAGTAAATTC
C

vegbac2 AATTGAATTCGCTATCCTCCTGTGCTCCCTCTGC

FIG. 14.

>3993180H1 LUNGNON03 INCYTE
CACAAATCACTCACCGACGTGGCCCTGGAGCACCATGAGGNGTGTGACTGTGTGTGCAGAGGGAGCACAGGAGGATAGCC
GCATCACCACCAGCAGCTCTTGCCCAGAGCTGTGCAGTGCAGTGGCTGATTCTATTAGAGAACGTATGCGTTATCTCCAT
CCTTAATCTCAGTTGTTTGCTTCAAGGACCTTTCATCTTCAGGATTTACAGTGCATTCTGAAAGAGGAGACATCAAACAG
AATTAGGAGTTGTGCAACAGCTCTTTTGAGAGGAGGCTAAAGGACAGGAGAANAGGTCTT
>3510192H1 CONCNOT01 INCYTE
TGCAGTGCAGTGGCTGATTCTATTAGAGAACGTATGCGTTATCTCCATCCTTAATCTCAGTTGTTTGCTTCAAGGACCTT
TCATCTTCAGGATTTACAGTGCATTCTGAAAGAGGAGACATCAAACAGAATTAGGAGTTGTGCAACAGCTCTTTTGAGAG
GAGGCCTAAAGGACAGGAGAAAAGGTCTTCAATCGTGGAAAGAAAATTAAATGTTGTATTAAATAGATCACCAGCTAGTT
TCAGAGTTACCATGTACGTATTCCACTAGCTGGGTTCTGTATTT
>2559870H1 ADRETUT01 INCYTE
CACGAGGTCCTTCAGTTGAGACCAAAGACCGGTGTCAGGGGATTGCACAAATCACTCACCGACGTGGCCCTGGAGCACCA
TGAGGAGTGTGACTGTGTGTGCAGAGGGAGCACAGGGGGATAGCCGCATCACCACCAGCAGCTCTTGCCCAGAGCTGTGC
AGTGCAGTGGCTGATTCTATTAGAGAACGTATGCGTTATCTCCATCCTTAATCTCAGTTGTTTGCTTCAAGGACCTTTCA
TCTTCAGGATTTACAGTGCATTCTGAAAGAGGAGA
>3979767H1 LUNGTUT08 INCYTE
GGAGGATAGCCGCATCACCACCAGCAGCTCTTGCCCAGAGCTGTGCAGTGCAGTGGCTGATTCTATTAGAGAACGTATGC
GTTATCTCCATCCTTAATCTCAGTTGTTTGCTTCAAGGACCTTTCATCTTCAGGATTTACAGTGCATTCTGAAAGAGGAG
ACATCAAACAGAATTAGGAGTTGTGCAACAGCTCTTTTGAGAGGAGGCCTAAAGGACAGGAGAAAAGGTCTTCAATCGTG
GAAAGAANATTAAATGTTGTATTAAATAGACACCAGCT
>3980011H1 LUNGTUT08 INCYTE
GGAGGATAGCCGCATCACCACCAGCAGCTCTTGCCCAGAGCTGTGCAGTGCAGTGGCTGATTCTATTAGAGAACGTATGC
GTTATCTCCATCCTTAATCTCAGTTGTTTGCTTCAAGGACCTTTCATCTTCAGGATTTACATGCATTCTGAAAGAGGAGA
CATCAAACAGAATTAGGAGTTGTGCAACAGCTCTTTTGAGAGGAGGCCTAAAGGACAGGAGAAAAGGTCTTCAATCGTGG
AAAGAAAATTAAATGTTGTATTAAATAGATCACCA
>4825396H1 BLADDIT01 INCYTE
GAGAACCGATACCATTTTCTGGCCAGGTTGTCTCCTGGTTAAACGCTGTGGTGGGAACTGTGCCTGTTGTCTCCACAATT
GCAATGAATGTCAATGTGTCCCAAGCAAAGTTACTAAAAAATACCACGAGGTCCTTCAGTTGAGACCAAAGACCGGTGTC
AGGGGATTGCACAAATCACTCACCGACGTGGCCCTGGAGCACCATGAGGAGTGTGACTGTGTGTGCAGAGGGAGCACAGG
AGGATAGCCGCATCACCACCA
>3073703H1 BONEUNT01 INCYTE
AGAAAATCCAGAGTGGTGGATCTGAACCTTCTAACAGAGGAGGTAAGATTATACAGCTGCACACCTCGTAACTTCTCAGT
GTCCATAAGGGAAGAACTAAAGAGAACCGATACCATTTTCTGGCCAGGTTGTCTCCTGGTTAAACGCTGTGGTGGGAACT
GTGCCTGTTGTCTCCACAATTGCAATGAATGTCAATGTGTCCCAAGCAAAGTTACTAAAAAATACCACGAGGTCCTTCAG
TTGAGACCAAAGACCGGTGTCAGGGGATTGCACAAATCA
>1302516H1 PLACNOT02 INCYTE
AGGAAATCAAATTAGGATAAGATTTGTATCTGATGAATATTTCCTTCTGAACCTTCTAACAGAGGAGGTAAGATTATAC
AGCTGCACACCTCGTAACTTCTCAGTGTCCATAAGGGAAGAACTAAAGAGAACCGATACCATTTTCTGGCCAGGTTGTCT
CCTGGTTAAACGCTGTGGTGGGAACTGTGCCTGTTGTCTCCCACAATTGCAATGAATGTCAATGTGTCCCAAGCAAAGTT
ACTAAAAAATACCACGAGGTCC
>3684109H1 HEAANOT01 INCYTE
ATTTCATCTTCAGGATTTACAGTGCATTCTGAAANAGGAGAAATCAAACANAATTAGGAGTTGTGCAACAGCTCTTTTGA
GAGGAGGCCTAAAGGACAGGAGAAAAGGTCTTCAATCGTGGAAANAAAATTAAATGTTGTATTAAATAGATCACCAGCTA
GTTTCAGAGTTACCATGTACGTATTCCACTAGCTGGGTTCTGTATTTCAGTTCTTTCGATACGGCTTAGGGTAATGTCAG
TACAGGAAAAAAACTGTGCAAGTGAGCACCTGATTCCGTTGCCTTGCTT
>4713188H1 BRAIHCT01 INCYTE
CAAAGTTACTAAAAAATACCACGAGGTCCTTCAGTTGAGACCAAAGACCGGTGTCAGGGGATTGCACAAATCACTCACCG
ACGTGGCCCTGGAGCACCATGAGGAGTGTGACTGTGTGTGCAGAGGGAGCACAGGAGGATAGCCGCATCACCACCAGCAG
CTCTTGCCCAGAGCTGTGCAGTGCAGTGGCTGATTCTATTAGAGAACGTATGCGTTATCTCCATCCTTAATCTCAGTTGT
TTGCT
>458823H1 KERANOT01 INCYTE
ANGAGTTGCCCAGAGCTGTGCAGTGCAGTGGCTGATTCTATTAGAGAACGTATGCGTTATCTCCATCCTTAATCTCAGTT
GTTTGNTTCAAGGACCTTTCATCTTCAGGATTTACAGTGCATTCTGAAAGAGGAGACATCAAACAGAATTAGGAGTTGTG
CAACAGCTCTTTTGAGAGGAGGCCTAAAGGNCAGGAGAAAAGGTCTTCAATCGTGGAAAGAAAATTAAATGTTGTATTAA
ATAGATC
>1303909H1 PLACNOT02 INCYTE
AGGAAATCAAATTAGGATAAGATTTGTATCTGATGAATATTTCCTTCTGAACCTTCTAACAGAGGAGGTAAGATTATAC
AGCTGCACACCTCGTAACTTCTCAGTGTCCATAAGGGAAGAACTAAAGAGAACCGATACCATTTTCTGGCCAGGTTGTCT
CCTGGTTAAACGCTGTGGTGGGAACTGTGCCTGTTGTCTCCACAATTGCAATGAATGTCAATGTGTCCCAAG

FIG. 14 (CONTINUED).

```
>2739211H1      OVARNOT09       INCYTE
GTGCATTCTGAAAGAGGAGACATCAAACAGAATTAGGAGTTGTGCAACAGCTCTTTTGAGAGGAGGCCTAAAGGACAGGA
GAAAAGGTCTTCAATCGTGGAAAGAAAATTAAATGTTGTATTAAATAGATCACCAGCTAGTTTCAGAGTTACCATGTACG
TATTCCACTAGCTGGGTTCTGTATTTCAGTTCTTTCGATACGGCTTAGGGTAATGTCAGTACAGGAAAAAAACTGTGCAA
GTGAGCACCTGAT
>3325591H1      PTHYNOT03       INCYTE
TGCAACAGCTCTTTTGAGAGGAGGCCTAAAGGACAGGAGAAAAGGTCTTCAATCGTGGAAAGAAAATTAAATGTTGTATT
AAATAGATCACCAGCTAGTTTCAGAGTTACCATGTACGTATTCCACTAGCTGGGTTCTGTATTTCAGTTCTTTCGATACG
GCTTAGGGTAATGTCAGTACAGGAAAAAAACTGTGCAAGTGAGCACCTGATTCCGTTGCCTTGCTTAACCCTAAAGCNCC
ATGTCNNGGGCNAAAANCGAAAAAT
>3733565H1      SMCCNOS01       INCYTE
CCTTAATCTCAGTTGTTTGCTTCAAGGACCTTTCATCTTCAGGATTTACAGTGCATTCTGNAAGANGAGACATCAAACAG
AATTAGGNGTTGTGCAAAAGCTCTTTTGAGAGGAGGCCTAAAGGACAGGAGAAAAGGTCTNCAATCGTGGAAAGNAAATT
AAATGTTGTATNAAATNGATCACCAGCTAGTTTCAGAGTTACCATGTACGTATTCCACTAGCTGGGNCNGTATTCAGTCT
TTCGGAACGGCTTAGGGTAATGTCAGTACAGGANAAAAACTGTGCAGTGAG
>3554223H1      SYNONOT01       INCYTE
ATTAAATAGATCACCAGCTAGTTTCAGAGTTACCATGTACGTATTCCACTAGCTGGGTTCTGTATTTCAGTTCTTTCGAT
ACGGCTTAGGGTAATGTCAGTACAGGAAAAAAACTGTGCAAGTGAGCACCTGATTCCGTTGCCTTGGCTTAACTCTAAAG
CTCCATGTCCTGGGCCTAAAATCGTATAAAATCTGGATTTTTTNTTTTTTTTGCGCATATTCACATATGTAAACCAGN
ACATTCTATGTACNACAAACCTGGTTTTTAAAAAGGAAC
>4507477H1      OVARTDT01       INCYTE
GGCTAGTTTCAGAGTTACCATGTACGTATTCCACTAGCTGGGTTCTGTATTTCAGTTCTTTCGATACGGCTTAGGGTAAT
GTCAGTACAGGAAAAAAACTGTGCAAGTGAGCACCTGATTCCGTTGCCTTGCTTAACTCTAAAGCTCCATGTCCTGGGCC
TAAAATCGTATAAAATCTGGA
>4163378H1      BRSTNOT32       INCYTE
AATAGATCACCAGCTAGTTTCAGAGTTACCATGTACGTATTCCACTAGCTGGGNTCTGTATTTCAGTTCCTTTCGATACG
GCTTAGGGTAATGTCAGTACAGGAAAAAAGCTGTGCAAGTGAGCACCTGATTCCGTTGCCTTGCTTAACTCTAAAGCTCC
ATGTCCTGGGCCTAAAATCGTATA
```

FIG. 15.

>2054675H1 BEPINOT01 INCYTE
AAAGGAACTATGTTGCTATGAATTAAACTTGTGTCGTGCTGATAGGACAGACTGGATTTTTCATATTTCTTATTAAAATT
TCTGCCATTTAGAAGAAGAGAACTACATTCATGGTTTGGAAGAGATAAACCTGAAAAGAAGAGTGGCCTTATCTTCACTT
TATCGATAAGTCAGTTTATTTGTTTCATTGTGTACATTTTTATATTCTCCTTTTGACATTATAACTGTTGGCTTTTCTAA
TCTTGTTAAATATATCTATTTTTACCAAAGGTATTTAATATTCTTTTTTA
>3993180H1 LUNGNON03 INCYTE
CACAAATCACTCACCGACGTGGCCCTGGAGCACCATGAGGNGTGTGACTGTGTGTGCAGAGGGAGCACAGGAGGATAGCC
GCATCACCACCAGCAGCTCTTGCCCAGAGCTGTGCAGTGCAGTGGCTGATTCTATTAGAGAACGTATGCGTTATCTCCAT
CCTTAATCTCAGTTGTTTGCTTCAAGGACCTTTCATCTTCAGGATTTACAGTGCATTCTGAAAGAGGAGACATCAAACAG
AATTAGGAGTTGTGCAACAGCTCTTTTGAGAGGAGGCTAAAGGACAGGAGAANAGGTCTT
>3510192H1 CONCNOT01 INCYTE
TGCAGTGCAGTGGCTGATTCTATTAGAGAACGTATGCGTTATCTCCATCCTTAATCTCAGTTGTTTGCTTCAAGGACCTT
TCATCTTCAGGATTTACAGTGCATTCTGAAAGAGGAGACATCAAACAGAATTAGGAGTTGTGCAACAGCTCTTTTGAGAG
GAGGCCTAAAGGACAGGAGAAAAGGTCTTCAATCGTGGAAAGAAAATTAAATGTTGTATTAAATAGATCACCAGCTAGTT
TCAGAGTTACCATGTACGTATTCCACTAGCTGGGTTCTGTATTT
>4164633H1 BRSTNOT32 INCYTE
CTTGTTAAATATATCTATTTTTACCAAAGGTATTTAATATTCTTTANTTATGACAACTTAGATCAACTATTTTTAGCTTG
GTAAATTTTTCTAAACACAATTGTTATAGCCAGAGGAACAAAGATGATATAAAATATTGTTGCTCTGACAAAAATACATG
TATTTCATTCTCGTATGGTGCTAGAGTTAGATTAATCTGCATTTTAAAAAACTGAATTGGAATAGAATTGGTAAGTTGCA
AAGACTTTTTGANAATAATTAAATTATCATATCTTCCATTCCTGTTATTGGGGGAGAAAAT
>2559870H1 ADRETUT01 INCYTE
CACGAGGTCCTTCAGTTGAGACCAAAGACCGGTGTCAGGGGATTGCACAAATCACTCACCGACGTGGCCCTGGAGCACCA
TGAGGAGTGTGACTGTGTGTGCAGAGGGAGCACAGGGGGATAGCCGCATCACCACCAGCAGCTCTTGCCCAGAGCTGTGC
AGTGCAGTGGCTGATTCTATTAGAGAACGTATGCGTTATCTCCATCCTTAATCTCAGTTGTTTGCTTCAAGGACCTTTCA
TCTTCAGGATTTACAGTGCATTCTGAAAGAGGAGA
>3817470H1 BONSTUT01 INCYTE
TTAAAAAGGAACTATGTTGCTATGAATTAAACTTGTGTCATGCTGATAGGACAGACTGGATTTTTCATATTTCTTATTAA
AATTTCTGCCATTTAGAAGAAGAGAACTACATTCATGGTTTGGAAGAGATAAACCTGAAAAGAAGAGTGGCCTTATCTTC
ACTTTATCGATAAGTCAGTTTATTTGTTTCATTGTGTACATTTTTATATTCTCCTTTTGACATTATAACTGTTGGCTTTC
TAATCTGTTAAATATATCTATTTTTACCAAAGGTATTTAATATTCTTT
>3979767H1 LUNGTUT08 INCYTE
GGAGGATAGCCGCATCACCACCAGCAGCTCTTGCCCAGAGCTGTGCAGTGCAGTGGCTGATTCTATTAGAGAACGTATGC
GTTATCTCCATCCTTAATCTCAGTTGTTTGCTTCAAGGACCTTTCATCTTCAGGATTTACAGTGCATTCTGAAAGAGGAG
ACATCAAACAGAATTAGGAGTTGTGCAACAGCTCTTTTGAGAGGAGGCCTAAAGGACAGGAGAAAAGGTCTTCAATCGTG
GAAAGAANATTAAATGTTGTATTAAATAGACACCAGCT
>3980011H1 LUNGTUT08 INCYTE
GGAGGATAGCCGCATCACCACCAGCAGCTCTTGCCCAGAGCTGTGCAGTGCAGTGGCTGATTCTATTAGAGAACGTATGC
GTTATCTCCATCCTTAATCTCAGTTGTTTGCTTCAAGGACCTTTCATCTTCAGGATTTACATGCATTCTGAAAGAGGAGA
CATCAAACAGAATTAGGAGTTGTGCAACAGCTCTTTTGAGAGGAGGCCTAAAGGACAGGAGAAAAGGTCTTCAATCGTGG
AAAGAAAATTAAATGTTGTATTAAATAGATCACCA
>4825396H1 BLADDIT01 INCYTE
GAGAACCGATACCATTTTCTGGCCAGGTTGTCTCCTGGTTAAACGCTGTGGTGGGAACTGTGCCTGTTGTCTCCACAATT
GCAATGAATGTCAATGTGTCCCAAGCAAAGTTACTAAAAAATACCACGAGGTCCTTCAGTTGAGACCAAAGACCGGTGTC
AGGGGATTGCACAAATCACTCACCGACGTGGCCCTGGAGCACCATGAGGAGTGTGACTGTGTGTGCAGAGGGAGCACAGG
AGGATAGCCGCATCACCACCA
>3073703H1 BONEUNT01 INCYTE
AGAAAATCCAGAGTGGTGGATCTGAACCTTCTAACAGAGGAGGTAAGATTATACAGCTGCACACCTCGTAACTTCTCAGT
GTCCATAAGGGAAGAACTAAAGAGAACCGATACCATTTTCTGGCCAGGTTGTCTCCTGGTTAAACGCTGTGGTGGGAACT
GTGCCTGTTGTCTCCACAATTGCAATGAATGTCAATGTGTCCCAAGCAAAGTTACTAAAAAATACCACGAGGTCCTTCAG
TTGAGACCAAAGACCGGTGTCAGGGGATTGCACAAATCA
>862169H1 BRAITUT03 INCYTE
AGATGATATAAAATATTGTTGCTCTGACAAAAATACATGTATTTCATTCTCGTATGGTGCTAGAGTTAGATTAATCTGCA
TTTTAAAAAACTGAATTGGAATAGAATTGGTAAGTTGCAAAGACTTTTTGAAAATAATTAAATTATCATATCTTCCATTC
CTGTTATTGGAGATGAAAATAAAAAGCAACTTATGAAAGTAGACATTCAGATCCAGCCATTACTAACCTATTCCTTTTTT
GGGGAAATCTGAGCCTAGC
>4201385H1 BRAITUT29 INCYTE
TTTTTAAAAAGGAACTATGTTGCTATGAATTAAACTTGTGTCGTGCTGATAGGACAGACTGGATTTTTCATATTTCTTAT
TAAAATTTCTGCCATTTAGAAGAAGAGAACTACATTCATGGTTTGGAAGAGATAAACCTGAAAAGAAGAGTGGCCTATCT
TCACTTTATCGATAAGTCAGTTTATTTGTTTCATTGTGTACATTTTTATATTCTCCTTTGACATATAACTGTTGGCTTTT

FIG. 15 (CONTINUED 1).

CTAATCTGTTAAATATATCTATTTTTACCAAAGGTATTTAATAT
>1302516H1 PLACNOT02 INCYTE
AGGAAATCAAATTAGGATAAGATTTGTATCTGATGAATATTTCCTTCTGAACCTTCTAACAGAGGAGGTAAGATTATAC
AGCTGCACACCTCGTAACTTCTCAGTGTCCATAAGGGAAGAACTAAAGAGAACCGATACCATTTTCTGGCCAGGTTGTCT
CCTGGTTAAACGCTGTGGTGGGAACTGTGCCTGTTGTCTCCCACAATTGCAATGAATGTCAATGTGTCCCAAGCAAAGTT
ACTAAAAAATACCACGAGGTCC
>3684109H1 HEAANOT01 INCYTE
ATTTCATCTTCAGGATTTACAGTGCATTCTGAAANAGGAGAAATCAAACANAATTAGGAGTTGTGCAACAGCTCTTTTGA
GAGGAGGCCTAAAGGACAGGAGAAAAGGTCTTCAATCGTGGAAANAAAATTAAATGTTGTATTAAATAGATCACCAGCTA
GTTTCAGAGTTACCATGTACGTATTCCACTAGCTGGGTTCTGTATTTCAGTTCTTTCGATACGGCTTAGGGTAATGTCAG
TACAGGAAAAAAAACTGTGCAAGTGAGCACCTGATTCCGTTGCCTTGCTT
>2549720H1 LUNGTUT06 INCYTE
TTAGCTTGGNAAATTTTTCTAAACACAATTGTTATAGCCAGAGGAACAAAGATGATATAAAATATTGTTGCTCTGACAAA
AATACATGTATTTCATTCTCGTATGGTGCTAGAGTTAGATTAATCTGCATTTTAAAAAACTGAATTGGAATAGAATTGGT
AAGTTGCAAAGACTTTTTGAAAATAATTAAATTATCATATCTTCCATTCCTGTTATTGGAGATGAAAATAAAAAGCAACT
TATGANAGTAG
>877279H1 LUNGAST01 INCYTE
CTTTTTTATGACAACTTAGATCAACTATTTTTAGCTTGGTAAATTTTTCTAAACACAATTGTTATAGCCAGAGGAACAAA
GATGATATAAAATATTGTTGCTCTGACAAAAATACATGTATTTCATTCTCGTATGGTGCTAGAGTTAGATTAATCTGCAT
TTTAAAAAACTGAATTGGAATAGAATTGGTAAGTTGCAAAGGCTTTTTGAAAATAATTAAATTATCATATCTTCCATTCC
TGTTATTGGNGG
>4713188H1 BRAIHCT01 INCYTE
CAAAGTTACTAAAAAATACCACGAGGTCCTTCAGTTGAGACCAAAGACCGGTGTCAGGGGATTGCACAAATCACTCACCG
ACGTGGCCCTGGAGCACCATGAGGAGTGTGACTGTGTGTGCAGAGGGAGCACAGGAGGATAGCCGCATCACCACCAGCAG
CTCTTGCCCAGAGCTGTGCAGTGCAGTGGCTGATTCTATTAGAGAACGTATGCGTTATCTCCATCCTTAATCTCAGTTGT
TTGCT
>2171082H1 ENDCNOT03 INCYTE
AGATAAACCTGAAAAGAAGAGTGGCCTTATCTTCACTTTATCGATAAGTCAGTTTATTTGTTTCATTGTGTACATTTTTA
TATTCTCCTTTTGACATTATAACTGTTGGCTTTTCTAATCTTGTTAAATATATCTATTTTTACCAAAGGTATTTAATATT
CTTTTTTATGACAACTTAGATCAACTATTTTTAGCTTGGTAAATTTTTCTAAACACAATTGTTATAGCCAGAGGAACAAA
GATGA
>875860H1 LUNGAST01 INCYTE
CTGGATTTTTCATATTTCTTATTAAAATTTCTGCCATTTAGAAGAAGAGAACTACATTCATGGTTTGGAAGAGATAAACC
TGAAAAGAAGAGTGGCCTTATCTTCACTTTATCGATAAGTCAGTTTATTTGTTTCATTGTGTACATTTTTATATTCTCCT
TTTGACATTATAACTGTTGGCTTTTCTAATCTTGTTAAATATATCTATTTTTACCAAAGGTATTTAATATTCTTTTTTAT
GAC
>706168H1 SYNORAT04 INCYTE
GCTCATATTCACATATGTAAACCAGAACATTCTATGTACTACAAACCTGGTTTTTAAAAAGGANCTATGTTGCTATGAAT
TAAACTTGTGTCGTGCTGATAGGACAGACTGGATTTTTCATATTTCTTATTAAAATTTCTGCCATTTAGAAGAAGAGAAC
TACATTCATGGTTTGGAAGAGATAAACCTGAAAAGAAGAGTGGCCTTATCTTCANTTTATCGATAAGTCAGTTTATTTGT
TTCA
>458823H1 KERANOT01 INCYTE
ANGAGTTGCCCAGAGCTGTGCAGTGCAGTGGCTGATTCTATTAGAGAACGTATGCGTTATCTCCATCCTTAATCTCAGTT
GTTTGNTTCAAGGACCTTTCATCTTCAGGATTTACAGTGCATTCTGAAAGAGGAGACATCAAACAGAATTAGGAGTTGTG
CAACAGCTCTTTTGAGAGGAGGCCTAAAGGNCAGGAGAAAAGGTCTTCAATCGTGGAAAGAAAATTAAATGTTGTATTAA
ATAGATC
>538436H1 LNODNOT02 INCYTE
AAAGATGATATAAAATATTGTTGCTCTGACAAAAATACATGTATTTCATTCTCGTATGGTGCTAGAGTTAGATTAATCTG
CATTTTAAAAAACTGAATTGGAATAGAATTGGTAAGTTGCAAAGACTTTTTGAAAATAATTAAATTATCATATCTTCCAT
TCCTGTTATTGGAGATGAAAATAAAAAGCAACTTATGAAAGTAGACATTCAGATCCAGCCATTACTAACCTAT
>1303909H1 PLACNOT02 INCYTE
AGGAAATCAAATTAGGATAAGATTTGTATCTGATGAATATTTCCTTCTGAACCTTCTAACAGAGGAGGTAAGATTATAC
AGCTGCACACCTCGTAACTTCTCAGTGTCCATAAGGGAAGAACTAAAGAGAACCGATACCATTTTCTGGCCAGGTTGTCT
CCTGGTTAAACGCTGTGGTGGGAACTGTGCCTGTTGTCTCCACAATTGCAATGAATGTCAATGTGTCCCAAG
>2739211H1 OVARNOT09 INCYTE
GTGCATTCTGAAAGAGGAGACATCAAACAGAATTAGGAGTTGTGCAACAGCTCTTTTGAGAGGAGGCCTAAAGGACAGGA
GAAAAGGTCTTCAATCGTGGAAAGAAAATTAAATGTTGTATTAAATAGATCACCAGCTAGTTTCAGAGTTACCATGTACG
TATTCCACTAGCTGGGTTCTGTATTTCAGTTCTTTCGATACGGCTTAGGGTAATGTCAGTACAGGAAAAAAACTGTGCAA
GTGAGCACCTGAT

FIG. 15(CONTINUED 2).

>2550343H1 LUNGTUT06 INCYTE
TGTACATTTTATATTCTCCTTTTGACATTATAACTGTTGGCTTTTCNAATCTTGTTAAATATATCTATTTTTACCAAAG
GTATTTAATATTCTTTTTTATGACAACTTAGATCAACTATTTTTAGCTTGGTAAATTTTTCTAAACACAATTGTTATAGC
CAGAGGAACAAAGATGATATAAAATATTGTTGCTCTGACAAAAATACATGTATTTCATTCTCGTATGGTGCTA
>5321148H1 FIBPFEN06 INCYTE
CACAATTGTTATAGCCAGAGGAACAAAGATGATATAAAATATTGTTGCTCTGNCAAAAATACATGTATTTCATTCTCGTA
TGGTGCTAGAGTTAGATTAATCTGCATTTTAAAAAACTGAATTGGAATAGAATTGGTAAGTTGCAAAGACTTTTTGAAAA
TAATTAAATTATCATATCTTCCATTCCTGTTATTGGAGATGAAAATAAAAAGCAACTTATGAAAGTAAATTCAGATCCAC
CATTACTAAC
>879495H1 THYRNOT02 INCYTE
ATTTCATTCTCGTATGGTGCTAGAGTTAGATTAATCTGCATTTTAAAAAACTGAATTGGAATAGAATTGGTAAGTTGCAA
AGACTTTTTGAAAATAATTAAATTATCATATCTTCCATTCCTGTTATTGGAGATGAAAATAAAAAGCAACTTATGAAAGT
AGACATTCAGATCCAGCCATTACTAACCTATTCCTTTTTGGGGAAATCTGAGCCTAGCTCAGAAAAACATAAAGCACCT
TGAAAAA
>3325591H1 PTHYNOT03 INCYTE
TGCAACAGCTCTTTTGAGAGGAGGCCTAAAGGACAGGAGAAAAGGTCTTCAATCGTGGAAAGAAAATTAAATGTTGTATT
AAATAGATCACCAGCTAGTTTCAGAGTTACCATGTACGTATTCCACTAGCTGGGTTCTGTATTTCAGTTCTTTCGATACG
GCTTAGGGTAATGTCAGTACAGGAAAAAAACTGTGCAAGTGAGCACCTGATTCCGTTGCCTTGCTTAACCCTAAAGCNCC
ATGTCNNGGGCNAAAANCGAAAAAT
>543890H1 OVARNOT02 INCYTE
TTTCTAAACACAATTGTTATAGCCAGAGGAACAAAGATGATATAAAATATTGTTGCTCTGACAAAAATACATGTATTTCA
TTCTCGTATGGTGCTAGAGTTAGATTAATCTGCATTTTAAAAAACTGAATTGGNATAGAATTGGTAAGTTGCAAAGNCTT
TTTGAAAATAATTAAATTATCATATCTTCCATTCCTGTTATTGGAGGATGGAAAATAAAAAGCAACTTATGGAAAGTAGG
ACATTCAGATC
>3733565H1 SMCCNOS01 INCYTE
CCTTAATCTCAGTTGTTTGCTTCAAGGACCTTTCATCTTCAGGATTTACAGTGCATTCTGNAAGANGAGACATCAAACAG
AATTAGGNGTTGTGCAAAAGCTCTTTTGAGAGGAGGCCTAAAGGACAGGAGAAAAGGTCTNCAATCGTGGAAAGNAAATT
AAATGTTGTATNAAATNGATCACCAGCTAGTTTCAGAGTTACCATGTACGTATTCCACTAGCTGGGNCNGTATTCAGTCT
TTCGGAACGGCTTAGGGTAATGTCAGTACAGGANAAAAACTGTGCAGTGAG
>4641939H1 PROSTMT03 INCYTE
GTACTACAAACCTGGTTTTTAAAAAGGAACTATGTTGCTATGAATTAAACTTGTGTCCATGCTGATAGGACAGACTGGAT
TTTNCATATTTCTTATTAAAATTTCTGCCATTTAGAAGAAGAGAACTACATTCATGGTTTGGNAGAGATAAACCTGAAAA
GAAGAGTGGCCTTATCTTCACTTTATCGATAAGTCAGTTTATTTGTTTCATGTGTACATTTTTATATTCTCCTTTGACAT
ATAACGTGGCTTT
>2007780H1 TESTNOT03 INCYTE
TTATATTCTCCTTTTGACATTATAACTGTTGGCTTTTCTAATCTTGTTAAATATATCTATTTTTACCAAAGGTATTTAAT
ATTCTTTTTTATGACAACTTAGATCAACTATTTTTAGCTTGGTAAATTTTTCTAAACACAATTGTTATAGCCAGAGGAAC
AAAGATGATATAAAATATTGTTGCTCTGANAAAAATACATGTAT
>3085331H1 HEAONOT03 INCYTE
GCTCATATTCACATATGTAAACCAGAACATTCTATGTACTACAAACCTGGTTTTTAAAAAGGAACTATTTGCTATGAATT
AAACTTGTGTCGTGCTGATAGGACAGACTGGNTTTTCATATTTCTTATTANAATTTCTGCCATTAGAAGAAGAGAACTA
CATTCATGGTTTGGAAGAGATAAACCTGAAAAGAAGAGTGGCCTATTTCACTTTATCGATAAGTCAGT
>3414043H1 PTHYNOT04 INCYTE
GCTCATATTCACATATGTAAACCAGAACATTCTATGTACTACAAACCTGGTTTTTAAAAAGGAACTATGTTGCTATGAAT
TAAACTTGTGTCGTGCTGATAGGACAGACTGGATTTTTCATATTTCTTATTAAAATTTCTGCCATTTAGAAGAAGAGAAC
TACATTCATGGTTTGGAAGAGATAAACCTGAAA
>3705963H1 PENCNOT07 INCYTE
ANACTGTGCAAGTGAGCACCTGATTCCGTTGCCTTGCTTAACTCTAAAGCTCCATGTCCTGGGCCTAAAATCGTATAAAA
TCTGGAnnnnnnnnnnnnnnnnnnGCTCATATTCACATATGTAAACCAGAACATTCTATGTACTACAAACCTGGTTTTTA
AAAAGGAACTATGTTGCTATGAATTAAACTTGTGTCGTGCTGATAGGACAGACTGGATTTTTCATATTTCTTATTAAAAT
TTCTGCCATTAGAAGAAGAGAACTACNTTCANGGTTTGGAAGAGATAACCCTGAAAAGANGGG
>5137051H1 OVARDIT04 INCYTE
AAAAAACTGAATTGGAATAGAATTGGTAAGTTGCAAAGACTNTTTGAAAATAATTAAATTATCATATCTTCCATTCCTGT
TATTGGAGATGAANATAAAAAGCAACTTATGAAAGTAGACATTCAGATCCAGCCATTACTAACCTATTCCTTTTTTGGGG
AAATCTGAGCCTAGCTCAGAAAAACATAAAGCACCTTGAAAAAGACTTGGCAGCTTCCTGATAAAGCGTGCTGTNTGTCA
GTAGGAACACATCCTATTTATTGTGATGNTGTGGTTTATTAT
>3554223H1 SYNONOT01 INCYTE
ATTAAATAGATCACCAGCTAGTTTCAGAGTTACCATGTACGTATTCCACTAGCTGGGTTCTGTATTTCAGTTCTTTCGAT
ACGGCTTAGGGTAATGTCAGTACAGGAAAAAAACTGTGCAAGTGAGCACCTGATTCCGTTGCCTTGGCTTAACTCTAAAG

FIG. 15 (CONTINUED 3).

CTCCATGTCCTGGGCCTAAAATCGTATAAAATCTGGATTTTTTNTTTTTTTTGCGCATATTCACATATGTAAACCAGN
ACATTCTATGTACNACAAACCTGGTTTTAAAAAGGAAC
>4507477H1 OVARTDT01 INCYTE
GGCTAGTTTCAGAGTTACCATGTACGTATTCCACTAGCTGGGTTCTGTATTTCAGTTCTTTCGATACGGCTTAGGGTAAT
GTCAGTACAGGAAAAAAACTGTGCAAGTGAGCACCTGATTCCGTTGCCTTGCTTAACTCTAAAGCTCCATGTCCTGGGCC
TAAAATCGTATAAAATCTGGA
>1955646H1 CONNNOT01 INCYTE
TGGTAAGTTGCAAAGACTTTTTGAAAATAATTAAATTATCATATCTTCCATTCCTGTTATTGGAGATGAAAATAAAAAGC
AACTTATGAAAGTAGACATTCAGATCCAGCCATTACTAACCTATTCCTTTTTGGGGAAATCTGAGCCTAGCTCAGAAAA
ACATAAAGCACCTTGAAAAAGACTTGGCAGCTTCCTGATAAAGCGTGCTGTGCTGTGCAGTAGGGAACACATCCTATTTA
TTGTGATGTTGTGGTTTATATCCTAAACC
>4163378H1 BRSTNOT32 INCYTE
AATAGATCACCAGCTAGTTTCAGAGTTACCATGTACGTATTCCACTAGCTGGGNTCTGTATTTCAGTTCCTTTCGATACG
GCTTAGGGTAATGTCAGTACAGGAAAAAAGCTGTGCAAGTGAGCACCTGATTCCGTTGCCTTGCTTAACTCTAAAGCTCC
ATGTCCTGGGCCTAAAATCGTATA
>5095141H1 EPIMNON05 INCYTE
AGATAAACCTGAAAAGAAGAGTGGCCTTATNTTCACTTTATCGATAAGTCAGNTTATTTGTTTCATTGTGTACATTTNNA
TATTCTCCTTTTGACATTATAACTGNTGGCTTTTCTAANCNTGTTAAATATATCTATTTTTACCAAAGGTATTTAATATT
CTTT
>943826H1 ADRENOT03 INCYTE
TATGGTGCTAGAGTTAGATTAATCTGCATTTTAAAAAACTGAATTGGAATAGAATTGGTAAGTTGCAAAGACTTTTTGAA
AATAATTAAATTATCATATCTTCCATTCCTGTTATTGGAGATGAAAATAAAAAGCAACTTATG
>3451273H1 UTRSNON03 INCYTE
TTTTTTNTTTTGCTCATATTCACATATGTAAACCNGAACATTCTATGTACNACAAACCTGGTTTTAAAAAGGAACTATG
TTGCTATGAATTAAACTTGTGTCGTGCTGATAGGACAGACTGGATTTTTCANATTTCTTANTAANNTTTCTGCCATTTAG
AAGA
>1402278H1 LATRTUT02 INCYTE
GTACAGGAAAAAAACTGTGCAAGTGAGCACCTGATTCCGTTGCCTTGCTTAACTCTAAAGCTCCATGTCCTGGGCCTAAA
ATCGTATAAAATCTGGAnnnnnnnnnnnnnnnnnnGCTCATATTCACATATGTAAACCAGAACATTCTATGTACTACAAA
CCTGGTTTTAAAAAGGAACTATGTTGCTATGAATTAAACTTGTGTCGTGCTGATAGGACAGACTGGATTTTTCATATTT
CTTA
>4361191H1 SKIRNOT01 INCYTE
GCAAAGACTTTTTGANAATNATTAANTTATCATATCTTCCATTCCTGTTATNGGAGATGANAATAAAAAGCAACTTATGA
AAGTAGACATTCAGATCCAGCCATTACTAACCTATTCCTTTTTGGGGAAATCTGAGCCTAGCNCAGAAAAACATAAAGC
ACCTTGAAAAAGACTTGGCAGCTTCCTGATAAAGCGTGCTGTGCTGTGCAGTAGGAACACATCCNATTTATTGTGNTGTN
GNGGTTTTATGATC
>1307017H1 PLACNOT02 INCYTE
TGTCAGTACAGGAAAAAAACTGTGCAAGTGAGCACCTGATTCCGTTGCCTTGCTTAACTCTAAAGCTCCATGTCCTGGGC
CTAAAATCGTATAAAATCTGGAnnnnnnnnnnnnnnnnnnGCTCATATTCACATATGTAAACCAGAACATTCTATGTACT
ACAAACCTGGTTTTAAAAAGGAACTATGTTGCTATGAATTAAACTTGTGTCATGCTGATAGGACAGACTGGATTTTTCA
TAT
>5032225H1 HEARFET03 INCYTE
AATTATCATATCTTCCATTCCTGTTATTGGAGATGNAAATAAAAAGCAACTTATGAAAGTAGACATTCAGATCCAGCCAT
TACTAACCTATTCCTTTTTGGGGAAATCTGAGCCTAGCTCAGAAAAACATAAAGCACCTTGAAAAAGACTGTCAGCTTC
CTGATAAAGCGTGCTGTGCTGTGCAGTAGGAACACATCCTATTTATTGTGATGTTGTGGTTTTATTATCTTAAACTCGTT
CCAT
>3732621H1 SMCCNOS01 INCYTE
ANAGATGATATAAAANATTGTTGCTCTGACAANNATACATGTATTTCATTCTCGTATGGTGCTAGAGTTAGATTAATCTG
CNTTTTAAAAAACTGANTTGGAATAGANTTGGTAAGTTGCAAAGNCNTTTGAAAATNATTAAGTTATCAGAT
>3530274H1 BLADNOT09 INCYTE
TTCCATTCCTGTTATTGGAGATGAAAATAAAAAGCAACTTATGAAAGTAGACATTCAGATCCAGCCATTACTAACCTATT
CCTTTTTTGGGGAAATCTGAGCCTAGCTCAGAAAAACATAAAGCACCTTGAAAAAGACTTGGCAGCTTCCTGATAAAGCG
TGCTGTGCTGTGCAGTAGGAACACATCCTATTTATTGTGATGTTGTGGTTTTATTATCTAAACTCTGTTCCATACACTTG
TATAAATACATGGATATTTTTATGTACAGAAGTATGTCTCTTAACCAGTTCA
>3530249H1 BLADNOT09 INCYTE
CTTCCATTCCTGTTATTGGAGATGAAAATAAAAAGCAACTTATGANAGTAGACATTCAGATCCAGCCATTACTAACCTAT
TCCTTTTTTGGGGAAATCTGAGCCTAGCTCAGAAAAACATAAAGCACCTTGAAAAAGACTTGGCAGCTTCCTGATAAAGC
GTGCTGTGCTGTGCAGTAGGAACACATCCTATTTATTGTGATGTTGTGGTTTTATTATCTTAAACTCTGTTCCATACACT
TGTATAAATACATGGATATTTTTATGTACAGAAGTATGTCTCTTAACCAGTTCACTTATTGTACCTGG

FIG. 16.

| | | |
|---|---|---|
| VEGFE1 | AAAATGTATGGATACAACTTAC | 22 |
| VEGFE2 | GTTTGATGAAAGATTTGGGCTTG | 23 |
| VEGFE3 | TTTCTAAAGGAAATCAAATTAG | 22 |
| VEGFE4 | GATAAGATTTGTATCTGATG | 20 |
| VEGFE5 | GATGTCTCCTCTTTCAG | 17 |
| VEGFE6 | GCACAACTCCTAATTCTG | 18 |
| VEGFE7 | AGCACCTGATTCCGTTGC | 19 |
| VEGFE8 | TAGTACATAGAATGTTCTGG | 20 |
| VEGFE9 | AAGAGACATACTTCTGTAC | 19 |
| VEGFE10 | CCAGGTACAATAAGTGAACTG | 21 |

FIG. 17.

```
     +3                                         M   N   I   F  L  L
N  L  L  T   E  E  V   R  L  Y
                                               ]------------------
--------------------------------
      1  AGGAAATCAA ATTAGGATAA GATTTGTATC TGATGAATAT TTTCCTTCTG
AACCTTCTAA CAGAGGAGGT AAGATTATAC
         TCCTTTAGTT TAATCCTATT CTAAACATAG ACTACTTATA AAAGGAAGAC
TTGGAAGATT GTCTCCTCCA TTCTAATATG

     +3  S  C  T  P   R  N  F   S  V  S   I  R  E  E   L  K  R
T  D  T   I  F  W  P   G  C  L
         --------------------------]----------------------------
--------------------------------
     81  AGCTGCACAC CTCGTAACTT CTCAGTGTCC ATAAGGGAAG AACTAAAGAG
AACCGATACC ATTTTCTGGC CAGGTTGTCT
         TCGACGTGTG GAGCATTGAA GAGTCACAGG TATTCCCTTC TTGATTTCTC
TTGGCTATGG TAAAAGACCG GTCCAACAGA
     -2                                                    <-----
--------------------------------

     +3  L  V  K   R  C  G  G   N  C  A   C  C  L   H  N  C  N
E  C  Q   C  V  P   S  K  V
         -------------------------------------------------------
--------------------------------
    161  CCTGGTTAAA CGCTGTGGTG GGAACTGTGC CTGTTGTCTC CACAATTGCA
ATGAATGTCA ATGTGTCCCA AGCAAAGTTA
         GGACCAATTT GCGACACCAC CCTTGACACG GACAACAGAG GTGTTAACGT
TACTTACAGT TACACAGGGT TCGTTTCAAT
     -2  -------------------------------------------------------
--------------------------------

     +3 T  K  K  Y   H  E  V   L  Q  L  R   P  K  T   G  V  R
G  L  H  K   S  L  T   D  V  A
         -------------------------------------------------------
--------------------------------
     +1                                                V  S  G
D  C  T   N  H  S  P   T  W  P
                                                       ]--------
--------------------------------
    241  CTAAAAAATA CCACGAGGTC CTTCAGTTGA GACCAAAGAC CGGTGTCAGG
GGATTGCACA AATCACTCAC CGACGTGGCC
         GATTTTTTAT GGTGCTCCAG GAAGTCAACT CTGGTTTCTG GCCACAGTCC
CCTAACGTGT TTAGTGAGTG GCTGCACCGG
     -2  -------------------------------------------------------
--------[

     +3  L  E  H  H   E  E  C   D  C  V   C  R  G  S   T  G  G
```

FIG. 17 (CONTINUED).

```
        ----------------------------------------------------------
>
     +2                                          V   Q   R   E   H   R   R
I  A  A  S   P  P  A   A  L  A
                                                 ]----------------------
--------------------------------
     +1  W  S  T   M  R  S  V   T  V  C   A  E  G   A  Q  E  D
S  R  I   T  T  S   S  S  C
        ----------------------------------------------------------
--------------------------------
     321  CTGGAGCACC ATGAGGAGTG TGACTGTGTG TGCAGAGGGA GCACAGGAGG
ATAGCCGCAT CACCACCAGC AGCTCTTGCC
          GACCTCGTGG TACTCCTCAC ACTGACACAC ACGTCTCCCT CGTGTCCTCC
TATCGGCGTA GTGGTGGTCG TCGAGAACGG

     +2  Q  S  C  A   V  Q  W   L  I  L   L  E  N  V   C  V  I
S  I  L   N  L  S  C   L  L  Q
        ----------------------------------------------------------
--------------------------------
     +1 P  E  L  C   S  A  V   A  D  S  I   R  E  R   M  R  Y
L  H  P
        ----------------------------------------------------------
-------->
     401  CAGAGCTGTG CAGTGCAGTG GCTGATTCTA TTAGAGAACG TATGCGTTAT
CTCCATCCTT AATCTCAGTT GTTTGCTTCA
          GTCTCGACAC GTCACGTCAC CGACTAAGAT AATCTCTTGC ATACGCAATA
GAGGTAGGAA TTAGAGTCAA CAAACGAAGT

     +2   G  P  F   I  F  R  I   Y  S  A   F
        ------------------------------------>
     481  AGGACCTTTC ATCTTCAGGA TTTACAGTGC ATTCTGAAAG AGGAGACATC
AAACAGAATT AGGAGTTGTG CAACAGCTCT
          TCCTGGAAAG TAGAAGTCCT AAATGTCACG TAAGACTTTC TCCTCTGTAG
TTTGTCTTAA TCCTCAACAC GTTGTCGAGA

     561  TTTGAGAGGA GGCCTAAAGG ACAGGAGAAA AGGTCTTCAA TCGTGGAAAG
AAAATTAAAT GTTGTATTAA ATAGATCACC
          AAACTCTCCT CCGGATTTCC TGTCCTCTTT TCCAGAAGTT AGCACCTTTC
TTTTAATTTA CAACATAATT TATCTAGTGG

     641  AGCTAGTTTC AGAGTTACCA TGTACGTATT CCACTAGCTG GGTTCTGTAT
TTCAGTTCTT TCGATACGGC TTAGGGTAAT
          TCGATCAAAG TCTCAATGGT ACATGCATAA GGTGATCGAC CCAAGACATA
AAGTCAAGAA AGCTATGCCG AATCCCATTA

     721  GTCAGTACAG GAAAAAAACT GTGCAAGTGA GCACCTGATT CCGTTGCCTT
GGCTTAACTC TAAAGCTCCA TGTCCTGGGC
          CAGTCATGTC CTTTTTTTGA CACGTTCACT CGTGGACTAA GGCAACGGAA
CCGAATTGAG ATTTCGAGGT ACAGGACCCG

     801  CTAAAATCGT ATAAAATCTG GA
          GATTTTAGCA TATTTTAGAC CT
```

FIG. 18.

```
       +3                                               M  N  I   F  L  L
N  L  L  T   E  E  V   R  L  Y
                                                      ]------------------
--------------------------------
       1  AGGAAATCAA ATTAGGATAA GATTTGTATC TGATGAATAT TTTCCTTCTG
AACCTTCTAA CAGAGGAGGT AAGATTATAC
          TCCTTTAGTT TAATCCTATT CTAAACATAG ACTACTTATA AAAGGAAGAC
TTGGAAGATT GTCTCCTCCA TTCTAATATG

       +3  S  C  T  P   R  N  F   S  V  S   I  R  E  E   L  K  R
T  D  T   I  F  W  P   G  C  L
          -------------------------]----------------------------
--------------------------------
      81  AGCTGCACAC CTCGTAACTT CTCAGTGTCC ATAAGGGAAG AACTAAAGAG
AACCGATACC ATTTTCTGGC CAGGTTGTCT
          TCGACGTGTG GAGCATTGAA GAGTCACAGG TATTCCCTTC TTGATTTCTC
TTGGCTATGG TAAAAGACCG GTCCAACAGA
       -2                                                  <-----
--------------------------------

       +3   L  V  K   R  C  G  G   N  C  A   C  C  L   H  N  C  N
E  C  Q   C  V  P   S  K  V
          ------------------------------------------------------
--------------------------------
     161  CCTGGTTAAA CGCTGTGGTG GGAACTGTGC CTGTTGTCTC CACAATTGCA
ATGAATGTCA ATGTGTCCCA AGCAAAGTTA
          GGACCAATTT GCGACACCAC CCTTGACACG GACAACAGAG GTGTTAACGT
TACTTACAGT TACACAGGGT TCGTTTCAAT
       -2 ------------------------------------------------------
--------------------------------

       +3 T  K  K  Y   H  E  V   L  Q  L  R   P  K  T   G  V  R
G  L  H  K   S  L  T   D  V  A
          ------------------------------------------------------
--------------------------------
       +1                                            V  S  G
D  C  T   N  H  S  P   T  W  P
                                                     ]--------
--------------------------------
     241  CTAAAAAATA CCACGAGGTC CTTCAGTTGA GACCAAAGAC CGGTGTCAGG
GGATTGCACA AATCACTCAC CGACGTGGCC
          GATTTTTTAT GGTGCTCCAG GAAGTCAACT CTGGTTTCTG GCCACAGTCC
CCTAACGTGT TTAGTGAGTG GCTGCACCGG
```

FIG. 18 (CONTINUED 1).

```
       -2 -------------------------------------------------------
--------[

       +3 L  E  H  H   E  E  C   D  C  V   C  R  G  S   T  G  G
          -------------------------------------------------------
>
       +2                                   V   Q  R  E   H  R  R
I  A  A  S   P  P  A   A  L  A
                                            ]----------------------
-------------------------------

       +1  W  S  T   M  R  S  V   T  V  C   A  E  G   A  Q  E  D
S  R  I   T  T  S   S  S  C
          -------------------------------------------------------
-------------------------------
     321  CTGGAGCACC ATGAGGAGTG TGACTGTGTG TGCAGAGGGA GCACAGGAGG
ATAGCCGCAT CACCACCAGC AGCTCTTGCC
          GACCTCGTGG TACTCCTCAC ACTGACACAC ACGTCTCCCT CGTGTCCTCC
TATCGGCGTA GTGGTGGTCG TCGAGAACGG

       +2 Q  S  C  A   V  Q  W   L  I  L   L  E  N  V   C  V  I
S  I  L   N  L  S  C   L  L  Q
          -------------------------------------------------------
-------------------------------
       +1 P  E  L  C   S  A  V   A  D  S  I   R  E  R   M  R  Y
L  H  P
          -------------------------------------------------------
-------->
     401  CAGAGCTGTG CAGTGCAGTG GCTGATTCTA TTAGAGAACG TATGCGTTAT
CTCCATCCTT AATCTCAGTT GTTTGCTTCA
          GTCTCGACAC GTCACGTCAC CGACTAAGAT AATCTCTTGC ATACGCAATA
GAGGTAGGAA TTAGAGTCAA CAAACGAAGT

       +2  G  P  F   I  F  R  I   Y  S  A   F
          ------------------------------------>
     481  AGGACCTTTC ATCTTCAGGA TTTACAGTGC ATTCTGAAAG AGGAGACATC
AAACAGAATT AGGAGTTGTG CAACAGCTCT
          TCCTGGAAAG TAGAAGTCCT AAATGTCACG TAAGACTTTC TCCTCTGTAG
TTTGTCTTAA TCCTCAACAC GTTGTCGAGA

     561  TTTGAGAGGA GGCCTAAAGG ACAGGAGAAA AGGTCTTCAA TCGTGGAAAG
AAAATTAAAT GTTGTATTAA ATAGATCACC
          AAACTCTCCT CCGGATTTCC TGTCCTCTTT TCCAGAAGTT AGCACCTTTC
TTTTAATTTA CAACATAATT TATCTAGTGG

     641  AGCTAGTTTC AGAGTTACCA TGTACGTATT CCACTAGCTG GGTTCTGTAT
TTCAGTTCTT TCGATACGGC TTAGGGTAAT
          TCGATCAAAG TCTCAATGGT ACATGCATAA GGTGATCGAC CCAAGACATA
AAGTCAAGAA AGCTATGCCG AATCCCATTA

     721  GTCAGTACAG GAAAAAAACT GTGCAAGTGA GCACCTGATT CCGTTGCCTT
GGCTTAACTC TAAAGCTCCA TGTCCTGGGC
```

FIG. 18(CONTINUED 2).

CAGTCATGTC CTTTTTTTGA CACGTTCACT CGTGGACTAA GGCAACGGAA
CCGAATTGAG ATTTCGAGGT ACAGGACCCG

801 CTAAAATCGT ATAAAATCTG GATTTTTTTN TTTTTTTTTG CGCATATTCA
CATATGTAAA CCAGAACATT CTATGTACTA
GATTTTAGCA TATTTTAGAC CTAAAAAAAN AAAAAAAAAC GCGTATAAGT
GTATACATTT GGTCTTGTAA GATACATGAT

881 CAAACCTGGT TTTTAAAAAG GAACTATGTT GCTATGAATT AAACTTGTGT
CGTGCTGATA GGACAGACTG GATTTTTCAT
GTTTGGACCA AAAATTTTTC CTTGATACAA CGATACTTAA TTTGAACACA
GCACGACTAT CCTGTCTGAC CTAAAAAGTA
-3 <-------------------------------------
------------------------------

961 ATTTCTTATT AAAATTTCTG CCATTTAGAA GAAGAGAACT ACATTCATGG
TTTGGAAGAG ATAAACCTGA AAAGAAGAGT
TAAAGAATAA TTTTAAAGAC GGTAAATCTT CTTCTCTTGA TGTAAGTACC
AAACCTTCTC TATTTGGACT TTTCTTCTCA
-3 ---------------------------------------------------
------------------------------

1041 GGCCTTATCT TCACTTTATC GATAAGTCAG TTTATTTGTT TCATTGTGTA
CATTTTTATA TTCTCCTTTT GACATTATAA
CCGGAATAGA AGTGAAATAG CTATTCAGTC AAATAAACAA AGTAACACAT
GTAAAAATAT AAGAGGAAAA CTGTAATATT
-3 -------------[

1121 CTGTTGGCTT TTCTAATCTT GTTAAATATA TCTATTTTTA CCAAAGGTAT
TTAATATTCT TTTTTATGAC AACTTAGATC
GACAACCGAA AAGATTAGAA CAATTTATAT AGATAAAAAT GGTTTCCATA
AATTATAAGA AAAAATACTG TTGAATCTAG

1201 AACTATTTTT AGCTTGGTAA ATTTTTCTAA ACACAATTGT TATAGCCAGA
GGAACAAAGA TGATATAAAA TATTGTTGCT
TTGATAAAAA TCGAACCATT TAAAAAGATT TGTGTTAACA ATATCGGTCT
CCTTGTTTCT ACTATATTTT ATAACAACGA

1281 CTGACAAAAA TACATGTATT TCATTCTCGT ATGGTGCTAG AGTTAGATTA
ATCTGCATTT TAAAAAACTG AATTGGAATA
GACTGTTTTT ATGTACATAA AGTAAGAGCA TACCACGATC TCAATCTAAT
TAGACGTAAA ATTTTTTGAC TTAACCTTAT

1361 GAATTGGTAA GTTGCAAAGA CTTTTTGAAA ATAATTAAAT TATCATATCT
TCCATTCCTG TTATTGGAGA TGAAAATAAA
CTTAACCATT CAACGTTTCT GAAAAACTTT TATTAATTTA ATAGTATAGA
AGGTAAGGAC AATAACCTCT ACTTTTATTT

1441 AAGCAACTTA TGAAAGTAGA CATTCAGATC CAGCCATTAC TAACCTATTC
CTTTTTTGGG GAAATCTGAG CCTAGCTCAG
TTCGTTGAAT ACTTTCATCT GTAAGTCTAG GTCGGTAATG ATTGGATAAG
GAAAAAACCC CTTTAGACTC GGATCGAGTC

FIG. 18 (CONTINUED 3).

1521 AAAAACATAA AGCACCTTGA AAAAGACTTG GCAGCTTCCT GATAAAGCGT GCTGTGCTGT GCAGTAGGAA CACATCCTAT
TTTTTGTATT TCGTGGAACT TTTTCTGAAC CGTCGAAGGA CTATTTCGCA CGACACGACA CGTCATCCTT GTGTAGGATA

1601 TTATTGTGAT GTTGTGGTTT TATTATCTTA AACTCTGTTC CATACACTTG TATAAATACA TGGATATTTT TATGTACAGA
AATAACACTA CAACACCAAA ATAATAGAAT TTGAGACAAG GTATGTGAAC ATATTTATGT ACCTATAAAA ATACATGTCT

1681 AGTATGTCTC TTAACCAGTT CACTTATTGT ACCTGG
TCATACAGAG AATTGGTCAA GTGAATAACA TGGACC

FIG. 19. **DNA and polypeptide sequence used for mammalian cell expression**

```
  +1             m  s  l  f  g  l  l  l  l  t  s  a  l  a  g  q  r
   1  GGATCCAAAA TGAGCCTCTT CGGGCTTCTC CTGCTGACAT CTGCCCTGGC CGGCCAGAGA

  +1  q  g  t  q  a  E  S  N  L  S  S  K  F  Q  F  S  S  N  K  E
  61  CAGGGGACTC AGGCGGAATC CAACCTGAGT AGTAAATTCC AGTTTTCCAG CAACAAGGAA

  +1  Q  N  G  V  Q  D  P  Q  H  E  R  I  I  T  V  S  T  N  G  S
 121  CAGAACGGAG TACAAGATCC TCAGCATGAG AGAATTATTA CTGTGTCTAC TAATGGAAGT

  +1  I  H  S  P  R  F  P  H  T  Y  P  R  N  T  V  L  V  W  R  L
 181  ATTCACAGCC CAAGGTTTCC TCATACTTAT CCAAGAAATA CGGTCTTGGT ATGGAGATTA

  +1  V  A  V  E  E  N  V  W  I  Q  L  T  F  D  E  R  F  G  L  E
 241  GTAGCAGTAG AGGAAAATGT ATGGATACAA CTTACGTTTG ATGAAAGATT TGGGCTTGAA

  +1  D  P  E  D  D  I  C  K  Y  D  F  V  E  V  E  E  P  S  D  G
 301  GACCCAGAAG ATGACATATG CAAGTATGAT TTTGTAGAAG TTGAGGAACC CAGTGATGGA

  +1  T  I  L  G  R  W  C  G  S  G  T  V  P  G  K  Q  I  S  K  G
 361  ACTATATTAG GCCGCTGGTG TGGTTCTGGT ACTGTACCAG GAAAACAGAT TTCTAAAGGA

  +1  N  Q  I  R  I  R  F  V  S  D  E  Y  F  P  S  E  P  G  F  C
 421  AATCAAATTA GGATAAGATT TGTATCTGAT GAATATTTTC CTTCTGAACC AGGGTTCTGC

  +1  I  H  Y  N  I  V  M  P  Q  F  T  E  A  V  S  P  S  V  L  P
 481  ATCCACTACA ACATTGTCAT GCCACAATTC ACAGAAGCTG TGAGTCCTTC AGTGCTACCC

  +1  P  S  A  L  P  L  D  L  L  N  N  A  I  T  A  F  S  T  L  E
 541  CCTTCAGCTT TGCCACTGGA CCTGCTTAAT AATGCTATAA CTGCCTTTAG TACCTTGGAA

  +1  D  L  I  R  Y  L  E  P  E  R  W  Q  L  D  L  E  D  L  Y  R
 601  GACCTTATTC GATATCTTGA ACCAGAGAGA TGGCAGTTGG ACTTAGAAGA TCTATATAGG

  +1  P  T  W  Q  L  L  G  K  A  F  V  F  G  R  K  S  R  V  V  D
 661  CCAACTTGGC AACTTCTTGG CAAGGCTTTT GTTTTTGGAA GAAAATCCAG AGTGGTGGAT

  +1  L  N  L  L  T  E  E  V  R  L  Y  S  C  T  P  R  N  F  S  V
 721  CTGAACCTTC TAACAGAGGA GGTAAGATTA TACAGCTGCA CACCTCGTAA CTTCTCAGTG

  +1  S  I  R  E  E  L  K  R  T  D  T  I  F  W  P  G  C  L  L  V
 781  TCCATAAGGG AAGAACTAAA GAGAACCGAT ACCATTTTCT GGCCAGGTTG TCTCCTGGTT

  +1  K  R  C  G  G  N  C  A  C  C  L  H  N  C  N  E  C  Q  C  V
 841  AAACGCTGTG GTGGGAACTG TGCCTGTTGT CTCCACAATT GCAATGAATG TCAATGTGTC

  +1  P  S  K  V  T  K  K  Y  H  E  V  L  Q  L  R  P  K  T  G  V
 901  CCAAGCAAAG TTACTAAAAA ATACCACGAG GTCCTTCAGT TGAGACCAAA GACCGGTGTC

  +1  R  G  L  H  K  S  L  T  D  V  A  L  E  H  H  E  E  C  D  C
 961  AGGGGATTGC ACAAATCACT CACCGACGTG GCCCTGGAGC ACCATGAGGA GTGTGACTGT

  +1  V  C  R  G  S  T  G  G  S  R  G  P  F  E  G  K  P  I  P  N
1021  GTGTGCAGAG GGAGCACAGG AGGATCTAGA GGGCCCTTCG AAGGTAAGCC TATCCCTAAC

  +1  P  L  L  G  L  D  S  T  R  T  G  H  H  H  H  H  H
1081  CCTCTCCTCG GTCTCGATTC TACGCGTACC GGTCATCATC ACCATCACCA TTGA
```

FIG. 20. **DNA and polypeptide sequence used for baculovirus/insect cell expression**

1 GAATTCAAAG GCCTGTATTT TACTGTTTTC GTAACAGTTT TGTAATAAAA AAACCTATAA

+3 m k f l v n v a l v f m v v y i s y i
61 ATATGAAATT CTTAGTCAAC GTTGCCCTTG TTTTTATGGT CGTATACATT TCTTACATCT

+3 y a <u>D P E S H H H H H H</u> E S N L S S K F
121 ATGCGGATCC GGAGTCTCAC CATCACCACC ATCATGAATC CAACCTGAGT AGTAAATTCC

+3 Q F S S N K E Q N G V Q D P Q H E R I I
181 AGTTTTCCAG CAACAAGGAA CAGAACGGAG TACAAGATCC TCAGCATGAG AGAATTATTA

+3 T V S T N G S I H S P R F P H T Y P R N
241 CTGTGTCTAC TAATGGAAGT ATTCACAGCC CAAGGTTTCC TCATACTTAT CCAAGAAATA

+3 T V L V W R L V A V E E N V W I Q L T F
301 CGGTCTTGGT ATGGAGATTA GTAGCAGTAG AGGAAAATGT ATGGATACAA CTTACGTTTG

+3 D E R F G L E D P E D D I C K Y D F V E
361 ATGAAAGATT TGGGCTTGAA GACCCAGAAG ATGACATATG CAAGTATGAT TTTGTAGAAG

+3 V E E P S D G T I L G R W C G S G T V P
421 TTGAGGAACC CAGTGATGGA ACTATATTAG GGCGCTGGTG TGGTTCTGGT ACTGTACCAG

+3 G K Q I S K G N Q I R I R F V S D E Y F
481 GAAAACAGAT TTCTAAAGGA AATCAAATTA GGATAAGATT TGTATCTGAT GAATATTTTC

+3 P S E P G F C I H Y N I V M P Q F T E A
541 CTTCTGAACC AGGGTTCTGC ATCCACTACA ACATTGTCAT GCCACAATTC ACAGAAGCTG

+3 V S P S V L P P S A L P L D L L N N A I
601 TGAGTCCTTC AGTGCTACCC CCTTCAGCTT TGCCACTGGA CCTGCTTAAT AATGCTATAA

+3 T A F S T L E D L I R Y L E P E R W Q L
661 CTGCCTTTAG TACCTTGGAA GACCTTATTC GATATCTTGA ACCAGAGAGA TGGCAGTTGG

+3 D L E D L Y R P T W Q L L G K A F V F G
721 ACTTAGAAGA TCTATATAGG CCAACTTGGC AACTTCTTGG CAAGGCTTTT GTTTTTGGAA

+3 R K S R V V D L N L L T E E V R L Y S C
781 GAAAATCCAG AGTGGTGGAT CTGAACCTTC TAACAGAGGA GGTAAGATTA TACAGCTGCA

+3 T P R N F S V S I R E E L K R T D T I F
841 CACCTCGTAA CTTCTCAGTG TCCATAAGGG AAGAACTAAA GAGAACCGAT ACCATTTTCT

+3 W P G C L L V K R C G G N C A C C L H N
901 GGCCAGGTTG TCTCCTGGTT AAACGCTGTG GTGGGAACTG TGCCTGTTGT CTCCACAATT

+3 C N E C Q C V P S K V T K K Y H E V L Q
961 GCAATGAATG TCAATGTGTC CCAAGCAAAG TTACTAAAAA ATACCACGAG GTCCTTCAGT

+3 L R P K T G V R G L H K S L T D V A L E
1021 TGAGACCAAA GACCGGTGTC AGGGGATTGC ACAAATCACT CACCGACGTG GCCCTGGAGC

+3 H H E E S D C V C R G S T G G
1081 ACCATGAGGA GTGTGACTGT GTGTGCAGAG GGAGCACAGG AGGATAGCTC TAGA

FIG. 21. **DNA and polypeptide sequence used for *E.coli* expression**

```
+3    Q  T  N   S  S  S   N  N  N  N   N  N  N   N  N  N   L  G  I
1     CGCAGACTAA TTCGAGCTCG AACAACAACA ACAATAACAA TAACAACAAC CTCGGGATCG

+3 E  G  R  I   S  E  F   E  S  N  L   S  S  K   F  Q  F   S  S  N
61    AGGGAAGGAT TTCAGAATTC GAATCCAACC TGAGTAGTAA ATTCCAGTTT TCCAGCAACA

+3 K  E  Q  N   G  V  Q   D  P  Q  H   E  R  I   I  T  V   S  T  N
121   AGGAACAGAA CGGAGTACAA GATCCTCAGC ATGAGAGAAT TATTACTGTG TCTACTAATG

+3 G  S  I  H   S  P  R   F  P  H  T   Y  P  R   N  T  V   L  V  W
181   GAAGTATTCA CAGCCCAAGG TTTCCTCATA CTTATCCAAG AAATACGGTC TTGGTATGGA

+3 R  L  V  A   V  E  E   N  V  W  I   Q  L  T   F  D  E   R  F  G
241   GATTAGTAGC AGTAGAGGAA AATGTATGGA TACAACTTAC GTTTGATGAA AGATTTGGGC

+3 L  E  D  P   E  D  D   I  C  K  Y   D  F  V   E  V  E   E  P  S
301   TTGAAGACCC AGAAGATGAC ATATGCAAGT ATGATTTTGT AGAAGTTGAG GAACCCAGTG

+3 D  G  T  I   L  G  R   W  C  G  S   G  T  V   P  G  K   Q  I  S
361   ATGGAACTAT ATTAGGGCGC TGGTGTGGTT CTGGTACTGT ACCAGGAAAA CAGATTTCTA

+3 K  G  N  Q   I  R  I   R  F  V  S   D  E  Y   F  P  S   E  P  G
421   AAGGAAATCA AATTAGGATA AGATTTGTAT CTGATGAATA TTTTCCTTCT GAACCAGGGT

+3 F  C  I  H   Y  N  I   V  M  P  Q   F  T  E   A  V  S   P  S  V
481   TCTGCATCCA CTACAACATT GTCATGCCAC AATTCACAGA AGCTGTGAGT CCTTCAGTGC

+3 L  P  P  S   A  L  P   L  D  L  L   N  N  A   I  T  A   F  S  T
541   TACCCCCTTC AGCTTTGCCA CTGGACCTGC TTAATAATGC TATAACTGCC TTTAGTACCT

+3 L  E  D  L   I  R  Y   L  E  P  E   R  W  Q   L  D  L   E  D  L
601   TGGAAGACCT TATTCGATAT CTTGAACCAG AGAGATGGCA GTTGGACTTA GAAGATCTAT

+3 Y  R  P  T   W  Q  L   L  G  K  A   F  V  F   G  R  K   S  R  V
661   ATAGGCCAAC TTGGCAACTT CTTGGCAAGG CTTTTGTTTT TGGAAGAAAA TCCAGAGTGG

+3 V  D  L  N   L  L  T   E  E  V  R   L  Y  S   C  T  P   R  N  F
721   TGGATCTGAA CCTTCTAACA GAGGAGGTAA GATTATACAG CTGCACACCT CGTAACTTCT

+3 S  V  S  I   R  E  E   L  K  R  T   D  T  I   F  W  P   G  C  L
781   CAGTGTCCAT AAGGGAAGAA CTAAAGAGAA CCGATACCAT TTTCTGGCCA GGTTGTCTCC

+3 L  V  K  R   C  G  G   N  C  A  C   C  L  H   N  C  N   E  C  Q
841   TGGTTAAACG CTGTGGTGGG AACTGTGCCT GTTGTCTCCA CAATTGCAAT GAATGTCAAT

+3 C  V  P  S   K  V  T   K  K  Y  H   E  V  L   Q  L  R   P  K  T
901   GTGTCCCAAG CAAAGTTACT AAAAAATACC ACGAGGTCCT TCAGTTGAGA CCAAAGACCG

+3 G  V  R  G   L  H  K   S  L  T  D   V  A  L   E  H  H   E  E  C
961   GTGTCAGGGG ATTGCACAAA TCACTCACCG ACGTGGCCCT GGAGCACCAT GAGGAGTGTG

+3 D  C  V  C   R  G  S   T  G  G  H   H  H  H   H  H  *
1021  ACTGTGTGTG CAGAGGGAGC ACAGGAGGAC ATCATCACCA TCACCATTGA TCTAGAGTCG

1081  ACCTGCAGGC AAGCTT
```

FIG. 22. **Disulphide-linked dimerisation of VEGF-X**

**(A) Mammalian cell expression**

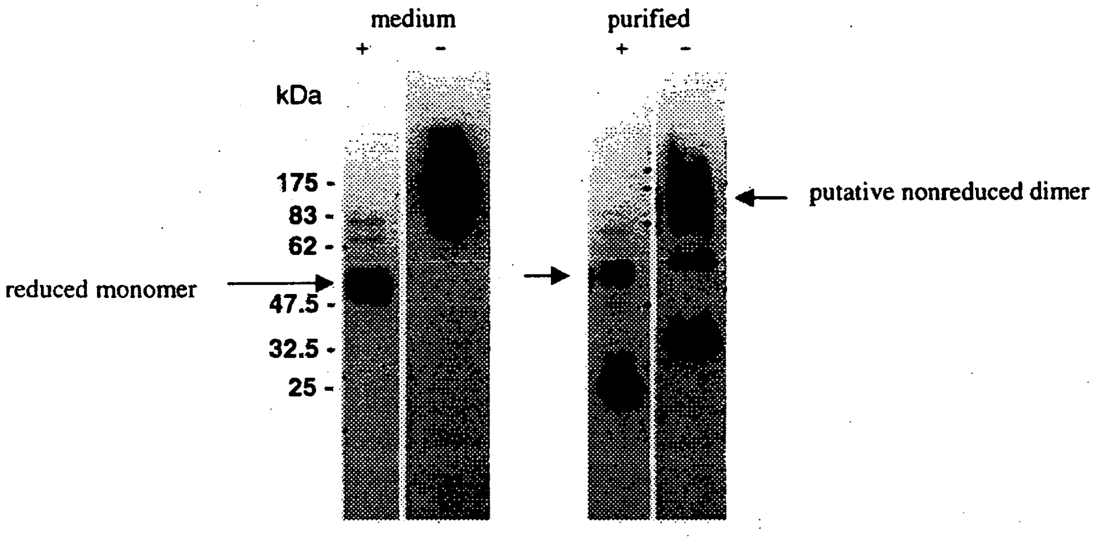


**(B)** ***E.coli*** **expression**

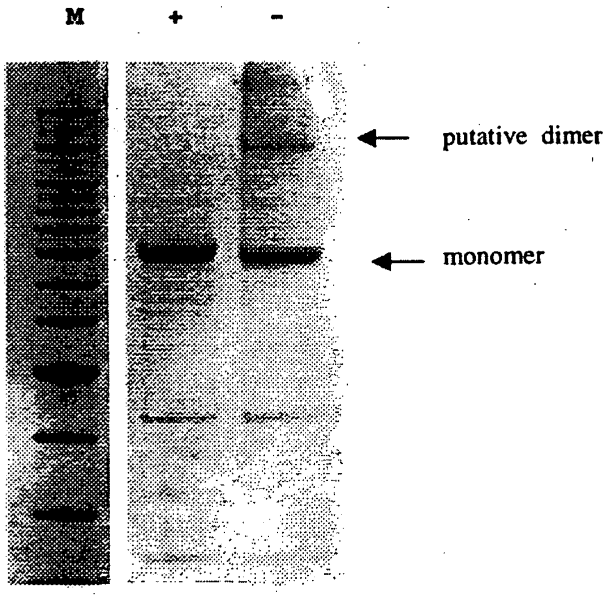

FIG. 23. Glycosylation of VEGF-X

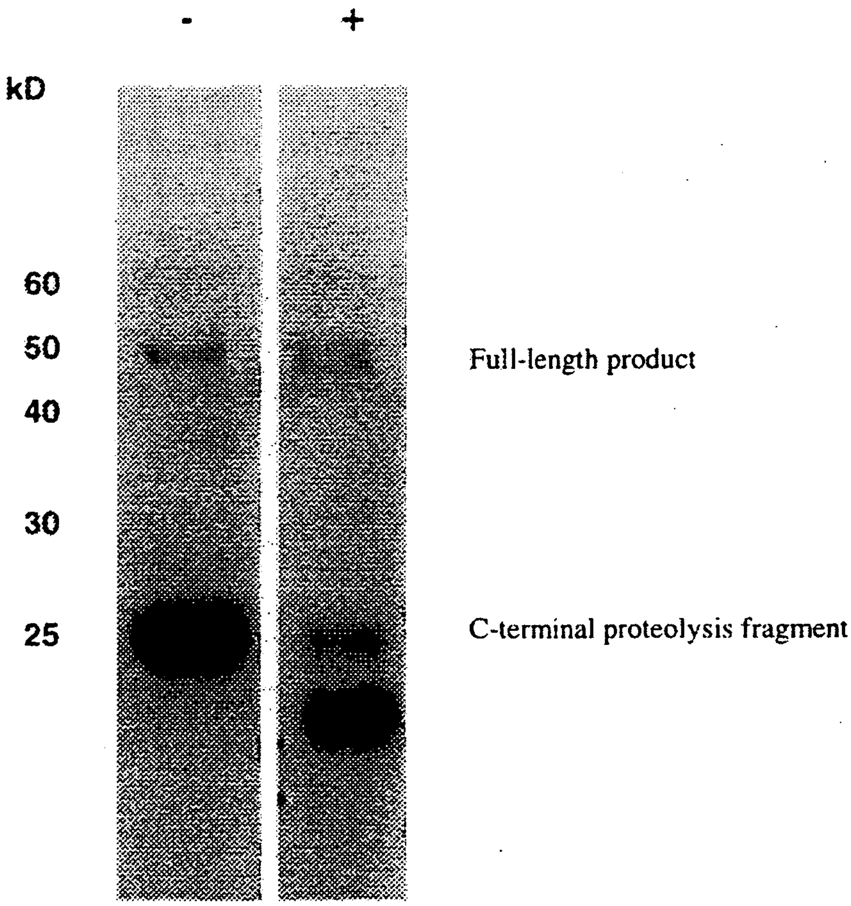

FIG. 24.

**DNA and polypeptide sequence used for *E.coli* expression of the PDGF-like domain**

```
+3                    M  R   G  S  H  H   H  H  H   H  G  M   A  S  M
1    AAGGAGATAT ACATATGCGG GGTTCTCATC ATCATCATCA TCATGGTATG GCTAGCATGA

+3   T  G  G  Q   Q  M  G   R  D  L  Y   D  D  D   D  K  D   P  G  R
61   CTGGTGGACA GCAAATGGGT CGGGATCTGT ACGACGATGA CGATAAGGAT CCGGGAAGAA

+3   K  S  R  V   V  D  L   N  L  L  T   E  E  V   R  L  Y   S  C  T
121  AATCCAGAGT GGTGGATCTG AACCTTCTAA CAGAGGAGGT AAGATTATAC AGCTGCACAC

+3   P  R  N  F   S  V  S   I  R  E  E   L  K  R   T  D  T   I  F  W
181  CTCGTAACTT CTCAGTGTCC ATAAGGGAAG AACTAAAGAG AACCGATACC ATTTTCTGGC

+3   P  G  C  L   L  V  K   R  C  G  G   N  C  A   C  C  L   H  N  C
241  CAGGTTGTCT CCTGGTTAAA CGCTGTGGTG GGAACTGTGC CTGTTGTCTC CACAATTGCA

+3   N  E  C  Q   C  V  P   S  K  V  T   K  K  Y   H  E  V   L  Q  L
301  ATGAATGTCA ATGTGTCCCA AGCAAAGTTA CTAAAAAATA CCACGAGGTC CTTCAGTTGA

+3   R  P  K  T   G  V  R   G  L  H  K   S  L  T   D  V  A   L  E  H
361  GACCAAAGAC CGGTGTCAGG GGATTGCACA AATCACTCAC CGACGTGGCC CTGGAGCACC

+3   H  E  E  C   D  C  V   C  R  G  S   T  G  G
421  ATGAGGAGTG TGACTGTGTG TGCAGAGGGA GCACAGGAGG ATAATGAATT CGAAGCTTGA

481  TCCGGCTGCT AACAAAGCCC
```

FIG. 25. Expression of PDGF domain in *E.coli*

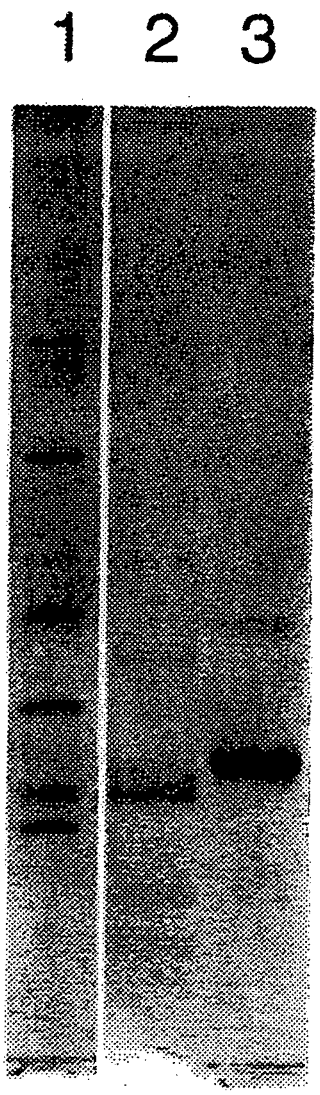

FIG. 26.

**DNA and polypeptide sequence used for *E.coli* expression of the CUB-like domain**

```
+2        M  A  M  D  I  G  I  N  S  D  P  E  S  H  H  H  H  H
1    GGCGATGGCC ATGGATATCG GAATTAATTC GGATCCGGAG TCTCACCATC ACCACCATCA

+2    E  S  N  L  S  S  K  F  Q  F  S  S  N  K  E  Q  N  G  V  Q
61   TGAATCCAAC CTGAGTAGTA AATTCCAGTT TTCCAGCAAC AAGGAACAGA ACGGAGTACA

+2    D  P  Q  H  E  R  I  I  T  V  S  T  N  G  S  I  H  S  P  R
121  AGATCCTCAG CATGAGAGAA TTATTACTGT GTCTACTAAT GGAAGTATTC ACAGCCCAAG

+2    F  P  H  T  Y  P  R  N  T  V  L  V  W  R  L  V  A  V  E  E
181  GTTTCCTCAT ACTTATCCAA GAAATACGGT CTTGGTATGG AGATTAGTAG CAGTAGAGGA

+2    N  V  W  I  Q  L  T  F  D  E  R  F  G  L  E  D  P  E  D  D
241  AAATGTATGG ATACAACTTA CGTTTGATGA AAGATTTGGG CTTGAAGACC CAGAAGATGA

+2    I  C  K  Y  D  F  V  E  V  E  E  P  S  D  G  T  I  L  G  R
301  CATATGCAAG TATGATTTTG TAGAAGTTGA GGAACCCAGT GATGGAACTA TATTAGGGCG

+2    W  C  G  S  G  T  V  P  G  K  Q  I  S  K  G  N  Q  I  R  I
361  CTGGTGTGGT TCTGGTACTG TACCAGGAAA ACAGATTTCT AAAGGAAATC AAATTAGGAT

+2    R  F  V  S  D  E  Y  F  P  S  E  P  G  F  C  I  H  Y  N  I
421  AAGATTTGTA TCTGATGAAT ATTTTCCTTC TGAACCAGGG TTCTGCATCC ACTACAACAT

+2    V  M  P  Q  F  T  E  A  V
481  TGTCATGCCA CAATTCACAG AAGCTGTGTA GTCGAGCTCC GTCGACAAGC TTGCGGCCGC

541  ACTCGAGCAC
```

FIG. 27. Expression of the CUB domain in *E.coli*

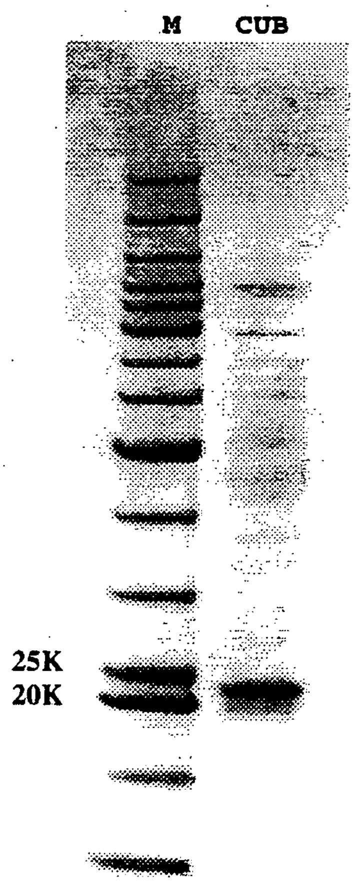

FIG. 28. The Effect of Truncated VEGF-X (CUB domain) on HUVEC Proliferation.

(A)

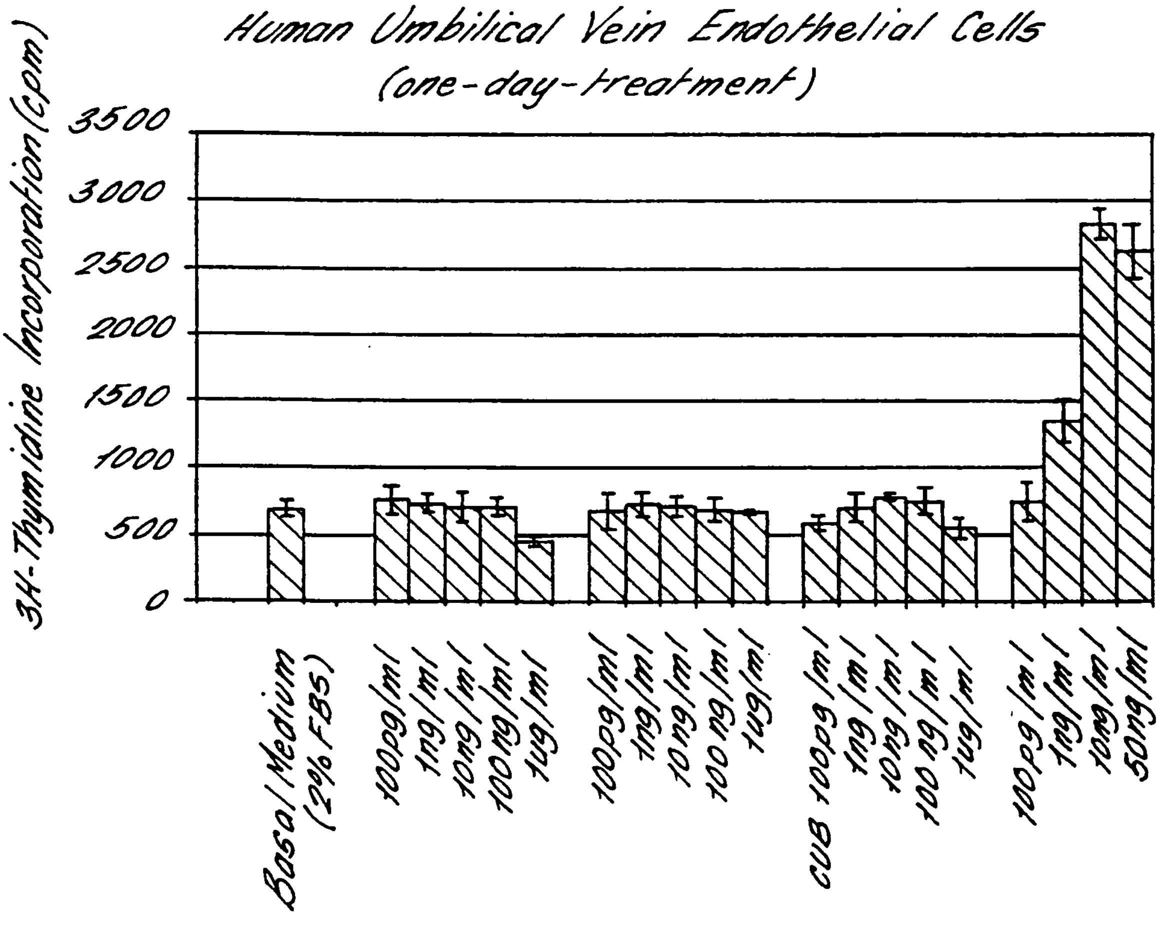

FIG. 28 (CONTINUED 1).

(B)

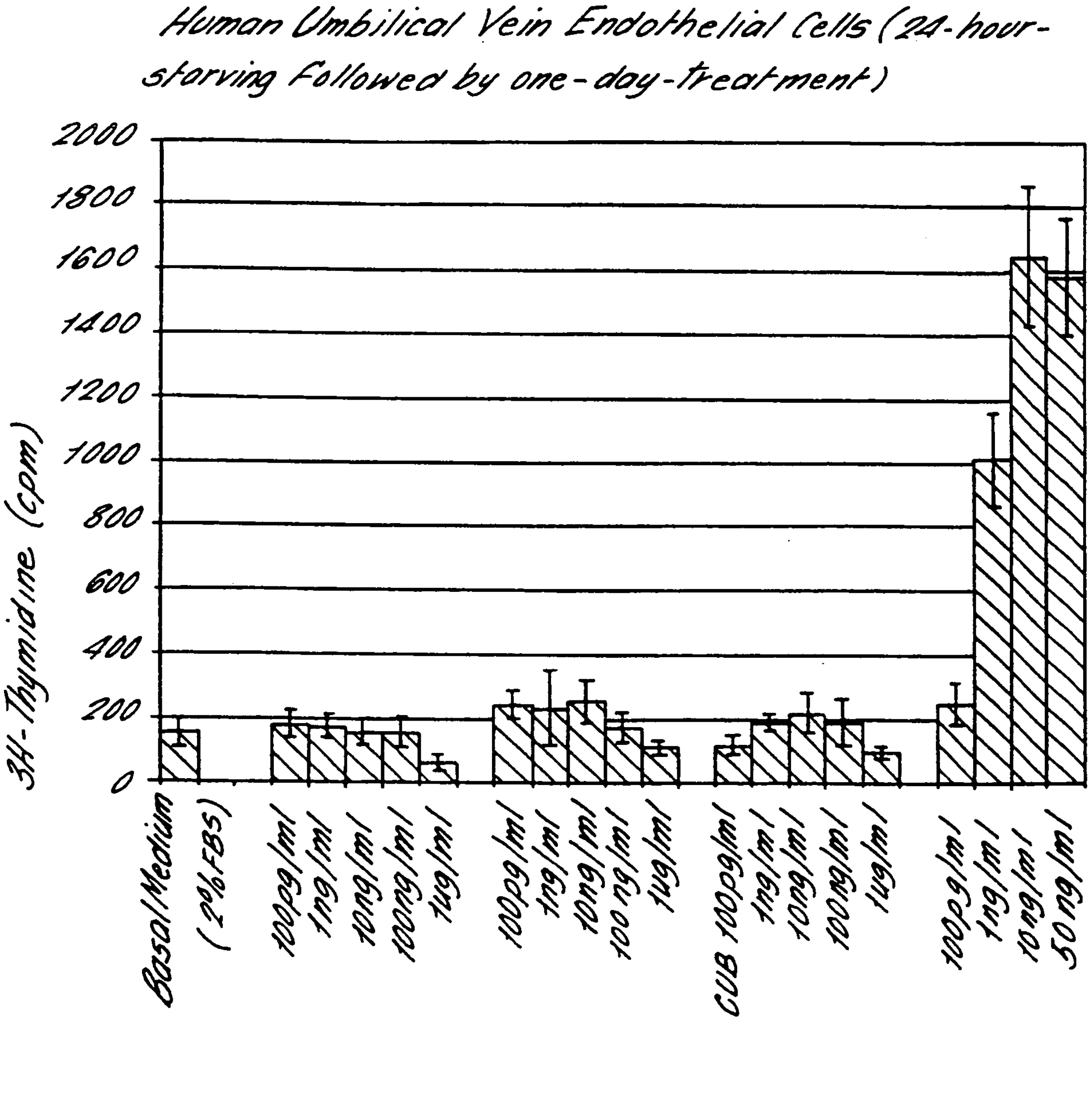

FIG. 28 (CONTINUED 2).

(C)

The effect of VEGF-$A_{165}$ and VEGF-X CUB domain on the proliferation of HUVEC (two-day-treatment).

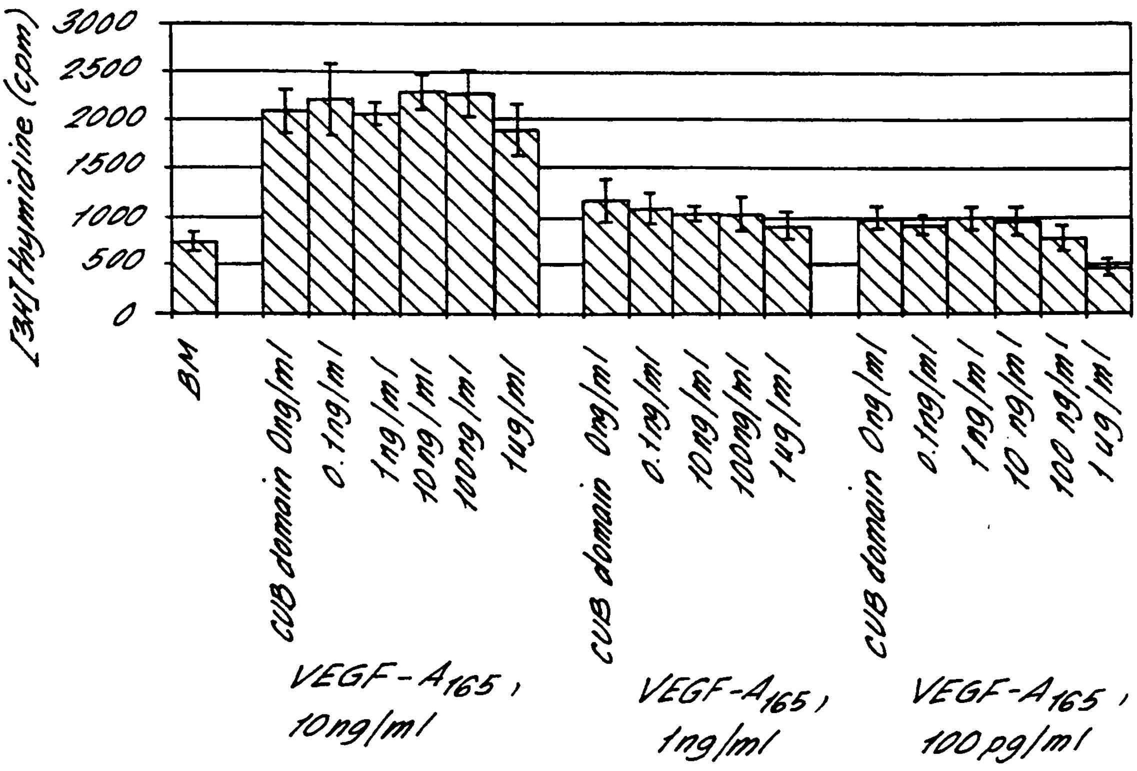

FIG. 29.

**Tissue distribution of mRNA**

**(A) - Normal tissues**

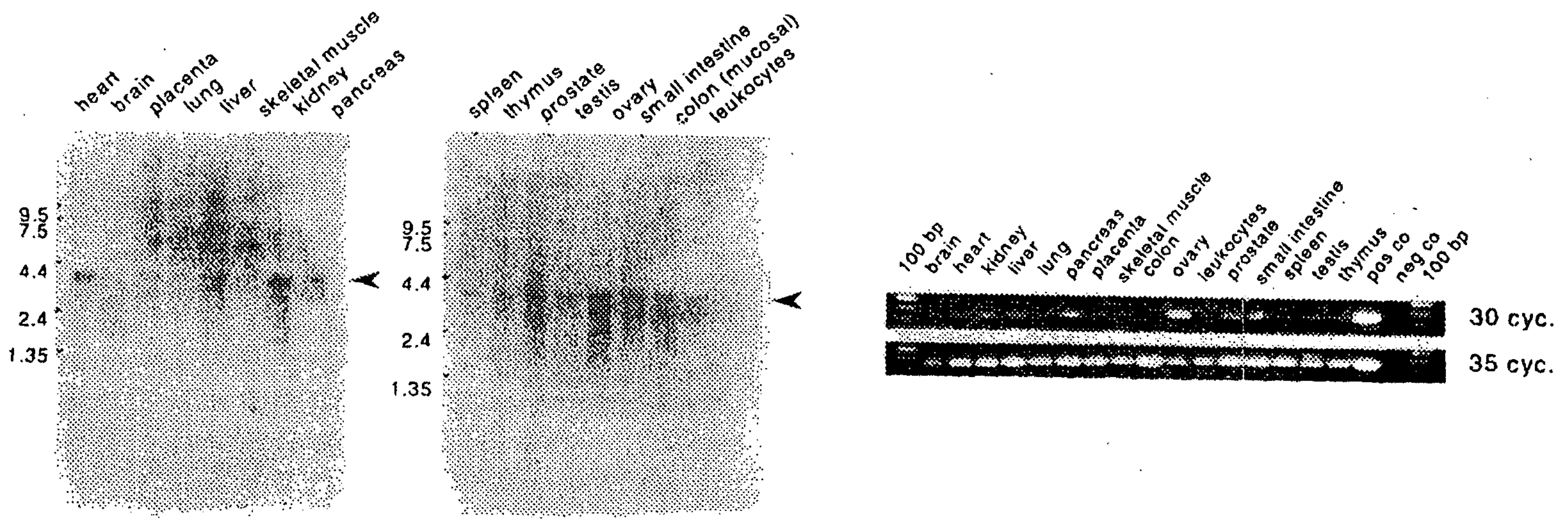

FIG. 29 (CONTINUED). (B)- Tumour tissue and cell lines

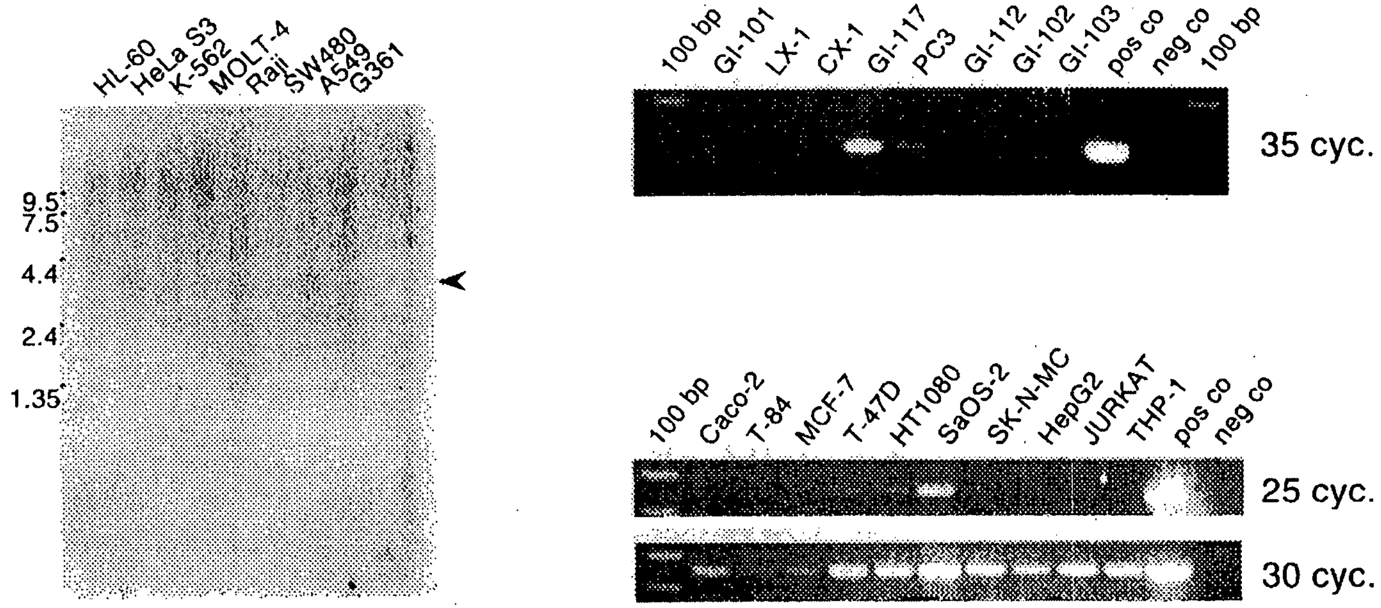

FIG. 30.

**Partial intron/exon structure of the VEGF-X gene**

**(A) - Genomic DNA sequences of 2 exons determined by sequencing**

tttcttttataccatatagtggtggatctgaaccagGGTTCTGCATCCACTACAACATTGTCATGCCACAATTCACAGAAGCTGTG
AGTCCTTCAGTGCTACCCCCTTCAGCTTTGCCACTGGACCTGCTTAATAATGCTATAACTGCCTTTAGTACCTTGGAAGACCTTAT
TCGATATCTTGAACCAGAGAGATGGCAGTTGGACTTAGAAGATCTATATAGGCCAACTTGGCAACTTCTTGGCAAGGCTTTTGTTT
TTGGAAGAAAATCCAGAGTGGTGGATCTGAACCTTCTAACAGAGGAGGTAAGATTATACAGCTGCACACCTCGTAACTTCTCAGTG
TCCATAAGGGAAGAACTAAAGAGAACCGATACCATTTTCTGGCCAGGTTGTCTCCTGGTTAAACGCTGTGGTGGGAACTGTGCCTG
TTGTCTCCACAATTGCAATGAATGTCAATGTGTCCCAAGCAAAGTTACTAAAAAATACCACGAGgtaggtatacaatttcttttt
ggtttccttcgggtattttatgtctt

aaagccagtcatagacattcgttgatttttaaaagtggcttactcttattccctttcagGTCCTTCAGTTGAGACCAAAGACCGGT
GTCAGGGGATTGCACAAATCACTCACCGACGTGGCCCTGGAGCACCATGAGGAGTGTGACTGTGTGTGCAGAGGGAGCACAGGAGG
ATAGCCGCATCACCACCAGCAGCTCTTGCCCAGAGCTGTGCAGTGCAGTGGCTGATTCTATTAGAGAACGTATGCGTTATCTCCAT
CCTTAATCTCAGTTGTTTGCTTCAAGGACCTTTCATCTTCAGGATTTACAGTGCATTCTGAAAGAGGAGACATCAAACAGAATTAG
GAGTTGTGCAACAGCTCTTTTGAGAGGAGGCCTAAAGGACAGGAGAAAAGGTCTTCAATCGTGGAAAGAAAATTAAATGTTGTATT
AAATAGATCACCAGCTAGTTTCAGAGTTACCATGTACGTATTCCACTAGCTGGGTTCTGTATTTCAGTTCTTTCGATACGGCTTAG
GGTAATGTCAGTACAGGAAAAAAACTGTGCAAGTGAGCACCTGATTCCGTTGCCTTGGCTTAACTCTAAAGCTCCATGTCCTGGGC
CTAAAATCGTATAAAATCTGGATTTTTTTTTTTTTTTTTGCGCATATTCACATATGTAAACCAGAACATTCTATGTACTACAAACC
TGGTTTTTAAAAAGGAACTATGTTGCTATGAATTAAACTTGTGTCATGCTGATAGGACAGACTGGATTTTTCATATTTCTTATTAA
AATTTCTGCCATTTAGAAGAAGAGAACTACATTCATGGTTTGGAAGAGATAAACCTGAAAAGAAGAGTGGCCTTATCTTCACTTTA
TCGATAAGTCAGTTTATTTGTTTCATTGTGTACATTTTTATATTCTCCTTTTGACATTATAACTGTTGGCTTTTCTAATCTTGTTA
AATATATCTATTTTTACCAAAGGTATTTAATATTCTTTTTTATGACAACTTAGATCAACTATTTTTAGCTTGGTAAATTTTTCTAA
ACACAATTGTTATAGCCAGAGGAACAAAGATGATATAAAATATTGTTGCTCTGACAAAAATACATGTATTTCATTCTCGTATGGTG
CTAGAGTTAGATTAATCTGCATTTTAAAAAACTGAATTGGAATAGAATTGGTAAGTTGCAAAGACTTTTTGAAAATAATTAAATTA
TCATATCTTCCATTCCTGTTATTGGAGATGAAAATAAAAAGCAACTTATGAAAGTAGACATTCAGATCCAGCCATTACTAACCTAT
TCCTTTTTTGGGGAAATCTGAGCCTAGCTCAGAAAAACATAAAGCACCTTGAAAAAGACTTGGCAGCTTCCTGATAAAGCGTGCTG
TGCTGTGCAGTAGGAACACATCCTATTTATTGTGATGTTGTGGTTTTATTATCTTAAACTCTGTTCCATACACTTGTATAAATACA
TGGATATTTTTATGTACAGAAGTATGTCTCTTAACCAGTTCACTTATTGTACTCTGGCAATTTAAAAGAAAATCAGTAAAATATTT
TGCTTGTAAAATGCTTAATATCGTGCCTAGGTTATGTGGTGACTATTTGAATCAAAAATGTATTGAATCATCAAATAAAAGAATGT
GGCTATTTTGGGGAGAAAATTatgtgtgtgtgtgctcaagatttatttcttggactctgagaaaatgaaagataaa

FIG. 30 (CONTINUED 1).

**(B) - Location of splice sites within the cDNA sequence**

```
   1  GAATTCGCCC TTTTGTTTAA ACCTTGGGAA CTGGTTCAGG TCCAGGTTTT GCTTTGATCC

  61  TTTTCAAAAA CTGGAGACAC AGAAGAGGGC TCTAGGAAAA AGTTTTGGAT GGGATTATGT

 121  GGAAACTACC CTGCGATTCT CTGCTGCCAG AGCAGGCTCG GCGCTTCCAC CCCAGTGCAG

 181  CCTTCCCCTG GCGGTGGTGA AAGAGACTCG GGAGTCGCTG CTTCCAAAGT GCCCGCCGTG

  +3                                M  S  L  F  G  L  L  L  L  T  S
 241  AGTGAGCTCT CACCCCAGTC AGCCAAATGA GCCTCTTCGG GCTTCTCCTG CTGACATCTG

  +3 A  L  A  G  Q  R  Q  G  T  Q  A  E  S  N  L  S  S  K  F  Q
 301  CCCTGGCCGG CCAGAGACAG GGGACTCAGG CGGAATCCAA CCTGAGTAGT AAATTCCAGT

  +3 F  S  S  N  K  E  Q  N  G  V  Q  D  P  Q  H  E  R  I  I  T
 361  TTTCCAGCAA CAAGGAACAG AACGGAGTAC AAGATCCTCA GCATGAGAGA ATTATTACTG

  +3 V  S  T  N  G  S  I  H  S  P  R  F  P  H  T  Y  P  R  N  T
 421  TGTCTACTAA TGGAAGTATT CACAGCCCAA GGTTTCCTCA TACTTATCCA AGAAATACGG

  +3 V  L  V  W  R  L  V  A  V  E  E  N  V  W  I  Q  L  T  F  D
 481  TCTTGGTATG GAGATTAGTA GCAGTAGAGG AAAATGTATG GATACAACTT ACGTTTGATG

  +3 E  R  F  G  L  E  D  P  E  D  D  I  C  K  Y  D  F  V  E  V
 541  AAAGATTTGG GCTTGAAGAC CCAGAAGATG ACATATGCAA GTATGATTTT GTAGAAGTTG

  +3 E  E  P  S  D  G  T  I  L  G  R  W  C  G  S  G  T  V  P  G
 601  AGGAACCCAG TGATGGAACT ATATTAGGGC GCTGGTGTGG TTCTGGTACT GTACCAGGAA

  +3 K  Q  I  S  K  G  N  Q  I  R  I  R  F  V  S  D  E  Y  F  P
 661  AACAGATTTC TAAAGGAAAT CAAATTAGGA TAAGATTTGT ATCTGATGAA TATTTTCCTT

  +3 S  E  P  G  F  C  I  H  Y  N  I  V  M  P  Q  F  T  E  A  V
 721  CTGAACCAGG GTTCTGCATC CACTACAACA TTGTCATGCC ACAATTCACA GAAGCTGTGA

  +3 S  P  S  V  L  P  P  S  A  L  P  L  D  L  L  N  N  A  I  T
 781  GTCCTTCAGT GCTACCCCCT TCAGCTTTGC CACTGGACCT GCTTAATAAT GCTATAACTG

  +3 A  F  S  T  L  E  D  L  I  R  Y  L  E  P  E  R  W  Q  L  D
 841  CCTTTAGTAC CTTGGAAGAC CTTATTCGAT ATCTTGAACC AGAGAGATGG CAGTTGGACT

  +3 L  E  D  L  Y  R  P  T  W  Q  L  L  G  K  A  F  V  F  G  R
 901  TAGAAGATCT ATATAGGCCA ACTTGGCAAC TTCTTGGCAA GGCTTTTGTT TTTGGAAGAA

  +3 K  S  R  V  V  D  L  N  L  L  T  E  E  V  R  L  Y  S  C  T
 961  AATCCAGAGT GGTGGATCTG AACCTTCTAA CAGAGGAGGT AAGATTATAC AGCTGCACAC

  +3 P  R  N  F  S  V  S  I  R  E  E  L  K  R  T  D  T  I  F  W
1021  CTCGTAACTT CTCAGTGTCC ATAAGGGAAG AACTAAAGAG AACCGATACC ATTTTCTGGC

  +3 P  G  C  L  L  V  K  R  C  G  G  N  C  A  C  C  L  H  N  C
1081  CAGGTTGTCT CCTGGTTAAA CGCTGTGGTG GGAACTGTGC CTGTTGTCTC CACAATTGCA

  +3 N  E  C  Q  C  V  P  S  K  V  T  K  K  Y  H  E  V  L  Q  L
1141  ATGAATGTCA ATGTGTCCCA AGCAAAGTTA CTAAAAAATA CCACGAGGTC CTTCAGTTGA
```

FIG. 30 (CONTINUED 2).

```
 +3 R  P  K  T   G  V  R   G  L  H  K   S  L  T   D  V  A   L  E  H
1201  GACCAAAGAC CGGTGTCAGG GGATTGCACA AATCACTCAC CGACGTGGCC CTGGAGCACC

 +3 H  E  E  C   D  C  V   C  R  G  S   T  G  G
1261  ATGAGGAGTG TGACTGTGTG TGCAGAGGGA GCACAGGAGG ATAGCCGCAT CACCACCAGC

1321  AGCTCTTGCC CAGAGCTGTG CAGTGCAGTG GCTGATTCTA TTAGAGAACG TATGCGTTAT

1381  CTCCATCCTT AATCTCAGTT GTTTGCTTCA AGGACCTTTC ATCTTCAGGA TTTACAGTGC

1441  ATTCTGAAAG AGGAGACATC AAACAGAATT AGGAGTTGTG CAACAGCTCT TTTGAGAGGA

1501  GGCCTAAAGG ACAGGAGAAA AGGTCTTCAA TCGTGGAAAG AAAATTAAAT GTTGTATTAA

1561  ATAGATCACC AGCTAGTTTC AGAGTTACCA TGTACGTATT CCACTAGCTG GGTTCTGTAT

1621  TTCAGTTCTT TCGATACGGC TTAGGGTAAT GTCAGTACAG GAAAAAAACT GTGCAAGTGA

1681  GCACCTGATT CCGTTGCCTT GCTTAACTCT AAAGCTCCAT GTCCTGGGCC TAAAATCGTA

1741  TAAAATCTGG ATTTTTTTTT TTTTTTTTTG CTCATATTCA CATATGTAAA CCAGAACATT

1801  CTATGTACTA CAAACCTGGT TTTTAAAAAG GAACTATGTT GCTATGAATT AAACTTGTGT

1861  CATGCTGATA GGACAGACTG GATTTTTCAT ATTTCTTATT AAAATTTCTG CCATTTAGAA

1921  GAAGAGAACT ACATTCATGG TTTGGAAGAG ATAAACCTGA AAAGAAGAGT GGCCTTATCT

1981  TCACTTTATC GATAAGTCAG TTTATTTGTT TCATTGTGTA CATTTTTATA TTCTCCTTTT

2041  GACATTATAA CTGTTGGCTT TTCTAATCTT GTTAAATATA TCTATTTTTA CCAAAGGTAT

2101  TTAATATTCT TTTTTATGAC AACTTAGATC AACTATTTTT AGCTTGGTAA ATTTTTCTAA

2161  ACACAATTGT TATAGCCAGA GGAACAAAGA TGATATAAAA TATTGTTGCT CTGACAAAAA

2221  TACATGTATT TCATTCTCGT ATGGTGCTAG AGTTAGATTA ATCTGCATTT TAAAAAACTG

2281  AATTGGAATA GAATTGGTAA GTTGCAAAGA CTTTTTGAAA ATAATTAAAT TATCATATCT

2341  TCCATTCCTG TTATTGGAGA TGAAAATAAA AAGCAACTTA TGAAAGTAGA CATTCAGATC

2401  CAGCCATTAC TAACCTATTC CTTTTTTGGG GAAATCTGAG CCTAGCTCAG AAAAACATAA

2461  AGCACCTTGA AAAAGACTTG GCAGCTTCCT GATAAAGCGT GCTGTGCTGT GCAGTAGGAA

2521  CACATCCTAT TTATTGTGAT GTTGTGGTTT TATTATCTTA AACTCTGTTC CATACACTTG

2581  TATAAATACA TGGATATTTT TATGTACAGA AGTATGTCTC TTAACCAGTT CACTTATTGT

2641  ACCTGGAAGG GCGAATTCTG CAGATATC
```

FIG. 31.

The Effect of Fl-VEGF-X on HUVEC Proliferation:
(24-hour serum starvation followed by
one day-treatment)

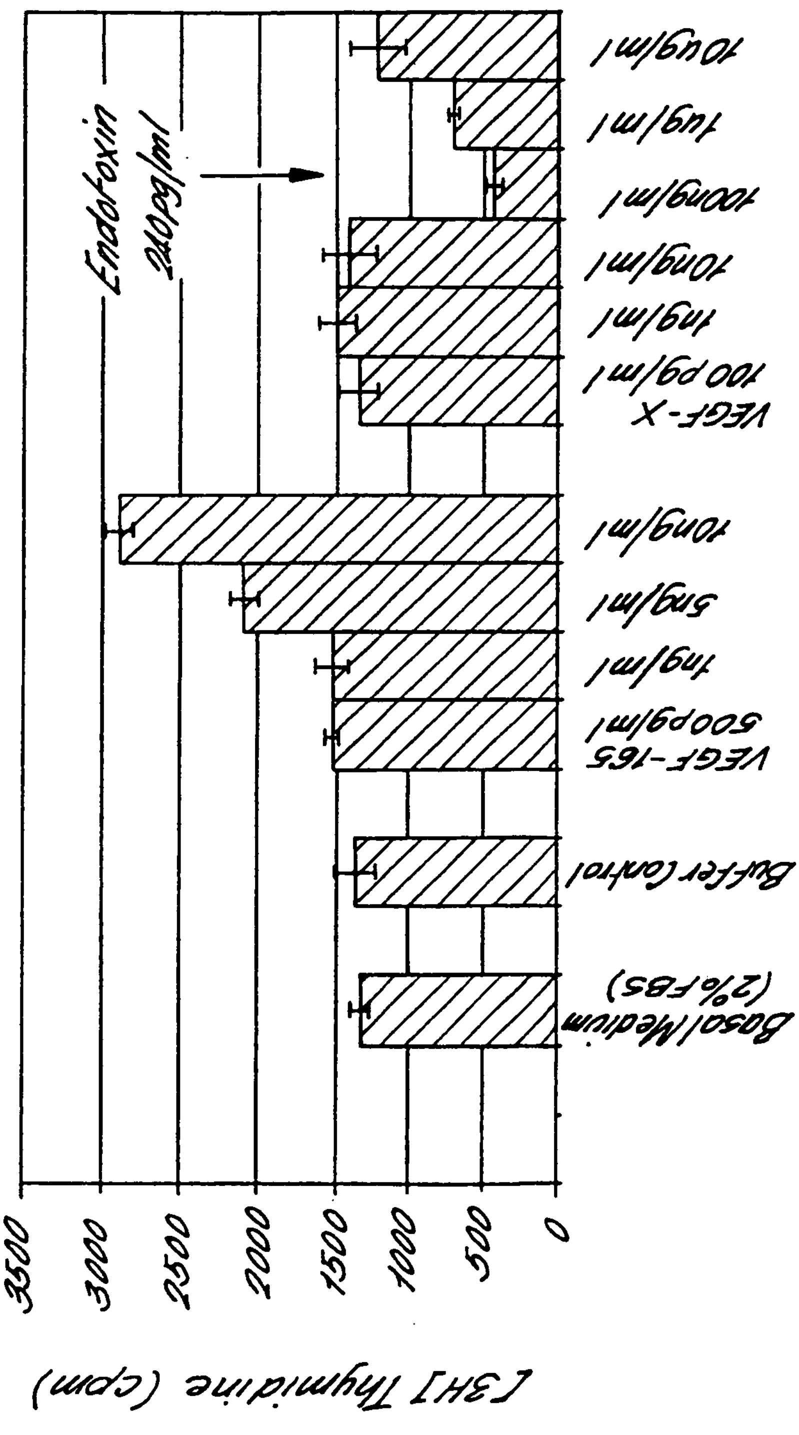

FIG. 32.

The Combined Effect of Truncated VEGF-X (CUB domain) and Human Recombinant $VEGF_{165}$ on HUVEC Proliferation: (24-hour serum starvation followed by two-day-treatment)

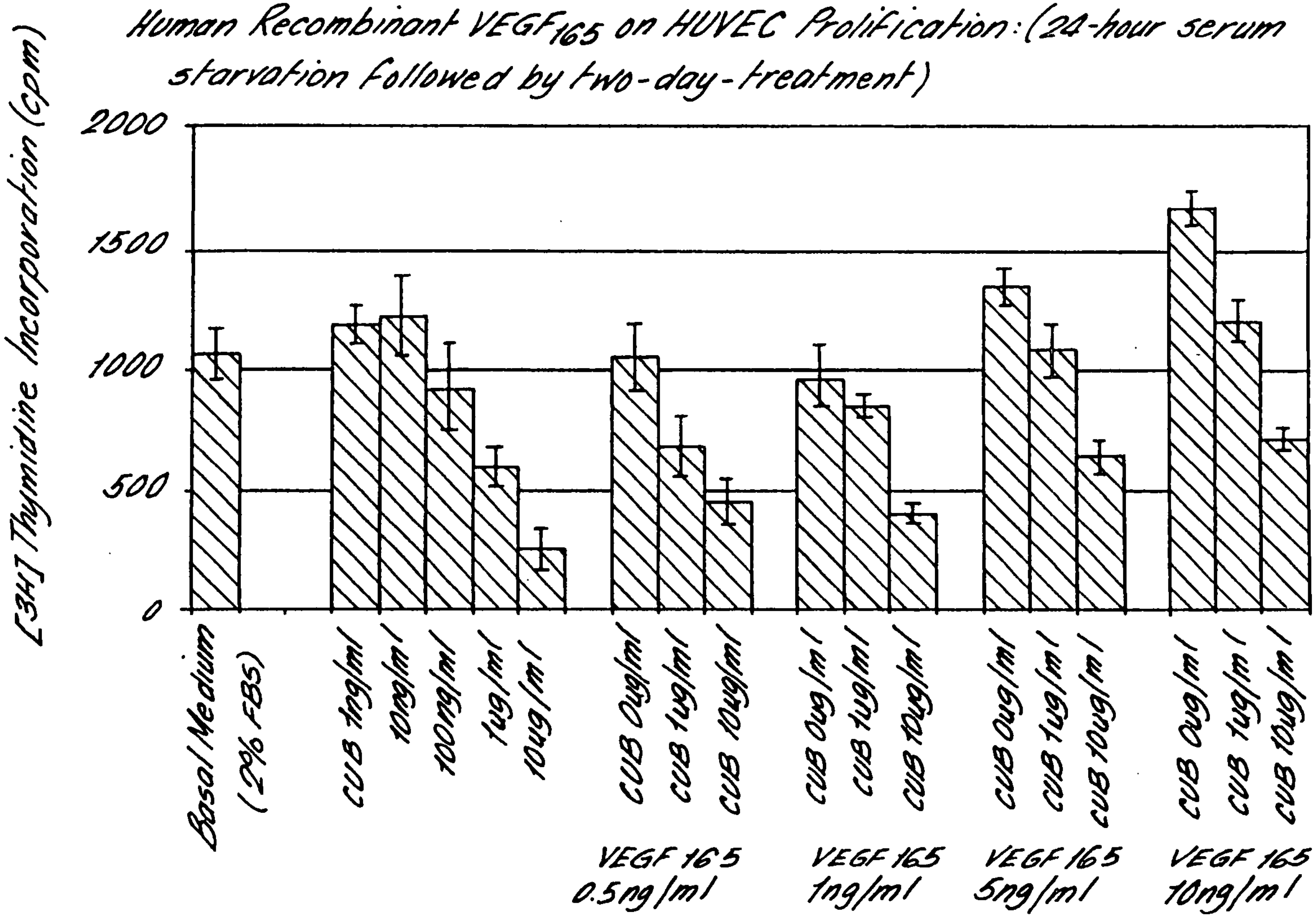

FIG. 33.

The Combined Effect of CUB Domain and Human Recombinant bFGF on HUVEC Proliferation: (24-hour serum starvation followed by two-day-treatment).

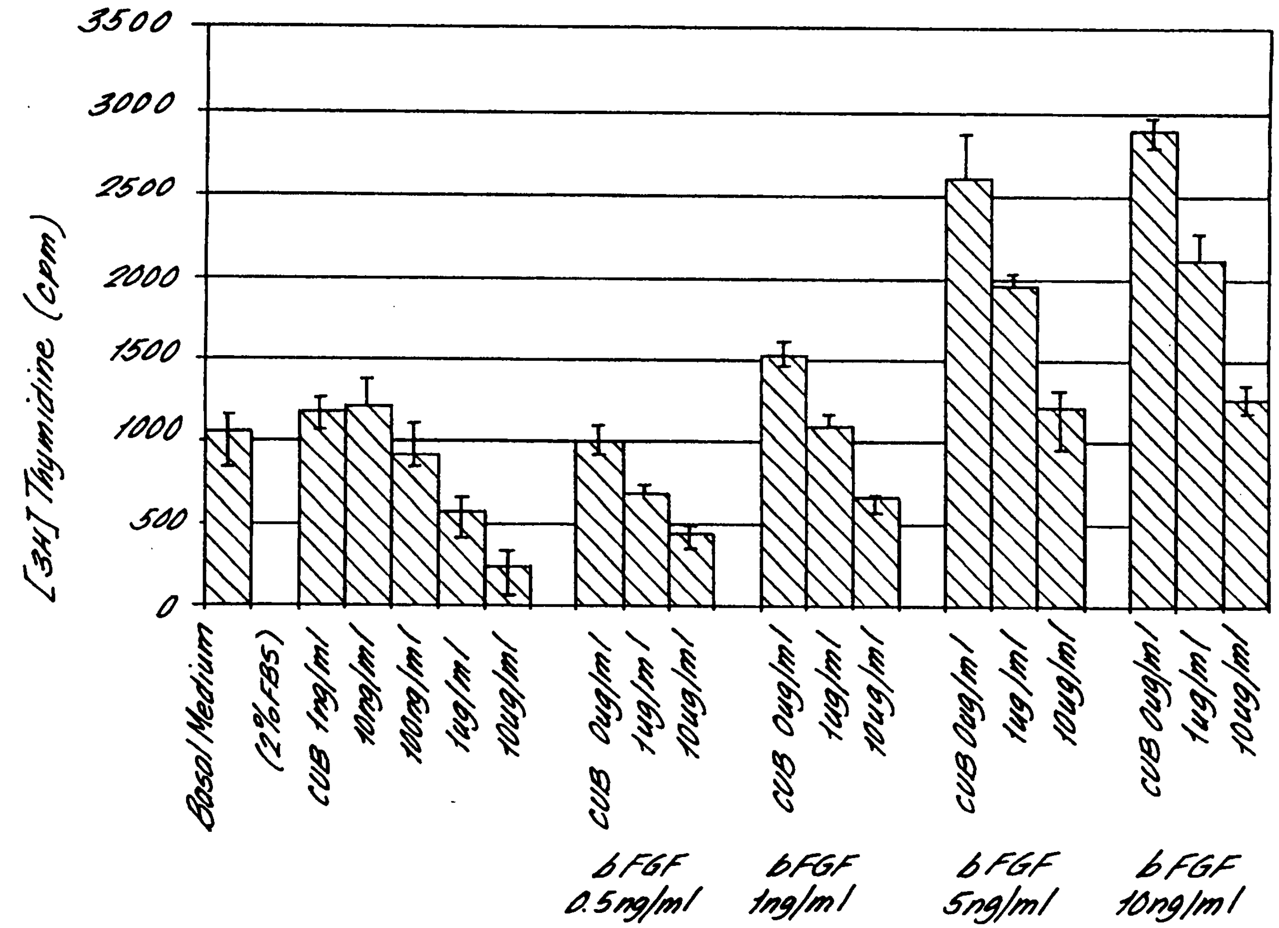

FIG. 34.

LDH Assay for Testing Cytotoxicity of CUB Domain or CUB Domain with rhVEGF$_{165}$

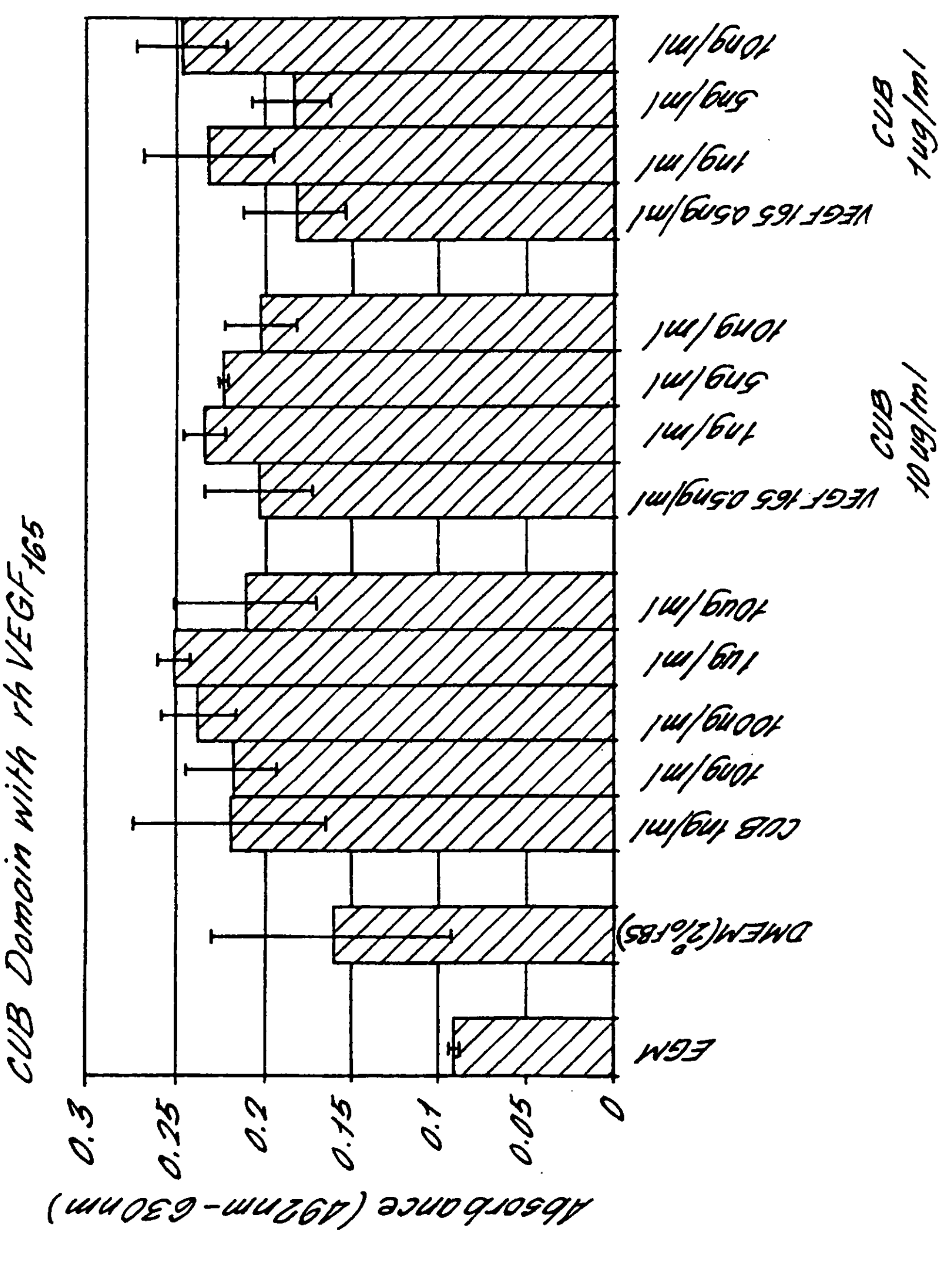

FIG. 35.

LDH Assay for Testing Cytotoxicity of CUB Domain or CUB Domain with rh-bFGF

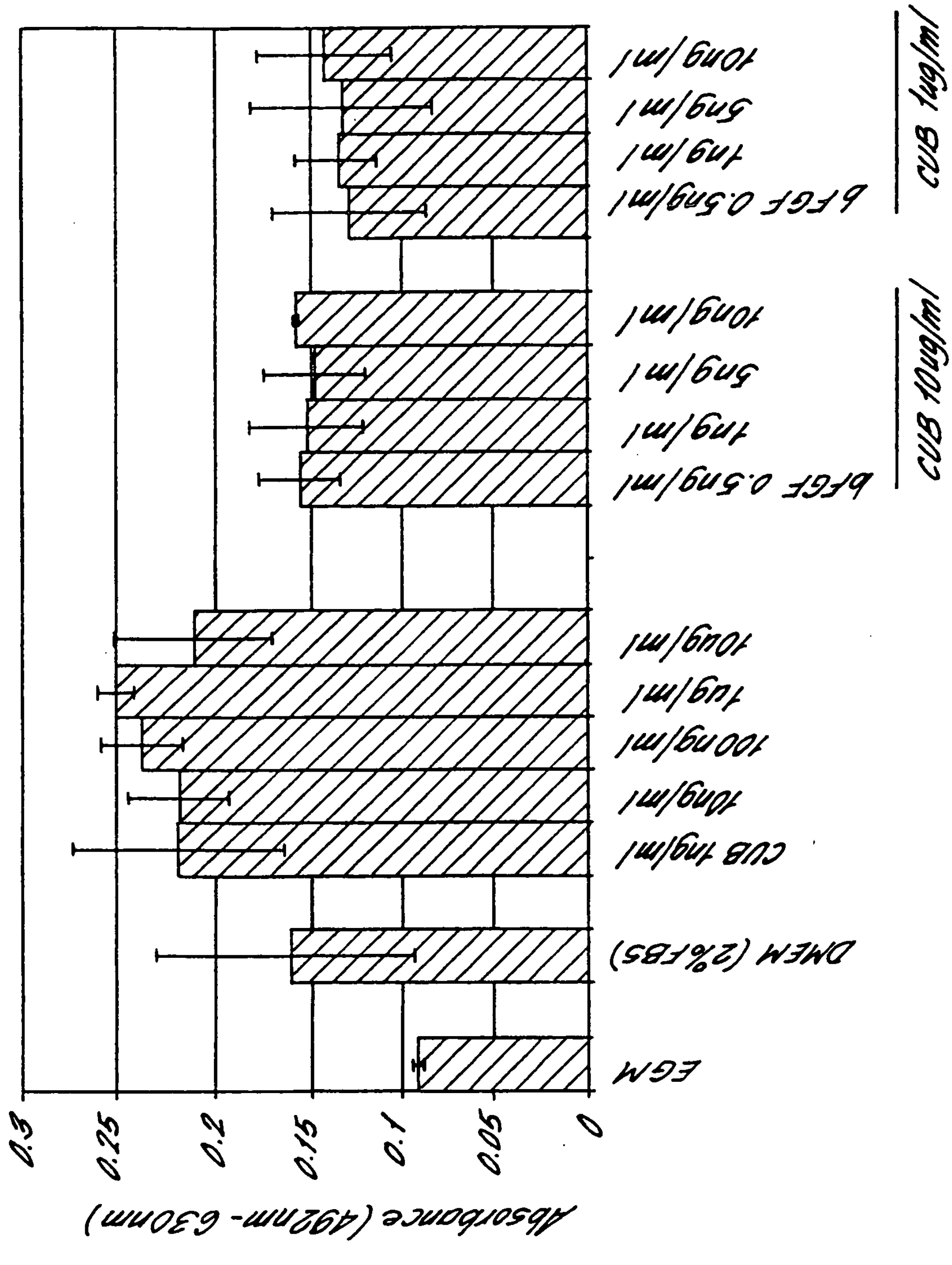

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9524473 A
- WO 9828621 A
- WO 9013649 A
- EP 0476983 A
- EP 0550296 A
- WO 9626736 A
- WO 9627007 A
- WO 9849300 A
- WO 9836075 A
- WO 98840124 A
- WO 9011084 A
- WO 9824811 A
- WO 9810071 A
- WO 9807832 A
- WO 9802543 A
- WO 9705250 A
- WO 9102058 A
- WO 9639421 A
- WO 9639515 A
- WO 9816551 A
- EP 0984 A
- EP 063 A
- US 9930503 W
- GB 9828377 A
- US 60124967 B
- US 60164131 B


### Non-patent literature cited in the description


- Vascular Endothelial Growth Factor: Basic Biology and Clinical Implications. **FERRARA ; I.D. GOLDBERG ; E.M. ROSEN et al.** Regulation of angiogenesis. Birkhauser Verlag, 1997
- **GAJDUSEK, C.M. ; CARBON, S.** *J., Cell Physiol.,* 1989, vol. 139, 570-579
- **MCNEIL, P.L. ; MUTHUKRISHNAN, L. ; WARDER, E. ; D'AMORE, P.A.** *J. Cell. Biol.,* 1989, vol. 109, 811-822
- **JAKEMAN, L.B. et al.** *Clin. Invest.,* 1989, vol. 89, 244-253
- **PLATE, K.H.** *Nature,* 1992, vol. 359, 845-348
- **KIM, K.J.** *Nature,* 1993, vol. 362, 841-844
- **KIM K. J. et al.** *Nature,* 1993, vol. 362, 841-844
- **FOLKMAN, J.** *Nature Medicine,* 1995, vol. 1, 27-31
- **AUSUBEL, F.M. et al.** Current Protocols in Molecular Biology. Green Inc, 1989, vol. I
- **LOCKHART et al.** Expression monitoring by hybridisation to high density oligonucleotide arrays. *Nature Biotechnology,* December 1996, vol. 14
- **SAMBROOK et al.** *Molecular Cloning: a Laboratory Manual,* 1989
- **KOHLER R. ; MILSTEIN C.** *Nature,* 1975, vol. 256, 495-497
- **CHIEN et al.** *Proc. Natl. Acad. Sci. USA,* 1991, vol. 88, 9578-9582
- **ALTSCHUL et al.** Basic Local Alignment Search Tool. *J. Mol. Biol.,* 1990, vol. 215, 403-410
- **VON HEIJNE.** *Nucleic Acids Res.,* 1986, vol. 14, 4683-4690
- **MULLER et al.** *Proc. Natl. Acad. Sci USA,* 1997, vol. 94, 7192-7197
- **SOKER et al.** *Cell,* 1998, vol. 92, 735-745
- **CHRISTINGER et al.** *PROTEINS: Structure, Function and Genetics,* 1996, vol. 26, 353-357
- **ACHEN et al.** *Proc. Natl. Acad. Sci USA,* 1998, vol. 95, 548-553
- Current Protocols in Molecular Biology. John Wiley and Sons, 1997
- **VON HEIJNE, G.** *Nucleic Acids Res.,* 1986, vol. 14, 4683-4690
- **MULLER, YA ; B LI ; HW CHRISTINGER ; JA WELLS ; BC CUNNINGHAM ; AM DE VOS.** Vascular endothelial growth factor: crystal structure and functional mapping of the kinase domain receptor binding site. *Proc. Natl. Acad. Sci USA,* 1997, vol. 94, 7192-7197
- **KORFF, T ; AUGUSTIC, H.G.** Integration of endothelial cells in multicellular spheroids prevents apoptosis and induced differentiation. *The Journal of Cell Biology,* 1998, vol. 143, 1341-1352
- **CHRISTINGER, HW ; YA MULLER ; LT BERLEAU ; BA KEYT ; BC CUNNINGHAM ; N FERRARA ; AM DE VOS.** *PROTEINS: Structure, Function and Genetics,* 1996, vol. 26, 353-357
- **ACHEN, MG ; M JELTSCH ; E KUKK ; T MAKINEN ; A VITALI ; AF WILKS ; K ALITALO ; SA STACKER.** *Proc. Natl. Acad. Sci USA,* 1998, vol. 95, 548-553

- **SIEMEISTER, G ; B SCHNURR ; K MOHRS ; C SCHACHTELE ; C MARME ; G MARTINY-BARON.** *Biochem. Biophys. Res. Commun.,* 1996, vol. 222, 249-255
- **SOKER, S ; S TAKASHIMA ; HQ MIAO ; G NEUFELD ; M KLAGSBRUN.** Neuropilin-1 is expressed by endothelial and tumor cells as an isoform-specific receptor for vascular endothelial growth factor. *Cell,* 1998, vol. 92, 735-745
- **NEUFELD, G ; T COHEN ; S GENGRINOVITCH ; Z POLTORAK.** Vascular endothelial growth factor and its receptors. *FASEB J.,* 1999, vol. 13, 9-22
- **OEFNER, C. ; D'ARCY, A. ; WINKLER, F.K. ; EGGIMANN, B. ; HOSANG, M.** Crystal structure of human platelet-derived growth factor BB. *EMBO J.,* 1992, vol. 11, 3921-3926
- **PASSANTI, A. ; TAYLOR, R.M. ; PILI, R. ; GUO, Y. ; LONG, P.V. ; HANEY, J.A. ; PAULY, R. ; GRANT, D.S. ; MARTIN, G.R.** A simple, quantitative method for assessing angiogenesis and antiangiogenic agents using reconstituted basement membrane, heparin and fibroblast growth factor. *Laboratory Investigation,* 1992, vol. 67, 519-528
- **ROCCHIGIANI, M. ; LESTINGI, M. ; LUDDI, A. ; ORLANDINI, M. ; FRANCO, B. ; ROSSI, E. ; BALLABIO, A. ; ZUFFARDI, 0. ; OLIVIERO, S.** Human FIGF: cloning, gene structure, and mapping to chromosome Xp22.1 between the PIGA and the GRPR genes. *Genomics,* 1990, vol. 47, 207-216
- **TAKAHASHI, Y. ; KITADAI, Y. ; BUCANA, C.D. ; CLEARY, K.R. ; ELLIS, L.M.** Expression of vascular endothelial growth factor and its receptor, KDR, correlates with vascularity, metastasis and proliferation of human colon cancer. *Cancer Research,* 1995, vol. 55, 3964-3968
- **TISCHER, E. ; MITCHELL, R. ; HARTMAN, T. ; SILVA, M. ; GOSPODAROWICZ, D. ; FIDDES, J.C. ; ABRAHAM, J.A.** The human gene for vascular endothelial growth factor: Multiple protein forms are encoded through alternative exon splicing. *J. Biol. Chem.,* 1991, vol. 266, 11947-11954

SEQUENCE LISTING

<110> Janssen Pharmaceutica N.V. et al
<120> Vascular Endothelial Growth Factor-X
<130> 51935/004
<140> PCT/US99/30503
<141> 1999-12-21
<150> GB 9828377.3
<151> 1998-12-22
<150> US 60/124,967
<151> 1999-03-18
<150> US 60/164,131
<151> 1999-11-08
<160> 97
<170> PatentIn Ver. 2.0
<210> 1
<211> 323
<212> PRT
<213> Homo sapiens
<400> 1

```
Glu Ser Asn Leu Ser Ser Lys Phe Gln Phe Ser Ser Asn Lys Glu Gln
 1               5                  10                  15

Tyr Gly Val Gln Asp Pro Gln His Glu Arg Ile Ile Thr Val Ser Thr
            20                  25                  30

Asn Gly Ser Ile His Ser Pro Arg Phe Pro His Thr Tyr Pro Arg Asn
        35                  40                  45

Thr Val Leu Val Trp Arg Leu Val Ala Val Glu Glu Asn Val Trp Ile
    50                  55                  60

Gln Leu Thr Phe Asp Glu Arg Phe Gly Leu Glu Asp Pro Glu Asp Asp
65                  70                  75                  80

Ile Cys Lys Tyr Asp Phe Val Glu Val Glu Glu Pro Ser Asp Gly Thr
                85                  90                  95
```

```
Ile Leu Gly Arg Trp Cys Gly Ser Gly Thr Val Pro Gly Lys Gln Ile
            100                 105                 110

Ser Lys Gly Asn Gln Ile Arg Ile Arg Phe Val Ser Asp Glu Tyr Phe
        115                 120                 125

Pro Ser Glu Pro Gly Phe Cys Ile His Tyr Asn Ile Val Met Pro Gln
    130                 135                 140

Phe Thr Glu Ala Val Ser Pro Ser Val Leu Pro Pro Ser Ala Leu Pro
145                 150                 155                 160

Leu Asp Leu Leu Asn Asn Ala Ile Thr Ala Phe Ser Thr Leu Glu Asp
                165                 170                 175

Leu Ile Arg Tyr Leu Glu Pro Glu Arg Trp Gln Leu Asp Leu Glu Asp
            180                 185                 190

Leu Tyr Arg Pro Thr Trp Gln Leu Leu Gly Lys Ala Phe Val Phe Gly
        195                 200                 205

Arg Lys Ser Arg Val Val Asp Leu Asn Leu Leu Thr Glu Glu Val Arg
    210                 215                 220

Leu Tyr Ser Cys Thr Pro Arg Asn Phe Ser Val Ser Ile Arg Glu Glu
225                 230                 235                 240

Leu Lys Arg Thr Asp Thr Ile Phe Trp Pro Gly Cys Leu Leu Val Lys
                245                 250                 255

Arg Cys Gly Gly Asn Cys Ala Cys Cys Leu His Asn Cys Asn Glu Cys
            260                 265                 270

Gln Cys Val Pro Ser Lys Val Thr Lys Lys Tyr His Glu Val Leu Gln
        275                 280                 285

Leu Arg Pro Lys Thr Gly Val Arg Gly Leu His Lys Ser Leu Thr Asp
    290                 295                 300

Val Ala Leu Glu His His Glu Glu Cys Asp Cys Val Cys Arg Gly Ser
305                 310                 315                 320

Thr Gly Gly
```

<210> 2
<211> 345
<212> PRT
<213> Homo sapiens
<400> 2

```
Met Ser Leu Phe Gly Leu Leu Leu Leu Thr Ser Ala Leu Ala Gly Gln
 1               5                  10                  15

Arg Gln Gly Thr Gln Ala Glu Ser Asn Leu Ser Ser Lys Phe Gln Phe
            20                  25                  30

Ser Ser Asn Lys Glu Gln Tyr Gly Val Gln Asp Pro Gln His Glu Arg
        35                  40                  45

Ile Ile Thr Val Ser Thr Asn Gly Ser Ile His Ser Pro Arg Phe Pro
    50                  55                  60

His Thr Tyr Pro Arg Asn Thr Val Leu Val Trp Arg Leu Val Ala Val
65                  70                  75                  80

Glu Glu Asn Val Trp Ile Gln Leu Thr Phe Asp Glu Arg Phe Gly Leu
                85                  90                  95

Glu Asp Pro Glu Asp Asp Ile Cys Lys Tyr Asp Phe Val Glu Val Glu
            100                 105                 110

Glu Pro Ser Asp Gly Thr Ile Leu Gly Arg Trp Cys Gly Ser Gly Thr
        115                 120                 125

Val Pro Gly Lys Gln Ile Ser Lys Gly Asn Gln Ile Arg Ile Arg Phe
    130                 135                 140

Val Ser Asp Glu Tyr Phe Pro Ser Glu Pro Gly Phe Cys Ile His Tyr
145                 150                 155                 160

Asn Ile Val Met Pro Gln Phe Thr Glu Ala Val Ser Pro Ser Val Leu
                165                 170                 175

Pro Pro Ser Ala Leu Pro Leu Asp Leu Leu Asn Asn Ala Ile Thr Ala
            180                 185                 190

Phe Ser Thr Leu Glu Asp Leu Ile Arg Tyr Leu Glu Pro Glu Arg Trp
        195                 200                 205

Gln Leu Asp Leu Glu Asp Leu Tyr Arg Pro Thr Trp Gln Leu Leu Gly
    210                 215                 220

Lys Ala Phe Val Phe Gly Arg Lys Ser Arg Val Val Asp Leu Asn Leu
225                 230                 235                 240
```

```
Leu Thr Glu Glu Val Arg Leu Tyr Ser Cys Thr Pro Arg Asn Phe Ser
                245                     250                 255

Val Ser Ile Arg Glu Glu Leu Lys Arg Thr Asp Thr Ile Phe Trp Pro
            260                     265                 270

Gly Cys Leu Leu Val Lys Arg Cys Gly Gly Asn Cys Ala Cys Cys Leu
        275                     280                 285

His Asn Cys Asn Glu Cys Gln Cys Val Pro Ser Lys Val Thr Lys Lys
    290                     295                 300

Tyr His Glu Val Leu Gln Leu Arg Pro Lys Thr Gly Val Arg Gly Leu
305                     310                 315                 320

His Lys Ser Leu Thr Asp Val Ala Leu Glu His His Glu Glu Cys Asp
                325                     330                 335

Cys Val Cys Arg Gly Ser Thr Gly Gly
            340                 345
```

<210> 3
<211> 1035
<212> DNA
<213> Homo sapiens
<400> 3

```
atgagcctct tcgggcttct cctgctgaca tctgccctgg ccggccagag acaggggact 60
caggcggaat ccaacctgag tagtaaattc cagttttcca gcaacaagga acagaacgga 120
gtacaagatc ctcagcatga gagaattatt actgtgtcta ctaatggaag tattcacagc 180
ccaaggtttc ctcatactta tccaagaaat acggtcttgg tatggagatt agtagcagta 240
gaggaaaatg tatggataca acttacgttt gatgaaagat ttgggcttga agacccagaa 300
gatgacatat gcaagtatga ttttgtagaa gttgaggaac ccagtgatgg aactatatta 360
gggcgctggt gtggttctgg tactgtacca ggaaaacaga tttctaaagg aaatcaaatt 420
aggataagat ttgtatctga tgaatatttt ccttctgaac cagggttctg catccactac 480
aacattgtca tgccacaatt cacagaagct gtgagtcctt cagtgctacc cccttcagct 540
ttgccactgg acctgcttaa taatgctata actgccttta gtaccttgga agaccttatt 600
cgatatcttg aaccagagag atggcagttg gacttagaag atctatatag gccaacttgg 660
caacttcttg gcaaggcttt tgtttttgga agaaaatcca gagtggtgga tctgaacctt 720
ctaacagagg aggtaagatt atacagctgc acacctcgta acttctcagt gtccataagg 780
gaagaactaa agagaaccga taccattttc tggccaggtt gtctcctggt taaacgctgt 840
ggtgggaact gtgcctgttg tctccacaat tgcaatgaat gtcaatgtgt cccaagcaaa 900
gttactaaaa aataccacga ggtccttcag ttgagaccaa agaccggtgt caggggattg 960
cacaaatcac tcaccgacgt ggccctggag caccatgagg agtgtgactg tgtgtgcaga 1020
gggagcacag gagga                                                  1035
```

<210> 4
<211> 22
<212> DNA
<213> Artificial Sequence
<220>
<223> Description of Artificial Sequence: primer
<400> 4
aaaatgtatg gatacaactt ac 22
<210> 5
<211> 23
<212> DNA
<213> Artificial Sequence
<220>
<223> Description of Artificial Sequence:primer
<400> 5
gtttgatgaa agatttgggc ttg 23
<210> 6
<211> 22
<212> DNA
<213> Artificial Sequence
<220>
<223> Description of Artificial Sequence: primer
<400> 6
tttctaaagg aaatcaaatt ag 22
<210> 7
<211> 20
<212> DNA
<213> Artificial Sequence
<220>
<223> Description of Artificial Sequence: primer
<400> 7
gataagattt gtatctgatg 20

<210> 8
<211> 17
<212> DNA
<213> Artificial Sequence
<220>
<223> Description of Artificial Sequence: primer
<400> 8
gatgtctcct ctttcag 17
<210> 9
<211> 18
<212> DNA
<213> Artificial Sequence
<220>
<223> Description of Artificial Sequence: primer
<400> 9
gcacaactcc taattctg 18
<210> 10
<211> 18
<212> DNA
<213> Artificial Sequence
<220>
<223> Description of Artificial Sequence: primer
<400> 10
agcacctgat tccgttgc 18
<210> 11
<211> 20
<212> DNA.
<213> Artificial Sequence
<220>
<223> Description of Artificial Sequence: primer
<400> 11
tagtacatag aatgttctgg 20
<210> 12
<211> 19
<212> DNA
<213> Artificial Sequence
<220>
<223> Description of Artificial Sequence: primer
<400> 12
aagagacata cttctgtac 19
<210> 13
<211> 21
<212> DNA
<213> Artificial Sequence
<220>
<223> Description of Artificial Sequence:primer
<400> 13
ccaggtacaa taagtgaact g 21
<210> 14
<211> 28
<212> DNA
<213> Artificial Sequence
<220>
<223> Description of Artificial Sequence:primer
<400> 14
cctttagaaa tctgttttcc tggtacag 28
<210> 15
<211> 31

<212> DNA
<213> Artificial Sequence
<220>
<223> Description of Artificial Sequence:primer
<400> 15
ggaaaatatt catcagatac aaatcttatc c 31
<210> 16
<211> 22
<212> DNA
<213> Artificial Sequence
<220>
<223> Description of Artificial Sequence:primer
<400> 16
ggtccagtgg caaagctgaa gg 22
<210> 17
<211> 29
<212> DNA
<213> Artificial Sequence
<220>
<223> Description of Artificial Sequence:primer
<400> 17
ctggttcaag atatcgaata aggtcttcc 29
<210> 18
<211> 24
<212> DNA
<213> Artificial Sequence
<220>
<223> Description of Artificial Sequence:primer
<400> 18
tttgtttaaa ccttgggaaa ctgg 24
<210> 19
<211> 21
<212> DNA
<213> Artificial Sequence
<220>
<223> Description of Artificial Sequence:primer
<400> 19
gtccaggttt tgctttgatc c 21
<210> 20
<211> 30
<212> DNA
<213> Artificial Sequence
<220>
<223> Description of Artificial Sequence:primer
<400> 20
aattggatcc gagagtggtg gatctgaacc 30
<210> 21
<211> 30
<212> DNA
<213> Artificial Sequence
<220>
<223> Description of Artificial Sequence:primer
<400> 21
aattggatcc gggaagaaaa tccagagtgg 30
<210> 22
<211> 40
<212> DNA
<213> Artificial Sequence

<220>
<223> Description of Artificial Sequence:primer
<400> 22
ggttgaattc attatttttt agtaactttg cttgggacac 40
<210> 23
<211> 31
<212> DNA
<213> Artificial Sequence
<220>
<223> Description of Artificial Sequence:primer
<400> 23
aattgaattc attatcctcc tgtgctccct c 31
<210> 24
<211> 60
<212> DNA
<213> Artificial Sequence
<220>
<223> Description of Artificial Sequence: primer
<400> 24
aattggatcc ggagtctcac catcaccacc atcatgaatc caacctgagt agtaaattcc 60
<210> 25
<211> 34
<212> DNA
<213> Artificial Sequence
<220>
<223> Description of Artificial Sequence: primer
<400> 25
aattgaattc gctatcctcc tgtgctccct ctgc 34
<210> 26
<211> 111
<212> PRT
<213> Homo sapiens
<400> 26

Gly Val Gln Asp Pro Gln His Glu Arg Ile Ile Thr Val Ser Thr Asn
1 5 10 15

Gly Ser Ile His Ser Pro Arg Phe Pro His Thr Tyr Pro Arg Asn Thr
20 25 30

Val Leu Val Trp Arg Leu Val Ala Val Glu Glu Asn Val Trp Ile Gln
35 40 45

Leu Thr Phe Asp Glu Arg Phe Gly Leu Glu Asp Pro Glu Asp Asp Ile
50 55 60

Cys Lys Tyr Asp Phe Val Glu Val Glu Glu Pro Ser Asp Gly Thr Ile
65 70 75 80

Leu Gly Arg Trp Cys Gly Ser Gly Thr Val Pro Gly Lys Gln Ile Ser
85 90 95

Lys Gly Asn Gln Ile Arg Ile Arg Phe Val Ser Asp Glu Tyr Phe
100 105 110

<210> 27
<211> 168
<212> PRT
<213> Homo sapiens
<400> 27

Met Ala Met Asp Ile Gly Ile Asn Ser Asp Pro Glu Ser His His His
1 5 10 15

His His His Glu Ser Asn Leu Ser Ser Lys Phe Gln Phe Ser Ser Asn
20 25 30

Lys Glu Gln Asn Gly Val Gln Asp Pro Gln His Glu Arg Ile Ile Thr
35 40 45

Val Ser Thr Asn Gly Ser Ile His Ser Pro Arg Phe Pro His Thr Tyr
50 55 60

```
Pro Arg Asn Thr Val Leu Val Trp Arg Leu Val Ala Val Glu Glu Asn
 65                 70                  75                  80

Val Trp Ile Gln Leu Thr Phe Asp Glu Arg Phe Gly Leu Glu Asp Pro
                85                  90                  95

Glu Asp Asp Ile Cys Lys Tyr Asp Phe Val Glu Val Glu Glu Pro Ser
            100                 105                 110

Asp Gly Thr Ile Leu Gly Arg Trp Cys Gly Ser Gly Thr Val Pro Gly
        115                 120                 125

Lys Gln Ile Ser Lys Gly Asn Gln Ile Arg Ile Arg Phe Val Ser Asp
    130                 135                 140

Glu Tyr Phe Pro Ser Glu Pro Gly Phe Cys Ile His Tyr Asn Ile Val
145                 150                 155                 160

Met Pro Gln Phe Thr Glu Ala Val
                165
```

<210> 28
<211> 504
<212> DNA
<213> Homo sapiens
<400> 28

```
atggccatgg atatcggaat taattcggat ccggagtctc accatcacca ccatcatgaa  60
tccaacctga gtagtaaatt ccagttttcc agcaacaagg aacagaacgg agtacaagat 120
cctcagcatg agagaattat tactgtgtct actaatggaa gtattcacag cccaaggttt 180
cctcatactt atccaagaaa tacggtcttg gtatggagat tagtagcagt agaggaaaat 240
gtatggatac aacttacgtt tgatgaaaga tttgggcttg aagacccaga agatgacata 300
tgcaagtatg attttgtaga agttgaggaa cccagtgatg gaactatatt agggcgctgg 360
tgtggttctg gtactgtacc aggaaaacag atttctaaag gaaatcaaat taggataaga 420
tttgtatctg atgaatattt tccttctgaa ccagggttct gcatccacta caacattgtc 480
atgccacaat tcacagaagc tgtg                                        504
```

<210> 29
<211> 132
<212> PRT
<213> Homo sapiens
<400> 29

Asp Leu Tyr Arg Pro Thr Trp Gln Leu Leu Gly Lys Ala Phe Val Phe
1 5 10 15

Gly Arg Lys Ser Arg Val Val Asp Leu Asn Leu Leu Thr Glu Glu Val

20 25 30

Arg Leu Tyr Ser Cys Thr Pro Arg Asn Phe Ser Val Ser Ile Arg Glu
35 40 45

Glu Leu Lys Arg Thr Asp Thr Ile Phe Trp Pro Gly Cys Leu Leu Val
50 55 60

Lys Arg Cys Gly Gly Asn Cys Ala Cys Cys Leu His Asn Cys Asn Glu
65 70 75 80

Cys Gln Cys Val Pro Ser Lys Val Thr Lys Lys Tyr His Glu Val Leu
85 90 95

Gln Leu Arg Pro Lys Thr Gly Val Arg Gly Leu His Lys Ser Leu Thr
100 105 110

Asp Val Ala Leu Glu His His Glu Glu Cys Asp Cys Val Cys Arg Gly
115 120 125

Ser Thr Gly Gly
130

<210> 30
<211> 300
<212> DNA
<213> Artificial Sequence
<220>
<223> Description of Artificial Sequence: Human EST
<400> 30

cacaaatcac tcaccgacgt ggccctggag caccatgagg ngtgtgactg tgtgtgcaga 60
gggagcacag gaggatagcc gcatcaccac cagcagctct tgcccagagc tgtgcagtgc 120
agtggctgat tctattagag aacgtatgcg ttatctccat ccttaatctc agttgtttgc 180
ttcaaggacc tttcatcttc aggatttaca gtgcattctg aaagaggaga catcaaacag 240
aattaggagt tgtgcaacag ctcttttgag aggaggctaa aggacaggag aanaggtctt 300

<210> 31
<211> 284
<212> DNA
<213> Artificial Sequence
<220>

<223> Description of Artificial Sequence:Human EST
<400> 31

```
tgcagtgcag tggctgattc tattagagaa cgtatgcgtt atctccatcc ttaatctcag 60
ttgtttgctt caaggacctt tcatcttcag gatttacagt gcattctgaa agaggagaca 120
tcaaacagaa ttaggagttg tgcaacagct cttttgagag gaggcctaaa ggacaggaga 180
aaaggtcttc aatcgtggaa agaaaattaa atgttgtatt aaatagatca ccagctagtt 240
tcagagttac catgtacgta ttccactagc tgggttctgt attt                 284
```

<210> 32
<211> 275
<212> DNA
<213> Artificial Sequence
<220>
<223> Description of Artificial Sequence: Human EST
<400> 32

```
cacgaggtcc ttcagttgag accaaagacc ggtgtcaggg gattgcacaa atcactcacc 60
gacgtggccc tggagcacca tgaggagtgt gactgtgtgt gcagagggag cacaggggga 120
tagccgcatc accaccagca gctcttgccc agagctgtgc agtgcagtgg ctgattctat 180
tagagaacgt atgcgttatc tccatcctta atctcagttg tttgcttcaa ggacctttca 240
tcttcaggat ttacagtgca ttctgaaaga ggaga                            275
```

<210> 33
<211> 278
<212> DNA
<213> Artificial Sequence
<220>
<223> Description of Artificial Sequence: Human EST
<400> 33

```
ggaggatagc cgcatcacca ccagcagctc ttgcccagag ctgtgcagtg cagtggctga 60
ttctattaga gaacgtatgc gttatctcca tccttaatct cagttgtttg cttcaaggac 120
ctttcatctt caggatttac agtgcattct gaaagaggag acatcaaaca gaattaggag 180
ttgtgcaaca gctcttttga gaggaggcct aaaggacagg agaaaaggtc ttcaatcgtg 240
gaaagaanat taaatgttgt attaaataga caccagct                         278
```

<210> 34
<211> 275
<212> DNA
<213> Artificial Sequence
<220>
<223> Description of Artificial Sequence: Human EST
<400> 34

ggaggatagc cgcatcacca ccagcagctc ttgcccagag ctgtgcagtg cagtggctga 60
ttctattaga gaacgtatgc gttatctcca tccttaatct cagttgtttg cttcaaggac 120
ctttcatctt caggatttac atgcattctg aaagaggaga catcaaacag aattaggagt 180

tgtgcaacag ctcttttgag aggaggccta aaggacagga gaaaaggtct tcaatcgtgg 240
aaagaaaatt aaatgttgta ttaaatagat cacca 275

<210> 35
<211> 261
<212> DNA
<213> Artificial Sequence
<220>
<223> Description of Artificial Sequence: Human EST
<400> 35

gagaaccgat accattttct ggccaggttg tctcctggtt aaacgctgtg gtgggaactg 60
tgcctgttgt ctccacaatt gcaatgaatg tcaatgtgtc ccaagcaaag ttactaaaaa 120
ataccacgag gtccttcagt tgagaccaaa gaccggtgtc aggggattgc acaaatcact 180
caccgacgtg gccctggagc accatgagga gtgtgactgt gtgtgcagag ggagcacagg 240
aggatagccg catcaccacc a 261

<210> 36
<211> 279
<212> DNA
<213> Artificial Sequence
<220>
<223> Description of Artificial Sequence: Human EST
<400> 36

agaaaatcca gagtggtgga tctgaacctt ctaacagagg aggtaagatt atacagctgc 60
acacctcgta acttctcagt gtccataagg gaagaactaa agagaaccga taccattttc 120
tggccaggtt gtctcctggt taaacgctgt ggtgggaact gtgcctgttg tctccacaat 180
tgcaatgaat gtcaatgtgt cccaagcaaa gttactaaaa aataccacga ggtccttcag 240
ttgagaccaa agaccggtgt caggggattg cacaaatca 279

<210> 37
<211> 262
<212> DNA
<213> Artificial Sequence
<220>
<223> Description of Artificial Sequence: Human EST
<400> 37

aggaaatcaa attaggataa gatttgtatc tgatgaatat tttccttctg aaccttctaa 60
cagaggaggt aagattatac agctgcacac ctcgtaactt ctcagtgtcc ataagggaag 120
aactaaagag aaccgatacc atttctggc caggttgtct cctggttaaa cgctgtggtg 180
ggaactgtgc ctgttgtctc ccacaattgc aatgaatgtc aatgtgtccc aagcaaagtt 240
actaaaaaat accacgaggt cc 262

<210> 38
<211> 289
<212> DNA
<213> Artificial Sequence
<220>
<223> Description of Artificial Sequence: Human EST
<400> 38

atttcatctt caggatttac agtgcattct gaaanaggag aaatcaaaca naattaggag 60
ttgtgcaaca gctcttttga gaggaggcct aaaggacagg agaaaaggtc ttcaatcgtg 120
gaaanaaaat taaatgttgt attaaataga tcaccagcta gtttcagagt taccatgtac 180
gtattccact agctgggttc tgtatttcag ttctttcgat acggcttagg gtaatgtcag 240
tacaggaaaa aaactgtgca agtgagcacc tgattccgtt gccttgctt 289

<210> 39
<211> 245
<212> DNA
<213> Artificial Sequence
<220>
<223> Description of Artificial Sequence: Human EST
<400> 39

caaagttact aaaaaatacc acgaggtcct tcagttgaga ccaaagaccg gtgtcagggg 60
attgcacaaa tcactcaccg acgtggccct ggagcaccat gaggagtgtg actgtgtgtg 120
cagagggagc acaggaggat agccgcatca ccaccagcag ctcttgccca gagctgtgca 180
gtgcagtggc tgattctatt agagaacgta tgcgttatct ccatccttaa tctcagttgt 240
ttgct 245

<210> 40
<211> 247
<212> DNA
<213> Artificial Sequence
<220>
<223> Description of Artificial Sequence: Human EST
<400> 40

angagttgcc cagagctgtg cagtgcagtg gctgattcta ttagagaacg tatgcgttat 60
ctccatcctt aatctcagtt gtttgnttca aggacctttc atcttcagga tttacagtgc 120
attctgaaag aggagacatc aaacagaatt aggagttgtg caacagctct tttgagagga 180
ggcctaaagg ncaggagaaa aggtcttcaa tcgtggaaag aaaattaaat gttgtattaa 240
atagatc 247

<210> 41
<211> 232
<212> DNA
<213> Artificial Sequence
<220>
<223> Description of Artificial Sequence: Human EST
<400> 41

aggaaatcaa attaggataa gatttgtatc tgatgaatat tttccttctg aaccttctaa 60
cagaggaggt aagattatac agctgcacac ctcgtaactt ctcagtgtcc ataagggaag 120
aactaaagag aaccgatacc attttctggc caggttgtct cctggttaaa cgctgtggtg 180
ggaactgtgc ctgttgtctc cacaattgca atgaatgtca atgtgtccca ag 232

<210> 42
<211> 253
<212> DNA
<213> Artificial Sequence
<220>
<223> Description of Artificial Sequence: Human EST
<400> 42

gtgcattctg aaagaggaga catcaaacag aattaggagt tgtgcaacag ctcttttgag 60
aggaggccta aaggacagga gaaaaggtct tcaatcgtgg aaagaaaatt aaatgttgta 120
ttaaatagat caccagctag tttcagagtt accatgtacg tattccacta gctgggttct 180
gtatttcagt tctttcgata cggcttaggg taatgtcagt acaggaaaaa aactgtgcaa 240
gtgagcacct gat 253

<210> 43
<211> 265
<212> DNA
<213> Artificial Sequence
<220>
<223> Description of Artificial Sequence: Human EST
<400> 43

tgcaacagct cttttgagag gaggcctaaa ggacaggaga aaaggtcttc aatcgtggaa 60
agaaaattaa atgttgtatt aaatagatca ccagctagtt tcagagttac catgtacgta 120
ttccactagc tgggttctgt atttcagttc tttcgatacg gcttagggta atgtcagtac 180
aggaaaaaaa ctgtgcaagt gagcacctga ttccgttgcc ttgcttaacc ctaaagcncc 240
atgtcnnggg cnaaaancga aaaat 265

<210> 44
<211> 291
<212> DNA
<213> Artificial Sequence
<220>
<223> Description of Artificial Sequence: Human EST
<400> 44

```
ccttaatctc agttgtttgc ttcaaggacc tttcatcttc aggatttaca gtgcattctg 60
naagangaga catcaaacag aattaggngt tgtgcaaaag ctcttttgag aggaggccta 120
aaggacagga gaaaaggtct ncaatcgtgg aaagnaaatt aaatgttgta tnaaatngat 180
caccagctag tttcagagtt accatgtacg tattccacta gctgggncng tattcagtct 240
ttcggaacgg cttagggtaa tgtcagtaca gganaaaaac tgtgcagtga g          291
```

<210> 45
<211> 279
<212> DNA
<213> Artificial Sequence
<220>
<223> Description of Artificial Sequence: Human EST
<400> 45

```
attaaataga tcaccagcta gtttcagagt taccatgtac gtattccact agctgggttc 60
tgtatttcag ttctttcgat acggcttagg gtaatgtcag tacaggaaaa aaactgtgca 120
agtgagcacc tgattccgtt gccttggctt aactctaaag ctccatgtcc tgggcctaaa 180
atcgtataaa atctggattt ttttnttttt ttttgcgcat attcacatat gtaaaccagn 240
acattctatg tacnacaaac ctggttttta aaaaggaac                         279
```

<210> 46
<211> 181
<212> DNA
<213> Artificial Sequence
<220>
<223> Description of Artificial Sequence: Human EST
<400> 46

```
ggctagtttc agagttacca tgtacgtatt ccactagctg ggttctgtat ttcagttctt 60
tcgatacggc ttagggtaat gtcagtacag gaaaaaaact gtgcaagtga gcacctgatt 120
ccgttgcctt gcttaactct aaagctccat gtcctgggcc taaaatcgta taaaatctgg 180
a                                                                 181
```

<210> 47
<211> 184
<212> DNA
<213> Artificial Sequence
<220>
<223> Description of Artificial Sequence: Human EST
<400> 47

```
aatagatcac cagctagttt cagagttacc atgtacgtat tccactagct gggntctgta 60
tttcagttcc tttcgatacg gcttagggta atgtcagtac aggaaaaaag ctgtgcaagt 120
gagcacctga ttccgttgcc ttgcttaact ctaaagctcc atgtcctggg cctaaaatcg 180
tata                                                              184
```

<210> 48
<211> 290
<212> DNA
<213> Artificial Sequence
<220>
<223> Description of Artificial Sequence: Human EST
<400> 48

aaaggaacta tgttgctatg aattaaactt gtgtcgtgct gataggacag actggatttt 60
tcatatttct tattaaaatt tctgccattt agaagaagag aactacattc atggtttgga 120
agagataaac ctgaaaagaa gagtggcctt atcttcactt tatcgataag tcagtttatt 180
tgtttcattg tgtacatttt tatattctcc ttttgacatt ataactgttg gcttttctaa 240
tcttgttaaa tatatctatt tttaccaaag gtatttaata ttcttttta 290

<210> 49
<211> 300
<212> DNA
<213> Artificial Sequence
<220>
<223> Description of Artificial Sequence: Human EST
<400> 49

cacaaatcac tcaccgacgt ggccctggag caccatgagg ngtgtgactg tgtgtgcaga 60
gggagcacag gaggatagcc gcatcaccac cagcagctct tgcccagagc tgtgcagtgc 120
agtggctgat tctattagag aacgtatgcg ttatctccat ccttaatctc agttgtttgc 180
ttcaaggacc tttcatcttc aggatttaca gtgcattctg aaagaggaga catcaaacag 240
aattaggagt tgtgcaacag ctcttttgag aggaggctaa aggacaggag aanaggtctt 300

<210> 50
<211> 284
<212> DNA
<213> Artificial Sequence
<220>
<223> Description of Artificial Sequence: Human EST
<400> 50

tgcagtgcag tggctgattc tattagagaa cgtatgcgtt atctccatcc ttaatctcag 60
ttgtttgctt caaggacctt tcatcttcag gatttacagt gcattctgaa agaggagaca 120
tcaaacagaa ttaggagttg tgcaacagct cttttgagag gaggcctaaa ggacaggaga 180
aaaggtcttc aatcgtggaa agaaaattaa atgttgtatt aaatagatca ccagctagtt 240
tcagagttac catgtacgta ttccactagc tgggttctgt attt 284

<210> 51
<211> 301
<212> DNA
<213> Artificial Sequence
<220>
<223> Description of Artificial Sequence: Human EST
<400> 51

cttgttaaat atatctattt ttaccaaagg tatttaatat tctttantta tgacaactta 60
gatcaactat ttttagcttg gtaaattttt ctaaacacaa ttgttatagc cagaggaaca 120
aagatgatat aaaatattgt tgctctgaca aaaatacatg tatttcattc tcgtatggtg 180
ctagagttag attaatctgc attttaaaaa actgaattgg aatagaattg gtaagttgca 240
aagacttttt ganaataatt aaattatcat atcttccatt cctgttattg ggggagaaaa 300
t 301

<210> 52
<211> 275
<212> DNA
<213> Artificial Sequence
<220>
<223> Description of Artificial Sequence: Human EST
<400> 52

cacgaggtcc ttcagttgag accaaagacc ggtgtcaggg gattgcacaa atcactcacc 60
gacgtggccc tggagcacca tgaggagtgt gactgtgtgt gcagagggag cacaggggga 120
tagccgcatc accaccagca gctcttgccc agagctgtgc agtgcagtgg ctgattctat 180
tagagaacgt atgcgttatc tccatcctta atctcagttg tttgcttcaa ggacctttca 240
tcttcaggat ttacagtgca ttctgaaaga ggaga 275

<210> 53
<211> 288
<212> DNA
<213> Artificial Sequence
<220>
<223> Description of Artificial Sequence: Human EST
<400> 53

ttaaaaagga actatgttgc tatgaattaa acttgtgtca tgctgatagg acagactgga 60
tttttcatat ttcttattaa aatttctgcc atttagaaga agagaactac attcatggtt 120
tggaagagat aaacctgaaa agaagagtgg ccttatcttc actttatcga taagtcagtt 180
tatttgtttc attgtgtaca tttttatatt ctccttttga cattataact gttggctttc 240
taatctgtta aatatatcta tttttaccaa aggtatttaa tattcttt 288

<210> 54
<211> 278
<212> DNA
<213> Artificial Sequence
<220>
<223> Description of Artificial Sequence: Human EST
<400> 54

```
ggaggatagc cgcatcacca ccagcagctc ttgcccagag ctgtgcagtg cagtggctga 60
ttctattaga gaacgtatgc gttatctcca tccttaatct cagttgtttg cttcaaggac 120
ctttcatctt caggatttac agtgcattct gaaagaggag acatcaaaca gaattaggag 180
ttgtgcaaca gctcttttga gaggaggcct aaaggacagg agaaaaggtc ttcaatcgtg 240
gaaagaanat taaatgttgt attaaataga caccagct                        278
```

<210> 55
<211> 275
<212> DNA
<213> Artificial Sequence
<220>
<223> Description of Artificial Sequence: Human EST
<400> 55

```
ggaggatagc cgcatcacca ccagcagctc ttgcccagag ctgtgcagtg cagtggctga 60
ttctattaga gaacgtatgc gttatctcca tccttaatct cagttgtttg cttcaaggac 120
ctttcatctt caggatttac atgcattctg aaagaggaga catcaaacag aattaggagt 180
tgtgcaacag ctcttttgag aggaggccta aaggacagga gaaaaggtct tcaatcgtgg 240
aaagaaaatt aaatgttgta ttaaatagat cacca                           275
```

<210> 56
<211> 261
<212> DNA
<213> Artificial Sequence
<220>
<223> Description of Artificial Sequence: Human EST
<400> 56

```
gagaaccgat accattttct ggccaggttg tctcctggtt aaacgctgtg gtgggaactg 60
tgcctgttgt ctccacaatt gcaatgaatg tcaatgtgtc ccaagcaaag ttactaaaaa 120
ataccacgag gtccttcagt tgagaccaaa gaccggtgtc aggggattgc acaaatcact 180
caccgacgtg gccctggagc accatgagga gtgtgactgt gtgtgcagag ggagcacagg 240
aggatagccg catcaccacc a                                          261
```

<210> 57
<211> 279
<212> DNA
<213> Artificial Sequence
<220>
<223> Description of Artificial Sequence: Human EST
<400> 57

```
agaaaatcca gagtggtgga tctgaacctt ctaacagagg aggtaagatt atacagctgc 60
acacctcgta acttctcagt gtccataagg gaagaactaa agagaaccga taccattttc 120
tggccaggtt gtctcctggt taaacgctgt ggtgggaact gtgcctgttg tctccacaat 180
tgcaatgaat gtcaatgtgt cccaagcaaa gttactaaaa aataccacga ggtccttcag 240
ttgagaccaa agaccggtgt caggggattg cacaaatca                       279
```

<210> 58

<211> 259
<212> DNA
<213> Artificial Sequence
<220>
<223> Description of Artificial Sequence: Human EST
<400> 58

```
agatgatata aaatattgtt gctctgacaa aaatacatgt atttcattct cgtatggtgc 60
tagagttaga ttaatctgca ttttaaaaaa ctgaattgga atagaattgg taagttgcaa 120
agactttttg aaaataatta aattatcata tcttccattc ctgttattgg agatgaaaat 180
aaaaagcaac ttatgaaagt agacattcag atccagccat tactaaccta ttcctttttt 240
ggggaaatct gagcctagc                                              259
```

<210> 59
<211> 284
<212> DNA
<213> Artificial Sequence
<220>
<223> Description of Artificial Sequence: Human EST
<400> 59

```
tttttaaaaa ggaactatgt tgctatgaat taaacttgtg tcgtgctgat aggacagact 60
ggatttttca tatttcttat taaaatttct gccatttaga agaagagaac tacattcatg 120
gtttggaaga gataaacctg aaaagaagag tggcctatct tcactttatc gataagtcag 180
tttatttgtt tcattgtgta catttttata ttctcctttg acatataact gttggctttt 240
ctaatctgtt aaatatatct atttttacca aaggtattta atat                  284
```

<210> 60
<211> 262
<212> DNA
<213> Artificial Sequence
<220>
<223> Description of Artificial Sequence: Human EST
<400> 60

```
aggaaatcaa attaggataa gatttgtatc tgatgaatat tttccttctg aaccttctaa 60
cagaggaggt aagattatac agctgcacac ctcgtaactt ctcagtgtcc ataagggaag 120
aactaaagag aaccgatacc attttctggc caggttgtct cctggttaaa cgctgtggtg 180
ggaactgtgc ctgttgtctc ccacaattgc aatgaatgtc aatgtgtccc aagcaaagtt 240
actaaaaaat accacgaggt cc                                          262
```

<210> 61
<211> 289
<212> DNA
<213> Artificial Sequence
<220>
<223> Description of Artificial Sequence: Human EST
<400> 61

```
atttcatctt caggatttac agtgcattct gaaanaggag aaatcaaaca naattaggag 60
ttgtgcaaca gctcttttga gaggaggcct aaaggacagg agaaaaggtc ttcaatcgtg 120
gaaanaaaat taaatgttgt attaaataga tcaccagcta gtttcagagt taccatgtac 180
gtattccact agctgggttc tgtatttcag ttctttcgat acggcttagg gtaatgtcag 240
tacaggaaaa aaactgtgca agtgagcacc tgattccgtt gccttgctt            289
```

<210> 62
<211> 251
<212> DNA
<213> Artificial Sequence
<220>
<223> Description of Artificial Sequence: Human EST
<400> 62

```
ttagcttggn aaatttttct aaacacaatt gttatagcca gaggaacaaa gatgatataa 60
aatattgttg ctctgacaaa aatacatgta tttcattctc gtatggtgct agagttagat 120
taatctgcat tttaaaaaac tgaattggaa tagaattggt aagttgcaaa gactttttga 180
aaataattaa attatcatat cttccattcc tgttattgga gatgaaaata aaaagcaact 240
tatganagta g                                                      251
```

<210> 63
<211> 252
<212> DNA
<213> Artificial Sequence
<220>
<223> Description of Artificial Sequence: Human EST
<400> 63

```
ctttttatg acaacttaga tcaactattt ttagcttggt aaatttttct aaacacaatt 60
gttatagcca gaggaacaaa gatgatataa aatattgttg ctctgacaaa aatacatgta 120

tttcattctc gtatggtgct agagttagat taatctgcat tttaaaaaac tgaattggaa 180
tagaattggt aagttgcaaa ggcttttga aaataattaa attatcatat cttccattcc 240
tgttattggn gg                                                    252
```

<210> 64
<211> 245
<212> DNA
<213> Artificial Sequence
<220>
<223> Description of Artificial Sequence: Human EST
<400> 64

```
caaagttact aaaaaatacc acgaggtcct tcagttgaga ccaaagaccg gtgtcagggg 60
attgcacaaa tcactcaccg acgtggccct ggagcaccat gaggagtgtg actgtgtgtg 120
cagagggagc acaggaggat agccgcatca ccaccagcag ctcttgccca gagctgtgca 180
gtgcagtggc tgattctatt agagaacgta tgcgttatct ccatccttaa tctcagttgt 240
ttgct                                                             245
```

<210> 65
<211> 245
<212> DNA
<213> Artificial Sequence
<220>
<223> Description of Artificial Sequence: Human EST
<400> 65

```
agataaacct gaaaagaaga gtggccttat cttcacttta tcgataagtc agtttatttg 60
tttcattgtg tacattttta tattctcctt ttgacattat aactgttggc ttttctaatc 120
ttgttaaata tatctatttt taccaaaggt atttaatatt ctttttatg acaacttaga 180
tcaactattt ttagcttggt aaatttttct aaacacaatt gttatagcca gaggaacaaa 240
gatga                                                             245
```

<210> 66
<211> 243
<212> DNA
<213> Artificial Sequence
<220>
<223> Description of Artificial Sequence: Human EST
<400> 66

```
ctggattttt catatttctt attaaaattt ctgccattta gaagaagaga actacattca 60
tggtttggaa gagataaacc tgaaaagaag agtggcctta tcttcacttt atcgataagt 120
cagtttattt gtttcattgt gtacattttt atattctcct tttgacatta taactgttgg 180
cttttctaat cttgttaaat atatctattt ttaccaaagg tatttaatat tctttttat 240
gac                                                               243
```

<210> 67
<211> 244
<212> DNA
<213> Artificial Sequence
<220>
<223> Description of Artificial Sequence: Human EST
<400> 67

```
gctcatattc acatatgtaa accagaacat tctatgtact acaaacctgg tttttaaaaa 60
gganctatgt tgctatgaat taaacttgtg tcgtgctgat aggacagact ggatttttca 120
tatttcttat taaaatttct gccatttaga agaagagaac tacattcatg gtttggaaga 180
gataaacctg aaaagaagag tggccttatc ttcantttat cgataagtca gtttatttgt 240
ttca                                                              244
```

<210> 68
<211> 247
<212> DNA
<213> Artificial Sequence
<220>
<223> Description of Artificial Sequence: Human EST
<400> 68

angagttgcc cagagctgtg cagtgcagtg gctgattcta ttagagaacg tatgcgttat 60
ctccatcctt aatctcagtt gtttgnttca aggacctttc atcttcagga tttacagtgc 120
attctgaaag aggagacatc aaacagaatt aggagttgtg caacagctct tttgagagga 180
ggcctaaagg ncaggagaaa aggtcttcaa tcgtggaaag aaaattaaat gttgtattaa 240
atagatc 247

<210> 69
<211> 233
<212> DNA
<213> Artificial Sequence
<220>
<223> Description of Artificial Sequence: Human EST
<400> 69

aaagatgata taaaatattg ttgctctgac aaaaatacat gtatttcatt ctcgtatggt 60
gctagagtta gattaatctg cattttaaaa aactgaattg gaatagaatt ggtaagttgc 120
aaagactttt tgaaaataat taaattatca tatcttccat tcctgttatt ggagatgaaa 180
ataaaaagca acttatgaaa gtagacattc agatccagcc attactaacc tat 233

<210> 70
<211> 232
<212> DNA
<213> Artificial Sequence
<220>
<223> Description of Artificial Sequence: Human EST
<400> 70

aggaaatcaa attaggataa gatttgtatc tgatgaatat tttccttctg aaccttctaa 60
cagaggaggt aagattatac agctgcacac ctcgtaactt ctcagtgtcc ataagggaag 120
aactaaagag aaccgatacc attttctggc caggttgtct cctggttaaa cgctgtggtg 180
ggaactgtgc ctgttgtctc cacaattgca atgaatgtca atgtgtccca ag 232

<210> 71
<211> 253
<212> DNA
<213> Artificial Sequence
<220>
<223> Description of Artificial Sequence: Human EST
<400> 71

gtgcattctg aaagaggaga catcaaacag aattaggagt tgtgcaacag ctcttttgag 60
aggaggccta aaggacagga gaaaaggtct tcaatcgtgg aaagaaaatt aaatgttgta 120
ttaaatagat caccagctag tttcagagtt accatgtacg tattccacta gctgggttct 180
gtatttcagt tctttcgata cggcttaggg taatgtcagt acaggaaaaa aactgtgcaa 240
gtgagcacct gat 253

<210> 72
<211> 233

<212> DNA
<213> Artificial Sequence
<220>
<223> Description of Artificial Sequence: Human EST
<400> 72

tgtacatttt tatattctcc ttttgacatt ataactgttg gcttttcnaa tcttgttaaa 60
tatatctatt tttaccaaag gtatttaata ttcttttta tgacaactta gatcaactat 120
ttttagcttg gtaaattttt ctaaacacaa ttgttatagc cagaggaaca aagatgatat 180
aaaatattgt tgctctgaca aaaatacatg tatttcattc tcgtatggtg cta 233

<210> 73
<211> 250
<212> DNA
<213> Artificial Sequence
<220>
<223> Description of Artificial Sequence: Human EST
<400> 73

cacaattgtt atagccagag gaacaaagat gatataaaat attgttgctc tgncaaaaat 60
acatgtattt cattctcgta tggtgctaga gttagattaa tctgcatttt aaaaaactga 120
attggaatag aattggtaag ttgcaaagac tttttgaaaa taattaaatt atcatatctt 180
ccattcctgt tattggagat gaaaataaaa agcaacttat gaaagtaaat tcagatccac 240
cattactaac 250

<210> 74
<211> 247
<212> DNA
<213> Artificial Sequence
<220>
<223> Description of Artificial Sequence: Human EST
<400> 74

atttcattct cgtatggtgc tagagttaga ttaatctgca ttttaaaaaa ctgaattgga 60
atagaattgg taagttgcaa agactttttg aaaataatta aattatcata tcttccattc 120
ctgttattgg agatgaaaat aaaaagcaac ttatgaaagt agacattcag atccagccat 180
tactaaccta ttcctttttt ggggaaatct gagcctagct cagaaaaaca taaagcacct 240
tgaaaaa 247

<210> 75
<211> 265
<212> DNA
<213> Artificial Sequence
<220>
<223> Description of Artificial Sequence: Human EST
<400> 75

```
tgcaacagct cttttgagag gaggcctaaa ggacaggaga aaaggtcttc aatcgtggaa 60
agaaaattaa atgttgtatt aaatagatca ccagctagtt tcagagttac catgtacgta 120
ttccactagc tgggttctgt atttcagttc tttcgatacg gcttagggta atgtcagtac 180
aggaaaaaaa ctgtgcaagt gagcacctga ttccgttgcc ttgcttaacc ctaaagcncc 240
atgtcnnggg cnaaaancga aaaat                                       265
```

<210> 76
<211> 251
<212> DNA
<213> Artificial Sequence
<220>
<223> Description of Artificial Sequence: Human EST
<400> 76

```
tttctaaaca caattgttat agccagagga acaaagatga tataaaatat tgttgctctg 60
acaaaaatac atgtatttca ttctcgtatg gtgctagagt tagattaatc tgcattttaa 120
aaaactgaat tggnatagaa ttggtaagtt gcaaagnctt tttgaaaata attaaattat 180
catatcttcc attcctgtta ttggaggatg gaaaataaaa agcaacttat ggaaagtagg 240
acattcagat c                                                      251
```

<210> 77
<211> 291
<212> DNA
<213> Artificial Sequence
<220>
<223> Description of Artificial Sequence: Human EST
<400> 77

```
ccttaatctc agttgtttgc ttcaaggacc tttcatcttc aggatttaca gtgcattctg 60
naagangaga catcaaacag aattaggngt tgtgcaaaag ctcttttgag aggaggccta 120
aaggacagga gaaaaggtct ncaatcgtgg aaagnaaatt aaatgttgta tnaaatngat 180
caccagctag tttcagagtt accatgtacg tattccacta gctgggncng tattcagtct 240
ttcggaacgg cttagggtaa tgtcagtaca gganaaaaac tgtgcagtga g          291
```

<210> 78
<211> 253
<212> DNA
<213> Artificial Sequence
<220>
<223> Description of Artificial Sequence: Human EST
<400> 78

gtactacaaa cctggttttt aaaaaggaac tatgttgcta tgaattaaac ttgtgtccat 60
gctgatagga cagactggat tttncatatt tcttattaaa atttctgcca tttagaagaa 120
gagaactaca ttcatggttt ggnagagata aacctgaaaa gaagagtggc cttatcttca 180
ctttatcgat aagtcagttt atttgtttca tgtgtacatt tttatattct cctttgacat 240
ataacgtggc ttt 253

<210> 79
<211> 204
<212> DNA
<213> Artificial Sequence
<220>
<223> Description of Artificial Sequence: Human EST
<400> 79

ttatattctc cttttgacat tataactgtt ggcttttcta atcttgttaa atatatctat 60
ttttaccaaa ggtatttaat attctttttt atgacaactt agatcaacta tttttagctt 120
ggtaaatttt tctaaacaca attgttatag ccagaggaac aaagatgata taaaatattg 180
ttgctctgan aaaaatacat gtat 204

<210> 80
<211> 303
<212> DNA
<213> Artificial Sequence
<220>
<223> Description of Artificial Sequence: Human EST
<400> 80

anactgtgca agtgagcacc tgattccgtt gccttgctta actctaaagc tccatgtcct 60
gggcctaaaa tcgtataaaa tctggannnn nnnnnnnnnn nnnngctcat attcacatat 120
gtaaaccaga acattctatg tactacaaac ctggttttta aaaaggaact atgttgctat 180
gaattaaact tgtgtcgtgc tgataggaca gactggattt ttcatatttc ttattaaaat 240
ttctgccatt agaagaagag aactacnttc anggtttgga agagataacc ctgaaaagan 300
ggg 303

<210> 81
<211> 228
<212> DNA
<213> Artificial Sequence
<220>
<223> Description of Artificial Sequence: Human EST
<400> 81

gctcatattc acatatgtaa accagaacat tctatgtact acaaacctgg tttttaaaaa 60
ggaactattt gctatgaatt aaacttgtgt cgtgctgata ggacagactg gnttttttcat 120
atttcttatt anaatttctg ccattagaag aagagaacta cattcatggt ttggaagaga 180
taaacctgaa aagaagagtg gcctatttca ctttatcgat aagtcagt 228

<210> 82
<211> 193
<212> DNA
<213> Artificial Sequence
<220>
<223> Description of Artificial Sequence: Human EST
<400> 82

gctcatattc acatatgtaa accagaacat tctatgtact acaaacctgg tttttaaaaa 60
ggaactatgt tgctatgaat taaacttgtg tcgtgctgat aggacagact ggatttttca 120
tatttcttat taaaatttct gccatttaga agaagagaac tacattcatg gtttggaaga 180
gataaacctg aaa 193

<210> 83
<211> 282
<212> DNA
<213> Artificial Sequence
<220>
<223> Description of Artificial Sequence: Human EST
<400> 83

aaaaaactga attggaatag aattggtaag ttgcaaagac tntttgaaaa taattaaatt 60
atcatatctt ccattcctgt tattggagat gaanataaaa agcaacttat gaaagtagac 120
attcagatcc agccattact aacctattcc ttttttgggg aaatctgagc ctagctcaga 180
aaaacataaa gcaccttgaa aaagacttgg cagcttcctg ataaagcgtg ctgtntgtca 240
gtaggaacac atcctattta ttgtgatgnt gtggtttatt at 282

<210> 84
<211> 279
<212> DNA
<213> Artificial Sequence
<220>
<223> Description of Artificial Sequence: Human EST
<400> 84

attaaataga tcaccagcta gtttcagagt taccatgtac gtattccact agctgggttc 60
tgtatttcag ttctttcgat acggcttagg gtaatgtcag tacaggaaaa aaactgtgca 120
agtgagcacc tgattccgtt gccttggctt aactctaaag ctccatgtcc tgggcctaaa 180
atcgtataaa atctggattt ttttnttttt ttttgcgcat attcacatat gtaaaccagn 240
acattctatg tacnacaaac ctggttttta aaaaggaac 279

<210> 85
<211> 181
<212> DNA
<213> Artificial Sequence
<220>
<223> Description of Artificial Sequence: Human EST
<400> 85

ggctagtttc agagttacca tgtacgtatt ccactagctg ggttctgtat ttcagttctt 60
tcgatacggc ttagggtaat gtcagtacag gaaaaaaact gtgcaagtga gcacctgatt 120
ccgttgcctt gcttaactct aaagctccat gtcctgggcc taaaatcgta taaaatctgg 180
a 181

<210> 86
<211> 269
<212> DNA
<213> Artificial Sequence
<220>
<223> Description of Artificial Sequence: Human EST
<400> 86

tggtaagttg caaagacttt ttgaaaataa ttaaattatc atatcttcca ttcctgttat 60
tggagatgaa aataaaaagc aacttatgaa agtagacatt cagatccagc cattactaac 120
ctattccttt tttggggaaa tctgagccta gctcagaaaa acataaagca ccttgaaaaa 180
gacttggcag cttcctgata aagcgtgctg tgctgtgcag tagggaacac atcctattta 240
ttgtgatgtt gtggtttata tcctaaacc 269

<210> 87
<211> 184
<212> DNA
<213> Artificial Sequence
<220>
<223> Description of Artificial Sequence: Human EST
<400> 87

aatagatcac cagctagttt cagagttacc atgtacgtat tccactagct gggntctgta 60
tttcagttcc tttcgatacg gcttagggta atgtcagtac aggaaaaaag ctgtgcaagt 120
gagcacctga ttccgttgcc ttgcttaact ctaaagctcc atgtcctggg cctaaaatcg 180
tata 184

<210> 88
<211> 164
<212> DNA
<213> Artificial Sequence
<220>
<223> Description of Artificial Sequence: Human EST
<400> 88

agataaacct gaaaagaaga gtggccttat nttcacttta tcgataagtc agnttatttg 60
tttcattgtg tacatttnna tattctcctt ttgacattat aactgntggc ttttctaanc 120
ntgttaaata tatctatttt taccaaaggt atttaatatt cttt 164

<210> 89
<211> 143
<212> DNA
<213> Artificial Sequence
<220>

<223> Description of Artificial Sequence: Human EST
<400> 89

tatggtgcta gagttagatt aatctgcatt ttaaaaaact gaattggaat agaattggta 60
agttgcaaag actttttgaa aataattaaa ttatcatatc ttccattcct gttattggag 120
atgaaaataa aaagcaactt atg 143

<210> 90
<211> 164
<212> DNA
<213> Artificial Sequence
<220>
<223> Description of Artificial Sequence: Human EST
<400> 90

ttttttnttt tgctcatatt cacatatgta aaccngaaca ttctatgtac nacaaacctg 60
gtttttaaaa aggaactatg ttgctatgaa ttaaacttgt gtcgtgctga taggacagac 120
tggatttttc anatttctta ntaanntttc tgccatttag aaga 164

<210> 91
<211> 244
<212> DNA
<213> Artificial Sequence
<220>
<223> Description of Artificial Sequence: Human EST
<400> 91

gtacaggaaa aaaactgtgc aagtgagcac ctgattccgt tgccttgctt aactctaaag 60
ctccatgtcc tgggcctaaa atcgtataaa atctggannn nnnnnnnnnn nnnnngctca 120
tattcacata tgtaaaccag aacattctat gtactacaaa cctggttttt aaaaaggaac 180
tatgttgcta tgaattaaac ttgtgtcgtg ctgataggac agactggatt tttcatattt 240
ctta 244

<210> 92
<211> 254
<212> DNA
<213> Artificial Sequence
<220>
<223> Description of Artificial Sequence: Human EST
<400> 92

gcaaagactt tttganaatn attaanttat catatcttcc attcctgtta tnggagatga 60
naataaaaag caacttatga aagtagacat tcagatccag ccattactaa cctattcctt 120
ttttggggaa atctgagcct agcncagaaa aacataaagc accttgaaaa agacttggca 180
gcttcctgat aaagcgtgct gtgctgtgca gtaggaacac atccnattta ttgtgntgtn 240
gnggttttat gatc 254

<210> 93
<211> 243

<212> DNA
<213> Artificial Sequence
<220>
<223> Description of Artificial Sequence: Human EST
<400> 93

tgtcagtaca ggaaaaaaac tgtgcaagtg agcacctgat tccgttgcct tgcttaactc 60
taaagctcca tgtcctgggc ctaaaatcgt ataaaatctg gannnnnnnn nnnnnnnnnn 120
gctcatattc acatatgtaa accagaacat tctatgtact acaaacctgg tttttaaaaa 180
ggaactatgt tgctatgaat taaacttgtg tcatgctgat aggacagact ggatttttca 240
tat 243

<210> 94
<211> 244
<212> DNA
<213> Artificial Sequence
<220>
<223> Description of Artificial Sequence: Human EST
<400> 94

aattatcata tcttccattc ctgttattgg agatgnaaat aaaaagcaac ttatgaaagt 60
agacattcag atccagccat tactaaccta ttcctttttt ggggaaatct gagcctagct 120
cagaaaaaca taaagcacct tgaaaaagac tgtcagcttc ctgataaagc gtgctgtgct 180
gtgcagtagg aacacatcct atttattgtg atgttgtggt tttattatct taaactcgtt 240
ccat 244

<210> 95
<211> 152
<212> DNA
<213> Artificial Sequence
<220>
<223> Description of Artificial Sequence: Human EST
<400> 95

anagatgata taaaanattg ttgctctgac aannatacat gtatttcatt ctcgtatggt 60
gctagagtta gattaatctg cnttttaaaa aactganttg gaatagantt ggtaagttgc 120
aaagncnttt gaaaatnatt aagttatcag at 152

<210> 96
<211> 292
<212> DNA
<213> Artificial Sequence
<220>
<223> Description of Artificial Sequence: Human EST
<400> 96

ttccattcct gttattggag atgaaaataa aaagcaactt atgaaagtag acattcagat 60
ccagccatta ctaacctatt cctttttggg ggaaatctga gcctagctca gaaaaacata 120

```
aagcaccttg aaaaagactt ggcagcttcc tgataaagcg tgctgtgctg tgcagtagga 180
acacatccta tttattgtga tgttgtggtt ttattatcta aactctgttc catacacttg 240
tataaataca tggatatttt tatgtacaga agtatgtctc ttaaccagtt ca         292
```

<210> 97
<211> 308
<212> DNA
<213> Artificial Sequence
<220>
<223> Description of Artificial Sequence: Human EST
<400> 97

```
cttccattcc tgttattgga gatgaaaata aaaagcaact tatganagta gacattcaga 60
tccagccatt actaacctat tcctttttg gggaaatctg agcctagctc agaaaaacat 120
aaagcacctt gaaaaagact tggcagcttc ctgataaagc gtgctgtgct gtgcagtagg 180
aacacatcct atttattgtg atgttgtggt tttattatct taaactctgt tccatacact 240
tgtataaata catggatatt tttatgtaca gaagtatgtc tcttaaccag ttcacttatt 300
gtacctgg                                                          308
```